# EUROPÄISCHE PATENTSCHRIFT

(11) **EP 2 964 631 B1**
(45) Veröffentlichungstag und Bekanntmachung des Hinweises auf die Patenterteilung: **30.08.2017**
(21) Anmeldenummer: 14706854.8
(22) Anmeldetag: 27.02.2014
(51) Int. Cl.: C07D 403/04, A61K 31/4155, A61K 31/4178, A01N 43/56, A01N 43/50, A61P 33/00, A61P 33/14

(54) **HALOGEN-SUBSTITUIERTE N-[3-(1H-PYRAZOL-4-YL)PHENYLMETHYL]-SULFONAMID-DERIVATE UND VERWANDTE VERBINDUNGEN ALS PESTIZIDE ZUR VERWENDUNG IM PFLANZENSCHUTZ, IN DER VETERINÄRMEDIZIN UND IM MATERIALSCHUTZ**
HALOGEN-SUBSTITUTED N-[3-(1H-PYRAZOL-4-YL)PHENYLMETHYL]-SULFONAMIDE DERIVATIVES AND RELATED COMPOUNDS AS PESTICIDES FOR USE IN CROP PROTECTION IN VETERINARY MEDICINE AND IN MATERIAL PROTECTION
DÉRIVÉS N-[3-(1H-PYRAZOL-4-YL)PHÉNYLMÉTHYL]-SULFONAMIDES SUBSTITUÉS PAR HALOGÈNES ET COMPOSÉS SIMILAIRES EN TANT QUE PESTICIDES À ÊTRE UTILISÉS DANS LA PROTECTION DES PLANTES, DANS LA MÉDECINE VÉTÉRINAIRE ET DANS LA PROTECTION DES MATÉRIAUX

(30) Priorität: 04.03.2013 EP 13157618
(43) Veröffentlichungstag der Anmeldung: 13.01.2016
(73) Patentinhaber: Bayer Animal Health GmbH, 51368 Leverkusen (DE)
(72) Erfinder: MAUE, Michael, 40764 Langenfeld (DE); DÉCOR, Anne, 40764 Langenfeld (DE); BRETSCHNEIDER, Thomas, 53797 Lohmar (DE); HAHN, Julia, Johanna, 40597 Düsseldorf (DE); HALLENBACH, Werner, 40789 Monheim am Rhein (DE); FISCHER, Reiner, 40789 Monheim (DE); SCHWARZ, Hans-Georg, 46282 Dorsten (DE); GÖRGENS, Ulrich, 40882 Ratingen (DE); ILG, Kerstin, 50670 Köln (DE); RAMING, Klaus, 51375 Leverkusen (DE); KÖBBERLING, Johannes, 41466 Neuss (DE); HÜBSCH, Walter, 42113 Wuppertal (DE); TÜRBERG, Andreas, 42781 Haan (DE)
(74) Vertreter: BIP Patents
(86) Internationale Anmeldenummer: PCT/EP2014/053838
(87) Internationale Veröffentlichungsnummer: WO 2014/135439

(56) Entgegenhaltungen:
- WO-A1-2012/107434

## Beschreibung

Die vorliegende Anmeldung betrifft neue Halogen-substituierte Verbindungen, Verfahren zu ihrer Herstellung und ihre Verwendung zur Bekämpfung von an Pflanzen parasitierenden Schädlingen, Vorratsschädlingen oder Schädlingen, die industrielle Materialien zerstören, vor allem von Arthropoden und insbesondere von Insekten, Spinnentieren und Nematoden.

Es ist bekannt, dass bestimmte Halogen-substituierte Verbindungen herbizid wirksam sind (vgl. J. Org. Chem. 1997, 62(17), 5908-5919, J. Heterocycl. Chem. 1998, 35(6), 1493-1499, WO 2004/035545, WO 2004/106324, US 2006/069132, WO 2008/029084).

Des Weiteren ist bekannt, daß bestimmte Halogen-substituierte Verbindungen insektizid wirksam sind (EP1911751, WO2012-069366, WO2012-080376 & WO2012-107434)
Ferner ist bekannt, dass bestimmte Halogen-substituierte Verbindungen FAAH-inhibitorische Aktivitäten aufweisen (WO 2009/151991).

Moderne Pflanzenschutzmittel müssen vielen Anforderungen genügen, beispielsweise in Bezug auf Höhe, Dauer und Breite ihrer Wirkung und möglichen Verwendung. Es spielen Fragen der Toxizität, der Kombinierbarkeit mit anderen Wirkstoffen oder Formulierhilfsmitteln eine Rolle sowie die Frage des Aufwands, der für die Synthese eines Wirkstoffs betrieben werden muss. Ferner können Resistenzen auftreten. Aus all diesen Gründen kann die Suche nach neuen Pflanzenschutzmitteln nie als abgeschlossen betrachtet werden und es besteht ständig Bedarf an neuen Verbindungen mit gegenüber den bekannten Verbindungen zumindest in Bezug auf einzelne Aspekte verbesserten Eigenschaften. Aufgabe der vorliegenden Erfindung war es, Verbindungen bereitzustellen, durch die das Spektrum der Schädlingsbekämpfungsmittel unter verschiedenen Aspekten verbreitert und/oder ihre Aktivität verbessert wird.

Es wurde nun überraschenderweise gefunden, dass bestimmte Halogen-substituierte Verbindungen, sowie deren *N*-Oxide und Salze biologische Eigenschaften aufweisen und sich insbesondere zur Bekämpfung von tierischen Schädlingen eignen, und deshalb besonders gut im agrochemischen Bereich und im Bereich der Tiergesundheit einsetzbar sind.

Die erfindungsgemäßen Halogen-substituierten Verbindungen sind durch die allgemeinen Formeln (I) und (II) 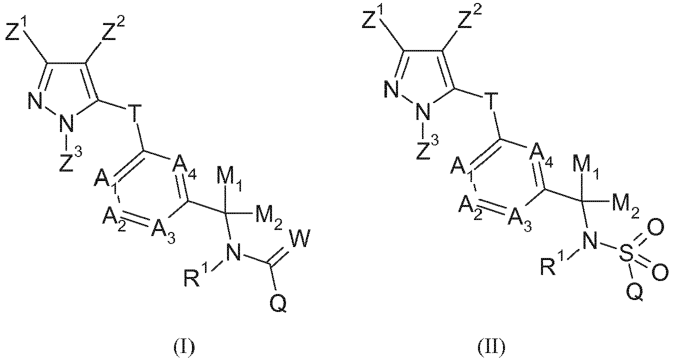 definiert, in denen
- R¹: für Wasserstoff, gegebenenfalls substituiertes C₁-C₆-Alkyl, C₃-C₆-Alkenyl, C₃-C₆-Alkinyl, C₃-C₇-Cycloalkyl, C₁-C₆-Alkylcarbonyl, C₁-C₆-Alkoxycarbonyl, Aryl(C₁-C₃)-alkyl, Heteroaryl(C₁-C₃)-alkyl,
- M¹ und M²: jeweils unabhängig voneinander für Wasserstoff, Cyano oder für ein gegebenenfalls ein oder mehrfach substituiertes C₁-C₆-Alkyl, C₂-C₆-Alkenyl, C₂-C₆-Alkinyl, C₃-C₆-Cycloalkyl, C₁-C₆-Alkoxycarbonyl stehen, oder
- M¹ und M²: mit dem Kohlenstoffatom, an das sie gebunden sind, einen gegebenenfalls substituierten 3-, 4-, 5- oder 6-gliedrigen Ring bilden, der gegebenenfalls 0, 1 oder 2 Stickstoffatome und/oder 0, 1 oder 2 Sauerstoffatome und/oder 0, 1 oder 2 Schwefelatome enthält,
die chemischen Gruppierungen
- A₁: für CR² oder Stickstoff,
- A₂: für CR³ oder Stickstoff,
- A₃: für CR⁴ oder Stickstoff, und
- A₄: für CR⁵ oder Stickstoff stehen,
wobei aber nicht mehr als drei der chemischen Gruppierungen A₁ bis A₄ gleichzeitig für Stickstoff stehen;
- R², R³, R⁴ und R⁵: unabhängig voneinander für Wasserstoff, Halogen, Cyano, Nitro, gegebenenfalls substituiertes C₁-C₆-Alkyl, C₃-C₆-Cycloalkyl, C₁-C₆-Alkoxy, *N*-C₁-C₆-Alkoxy-imino-C₁-C₃-alkyl, C₁-C₆-Alkylsulfanyl, C₁-C₆-Alkylsulfinyl, C₁-C₆-Alkylsulfonyl, *N*-C₁-C₆-Alkylamino oder *N,N-*D₁-C₁-C₆-alkylamino, stehen; wenn keine der Gruppierungen A₂ und A₃ für Stickstoff steht, können R³ und R⁴ gemeinsam mit dem Kohlenstoffatom, an das sie gebunden sind, einen 5- oder 6-gliedrigen Ring bilden, der 0, 1 oder 2 Stickstoffatome und/oder 0 oder 1 Sauerstoffatom und/oder 0 oder 1 Schwefelatom enthält, oder
wenn keine der Gruppierungen A₁ und A₂ für Stickstoff steht, können R² und R³ gemeinsam mit dem Kohlenstoffatom, an das sie gebunden sind, einen 6-gliedrigen Ring bilden, der 0, 1 oder 2 Stickstoffatome enthält;
- W: für Sauerstoff oder Schwefel steht;
- Q: für Wasserstoff, Hydroxy, Amino oder eine der gegebenenfalls substituierten Gruppierungen Alkyl, Alkoxy, Alkenyl, Alkinyl, Cycloalkyl, Heterocycloalkyl, Cycloalkylalkyl, Arylalkyl, Heteroarylalkyl oder für eine Gruppierung *N-*Alkylamino, *N*-Alkylcarbonylamino, *N,N-*Dialkylamino steht; oder
- Q: für einen gegebenenfalls einfach bis mehrfach mit V substituierten, ungesättigten 6-gliedrigen Carbozyklus steht, oder für einen gegebenenfalls einfach bis mehrfach mit V substituierten, ungesättigten 5- bzw. 6-gliedrigen, heterozyklischen Ring steht, wobei
- V: unabhängig voneinander für Halogen, Cyano, Nitro, gegebenenfalls substituiertes Alkyl, Alkenyl, Alkinyl, Cycloalkyl, Alkoxy, *N*-Alkoxyiminoalkyl, Alkylsulfanyl, Alkylsulfinyl, Alkylsulfonyl, *N,N*-Dialkylamino steht
- T: für einen der nachfolgend aufgeführten 5 gliedrigen Heteroaromaten T1-T8 steht, wobei die Bindung zur Pyrazolkopfgruppe mit einem Sternchen gekennzeichnet ist, 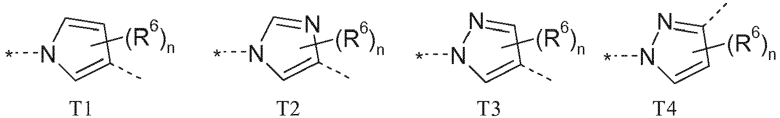 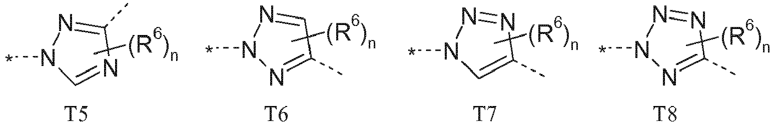
wobei
- R⁶: unabhängig voneinander für Halogen, Cyano, Nitro, Amino oder ein gegebenenfalls substituiertes C₁-C₆-Alkyl, C₁-C₆-Alkoxy, C₁-C₆-Alkylcarbonyl, C₁-C₆-Alkylsulfanyl, C₁-C₆-Alkylsulfinyl, C₁-C₆-Alkylsulfonyl, und
- n: für die Werte 0-2 stehen;
- Z¹: für ein gegebenenfalls substituiertes Alkyl und Cycloalkyl, und
- Z²: für Wasserstoff, Halogen, Cyano, Nitro, Amino oder ein gegebenenfalls substituiertes Alkyl, Alkylcarbonyl, Alkylsulfanyl, Alkylsulfinyl, Alkylsulfonyl, und
- Z³: für Wasserstoff oder ein gegebenenfalls substituiertes Alkyl, Cycloalkyl, Alkenyl, Alkinyl, Aryl oder Hetaryl stehen;

Ferner haben die Reste R¹, M¹, M² Q, V und R⁶ folgende alternative Bedeutungen:
- R¹: für Wasserstoff, gegebenenfalls substituiertes C₁-C₆-Alkyl, C₂-C₆-Alkenyl, C₂-C₆-Alkinyl, C₃-C₇-Cycloalkyl, C₁-C₆-Alkylcarbonyl, C₁-C₆-Alkoxycarbonyl, Aryl(C₁-C₃)-alkyl, Heteroaryl(C₁-C₃)-alkyl,
- M¹ und M²: jeweils unabhängig voneinander für Wasserstoff, Cyano oder für ein gegebenenfalls ein oder mehrfach substituiertes C₁-C₆-Alkyl, C₂-C₆-Alkenyl, C₂-C₆-Alkinyl, C₃-C₆-Cycloalkyl, C₂-C₇-Alkoxycarbonyl stehen, oder
- Q: für einen gegebenenfalls einfach bis mehrfach mit V substituierten, ungesättigten 6-gliedrigen Carbozyklus steht, oder für einen gegebenenfalls einfach bis mehrfach mit V substituierten, ungesättigten 5- bzw. 6-gliedrigen, heterozyklischen Ring steht, wobei
- V: unabhängig voneinander für Halogen, Cyano, Nitro, gegebenenfalls substituiertes Alkyl, Alkenyl, Alkinyl, Cycloalkyl, Alkoxy, *N*-Alkoxyiminoalkyl, Alkylsulfanyl, Alkylsulfinyl, Alkylsulfonyl, *N,N-*Dialkylamino steht
- R⁶: unabhängig voneinander für Halogen, Cyano, Nitro, Amino oder ein gegebenenfalls substituiertes C₁-C₆-Alkyl, C₁-C₆-Alkoxy, C₁-C₆-Alkylcarbonyl, C₁-C₆-Alkylsulfanyl, C₁-C₆-Alkylsulfinyl, C₁-C₆-Alkylsulfonyl, und

Generell bevorzugt im Sinne dieser Anmeldung sind immer die Verbindungen der allgemeinen Formel (II) unabhängig vom Bevorzugungsgrad der jeweiligen Restedefinitionen.

Bevorzugt sind Verbindungen der Formeln (I) und (II) 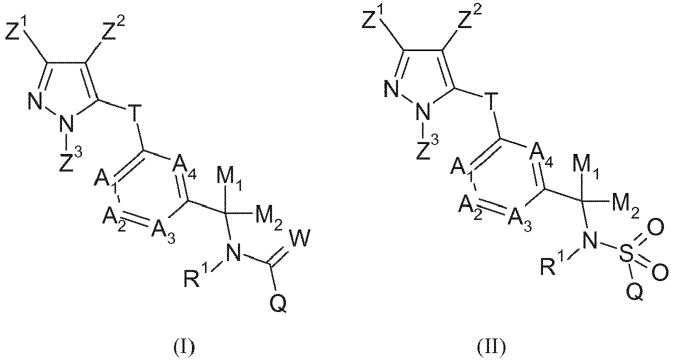 definiert, in denen
- R¹: für Wasserstoff, gegebenenfalls substituiertes C₁-C₆-Alkyl, C₃-C₆-Alkenyl, C₃-C₆-Alkinyl, C₃-C₇-Cycloalkyl, C₁-C₆-Alkylcarbonyl, C₁-C₆-Alkoxycarbonyl, Aryl(C₁-C₃)-alkyl, Heteroaryl(C₁-C₃)-alkyl steht;
- M¹ und M²: jeweils unabhängig voneinander für Wasserstoff, Cyano oder für ein gegebenenfalls ein oder mehrfach substituiertes C₁-C₆-Alkyl, C₂-C₆-Alkenyl, C₂-C₆-Alkinyl, C₃-C₆-Cycloalkyl, C₁-C₆-Alkoxycarbonyl stehen, oder
- M¹: und M² mit dem Kohlenstoffatom, an das sie gebunden sind, einen gegebenenfalls substituierten 3-, 4-, 5- oder 6-gliedrigen Ring bilden, der gegebenenfalls 0, 1 oder 2 Stickstoffatome und/oder 0, 1 oder 2 Sauerstoffatome und/oder 0, 1 oder 2 Schwefelatome enthält,
die chemischen Gruppierungen
- A₁: für CR² oder Stickstoff,
- A₂: für CR³ oder Stickstoff,
- A₃: für CR⁴ oder Stickstoff, und
- A₄: für CR⁵ oder Stickstoff stehen,
wobei aber nicht mehr als drei der chemischen Gruppierungen A₁ bis A₄ gleichzeitig für Stickstoff stehen;
- R², R³, R⁴ und R⁵: unabhängig voneinander für Wasserstoff, Halogen, Cyano, Nitro, gegebenenfalls substituiertes C₁-C₆-Alkyl, C₃-C₆-Cycloalkyl, C₁-C₆-Alkoxy, *N*-C₁-C₆-Alkoxyimino- C₁-C₃-alkyl, C₁-C₆-Alkylsulfanyl, C₁-C₆-Alkylsulfinyl, C₁-C₆-Alkylsulfonyl, *N*-C₁-C₆-Alkylamino, *N,N-*Di*-*C₁-C₆-alkylamino, stehen;
wenn keine der Gruppierungen A₂ und A₃ für Stickstoff steht, können R³ und R⁴ gemeinsam mit dem Kohlenstoffatom, an das sie gebunden sind, einen 5- oder 6-gliedrigen Ring bilden, der 0, 1 oder 2 Stickstoffatome und/oder 0 oder 1 Sauerstoffatom und/oder 0 oder 1 Schwefelatom enthält, oder
wenn keine der Gruppierungen A₁ und A₂ für Stickstoff steht, können R² und R³ gemeinsam mit dem Kohlenstoffatom, an das sie gebunden sind, einen 6-gliedrigen Ring bilden, der 0, 1 oder 2 Stickstoffatome enthält;
- W: für Sauerstoff oder Schwefel steht;
- Q: für Wasserstoff, Formyl, Hydroxy, Amino oder eine der gegebenenfalls substituierten Gruppierungen C₁-C₆-Alkyl, C₂-C₆-Alkenyl, C₂-C₆-Alkinyl, C₃-C₆-Cycloalkyl, C₂-C₇-Heterocycloalkyl, C₁-C₄-Alkoxy, C₃-C₆-Cycloalkyl-C₁-C₆-alkyl, Aryl-(C₁-C₃)-alkyl, Heteroaryl-(C₁-C₃)-alkyl oder für eine Gruppierung *N-*C₁-C₄-Alkylamino, *N*-C₁-C₄-Alkylcarbonylamino, *N*,*N-*Di-C₁-C₄-alkylamino steht; oder
- Q: für einen gegebenenfalls einfach bis mehrfach mit V substituierten, ungesättigten 6-gliedrigen Carbozyklus steht, oder für einen gegebenenfalls einfach bis mehrfach mit V substituierten, ungesättigten 5- bzw. 6-gliedrigen, heterozyklischen Ring steht, wobei
- V: unabhängig voneinander für Halogen, Cyano, Nitro, gegebenenfalls substituiertes C₁-C₆-Alkyl, C₂-C₄-Alkenyl, C₂-C₄-Alkinyl, C₃-C₆-Cycloalkyl, C₁-C₆-Alkoxy, *N*-C₁-C₆-Alkoxy-imino-C₁-C₃-alkyl, C₁-C₆-Alkylsulfanyl, C₁-C₆-Alkylsulfinyl, C₁-C₆-Alkylsulfonyl,*N*,*N*-Di-(C₁-C₆-alkyl)amino steht;
- T: für einen der nachfolgend aufgeführten 5 gliedrigen Heteroaromaten T1-T8 steht, wobei die Bindung zur Pyrazolkopfgruppe mit einem Sternchen gekennzeichnet ist, 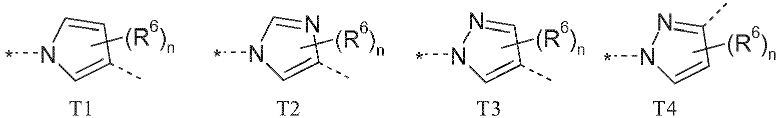 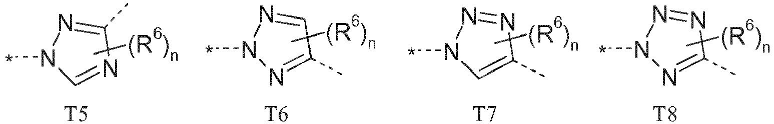
wobei
- R⁶: unabhängig voneinander für Halogen, Cyano, Nitro, Amino oder ein gegebenenfalls mit Halogen substituiertes C₁-C₆-Alkyl, C₁-C₆-Alkoxy, C₁-C₆-Alkylcarbonyl, C₁-C₆-Alkylsulfanyl, C₁-C₆-Alkylsulfinyl, C₁-C₆-Alkylsulfonyl, und
- n: für die Werte 0-1 stehen;
- Z¹: für ein gegebenenfalls substituiertes C₁-C₆-Halogenalkyl, C₃-C₆-Halogencycloalkyl, und
- Z²: für Wasserstoff Halogen, Cyano, Nitro, Amino oder ein gegebenenfalls substituiertes C₁-C₆-Alkyl, C₁-C₆-Alkylcarbonyl, C₁-C₆-Alkylsulfanyl, C₁-C₆- Alkylsulfinyl, C₁-C₆-Alkylsulfonyl, und
- Z³: für Wasserstoff oder ein gegebenenfalls substituiertes C₁-C₆-Alkyl, C₃-C₆-Cycloalkyl, C₃-C₆-Alkenyl, C₃-C₆-Alkinyl, Aryl oder Hetaryl stehen;

Ferner bevorzugt sind auch die Verbindungen der Formeln (I) und (II), in denen die untenstehenden Reste wie folgt alternativ definiert sind:
- R¹: für Wasserstoff, gegebenenfalls substituiertes C₁-C₆-Alkyl, C₂-C₆-Alkenyl, C₂-C₆-Alkinyl, C₃-C₇-Cycloalkyl, C₁-C₆-Alkylcarbonyl, C₁-C₆-Alkoxycarbonyl, Aryl(C₁-C₃)-alkyl, Heteroaryl(C₁-C₃)-alkyl steht;
- M¹: und M² jeweils unabhängig voneinander für Wasserstoff, Cyano oder für ein gegebenenfalls ein oder mehrfach substituiertes C₁-C₆-Alkyl, C₂-C₆-Alkenyl, C₂-C₆-Alkinyl, C₃-C₆-Cycloalkyl, C₂-C₇-Alkoxycarbonyl stehen, oder
- R², R³, R⁴ und R³: unabhängig voneinander für Wasserstoff, Halogen, Cyano, Nitro, gegebenenfalls substituiertes C₁-C₆-Alkyl, C₃-C₆-Cycloalkyl, C₁-C₆-Alkoxy, *N*-C₁-C₆-Alkoxyimino-C₁-C₃-alkyl, C₁-C₆-Alkylsulfanyl, C₁-C₆-Alkylsulfinyl, C₁-C₆-Alkylsulfonyl, *N*-C₁-C₆-Alkylamino, *N*,*N*-Di-C₁-C₆-alkylamino, stehen;
- Q: für Wasserstoff, Formyl, Hydroxy, Amino oder eine der gegebenenfalls substituierten Gruppierungen C₁-C₆-Alkyl, C₂-C₆-Alkenyl, C₂-C₆-Alkinyl, C₃-C₆-Cycloalkyl, C₁-C₅-Heterocycloalkyl, C₁-C₄-Alkoxy, C₁-C₆-Alkyl-C₃-C₆-cycloalkyl, , Aryl-(C₁-C₃)-alkyl, Heteroaryl-(C₁-C₃)-alkyl oder für eine Gruppierung *N*-C₁-C₄-Alkylamino, *N*-C₁-C₄-Alkylcarbonylamino, *N,N-*Di-C₁-C₄-alkylamino steht; oder
- Q: für einen gegebenenfalls einfach bis mehrfach mit V substituierten, ungesättigten 6-gliedrigen Carbozyklus steht, oder für einen gegebenenfalls einfach bis mehrfach mit V substituierten, ungesättigten 5- bzw. 6-gliedrigen, heterozyklischen Ring steht, wobei

- V: unabhängig voneinander für Halogen, Cyano, Nitro, gegebenenfalls substituiertes C₁-C₆-Alkyl, C₁-C₄-Alkenyl, C₁-C₄-Alkinyl, C₃-C₆-Cycloalkyl, C₁-C₆-Alkoxy, *N*-C₁-C₆-Alkoxy-imino-C₁-C₃-alkyl, C₁-C₆-Alkylsulfanyl, C₁-C₆-Alkylsulfinyl, C₁-C₆-Alkylsulfonyl, *N,N-*Di-(C₁-C₆-alkyl)amino steht;
- Z¹: für ein gegebenenfalls substituiertes C₁-C₆-Halogenalkyl, C₃-C₆-Cycloalkyl, C₃-C₆-Halogencyclo-alkyl, und
- Z³: für Wasserstoff oder ein gegebenenfalls substituiertes Alkyl, Cycloalkyl, Alkenyl, Alkinyl, Aryl oder Hetaryl stehen;

Besonders bevorzugt sind Verbindungen der Formeln (I) und (II) 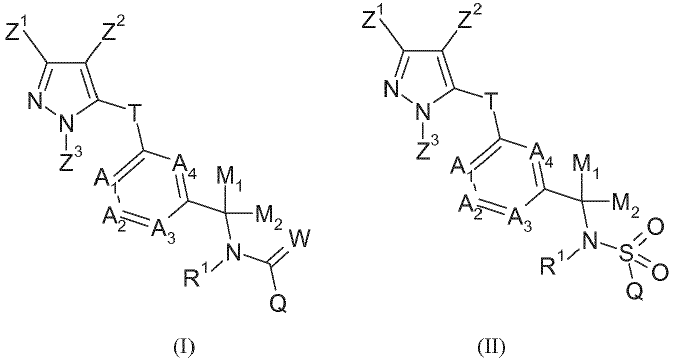 definiert, in denen
- R¹: für Wasserstoff, gegebenenfalls ein- bis siebenfach unabhängig voneinander mit Fluor, Chlor, Cyano, C₁-C₆-Alkoxy und C₁-C₆-Alkoxycarbonyl substituiertes C₁-C₆-Alkyl, C₃-C₆-Alkenyl, C₃-C₆-Alkinyl, C₃-C₇-Cycloalkyl, C₁-C₆-Alkylcarbonyl, C₁-C₆-Alkoxycarbonyl, Aryl(C₁-C₃)-alkyl, Heteroaryl(C₁-C₃₎-alkyl,
- M¹: für Wasserstoff steht,
- M²: für Wasserstoff, Cyano, gegebenenfalls ein- bis fünffach unabhängig voneinander durch Fluor, Chlor, Cyano oder C₁-C₃-Alkoxy substituiertes C₁-C₆-Alkyl, C₂-C₆-Alkenyl, C₃-C₆-Cycloalkyl, C₁-C₃-Alkoxycarbonyl steht,
- M¹ und M²: mit dem Kohlenstoffatom, an das sie gebunden sind, einen gegebenenfalls substituierten 3-gliedrigen Ring bilden,
die chemischen Gruppierungen
- A₁: für CR² oder Stickstoff,
- A₂: für CR³ oder Stickstoff,
- A₃: für CR⁴ oder Stickstoff, und
- A₄: für CR⁵ oder Stickstoff stehen,
wobei aber nicht mehr als drei der chemischen Gruppierungen A₁ bis A₄ gleichzeitig für Stickstoff stehen;
- R², R³, R⁴ und R⁵: unabhängig voneinander für Wasserstoff, Fluor, Chlor, Brom, Iod, Cyano, Nitro, gegebenenfalls einfach bis fünffach unabhängig voneinander durch Fluor, Chlor, Cyano, Hydroxycarbonyl, C₁-C₆-Alkoxycarbonyl, C₁-C₆-Alkylcarbonyl oder Phenyl substituiertes C₁-C₆-Alkyl, C₃-C₆-Cycloalkyl, C₁-C₆-Alkoxy, *N*-C₁-C₆-Alkoxy-imino-C₁-C₃-alkyl, C₁-C₆-Alkylsulfanyl, C₁-C₆-Alkylsulfinyl, C₁-C₆-Alkylsulfonyl, *N*-C₁-C₆-Alkylamino oder *N,N* Di-C₁-C₆-alkylamino stehen;
- W: für Sauerstoff oder Schwefel steht;
- Q: für Wasserstoff, Amino oder eine der gegebenenfalls unabhängig von einander ein- bis fünffach mit Hydroxy, Nitro, Amino, Fluor, Chlor, C₁-C₆-Alkyl, C₁-C₆-Alkoxy, Cyano, Hydroxycarbonyl, C₁-C₄-Alkoxycarbonyl, C₁-C₄-Alkylcarbamoyl, C₃-C₇-Cycloalkylcarbamoyl, Phenyl substituierten Gruppierungen C₁-C₆-Alkyl, C₂-C₆-Alkenyl, C₂-C₆-Alkinyl, C₃-C₆-Cycloalkyl, C₂-C₅-Heterocycloalkyl, C₁-C₄-Alkoxy, C₃-C₆-Cycloalkyl-C₁-C₆-alkyl, Aryl(C₁-C₃)-alkyl, Heteroaryl(C₁-C₃)-alkyl, *N*-C₁-C₄-Alkylamino, *N*-C₁-C₄-Alkylcarbonylamino oder *N*,*N*-Di-C₁-C₄-alkylamino steht; oder
- Q: für ein mit 0 - 4 Substituenten V substituiertes Aryl oder für ein mit 0 - 4 Substituenten V substituierten 5 bzw. 6 gliedrigen Heteroaromaten steht, wobei
- V: unabhängig voneinander für Halogen, Cyano, Nitro, gegebenenfalls substituiertes C₁-C₆-Alkyl, C₂-C₄-Alkenyl, C₂-C₄-Alkinyl, C₃-C₆-Cycloalkyl, C₁-C₆-Alkoxy, *N*-C₁-C₆-Alkoxy-imino-C₁-C₃-alkyl, C₁-C₆-Alkylsulfanyl, C₁-C₆-Alkylsulfinyl, C₁-C₆-Alkylsulfonyl, *N*,*N*-Di-(C₁-C₆-Alkyl)amino steht;
- T: für einen der nachfolgend aufgeführten 5 gliedrigen Heteroaromaten T1-T8 steht, wobei die Bindung zur Pyrazolkopfgruppe mit einem Sternchen gekennzeichnet ist, 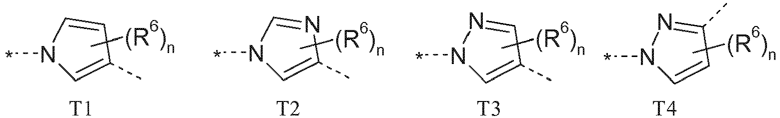 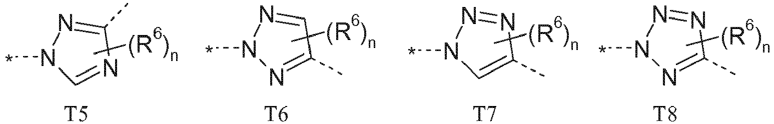
wobei
- R⁶: unabhängig voneinander für Fluor, Chlor, Brom, Iod, Cyano, Nitro, Amino oder ein gegebenenfalls unabhängig voneinander ein- bis fünffach mit Fluor und/oder Chlor substituiertes C₁-C₆-Alkyl, C₁-C₆-Alkox, C₁-C₆-Alkylcarbonyl, C₁-C₆-Alkylsulfanyl, C₁-C₆-Alkylsulfinyl, C₁-C₆-Alkylsulfonyl, und
- n: für die Werte 0-1 stehen;
- Z¹: für ein gegebenenfalls substituiertes C₁-C₆-Halogenalkyl, C₃-C₆-Halogencycloalkyl, und
- Z²: für Wasserstoff, Fluor, Chlor, Brom, Iod, Cyano, Nitro, Amino oder ein gegebenenfalls unabhängig voneinander ein- bis fünffach mit Fluor und/oder Chlor substituiertes C₁-C₆-Alkyl, C₁-C₆-Alkylcarbonyl, C₁-C₆-Alkylsulfanyl, C₁-C₆-Alkylsulfinyl, C₁-C₆-Alkylsulfonyl, und
- Z³: für Wasserstoff oder ein gegebenenfalls substituiertes C₁-C₆-Alkyl, C₃-C₆-Cycloalkyl C₃-C₄-Alkenyl, C₃-C₄-Alkinyl, Aryl und Hetaryl stehen;

Ferner besonders bevorzugt sind auch die Verbindungen der Formeln (I) und (II), in denen die untenstehenden Reste wie folgt alternativ definiert sind:
- R⁶: unabhängig voneinander für Halogen, Cyano, Nitro, Amino oder ein gegebenenfalls unabhängig voneinander ein- bis fünffach mit Halogen substituiertes C₁-C₆-Alkyl, C₁-C₆-Alkox, C₁-C₆-Alkylcarbonyl, C₁C₆-Alkysulfayl, C₁C₆-Alkylsulfinyl, C₁C₆-Alkylsulfonyl, und
- Z¹: für ein gegebenenfalls substituiertes C₁C₆-alkyl, C₃-C₆-cycloalkyl, und
- Z²: für Wasserstoff, Halogen, Cyano, Nitro, Amino oder ein gegebenenfalls unabhängig voneinander ein- bis fünffach mit Fluor und/oder Chlor substituiertes C₁C₆-Alkyl, C₁-₆-Alkylcarbonyl, C₁-C₆-Alkylsulfanyl, C₁-₆-Alkylsulfinyl, C₁-₆-Alkylsulfonyl, und

- Z³: für Wasserstoff oder ein gegebenenfalls substituiertes C₁-C₆-Alkyl, C₃-C₆-Cycloalkyl C₁-C₄-Alkenyl, C₁-C₄-Alkinyl, Aryl und Hetaryl stehen;

Ganz besonders bevorzugt sind Verbindungen der Formeln (I) und (II) 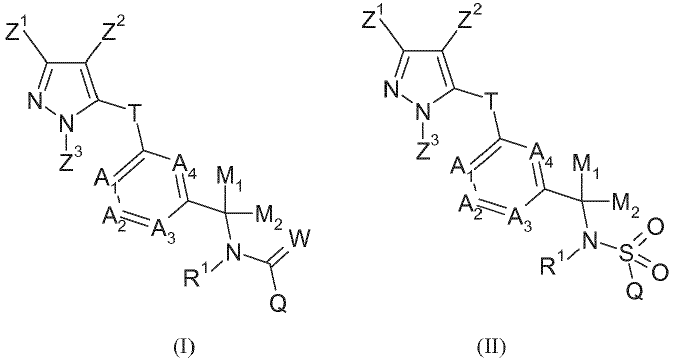 definiert, in denen
- R¹: für Wasserstoff, gegebenenfalls ein- oder fünffach unabhängig voneinander mit Fluor, Chlor, Cyano, C₁-C₄-Alkoxy und C₁-C₄-Alkoxycarbonyl substituiertes C₁-C₄-Alkyl, C₃-C₄-Alkenyl, C₃-C₄-Alkinyl, C₃-C₆-Cycloalkyl, C₁-C₄-Alkylcarbonyl, C₁-C₄-Alkoxycarbonyl, Aryl(C₁-C₂)-alkyl, Heteroaryl(C₁-C₂)-alkyl,
- M¹: für Wasserstoff steht,
- M²: für Wasserstoff, gegebenenfalls ein- oder fünffach fünffach unabhängig voneinander mit Halogen, Cyano, Alkoxy und Alkoxycarbonyl substituiertes C₁-C₃-Alkyl, C₂-C₆-Alkenyl, C₃-C₆-Cycloalkyl, C₁-C₃-Alkoxycarbonyl steht,
- M¹ und M²: mit dem Kohlenstoffatom, an das sie gebunden sind, einen gegebenenfalls substituierten 3-gliedrigen Ring bilden,
die chemischen Gruppierungen
- A₁: für CR² oder Stickstoff,
- A₂: für CR³ oder Stickstoff,
- A₃: für CR⁴ oder Stickstoff, und
- A₄: für CR⁵ oder Stickstoff stehen,
wobei aber nicht mehr als drei der chemischen Gruppierungen A₁ bis A₄ gleichzeitig für Stickstoff stehen;
- R², R³, R⁴ und R⁵: unabhängig voneinander für Wasserstoff, Fluor, Chlor, Brom, Cyano, Nitro, gegebenenfalls unabhängig von einander ein- bis fünffach mit Hydroxy, Nitro, Amino, Fluor, Chlor, C₁-C₄-Alkoxy, Cyano, Hydroxycarbonyl. C₁-C₄-Alkoxycarbonyl, C₁-C₄-Alkylcarbamoyl, C₃-C₇-Cycloallcylcarbamoyl, Phenyl substituierten C₁-C₄-Alkyl, C₃-C₆-Cycloalkyl, C₁-C₄-Alkoxy, *N*-C₁-C₄-Alkoxy-imino-C₁-C₂-alkyl, C₁-C₄-Alkylsulfanyl, C₁-C₄-Alkylsulfinyl, C₁-C₄-Alkylsulfonyl, *N*-C₁-C₄-Alkylamino oder *N,N-*Di-C₁-C₄-alkylamino stehen;
- W: für Sauerstoff oder Schwefel steht;
- Q: für Wasserstoff, Amino oder eine der gegebenenfalls unabhängig von einander ein- bis fünffach mit Hydroxy, Nitro, Amino, Halogen, C₁-C₄-Alkyl, C₁-C₄-Alkoxy, Cyano, Hydroxycarbonyl. C₁-C₄-Alkoxycarbonyl, C₁-C₄-Alkylcarbamoyl, C₃-C₆-Cycloalkylcarbamoyl, Phenyl substituierten Gruppierungen C₁-C₄-Alkyl, C₂-C₆-Alkenyl, C₂-C₆-Alkinyl, C₃-C₆-Cycloalkyl, C₂-C₅-Heterocycloalkyl, C₁-C₄-Alkoxy, C₃-C₆-Cycloalkyl-C₁-C₆-alkyl, Aryl(C₁-C₃)-alkyl, Heteroaryl(C₁-C₃)-alkyl, *N*-C₁-C₄-Alkylamino, *N*-C₁-C₄-Alkylcarbonylamino oder *N,N-*Di-C₁-C₄-alkylamino steht; oder
- Q: für ein mit 0, 1, 2, 3 oder 4 Substituenten V substituiertes Aryl oder für ein mit 0, 1, 2, 3 oder 4 Substituenten V substituierten 5 bzw. 6 gliedrigen Heteroaromaten steht, wobei
- V: unabhängig voneinander für Fluor, Chlor, Brom, Iod, Cyano, Nitro, gegebenenfalls unabhängig von einander ein- bis fünffach mit Hydroxy, Nitro, Amino, Fluor, Chlor, Brom, Iod, C₁-C₄-Alkyl, C₁-C₄-Alkoxy, Cyano, Hydroxycarbonyl. C₁-C₄-Alkoxycarbonyl, C₁-C₄-Alkylcarbamoyl, C₃-C₆-Cycloalkylcarbamoyl, Phenyl substituiertes C₁-C₆-Alkyl, C₂-C₄-Alkenyl, C₂-C₄-Alkinyl, C₃-C₆-Cycloalkyl, C₁-C₆-Alkoxy, *N*-C₁-C₆-Alkoxy-imino-C₁-C₃-alkyl, C₁-C₆-Alkylsulfanyl, C₁-C₆-Alkylsulfinyl, C₁-C₆-Alkylsulfonyl, *N*,*N*-Di-(C₁-C₆-alkyl)amino steht;
- T: für einen der nachfolgend aufgeführten 5 gliedrigen Heteroaromaten T1-T8 steht, wobei die Bindung zur Pyrazolkopfgruppe mit einem Sternchen gekennzeichnet ist, 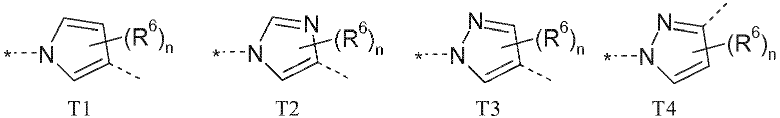 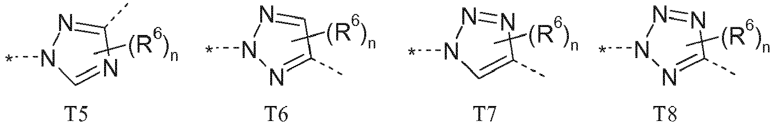
wobei
- R⁶: unabhängig voneinander für Fluor, Chlor, Cyano, Nitro, Amino oder ein gegebenenfalls einfach bis fünffach mit Fluor und/oder Chlor substituiertes C₁-C₆-Alkyl, C₁-C₆-Alkoxy, C₁-C₆-Alkylcarbonyl, C₁-C₆-Alkylsulfanyl, C₁-C₆-Alkylsulfinyl, C₁-C₆-Alkylsulfonyl, und
- n: für die Werte 0-1 stehen;
- Z¹: für ein gegebenenfalls ein- bis zweifach mit C₁-C₄-Alkoxy, Cyano, Hydroxycarbonyl. C₁-C₄-Alkoxycarbonyl, C₁-C₄-Alkylcarbamoyl, C₃-C₆-Cycloalkylcarbamoyl, Phenyl substituiertes C₁-C₄-Halogenalkyl, C₃-C₆-Halogencycloalkyl, und
- Z²: für Wasserstoff, Fluor, Chlor, Brom, Iod, Cyano, Nitro, Amino oder ein gegebenenfalls unabhängig von einander ein- bis dreifach mit Hydroxy, Nitro, Amino, Fluor, Chlor, C₁-C₄-Alkoxy, Cyano, Hydroxycarbonyl. C₁-C₄-Alkoxycarbonyl, C₁-C₄-Alkylcarbamoyl, C₃-C₆-Cycloalkylcarbamoyl, Phenyl substituiertes C₁-C₄-Alkyl, C₁-C₄-Alkylcarbonyl, C₁-C₄-Alkylsulfanyl, C₁-C₄-Alkylsulfinyl, C₁-C₄-Alkylsulfonyl, und
- Z³: für Wasserstoff oder ein gegebenenfalls unabhängig von einander ein- bis dreifach mit Hydroxy, Nitro, Amino, C₁-C₄-Alkoxy, Cyano, Fluor, Chlor, Hydroxycarbonyl. C₁-C₄-Alkoxycarbonyl, C₁-C₄-Alkylcarbamoyl, C₃-C₆-Cycloalkylcarbamoyl, Phenyl substituiertes C₁-C₄-Alkyl, C₃-C₆-Cycloalkyl, C₃-C₄-Alkenyl, C₃-C₄-Alkinyl, Phenyl und Hetaryl stehen;

Insbesondere bevorzugt sind Verbindungen der Formeln (I) und (II) 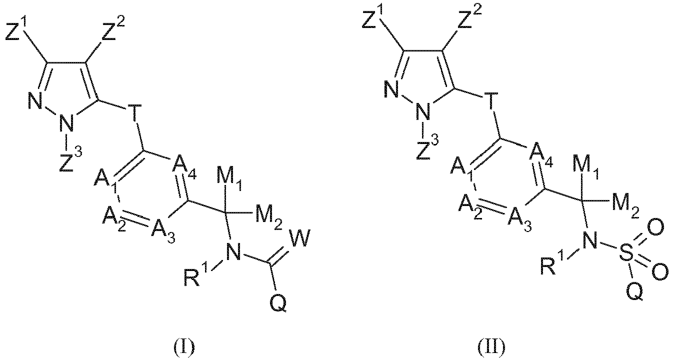 definiert, in denen
- R¹: für Wasserstoff, Methyl, Ethyl, n-Propyl, Isopropyl, n-Butyl, Isobutyl, s-Butyl, t-Butyl, Methoxymethyl, Ethoxymethyl, Propoxymethyl, Methylcarbonyl, Ethylcarbonyl, n-Propylcarbonyl, Isopropylcarbonyl, n-Butylcarbonyl, i-Butylcarbonyl, s-Butylcarbonyl, t-Butylcarbonyl, Methoxycarbonyl, Ethoxycarbonyl, n-Propoxycarbonyl, Isopropoxycarbonyl, n-Butoxycarbonyl, i-Butoxycarbonyl, s-Butoxycarbonyl, t-Butoxycarbonyl, Cyanomethyl, 2-Cyanoethyl, Benzyl, 4-Methoxybenzyl, Pyrid-2-yl-methyl, Pyrid-3-yl-methyl, Pyrid-4-yl-methyl, 6-Chlor-pyrid-3-yl-methyl steht;
- M¹: für Wasserstoff steht,
- M²: Wasserstoff, Methyl, Ethyl, Difluormethyl, Trichlormethyl, Dichlorfluormethyl, Trifluormethyl, Cyclopropyl, Cyclobutyl, Methoxycarbonyl, Ethoxycarbonyl,
- M¹ und M²: mit dem Kohlenstoffatom, an das sie gebunden sind, einen 3-gliedrigen Carbozyklus bilden,
die chemischen Gruppierungen
- A₁: für CR² oder Stickstoff,
- A₂: für CR³ oder Stickstoff,
- A₃: für CR⁴ oder Stickstoff, und
- A₄: für CR⁵ oder Stickstoff stehen,
wobei aber nicht mehr als drei der chemischen Gruppierungen A₁ bis A₄ gleichzeitig für Stickstoff stehen;
R² und R⁵ unabhängig voneinander für Wasserstoff, Methyl, Fluor und Chlor stehen und
R³ und R⁴ unabhängig voneinander für Wasserstoff, Fluor, Chlor, Brom, Iod, Cyano, Nitro, Methyl, Ethyl, Fluormethyl, Difluormethyl, Chlordifluormethyl, Trifluormethyl, 2,2,2-Trifluorethyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N-*Methoxyiminomethyl, 1-(*N*-Methoxyimino)-ethyl, 1, Methylsulfanyl, Trifluormethylsulfanyl, Methylsulfonyl, Methylsulfinyl, Trifluormethylsulfonyl, Trifluonnethylsulfinyl stehen
- W: für Sauerstoff oder Schwefel steht;
- Q: für für Wasserstoff, Methyl, Ethyl, n-Propyl, Isopropyl, t-Butyl, 1-Methylpropyl, n-Butyl, 2-Methylpropyl, 2-Methylbutyl, Hydroxymethyl, 2-Hydroxyethyl, 2-Hydroxypropyl, Cyanomethyl, 2-Cyanoethyl, Trifluormethyl, 1-Fluorethyl, 2-Fluorethyl, 2,2-Difluorethyl, 2,2,2-Trifluorethyl, 1-Trifluormethylethyl, 2,2-Difluorpropyl, 3,3,3-Trifluorpropyl, 2,2-Dimethyl-3-fluorpropyl, Cyclopropyl, 1-Cyano-cyclopropyl, Cyclopropyl-methyl, Cyclobutyl, Cyclopentyl, Cyclohexyl, 1-Cyclopropylethyl, Bis(cyclopropyl)methyl, 2,2-Dimethylcyclopropyl-methyl, 2-Phenylcyclopropyl, 2,2-Dichlorcyclopropyl, trans-2-Chlorcyclopropyl, cis-2-Chlorcyclopropyl, 2,2-Difluorcyclopropyl, trans-2-Fluorcyclopropyl, cis-2-Fluorcyclopropyl, trans-4-Hydroxycyclohexyl, 4-Trifluormethylcyclohexyl, Ethenyl, 1-Methylethenyl, Prop-1-enyl, 2-Methylprop-1-enyl, 3-Methylbut-1-enyl, 3,3,3-Trifluorprop-1-enyl, 1-Ethyl-ethenyl, 1-Methylprop-1-enyl, Prop-2-inyl, 3-Fluor-prop-2-enyl,, Oxetan-3-yl, Thietan-3-yl, 1-Oxido-thietan-3-yl, 1,1-Dioxido-thietan-3-yl, Tetrahydrofuran-3-yl, 1,1-Dioxido-tetrahydrothiophen-3-yl, Isoxazol-3-ylmethyl, 1,2,4-Triazol-3-ylmethyl, 3-Methyloxetan-3-ylmethyl, Benzyl, 2,6-Difluorphenylmethyl, 3-Fluorphenylmethyl, 2-Fluorphenylmethyl, 2,5-Difluorphenylmethyl, 1-Phenylethyl, 4-Chloiphenyletlryl, 2-Trifluormethylphenylethyl, 1-Pyridin-2-ylethyl, Pyridin-2-ylmethyl, 5-Fluorpyridin-2-ylmethyl, (6-Chlor-pyridin-3-yl)methyl, Pyrimidin-2-ylmethyl, Thiophen-2-yl-methyl. 2-Ethoxyethyl, 2-Methoxyethyl, 1-(Methylsulfanyl)ethyl, 2-(Methylsulfanyl)ethyl, 1-Methyl-2-(ethylsulfanyl)ethyl, 2-Methyl-1-(methylsulfanyl)propan-2-yl, Methoxycarbonyl, Ethoxycarbonyl, Methoxycarbonylmethyl, NH₂, *N*-Ethylamino, *N*-Allylamino, *N*,*N*-Dimethylamino, *N,N*-Diethylamino, Methoxy, Ethoxy, Propoxy, iso-Propoxy, tert-Butoxy steht; oder
- Q: für ein mit 0,1,2 oder 3Substituenten V substituiertes Phenyl, Naphthyl, Pyridazin, Pyrazin, Pyrimidin, Triazin, Pyridin, Pyrazol, Thiazol, Isothiazol, Oxazol, Isoxazol, Triazol, Imidazol, Furan, Thiophen, Pyrrol, Oxadiazol, Thiadiazol steht, wobei
- V: unabhängig voneinander für Fluor, Chlor, Brom, Iod, Cyano, Nitro, Methyl, Ethyl, Difluormethyl, Trichlormethyl, Chlordifluormethyl, Dichlorfluormethyl, Trifluormethyl, 1-Fluorethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Dilfluorethyl, Pentafluorethyl Heptafluor-n-propyl, Heptafluor-isopropyl, Nonafluor-n-butyl, Cyclopropyl, Cyclobutyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N*-Methoxyiminomethyl, 1-(*N-*Methoxyimino)-ethyl, Methylsulfanyl, Methylsulfonyl, Methylsulfinyl, Trifluormethylsulfonyl, Trifluormethylsulfinyl, Trifluormethylsulfanyl, *N*,*N-*Dimethylamino steht;
- T: für einen der nachfolgend aufgeführten 5 gliedrigen Heteroaromaten T1-T8 steht, wobei die Bindung zur Pyrazolkopfgruppe mit einem Sternchen gekennzeichnet ist, 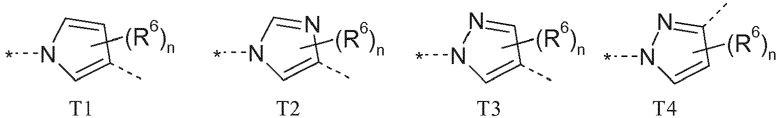 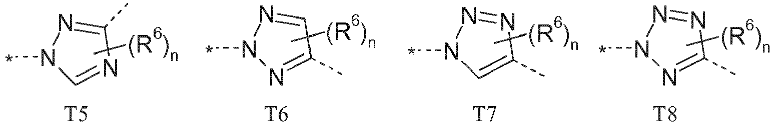
wobei
- R⁶: unabhängig voneinander für Fluor, Chlor, Cyano, Nitro, Amino, Methyl, Methyl, Propyl, 1-Methylethyl, tert-Butyl, Trifluormethyl, Difluormethyl, Methoxy, Ethoxy, Trifluormethoxy, 2,2-Difluorethoxy, 2,2,2-Trifluorethoxy, Methylcarbonyl, Ethylcarbonyl, Trifluormethylcarbonyl, Methylsulfanyl, Methylsulfinyl, Methylsulfonyl, Trifluormethylsulfonyl, Trifluormethylsulfanyl, Trilfuormethylsulfinyl, und
- n: für die Werte 0-1 stehen;
- Z¹: für Difluormethyl, Trichlormethyl, Chlordifluormethyl, Dichlorfluormethyl, Trifluormethyl, Bromdichlormethyl, 1-Fluorethyl, 1-Fluor-1-methylethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Difluorethyl, Pentafluorethyl Heptafluor-n-propyl, Heptafluor-isopropyl, Nonafluor-n-butyl, Cyclopropyl, 1-Chlorcyclopropyl, 1-Fluorcyclopropyl, 1-Bromcyclopropyl, 1-Cyan-cyclopropyl, 1-Trifluormehtyl-cyclopropyl, Cyclobutyl und 2,2-Difluor-1 -methyl-cyclopropyl, und
- Z²: für Wasserstoff, Fluor, Chlor, Brom, Iod, Cyano, Nitro,, Amino, Methyl, Ethyl, 1,1-t-Butyl, Difluormethyl, Trichlormethyl, Chlordifluormethyl, Dichlorfluormethyl, Trifluormethyl, Bromdichlormethyl, 1-Fluorethyl, 1-Fluor-1-methylethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Difluorethyl, Pentafluorethyl Heptafluor-n-propyl, Heptafluor-isopropyl, Nonafluor-n-butyl, Methylsulfanyl, Methylsulfinyl, Methylsulfonyl, Ethylthio, Ethylsulfinyl, Ethylsulfonyl, Trifluormethylsulfanyl, Trifluormethylsulfinyl, Trifluormethylsulfonyl, Chlor-difluormethylsulfanyl, Chlor-difluormethylsulfinyl, Chlor-difluormethylsulfonyl, Dichlor-fluormethylsulfanyl, Dichlor-fluormethylsulfinyl, Dichlor-fluormethylsulfonyl und
- Z³: für Wasserstoff, Methyl, Ethyl, n-Propyl, Isopropyl, n-Butyl, Isobutyl, s-Butyl, t-Butyl, 1-Propenyl, 1-Propinyl, 1-Butinyl, Difluormethyl, Trichlormethyl, Chlordifluormethyl, Dichlorfluormethyl, Trifluormethyl, 1-Fluorethyl, 1-Fluor-1-methylethyl, 2-Fluorethyl, 2,2-Difluorethyl, 2,2,2-Trifluorethyl, Phenyl, 2-Chlorphenyl, 3-Chlorphenyl. 4-Chlorphenyl, 2,4-Dichlorpenyl, 2,5-Dichlorphenyl, 3,4-Dichlorphenyl, 2,6-Dichlorphenyl 2,6-Dichlor-4-trifluormehtylphenyl, 3-Chlor-5-trifluormethylpyridin-2-yl stehen;

Ferner insbesondere bevorzugt sind auch die Verbindungen der Formeln (I) und (II), in denen die untenstehenden Reste wie folgt alternativ definiert sind:
- R¹: für Wasserstoff, Methyl, Ethyl, n-Propyl, Isopropyl, n-Butyl, Isobutyl, s-Butyl, t-Butyl, Methoxymethyl, Ethoxymethyl, Propoxymethyl, Methylcarbonyl, Ethylcarbonyl, n-Propylcarbonyl, Isopropylcarbonyl, s-Butylcarbonyl, t-Butylcarbonyl, Methoxycarbonyl, Ethoxycarbonyl, n-Propoxycarbonyl, Isopropoxycarbonyl, s-Butoxycarbonyl, t-Butoxycarbonyl, Cyanomethyl, 2-Cyanoethyl, Benzyl, 4-Methoxybenzyl, Pyrid-2-yl-methyl, Pyrid-3-yl-methyl, Pyrid-4-yl-methyl, 6-Chlor-pyrid-3-yl-methyl steht;
- M²: für Wasserstoff, C₁-C₃-Alkyl, C₂-C₃-Alkenyl, C₃-C₆-Cycloalkyl, C₁-C₃-Alkoxycarbonyl, Cyano, oder Cyano-C₁-C₂-alkyl steht,
- Q: für für Wasserstoff, Methyl, Ethyl, n-Propyl, 1-Methylethyl, 1,1-Dimethylethyl, 1-Methylpropyl, n-Butyl, 2-Methylpropyl, 2-Methylbutyl, Hydroxymethyl, 2-Hydroxypropyl, Cyanomethyl, 2-Cyanoethyl, 2-Fluorethyl, 2,2-Difluorethyl, 2,2,2-Trifluorethyl, 1-Trifluormethylethyl, 2,2-Difluorpropyl, 3,3,3-Trifluropropyl, 2,2-Dimethyl-3-fluorpropyl, Cyclopropyl, 1-Cyano-cyclopropyl, 1-Methoxycarbonyl-cyclopropyl, 1-(*N*-Methylcarbamoyl)cyclopropyl, 1-(*N-*Cyclopropylcarbamoyl)cyclopropyl, Cyclopropyl-methyl, Cyclobutyl, Cyclopentyl, Cyclohexyl, 1-Cyclopropylethyl, Bis(cyclopropyl)methyl, 2,2-Dimethylcyclopropyl-methyl, 2-Phenylcyclopropyl, 2,2-Dichlorcyclopropyl, trans-2-Chlorcyclopropyl, cis-2-Chlorcyclopropyl, 2,2-Difluorcyclopropyl, trans-2-Fluorcyclopropyl, cis-2-Fluorcyclopropyl, trans-4-Hydroxycyclohexyl, 4-Trifluormethylcyclohexyl, Prop-2-enyl, 2-Methylprop-2-enyl, Prop-2-inyl, 1,1-Dimethylbut-2-inyl, 3-Chlor-prop-2-enyl" 3,3-Dichlor-prop-2-enyl, 3,3-Dichlor-1,1-dimethylprop-2-enyl, Phenyl, 2-Chlorphenyl, 3-Chlorphenyl, 4-Chlorphenyl, Oxetan-3-yl, Thietan-3-yl, 1-Oxido-thietan-3-yl, 1,1-Dioxido-thietan-3-yl, Isoxazol-3-ylmethyl, 1,2,4-Triazol-3-ylmethyl, 3-Methyloxetan-3-ylmethyl, Benzyl, 2,6-Difluorphenylmethyl, 3-Fluorphenylmethyl, 2-Fluorphenylmethyl, 2,5-Difluorphenylmethyl, 1-Phenylethyl, 4-Chlorphenylethyl, 2-Trifluormethylphenylethyl, 1-Pyridin-2-ylethyl, Pyridin-2-ylmethyl, 5-Fluorpyridin-2-ylmethyl, (6-Chlor-pyridin-3-yl)methyl, Pyrimidin-2-ylmethyl, Methoxy, 2-Ethoxyethyl, 2-(Methylsulfanyl)ethyl, 1-Methyl-2-(ethylsulfanyl)ethyl, 2-Methyl-1-(methylsulfanyl)propan-2-yl, Methoxycarbonyl, Methoxycarbonylmethyl, NH₂, *N*-Ethylamino, *N*-Allylamino, *N*,*N-*Dimethylamin, *N*,*N*-Diethylamino steht; oder
- Q: für ein mit 0 - 4 Substituenten V substituiertes Phenyl, Naphthyl, Pyridazin, Pyrazin, Pyrimidin, Triazin, Pyridin, Pyrazol, Thiazol, Isothiazol, Oxazol, Isoxazol, Triazol, Imidazol, Furan, Thiophen, Pynol, Oxadiazol, Thiadiazol steht, wobei
- V: unabhängig voneinander für Fluor, Chlor, Brom, Iod, Cyano, Nitro, Methyl, Ethyl, Difluormethyl, Trichlormethyl, Chlordifluormethyl, Dichlorfluormethyl, Trifluormethyl, Chlormethyl, Brommethyl, 1-Fluorethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Dilfluorethyl, Pentafluorethyl Pentafluor-tert-butyl, Heptafluor-n-propyl, Heptafluor-isopropyl, Nonafluor-n-butyl, Cyclopropyl, Cyclobutyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N*-Methoxyiminomethyl, 1-(*N*-Methoxyimino)-ethyl, Methylsulfanyl, Methylsulfonyl, Methylsulfinyl, Trifluormethylsulfonyl, Trifluormethylsulfinyl, Trifluormethylsulfanyl, *N*,*N*-Dimethylamino steht;
- R⁶: unabhängig voneinander für Halogen, Cyano, Nitro, Amino, Methyl, Ethyl, 1-Methylethyl, tert-Butyl, Trifluormethyl, Difluormethyl, Methoxy, Ethoxy, Trifluormethoxy, 2,2-Difluorethoxy, 2,2,2-Trifluorethoxy, Methylcarbonyl, Ethylcarbonyl, Trifluormethylcarbonyl, Methylsulfanyl, Methylsulfinyl, Methylsulfonyl, Trifluormethylsulfonyl, Trifluonnethylsulfanyl, Trilfuormethylsulfinyl, und
- n: für die Werte 0-1 stehen;
- Z¹: für Methyl, Ethyl, 1,1-Dimethylethyl, Difluormethyl, Trichlormethyl, Chlordifluormethyl, Dichlorfluormethyl, Trifluormethyl, Bromdichlormethyl, Chlormethyl, Brommethyl, 1-Fluorethyl, 1-Fluor-1-methylethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Dilfluorethyl, Pentafluorethyl Pentafluor-t-butyl, Heptafluor-n-propyl, Heptafluor-isopropyl, Nonafluor-n-butyl, Cyclopropyl, 1-Chlorcyclopropyl, 1-Fluorcyclopropyl, 1-Bromcyclopropyl, 1-Cyan-cyclopropyl, 1-Trifluormehtyl-cyclopropyl, Cyclobutyl und 2,2-Difluor-1-methyl-cyclopropyl, und

- Z²: für Wasserstoff, Halogen, Cyano, Nitro" Amino, Methyl, Ethyl, 1,1-Dimethylethyl, Difluormethyl, Trichlormethyl, Chlordifluormethyl, Dichlorfluormethyl, Trifluormethyl, Bromdichlormethyl, Chlormethyl, Brommethyl, 1-Fluorethyl, 1-Fluor-1-methylethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Dilfluorethyl, Pentafluorethyl Pentafluor-t-butyl, Heptafluor-n-propyl, Heptafluor-isopropyl, Nonafluor-n-butyl, Methylsulfanyl, Methylsulfinyl, Methylsulfonyl, Ethylthio, Ethylsulfinyl, Ethylsulfonyl, Trifluormethylsulfanyl, Trifluormethylsulfinyl, Trifluormethylsulfonyl, Chlor-difluormethylsulfanyl, Chlor-difluormethylsulfinyl, Chlor-difluormethylsulfonyl, Dichlor-fluormethylsulfanyl, Dichlor-fluormethylsulfinyl, Dichlor-fluormethylsulfonyl und
- Z³: für Wasserstoff, Methyl, Ethyl, n-Propyl, Isopropyl, n-Butyl, Isobutyl, s-Butyl, t-Butyl, Ethenyl, 1-Propenyl, 2-Propenyl, 1-Propinyl, 1-Butinyl, Difluormethyl, Trichlormethyl, Chlordifluormethyl, Dichlorfluormethyl, Trifluormethyl, Chlormethyl, Brommethyl, 1-Fluorethyl, 1-Fluor-1-methylethyl, 2-Fluorethyl, 2,2-Difluorethyl, 2,2,2-Trifluorethyl, Phenyl, 2-Chlorphenyl, 3-Chlrophenyl. 4-Chlophenyl, 2,5-Dichlorphenyl, 3,4-Dichlorphenyl, 2,6-Dichlorphenyl 2,6-Dichlor-4-trifluormehtylphenyl, 3-Chlor-5-trifluormethylpyridin-2-yl stehen;

Insbesondere speziell bevorzugte Verbindungen im Sinne der Erfindung sind solche der allgemeinen Formeln (I) und (II), in denen
- Z¹: für Trifluormethyl, 1-Chlor-cyclopropyl, 1-Fluor-cyclopropyl oder Pentafluorethyl steht,
- Z²: für Trifluormethyl, Nitro, Methylsulfanyl, Methylsulfonyl, Methylsulfonyl, Fluor, Chlor, Brom, Cyano oder Iod steht,
- Z³: für Methyl, Ethyl, n-Propyl oder Wasserstoff steht,
- R¹: für Wasserstoff, Methyl, Ethyl, n-Propyl, Isopropyl, n-Butyl, i-Butyl, s-Butyl, t-Butyl, Methoxymethyl, Ethoxymethyl, Propoxymethyl, Methylcarbonyl, Ethylcarbonyl, n-Propylcarbonyl, Isopropylcarbonyl, n-Butylcarbonyl, i-Butylcarbonyl, s-Butylcarbonyl, t-Butylcarbonyl, Methoxycarbonyl, Ethoxycarbonyl, n-Propoxycarbonyl, Isopropoxycarbonyl, n-Butoxycarbonyl, i-Butoxycarbonyl, s-Butoxycarbonyl, t-Butoxycarbonyl, Cyanomethyl, 2-Cyanoethyl, Benzyl, 4-Methoxybenzyl, Pyrid-2-yl-methyl, Pyrid-3-yl-methyl, Pyrid-4-yl-methyl, 6-Chlor-pyrid-3-yl-methyl steht,
- M¹: für Wasserstoff steht;
- M²: für Wasserstoff oder Methyl steht;
- M¹ und M²: mit dem Kohlenstoffatom, an das sie gebunden sind, einen 3-gliedrigen Carbozyklus bilden,
- A¹, und A⁴: für CH steht
- A2: für CH oder N steht,
- A₃: für CR⁴ und
- R⁴: für Methyl, Ethyl, Fluor, Chlor, Brom oder Iod steht
- T: für einen der nachfolgend aufgeführten 5 gliedrigen Heteroaromaten T1-T8 steht, wobei die Bindung zur Pyrazolkopfgruppe mit einem Sternchen gekennzeichnet ist, 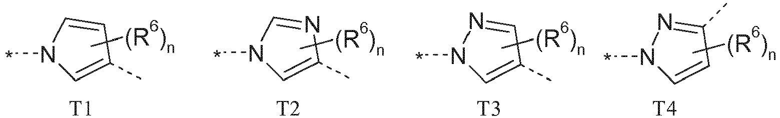 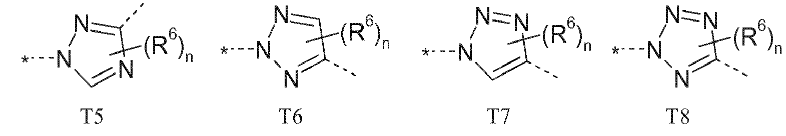
wobei
- R⁶: für Wasserstoff, Methyl, Ethyl, 2-Methylethyl, 2,2-Dimethylethyl, Fluor, Chlor, Brom, Iod, Nitro, Trifluormethyl, Amino steht,
- W: für Sauerstoff steht und
- Q: für für Wasserstoff, Methyl, Ethyl, n-Propyl, 1-Methylethyl, 1,1-Dimethylethyl, n-Butyl, 1-Methylpropyl, 2-Methylpropyl, 2-Methylbutyl, Hydroxymethyl2-Hydroxypropyl, Cyanomethyl, 2-Cyanoethyl, 2-Fluorethyl, 2,2-Difluorethyl, 2,2,2-Trifluorethyl, 1-Trifluormethylethyl, 2,2-Difluorpropyl, 3,3,3-Triflurpropyl, 2,2-Dimethyl-3-fluorpropyl, Cyclopropyl, Cyclopropyl-methyl, Cyclobutyl, Cyclopentyl, Cyclohexyl, 1-Cyclopropylethyl, Bis(cyclopropyl)methyl, 2,2-Dimethylcyclopropyl-methyl, 2-Phenylcyclopropyl, 2,2-Dichlorcyclopropyl, trans-2-Chlorcyclopropyl, cis-2-Chlorcyclopropyl, 2,2-Difluorcyclopropyl, trans-2-Fluorcyclopropyl, cis-2-Fluorcyclopropyl, trans-4-Hydroxycyclohexyl, 4-Trifluormethylcyclohexyl, Ethenyl, 1-Methylethenyl, Prop-1-enyl, 2-Methylprop-1-enyl, 3-Methylbut-1-enyl, 3,3,3-Trifluorprop-1-enyl, 1-Ethyl-ethenyl, 1-Methylprop-1-enyl, Prop-2-inyl, 3-Fluor-prop-2-enyl, Oxetan-3-yl, Thietan-3-yl, 1-Oxido-thietan-3-yl, 1,1-Dioxido-thietan-3-yl, Isoxazol-3-ylmethyl, 1,2,4-Triazol-3-ylmethyl, 3-Methyloxetan-3-ylmethyl, Benzyl, 2,6-Difluorphenylmethyl, 3-Fluorphenylmethyl, 2-Fluorphenylmethyl, 2,5-Difluorphenylmethyl, 1-Phenylethyl, 4-Chlorphenylethyl, 2-Trifluormethylphenylethyl, 1-Pyridin-2-ylethyl, Pyridin-2-ylmethyl, (6-Chlor-pyridin-3-yl)methyl, 5-Fluorpyridin-2-ylmethyl, Pyrimidin-2-ylmethyl, Methoxy, 2-Ethoxyethyl, 2-(Methylsulfanyl)ethyl, 1-Methyl-2-(ethylsufanyl)ethyl, 2-Methyl-1-(methylsulfanyl)propan-2-yl, Methoxycarbonyl, Methoxycarbonylmethyl, NH₂, *N-*Ethylamino, *N*-Allylamino, *N*,*N-*Dimethylamino, *N,N-*Diethylamino, Methoxy, Ethoxy, Propoxy, iso-Propoxy, tert-Butoxy steht; oder
- Q: für ein mit 0, 1, 2 oer 3 Substituenten V substituiertes Phenyl, Naphthyl, Pyridazin, Pyrazin, Pyrimidin, Triazin, Pyridin, Pyrazol, Thiazol, Isothiazol, Oxazol, Isoxazol, Triazol, Imidazol, Furan, Thiophen, Pyrrol, Oxadiazol, Thiadiazol steht, wobei
- V: unabhängig voneinander für Fluor, Chlor, Brom, Iod, Cyano, Nitro, Methyl, Ethyl, Difluormethyl, Trichlormethyl, Chlordifluormethyl, Dichlorfluormethyl, Trifluormethyl, 1-Fluorethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Dilfluorethyl, Pentafluorethyl, Heptafluor-n-propyl, Heptafluor-isopropyl, Nonafluor-n-butyl, Cyclopropyl, Cyclobutyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N*-Methoxyiminomethyl, 1-(*N*-Methoxyimino)-ethyl, 1, Methylsulfanyl, Methylsulfonyl, Methylsulfinyl, Trifluormethylsulfonyl, Trifluormethylsulfinyl, Trifluormethylsulfanyl, *N*,*N*-Dimethylamino steht;

Ferner insbesondere speziell bevorzugte Verbindungen sind auch solche in denen die nachfolgenden Reste alternativ wie folgt definiert sind:
- R¹: für Wasserstoff, Methyl, Ethyl, n-Propyl, Isopropyl, n-Butyl, i-Butyl, s-Butyl, t-Butyl, Methoxymethyl, Ethoxymethyl, Propoxymethyl, Methylcarbonyl, Ethylcarbonyl, n-Propylcarbonyl, Isopropylcarbonyl, s-Butylcarbonyl, t-Butylcarbonyl, Methoxycarbonyl, Ethoxycarbonyl, n-Propoxycarbonyl, Isopropoxycarbonyl, s-Butoxycarbonyl, t-Butoxycarbonyl, Cyanomethyl, 2-Cyanoethyl, Benzyl, 4-Methoxybenzyl, Pyrid-2-yl-methyl, Pyrid-3-yl-methyl, Pyrid-4-yl-methyl, 6-Chlor-pyrid-3-yl-methyl steht,
- A¹, A²: und A⁴ für CH steht,
- R⁴: für Fluor, Chlor, Brom oder Iod steht
- Q: für für Wasserstoff, Methyl, Ethyl, n-Propyl, 1-Methylethyl, 1,1-Dimethylethyl, n-Butyl, 1-Methylpropyl, 2-Methylpropyl, 2-Methylbutyl, Hydroxymethyl, 2-Hydroxypropyl, Cyanomethyl, 2-Cyanoethyl, 2-Fluorethyl, 2,2-Difluorethyl, 2,2,2-Trifluorethyl, 1-Trifluormethylethyl, 2,2-Difluorpropyl, 3,3,3-Trifluropropyl, 2,2-Dimethyl-3-fluorpropyl, Cyclopropyl, 1-Cyano-cyclopropyl, 1-Methoxycarbonyl-cyclopropyl, 1-(*N*-Methylcarbamoyl)cyclopropyl, 1-(*N-*Cyclopropylcarbamoyl)cyclopropyl, Cyclopropyl-methyl, Cyclobutyl, Cyclopentyl, Cyclohexyl, 1-Cyclopropylethyl, Bis(cyclopropyl)methyl, 2,2-Dimethylcyclopropyl-methyl, 2-Phenylcyclopropyl, 2,2-Dichlorcyclopropyl, trans-2-Chlorcyclopropyl, cis-2-Chlorcyclopropyl, 2,2-Difluorcyclopropyl, trans-2-Fluorcyclopropyl, cis-2-Fluorcyclopropyl, trans-4-Hydroxycyclohexyl, 4-Trifluormethylcyclohexyl, Prop-2-enyl, 2-Methylprop-2-enyl, Prop-2-inyl, 1,1-Dimethylbut-2-inyl, 3-Chlor-prop-2-enyl" 3,3-Dichlor-prop-2-enyl, 3,3-Dichlor-1,1-dimethylprop-2-enyl, Phenyl, 2-Chlorphenyl, 3-Chlorphenyl, 4-Chlorphenyl, Oxetan-3-yl, Thietan-3-yl, 1-Oxido-thietan-3-yl, 1,1-Dioxido-thietan-3-yl, Isoxazol-3-ylmethyl, 1,2,4-Triazol-3-ylmethyl, 3-Methyloxetan-3-ylmethyl, Benzyl, 2,6-Difluorphenylmethyl, 3-Fluorphenylmethyl, 2-Fluorphenylmethyl, 2,5-Difluorphenylmethyl, 1-Phenylethyl, 4-Chlorphenylethyl, 2-Trifluormethylphenylethyl, 1-Pyridin-2-ylethyl, Pyridin-2-ylmethyl, (6-Chlor-pyridin-3-yl)methyl, 5-Fluorpyridin-2-ylmethyl, Pyrimidin-2-ylmethyl, Methoxy, 2-Ethoxyethyl, 2-(Methylsulfanyl)ethyl, 1-Methyl-2-(ethylsulfanyl)ethyl, 2-Methyl-1-(methylsulfanyl)propan-2-yl, Methoxycarbonyl, Methoxycarbonylmethyl, NH₂, *N*-Ethylamino, *N*-Allylamino, *N,N-*Dimethylamino, *N*,*N*-Diethylamino steht; oder
- Q: für ein mit 0 - 4 Substituenten V substituiertes Phenyl, Naphthyl, Pyridazin, Pyrazin, Pyrimidin, Triazin, Pyridin, Pyrazol, Thiazol, Isothiazol, Oxazol, Isoxazol, Triazol, Imidazol, Furan, Thiophen, Pyrrol, Oxadiazol, Thiadiazol steht, wobei
- V: unabhängig voneinander für Fluor, Chlor, Brom, Iod, Cyano, Nitro, Methyl, Ethyl, Difluormethyl, Trichlormethyl, Chlordifluormethyl, Dichlorfluormethyl, Trifluormethyl, Chlormethyl, Brommethyl, 1-Fluorethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Dilfluorethyl, Pentafluorethyl Pentafluor-tert-butyl, Heptafluor-n-propyl, Heptafluor-isopropyl, Nonafluor-n-butyl, Cyclopropyl, Cyclobutyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N*-Methoxyiminomethyl, 1-(*N-*Methoxyimino)-ethyl, Methylsulfanyl, Methylsulfonyl, Methylsulfinyl, Trifluormethylsulfonyl, Trifluormethylsulfinyl, Trifluormethylsulfanyl, *N*,*N-*Dimethylamino steht;
Speziell hervorgehobene Verbindungen im Sinne der Erfindung sind solche der allgemeinen Formeln (Ia), (Ib), (Ic), (Id), (Ie), (If), (Ig), (Ih), bei denen die Reste A₁-A₄, n, W, Q, R¹, R⁶, M¹, M² und Z¹-Z³ die oben beschriebenen Bedeutungen haben.

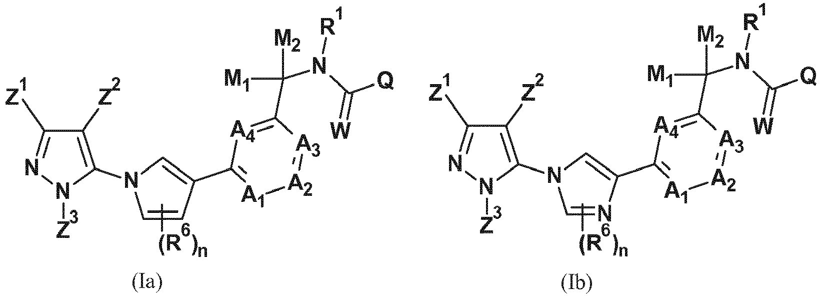

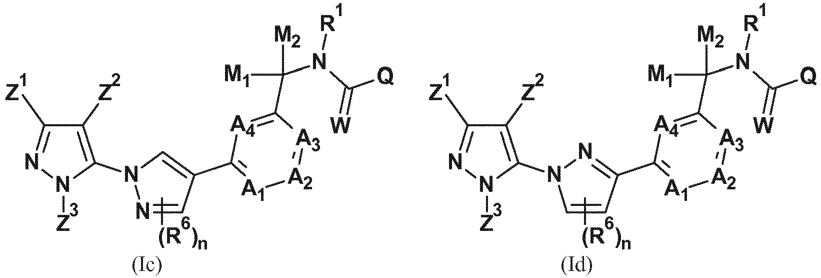

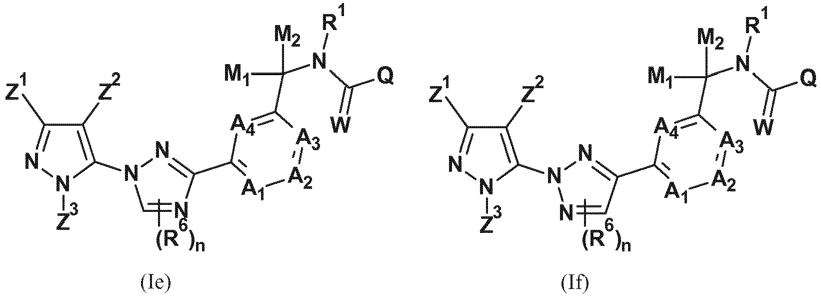

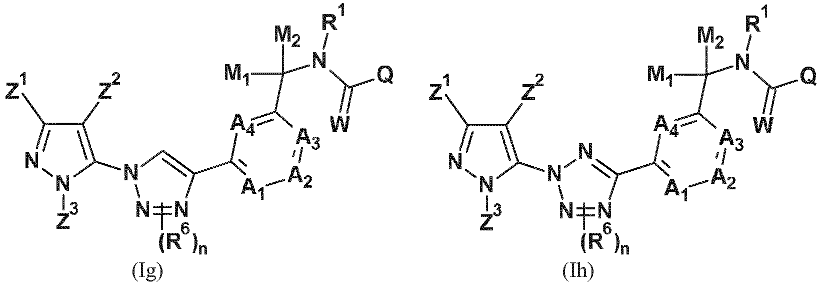

Speziell hervorgehobene Verbindungen im Sinne der Erfindung sind ferner solche der allgemeinen Formeln (IIa), (IIb), (IIc), (IId), (IIe), (IIf), (IIg), (IIh), bei denen die Reste A₁-A₄, n, W, Q, R¹, R⁶, M¹, M²und Z¹-Z³ die oben beschriebenen Bedeutungen haben.

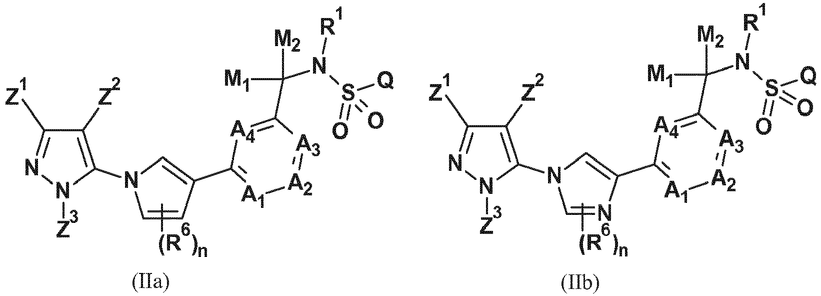

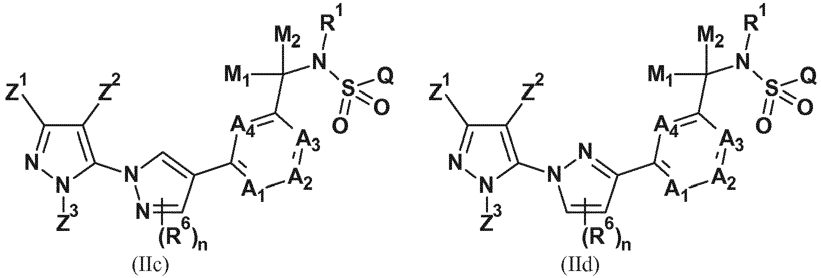

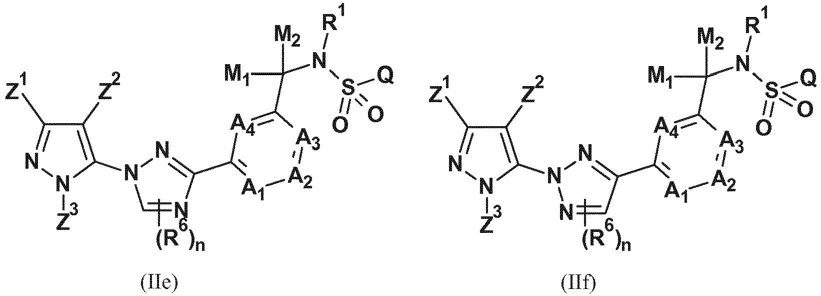

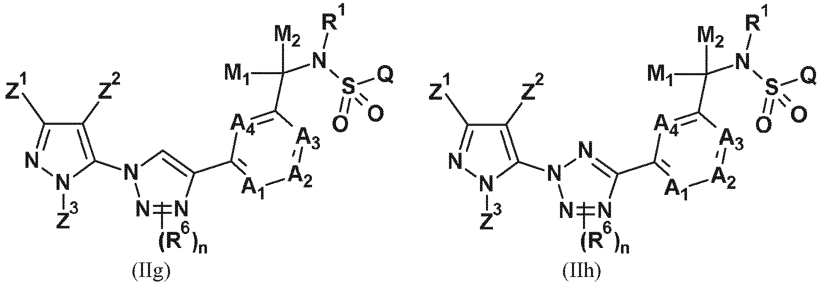

Erfindungsgemäß steht "Alkyl" - in Alleinstellung oder als Bestandteil einer chemischen Gruppe - für geradkettige oder verzweigte Kohlenstoffwasserstoffe, vorzugsweise mit 1 bis 6 Kohlenstoffatomen, wie beispielsweise Methyl, Ethyl, n-Propyl, Isopropyl, n-Butyl, Isobutyl, s-Butyl, t-Butyl, Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,2-Dimethylpropyl, 1,3-Dimethylbutyl, 1,4-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl und 2-Ethylbutyl. Ferner bevorzugt für Alkyle mit 1 bis 4 Kohlenstoffatomen, wie unter anderem Methyl, Ethyl, Ethyl, n-Propyl, Isopropyl, n-Butyl, Isobutyl, s-Butyl oder t-Butyl. Die erfindungsgemäßen Alkyle können mit einem oder mehreren, gleichen oder verschiedenen Resten substituiert sein.

Erfindungsgemäß steht "Alkenyl" - in Alleinstellung oder als Bestandteil einer chemischen Gruppe - für geradkettige oder verzweigte Kohlenstoffwasserstoffe, vorzugsweise mit 2 bis 6 Kohlenstoffatomen und mindestens einer Doppelbindung, wie beispielsweise Vinyl, 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-dimethyl-3-butenyl, 1,2-Dimethyl-2-butenyl, 1,2-Dimethyl-3-butenyl, 1,3-Dimethyl-2-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl. Ferner bevorzugt für Alkenyle mit 2 bis 4 Kohlenstoffatomen, wie unter anderem 2-Propenyl, 2-Butenyl oder 1-Methyl-2-propenyl. Die erfindungsgemäßen Alkenyle können mit einem oder mehreren, gleichen oder verschiedenen Resten substituiert sein.

Erfindungsgemäß steht "Alkinyl" - in Alleinstellung oder als Bestandteil einer chemischen Gruppe - für geradkettige oder verzweigte Kohlenstoffwasserstoffe, vorzugsweise mit 2 bis 6 Kohlenstoffatomen und mindestens einer Dreifachbindung wie beispielsweise 2-Propinyl, 2-Butinyl, 3-Butinyl, 1-Methyl-2-propinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-3-butiuyl, 1,2-Dimethyl-3-butinyl, 2,2-Dimethyl-3-butinyl, 1-Ethyl-3-butinyl, 2-Ethyl-3-butinyl, 1-Ethyl-1-methyl-2-propinyl und 2,5-Hexadiynyl. Ferner bevorzugt für Alkinyle mit 2 bis 4 Kohlenstoffatomen wie unter anderem Ethinyl, 2-Propinyl oder 2-Butinyl-2-propenyl. Die erfindungsgemäßen Alkinyle können mit einem oder mehreren, gleichen oder verschiedenen Resten substituiert sein.

Erfindungsgemäß steht "Cycloalkyl" - in Alleinstellung oder als Bestandteil einer chemischen Gruppe - für mono-, bi- oder tricyclische Kohlenwasserstoffe, vorzugsweise mit 3 bis 10 Kohlenstoffen wie beispielsweise Cyclopropyl, Cyclobutyl, Cyclopentyl, Cyclohexyl, Cycloheptyl, Cyclooctyl, Bicyclo[2.2.1]heptyl, Bicyclo[2.2.2]octyl oder Adamantyl. Ferner bevorzugt für Cycloalkyle mit 3, 4, 5, 6 oder 7 Kohlenstoffatomen, wie unter anderem Cyclopropyl oder Cyclobutyl. Die erfindungsgemäßen Cycloalkyle können mit einem oder mehreren, gleichen oder verschiedenen Resten substituiert sein.

Erfindungsgemäß steht "Alkylcycloalkyl" für mono-, bi- oder tricyclisches Alkylcycloalkyl, vorzugsweise mit 4 bis 10 oder 4 bis 7 Kohlenstoffatomen, wie beispielsweise Ethylcyclopropyl, Isopropylcyclobutyl, 3-Methylcyclopentyl und 4-Methyl-cyclohexyl. Ferner bevorzugt für Alkylcycloalkyle mit 4, 5 oder 7 Kohlenstoffatomen wie unter anderen Ethylcyclopropyl oder 4-Methyl-cyclohexyl. Die erfindungsgemäßen Alkylcycloalkyle können mit einem oder mehreren, gleichen oder verschiedenen Resten substituiert sein.

Erfindungsgemäß steht "Cycloalkylalkyl" für mono, bi- oder tricyclisches Cycloalkylalkyl, vorzugsweise mit 4 bis 10 oder 4 bis 7 Kohlenstoffatomen, wie beispielsweise Cyclopropylmethyl, Cyclobutylmethyl, Cyclopentylmethyl, Cyclohexylmethyl und Cyclopentylethyl. Ferner bevorzugt für Cycloalkylalkyle mit 4, 5 oder 7 Kohlenstoffatomen wie unter anderen Cyclopropylmethyl oder Cyclobutylmethyl. Die erfindungsgemäßen Cycloalkylalkyle können mit einem oder mehreren, gleichen oder verschiedenen Resten substituiert sein.

Erfindungsgemäß steht "Halogen" für Fluor, Chlor, Brom oder Iod, insbesondere für Fluor, Chlor oder Brom.

Die erfindungsgemäßen mit Halogen substituierten chemischen Gruppen, wie beispielsweise Halogenalkyl, Halogencycloalkyl, HalogenAlkoxy, HalogenAlkylsulfanyl, Halogenalkylsulfinyl oder Halogenalkylsulfonyl sind einfach oder mehrfach bis zur maximal möglichen Substituentenzahl mit Halogen substituiert. Bei mehrfacher Substitution mit Halogen, können die Halogenatome gleich oder verschieden sein und können alle an eines oder an mehrere Kohlenstoffatome gebunden sein. Dabei steht Halogen insbesondere für Fluor, Chlor, Brom oder Iod, vorzugsweise für Fluor, Chlor oder Brom und besonders bevorzugt für Fluor.

Erfindungsgemäß steht "Halogencycloalkyl" für mono-, bi- oder tricyclisches Halogencycloalkyl, vorzugsweise mit 3 bis 10 Kohlenstoffatomen, wie unter anderen 1-Fluor-cyclopropyl, 2-Fluor-cyclopropyl oder 1-Fluor-cyclobutyl. Ferner bevorzugt für Halogencycloalkyl mit 3, 5 oder 7 Kohlenstoffatomen. Die erfindungsgemäßen Halogencycloalkyle können mit einem oder mehreren, gleichen oder verschiedenen Resten substituiert sein.

Erfindungsgemäß steht "Halogenalkyl" "Halogenalkenyl" oder "Halogenalkinyl" für mit Halogen substituierte Alkyle, Alkenyle oder Alkinyle mit vorzugsweise 1 bis 9 gleichen oder verschiedenen Halogenatomen, wie beispielsweise Monohaloalkyl (= Monohalogenalkyl) wie CH₂CH₂Cl, CH₂CH₂F, CHClCH₃, CHFCH₃, CH₂Cl, CH₂F; Perhaloalkyl wie CCl₃ oder CF₃ oder CF₂CF₃; Polyhaloalkyl wie CHF₂, CH₂F, CH₂CHFCl, CHCl₂, CF₂CF₂H, CH₂CF₃. Entsprechendes gilt für Haloalkenyl und andere durch Halogen substituierten Reste. Haloalkoxy ist z.B. OCF₃, OCHF₂, OCH₂F, OCF₂CF₃, OCH₂CF₃ und OCH₂CH₂Cl;

Weitere Beispiele für Halogenalkyle sind Trichlormethyl, Chlordifluormethyl, Dichlorfluormethyl, Chlormethyl, Brommethyl, 1-Fluorethyl, 2-Fluorethyl, 2,2-Difluorethyl, 2,2,2-Trifluorethyl, 2,2,2-Trichlorethyl, 2-Chlor-2,2-difluorethyl, Pentafluorethyl und Pentafluor-t-butyl. Bevorzugt sind Halogenalkyle mit 1 bis 4 Kohlenstoffatomen und 1 bis 9, vorzugsweise 1 bis 5 gleichen oder verschiedenen Halogenatomen, die ausgewählt sind unter Fluor, Chlor oder Brom. Besonders bevorzugt sind Halogenalkyle mit 1 oder 2 Kohlenstoffatomen und mit 1 bis 5 gleichen oder verschiedenen Halogenatomen, die ausgewählt sind unter Fluor oder Chlor, wie unter anderen Difluormethyl, Trifluormethyl oder 2,2-Difluorethyl.

Erfindungsgemäß steht "Hydroxyalkyl" für geradkettigen oder verzweigten Alkohol, vorzugsweise mit 1 bis 6 Kohlenstoffatomen, wie beispielsweise Methanol, Ethanol, n-Propanol, Isopropanol, n-Butanol, Isobutanol, s-Butanol und t-Butanol. Ferner bevorzugt für Hydroxyalkylgruppen mit 1 bis 4 Kohlenstoffatomen. Die erfindungsgemäßen Hydroxyalkylgruppen können mit einem oder mehreren, gleichen oder verschiedenen Resten substituiert sein

Erfindungsgemäß steht "Alkoxy" für geradkettiges oder verzweigtes O-Alkyl, vorzugsweise mit 1 bis 6 Kohlenstoffatomen, wie beispielsweise Methoxy, Ethoxy, n-Propoxy, Isopropoxy, n-Butoxy, Isobutoxy, s-Butoxy und t-Butoxy. Ferner bevorzugt für Alkoxygruppen mit 1 bis 4 Kohlenstoffatomen. Die erfindungsgemäßen Alkoxygruppen können mit einem oder mehreren, gleichen oder verschiedenen Resten substituiert sein.

Erfindungsgemäß steht "Halogenalkoxy" für mit Halogen substituiertes geradkettiges oder verzweigtes O-Alkyl, vorzugsweise mit 1 bis 6 Kohlenstoffatomen, wie unter anderem Difluormethoxy, Trifluormethoxy, 2,2-Difluorethoxy, 1,1,2,2-Tetrafluorethoxy, 2,2,2-Trifluorethoxy und 2-Chlor-1,1,2-trifluorethoxy. Ferner bevorzugt für Halogenalkoxygruppen mit 1 bis 4 Kohlenstoffatomen. Die erfindungsgemäßen Halogenalkoxygruppen können mit einem oder mehreren, gleichen oder verschiedenen Resten substituiert sein.

Erfindungsgemäß steht "Alkylsulfanyl" für geradkettiges oder verzweigtes S-Alkyl, vorzugsweise mit 1 bis 6 Kohlenstoffatomen, wie beispielsweise Methylthio, Ethylthio, n-Propylthio, Isopropylthio, n-Butylthio, Isobutylthio, s-Butylthio und t-Butylthio. Ferner bevorzugt für Alkylsulfanylgruppen mit 1 bis 4 Kohlenstoffatomen. Die erfindungsgemäßen Alkylsulfanylgruppen können mit einem oder mehreren, gleichen oder verschiedenen Resten substituiert sein.

Beispiele für Halogenalkylsulfanylalkyle, d.h. mit Halogen substituierte Alkylsulfanylgruppen, sind unter anderem Difluormethylthio, Trifluormethylthio, Trichlormethylthio, Chlordifluormethylthio, 1-Fluorethylthio, 2-Fluorethylthio, 2,2-Difluorethylthio, 1,1,2,2-Tetrafluorethylthio, 2,2,2-Trifluorethylthio oder 2-Chlor-1,1,2-trifluorethylthio.

Erfindungsgemäß steht "Alkylsulfinyl" für geradkettiges oder verzweigtes Alkylsulfinyl, vorzugsweise mit 1 bis 6 Kohlenstoffatomen wie beispielsweise Methylsulfinyl, Ethylsulfinyl, n-Propylsulfinyl, Isopropylsulfinyl, n-Butylsulfinyl, Isobutylsulfinyl, s-Butylsulfinyl und t-Butylsulfinyl. Ferner bevorzugt für Alkylsulfinylgruppen mit 1 bis 4 Kohlenstoffatomen. Die erfindungsgemäßen Alkylsulfinylgruppen können mit einem oder mehreren, gleichen oder verschiedenen Resten substituiert sein.

Beispiele für Halogenalkylsulfinylgrupen, d.h. mit Halogen substituierte Alkylsulfinylgruppen, sind unter anderem Difluormethylsulfinyl, Trifluormethylsulfinyl, Trichlormethylsulfinyl, Chlordifluormethylsulfinyl, 1-Fluorethylsulfinyl, 2-Fluorethylsulfinyl, 2,2-Difluorethylsulfinyl, 1,1,2,2-Tetrafluorethylsulfinyl, 2,2,2-Trifluorethylsulfinyl und 2-Chlor-1,1,2-trifluorethylsulfinyl.

Erfindungsgemäß steht "Alkylsulfonyl" für geradkettiges oder verzweigtes Alkylsulfonyl, vorzugsweise mit 1 bis 6 Kohlenstoffatomen wie beispielsweise Methylsulfonyl, Ethylsulfonyl, n-Propylsulfonyl, Isopropylsulfonyl, n-Butylsulfonyl, Isobutylsulfonyl, s-Butylsulfonyl und t-Butylsulfonyl. Ferner bevorzugt für Alkylsulfonylgruppen mit 1 bis 4 Kohlenstoffatomen. Die erfindungsgemäßen Alkylsulfonylgruppen können mit einem oder mehreren, gleichen oder verschiedenen Resten substituiert sein.

Beispiele für Halogenalkylsulfonylgrupen, d.h. mit Halogen substituierte Alkylsulfonylgruppen sind unter anderem Difluormethylsulfonyl, Trifluormethylsulfonyl, Trichlormethylsulfonyl, Chlordifluormethylsulfonyl, 1-Fluorethylsulfonyl, 2-Fluorethylsulfonyl, 2,2-Difluorethylsulfonyl, 1,1,2,2-Tetrafluorethylsulfonyl, 2,2,2-Trifluorethylsulfonyl und 2-Chlor-1,1,2-trifluorethylsulfonyl.

Erfindungsgemäß steht "Alkylcarbonyl" für geradkettiges oder verzweigtes Alkyl-C(=O), vorzugsweise mit 2 bis 7 Kohlenstoffatomen, wie Methylcarbonyl, Ethylcarbonyl, n-Propylcarbonyl, Isopropylcarbonyl, s-Butylcarbonyl und t-Butylcarbonyl. Ferner bevorzugt für Alkylcarbonyle mit 1 bis 4 Kohlenstoffatomen. Die erfindungsgemäßen Alkylcarbonyle können mit einem oder mehreren, gleichen oder verschiedenen Resten substituiert sein.

Erfindungsgemäß steht "Cycloalkylcarbonyl" für geradkettiges oder verzweigtes Cycloalkylcarbonyl, vorzugsweise mit 3 bis 10 Kohlenstoffatomen im Cycloalkylteil, wie beispielsweise Cyclopropylcarbonyl, Cyclobutylcarbonyl, Cyclopentylcarbonyl, Cyclohexyl-carbonyl, Cycloheptylcarbonyl, Cyclooctylcarbonyl, Bicyclo[2.2.1]heptyl, Bycyclo[2.2.2]octylcarbonyl und Adamantylcarbonyl. Ferner bevorzugt für Cycloalkylcarbonyl mit 3, 5 oder 7 Kohlenstoffatomen im Cycloalkylteil. Die erfindungsgemäßen Cycloalkylcarbonylgruppen können mit einem oder mehreren, gleichen oder verschiedenen Resten substituiert sein.

Erfindungsgemäß steht "Alkoxycarbonyl" - in Alleinstellung oder als Bestandteil einer chemischen Gruppe - für geradkettiges oder verzweigtes Alkoxycarbonyl, vorzugsweise mit 1 bis 6 Kohlenstoffatomen oder 1 bis 4 Kohlenstoffatomen im Alkoxyteil, wie beispielsweise Methoxycarbonyl, Ethoxycarbonyl, n-Propoxycarbonyl, Isopropoxycarbonyl, s-Butoxycarbonyl und t-Butoxycarbonyl. Die erfindungsgemäßen Alkoxycarbonylgruppen können mit einem oder mehreren, gleichen oder verschiedenen Resten substituiert sein.

Erfindungsgemäß steht "Alkylaminocarbonyl" für geradkettiges oder verzweigtes Alkylaminocarbonyl mit vorzugsweise 1 bis 6 Kohlenstoffatomen oder 1 bis 4 Kohlenstoffatomen im Alkylteil, wie beispielsweise Methylaminocarbonyl, Ethylaminocarbonyl, n-Proylaminocarbonyl, Isopropylaminocarbonyl, s-Butylaminocarbonyl und t-Butylaminocarbonyl. Die erfindungsgemäßen Alkylaminocarbonylgruppen können mit einem oder mehreren, gleichen oder verschiedenen Resten substituiert sein.

Erfindungsgemäß steht *"N,N-*Dialkylamino-carbonyl" für geradkettiges oder verzweigtes *N*,*N-*Dialkylaminocarbonyl mit vorzugsweise 1 bis 6 Kohlenstoffatomen oder 1 bis 4 Kohlenstoffatomen im Alkylteil, wie beispielsweise *N,N-*Dimethylamino-carbonyl, N.N-Diethylamino-carbonyl, *N*,*N*-Di(n-propylamino)-carbonyl, *N*,*N*-Di-(isopropylamino)-carbonyl und *N*,*N*-Di-(s-butylamino)-carbonyl. Die erfindungsgemäßen *N*,*N-*Dialkylamino-carbonylgruppen können mit einem oder mehreren, gleichen oder verschiedenen Resten substituiert sein.

Erfindungsgemäß steht "Aryl" für ein mono-, bi- oder polycyclisches aromatisches System mit vorzugsweise 6 bis 14, insbesondere 6 bis 10 Ring-Kohlenstoffatomen, wie beispielsweise Phenyl, Naphthyl, Anthryl, Phenanthrenyl, vorzugsweise Phenyl. Ferner steht Aryl auch für mehrcyclische Systeme, wie Tetrahydronaphtyl, Indenyl, Indanyl, Fluorenyl, Biphenyl, wobei die Bindungsstelle am aromatischen System ist. Die erfindungsgemäßen Arylgruppen können mit einem oder mehreren, gleichen oder verschiedenen Resten substituiert sein.

Beispiele substitutierter Aryle stellen die Arylalkyle dar, die gleichfalls mit einem oder mehreren, gleichen oder verschiedenen Resten im Alkyl- und/oder Arylteil substituiert sein können. Beispiele solcher Arylalkyle sind unter anderem Benzyl und 1-Phenylethyl.

Erfindungsgemäß steht "Heterocyclus", "heterocyclischer Ring" oder "heterocyclisches Ringsystem" für ein carbocyclisches Ringsystem mit mindestens einem Ring, in dem mindestens ein Kohlenstoffatom durch ein Heteroatom ersetzt ist, vorzugsweise durch ein Heteroatom aus der Gruppe N, O, S, P, B, Si, Se und der gesättigt, ungesättigt oder heteroaromatisch ist und dabei unsubstituiert oder mit einem Substituenten Z substituiert sein kann, wobei die Bindungsstelle an einem Ringatom lokalisiert ist. Wenn nicht anders definiert, enthält der heterocyclische Ring vorzugsweise 3 bis 9 Ringatome, insbesondere 3 bis 6 Ringatome, und ein oder mehrere, vorzugsweise 1 bis 4, insbesondere 1, 2 oder 3 Heteroatome im heterocyclischen Ring, vorzugsweise aus der Gruppe N, O, und S, wobei jedoch nicht zwei Sauerstoffatome direkt benachbart sein sollen. Die heterocyclischen Ringe enthalten gewöhnlicherweise nicht mehr als 4 Stickstoffatome, und/oder nicht mehr als 2 Sauerstoffatome und/oder nicht mehr als 2 Schwefelatome. Ist der Heterocyclylrest oder der heterocyclische Ring gegebenenfalls substituiert, kann er mit anderen carbocyclischen oder heterocyclischen Ringen annelliert sein. Im Falle von gegebenenfalls substituiertem Heterocyclyl werden erfindungsgemäß auch mehrcyclische Systeme umfaßt, wie beispielsweise 8-Aza-bicyclo[3.2.1]octanyl oder 1-Aza-bicyclo[2.2.1]heptyl. Im Falle von gegebenenfalls substituiertem Heterocyclyl werden erfindungsgemäß auch spirocyclische Systeme umfasst, wie beispielsweise 1-Oxa-5-aza-spiro[2.3]hexyl.

Erfindungsgemäße Heterocyclylgruppen sind beispielsweise Piperidinyl, Piperazinyl, Morpholinyl, Thiomorpholinyl, Dihydropyranyl, Tetrahydropyranyl, Dioxanyl, Pyrrolinyl, Pyrrolidinyl, Imidazolinyl, Imidazolidinyl, Thiazolidinyl, Oxazolidinyl, Dioxolanyl, Dioxolyl, Pyrazolidinyl, Tetrahydrofuranyl, Dihydrofuranyl, Oxetanyl, Oxiranyl, Azetidinyl, Aziridinyl, Oxazetidinyl, Oxaziridinyl, Oxazepanyl, Oxazinanyl, Azepanyl, Oxopyrrolidinyl, Dioxopyrrolidinyl, Oxomorpholinyl, Oxopiperazinyl und Oxepanyl.

Eine besondere Bedeutung kommt Heteroarylen, also heteroaromatischen Systemen zu. Erfindungsgemäß steht der Ausdruck Heteroaryl für heteroaromatische Verbindungen, das heißt vollständig ungesättigte aromatische heterocyclische Verbindungen, die unter die vorstehende Definiton von Heterocyclen fallen. Vorzugsweise für 5- bis 7-gliedrige Ringe mit 1 bis 3, vorzugsweise 1 oder 2 gleichen oder verschiedenen Heteroatomen aus der oben genannten Gruppe. Erfindungsgemäße Heteroaryle sind beispielsweise Furyl, Thienyl, Pyrazolyl, Imidazolyl, 1,2,3- und 1,2,4-Triazolyl, Isoxazolyl, Thiazolyl, Isothiazolyl, 1,2,3-, 1,3,4-, 1,2,4- und 1,2,5-Oxadiazolyl, Azepinyl, Pyrrolyl, Pyridyl, Pyridazinyl, Pyrimidinyl, Pyrazinyl, 1,3,5-, 1,2,4- und 1,2,3-Triazinyl, 1,2,4-, 1,3,2-, 1,3,6- und 1,2,6-Oxazinyl, Oxepinyl, Thiepinyl, 1,2,4-Triazolonyl und 1,2,4-Diazepinyl. Die erfindungsgemäßen Heteroarylgruppen können ferner mit einem oder mehreren, gleichen oder verschiedenen Resten substituiert sein.

Substituierte Gruppen, wie ein substituierter Alkyl-, Alkenyl-, Alkinyl-, Cycloalkyl-, Aryl-, Phenyl-, Benzyl-, Heterocyclyl- und Heteroarylrest, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Alkylsulfanyl, Hydroxy, Amino, Nitro, Carboxy oder eine der Carboxygruppe äquivalente Gruppe, Cyano, Isocyano, Azido, Alkoxycarbonyl, Alkylcarbonyl, Formyl, Carbamoyl, Mono- und *N*,*N*-Dialkylamino-carbonyl, substituiertes Amino, wie Acylamino, Mono- und *N*,*N*-Dialkylamino, Trialkylsilyl und gegebenenfalls substituiertes Cycloalkyl, gegebenenfalls substituiertes Aryl, gegebenenfalls substituiertes Heterocyclyl, wobei jeder der letztgenannten cyclischen Gruppen auch über Heteroatome oder divalente funktionelle Gruppen wie bei den genannten Alkylresten gebunden sein kann, und Alkylsulfinyl, wobei beide Enantiomere der Alkylsulfonylgruppe umfasst sind, Alkylsulfonyl, Alkylphosphinyl, Alkylphosphonyl und, im Falle cyclischer Reste (= "cyclischer Grundkörper"), auch Alkyl, Haloalkyl, Alkylsulfanylalkyl, Alkoxy-alkyl, gegebenfalls substituiertes Mono- und *N*,*N*-Dialkyl-aminoalkyl und Hydroxyalkyl bedeutet.

Im Begriff "substituierte Gruppen" wie substituiertes Alkyl etc. sind als Substituenten zusätzlich zu den genannten gesättigten kohlenwasserstoffhaltigen Resten entsprechende ungesättigte aliphatische und aromatische Reste, wie gegebenenfalls substituiertes Alkenyl, Alkinyl, Alkenyloxy, Alkinyloxy, Alkenylthio, Alkinylthio, Alkenyloxycarbonyl, Alkinyloxycarbonyl, Alkenylcarbonyl, Alkinylcarbonyl, Mono- und *N*,*N*-Dialkenylamino-carbonyl, Mono- und Dialkinylaminocarbonyl, Mono- und *N,N-*Dialkenylamino, Mono- und *N*,*N*-Dialkinylamino, Trialkenylsilyl, Trialkinylsilyl, gegebenenfalls substituiertes Cycloalkenyl, gegebenenfalls substituiertes Cycloalkinyl, Phenyl, Phenoxy etc. eingeschlossen. Im Falle von substituierten cyclischen Resten mit aliphatischen Anteilen im Ring werden auch cyclische Systeme mit solchen Substituenten umfaßt, die mit einer Doppelbindung am Ring gebunden sind, z. B. mit einer Alkylidengruppe wie Methyliden oder Ethyliden oder einer Oxogruppe, Iminogruppe sowie einer substituierten Iminogruppe.

Wenn zwei oder mehrere Reste einen oder mehrere Ringe bilden, so können diese carbocyclisch, heterocyclisch, gesättigt, teilgesättigt, ungesättigt, beispielsweise auch aromatisch und weiter substituiert sein.

Die beispielhaft genannten Substituenten ("erste Substituentenebene") können, sofern sie kohlenwasserstoffhaltige Anteile enthalten, dort gegebenenfalls weiter substituiert sein ("zweite Substitutentenebene"), beispielsweise durch einen der Substituenten, wie er für die erste Substituentenebene definiert ist. Entsprechende weitere Substituentenebenen sind möglich. Vorzugsweise werden vom Begriff "substituierter Rest" nur ein oder zwei Substitutentenebenen umfasst.

### Bevorzugte Substituenten für die Substituentenebenen sind beispielsweise

Amino, Hydroxy, Halogen, Nitro, Cyano, Isocyano, Mercapto, Isothiocyanato, Carboxy, Carbonamid, SF₅, Aminosulfonyl, Alkyl, Cycloalkyl, Alkenyl, Cycloalkenyl, Alkinyl, *N-*Mono-alkyl-amino, *N*,*N-*Dialkylamino, *N-*Alkanoylamino, Alkoxy, Alkenyloxy, Alkinyloxy, Cycloalkoxy, Cycloalkenyloxy, Alkoxycarbonyl, Alkenyloxycarbonyl, Alkinyloxycarbonyl, Aryloxycarbonyl, Alkanoyl, Alkenylcarbonyl, Alkinylcarbonyl, Arylcarbonyl, Alkylsulfanyl, Cycloalkylsulfanyl, Alkenylthio, Cycloalkenylthio, Alkinylthio, Alkylsulfenyl und Alkylsulfinyl, wobei beide Enantiomere der Alkylsulfinylgruppe umfasst sind, Alkylsulfonyl, *N-*Mono-alkyl-aminosulfonyl, *N*,*N*-Dialkyl-aminosulfonyl, Alkylphosphinyl, Alkylphosphonyl, wobei für Alkylphosphinyl bzw. Alkylphosphonyl beide Enantiomere umfasst sind, *N*-Alkyl-aminocarbonyl, *N*,*N-*Dialkyl-amino-carbonyl, N-Alkanoylamino-carbonyl, N-Alkanoyl-N-alkyl-aminocarbonyl, Aryl, Aryloxy, Benzyl, Benzyloxy, Benzylthio, Arylthio, Arylamino, Benzylamino, Heterocyclyl und Trialkylsilyl.

Substituenten, die aus mehreren Substituentenebenen zusammengesetzt sind, sind bevorzugt Alkoxyalkyl, Alkylsulfanylalkyl, Alkylsulfanylalkoxy, Alkoxyalkoxy, Phenethyl, Benzyloxy, Halogenalkyl, Halogencycloalkyl, Halogenalkoxy, Halogenalkylsulfanyl, Halogenalkylsulfinyl, Halogenalkylsulfonyl, Halogenalkanoyl, Halogenalkylcarbonyl, Halogenalkoxycarbonyl, Halogenalkoxyalkoxy, Halogenalkoxyalkylsulfanyl, Halogenalkoxyalkanoyl, Halogenalkoxyalkyl.

Bei Resten mit C-Atomen sind solche mit 1 bis 6 C-Atomen, vorzugsweise 1 bis 4 C-Atomen, insbesondere 1 oder 2 C-Atomen bevorzugt. Bevorzugt sind in der Regel Substituenten aus der Gruppe Halogen, z.B. Fluor und Chlor, (C₁-C₄)Alkyl, vorzugsweise Methyl oder Ethyl, (C₁-C₄)Haloalkyl, vorzugsweise Trifluormethyl, (C₁-C₄)Alkoxy, vorzugsweise Methoxy oder Ethoxy, (C₁-C₄)Haloalkoxy, Nitro und Cyano. Besonders bevorzugt sind dabei die Substituenten Methyl, Methoxy, Fluor und Chlor.

Substituiertes Amino wie mono- oder disubstituiertes Amino bedeutet einen Rest aus der Gruppe der substituierten Aminoreste, welche beispielsweise durch einen bzw. zwei gleiche oder verschiedene Reste aus der Gruppe Alkyl, Hydroxy, Amino, Alkoxy, Acyl und Aryl *N-*substituiert sind; vorzugsweise *N-*Mono- und *N*,*N*-Dialkylamino, (z.B. Methylamino, Ethylamino, *N*,*N-*Dimethylamino, *N,N-*Diethylamino, *N,N-*Di-n-propylamino, *N,N*-Diisopropylamino oder *N*,*N-*Dibutylamino), *N*-Mono- oder *N*,*N*-Dialkoxyalkylaminogruppen (z.B. *N*-Methoxymethylamino, *N*-Methoxyethylamino, *N,N-Di-*(methoxymethyl)-amino oder *N*,*N*-Di-(methoxyethyl)-amino), *N*-Mono- und *N*,*N*-Diarylamino, wie gegebenenfalls substituierte Aniline, Acylamino, *N,N-*diacylamino, *N*-Alkyl-*N*-arylamino, *N-*Alkyl-*N-*acylamino sowie gesättigte *N-*Heterocyclen; dabei sind Alkylreste mit 1 bis 4 C-Atomen bevorzugt; Aryl ist dabei vorzugsweise Phenyl oder substituiertes Phenyl; für Acyl gilt dabei die weiter unten genannte Definition, vorzugsweise (C₁-C₄)Alkanoyl. Entsprechenes gilt für substituiertes Hydroxylamino oder Hydrazino.

Erfindungsgemäß umfasst der Begriff "cyclische Aminogruppen" heteroaromatische oder aliphatische Ringsysteme mit einem oder mehreren Stickstoffatomen. Die Heterocyclen sind gesättigt oder ungesättigt, bestehen aus einem oder mehreren, gegebenenfalls kondensierten Ringsystemen und beinhalten gegebenenfalls weitere Heteroatome, wie beispielsweise ein oder zwei Stickstoff-, Sauerstoff- und/oder Schwefelatome. Ferner umfasst der Begriff auch solche Gruppen, die einen Spiroring oder verbrücktes Ringsystem aufweisen. Die Anzahl der Atome, die die cyclische Aminogruppe bilden, ist beliebig und kann z.B. im Falle eines Einringsystems aus 3 bis 8 Ringatomen und im Falle eines Zweiringsystems aus 7 bis 11 Atomen.

Beispielhaft für cyclische Aminogruppen mit gesättigten und ungesättigten monocyclischen Gruppen mit einem Stickstoffatom als Heteroatom seien 1-Azetidinyl, Pyrrolidino, 2-Pyrrolidin-1-yl, 1-Pyrrolyl, Piperidino, 1,4-Dihydropyrazin-1-yl, 1,2,5,6-Tetrahydropyrazin-1-yl, 1,4-Dihydropyridin-1-yl, 1,2,5,6-Tetrahydropyridin-1-yl, Homopiperidinyl genannt; beispielhaft für cyclische Aminogruppen mit gesättigten und ungesättigten monocyclischen Gruppen mit zwei oder mehreren Stickstoffatomen als Heteroatome seien 1-Imidazolidinyl, 1-Imidazolyl, 1-Pyrazolyl, 1-Triazolyl, 1-Tetrazolyl, 1-Piperazinyl, 1-Homopiperazinyl, 1,2-Dihydro-piperazin-1-yl, 1,2-Dihydro-pyrimidin-1-yl, Perhydropyrimidin-1-yl, 1,4-Diazacycloheptan-1-yl, genannt; beispielhaft für cyclische Aminogruppen mit gesättigten und ungesättigten monocyclischen Gruppen mit einem oder zwei Sauerstoffatomen und einem bis drei Stickstoffatomen als Heteroatome, wie beispielsweise Oxazolidin-3-yl, 2,3-Dihydroisoxazol-2-yl, Isoxazol-2-yl, 1,2,3-Oxadiazin-2-yl, Morpholino, beispielhaft für cyclische Aminogruppen mir gesättigten und ungesättigten monocyclischen Gruppen mit einem bis drei Stickstoffatomen und einem bis zwei Schwefelatomen als Heteroatome seien Thiazolidin-3-yl, Isothiazolin-2-yl, Thiomorpholino, oder Dioxothiomorpholino genannt; beispielhaft für cyclische Aminogruppen mit gesättigten und ungesättigten kondensierten cyclischen Gruppen seien Indol-1-yl, 1,2-Dihydrobenzimidazol-1-yl, Perhydropyrrolo[1,2-a]pyrazin-2-yl genannt; beispielhaft für cyclische Aminogruppen mit spirocyclischen Gruppen sei das 2-Azaspiro[4,5]decan-2-yl genannt; beispielhaft für cyclische Aminogruppen mit verbrückten heterocyclischen Gruppen sei das 2-Azabicyclo[2,2,1]heptan-7-yl genannt.

Substituiertes Amino schließt auch quartäre Ammoniumverbindungen (Salze) mit vier organischen Substituenten am Stickstoffatom ein.

Gegebenenfalls substituiertes Phenyl ist vorzugsweise Phenyl, das unsubstituiert oder ein- oder mehrfach, vorzugsweise bis zu dreifach durch gleiche oder verschiedene Reste aus der Gruppe Halogen, (C₁-C₄)Alkyl, (C₁-C₄)Alkoxy, (C₁-C₄)Alkoxy-(C₁-C₄)alkoxy , (C₁-C₄)Alkoxy-(C₁-C₄)alkyl, (C₁-C₄)Halogenalkyl, (C₁-C₄)Halogenalkoxy, (C₁-C₄)Alkylsulfanyl, (C₁-C₄)Halogenalkylsulfanyl, Cyano, Isocyano und Nitro substituiert ist, z.B. o-, m- und p-Tolyl, Dimethylphenyle, 2-, 3- und 4-Chlorphenyl, 2-, 3- und 4-Fluorphenyl, 2-, 3- und 4-Trifluormethyl- und -Trichlormethylphenyl, 2,4-, 3,5-, 2,5- und 2,3-Dichlorphenyl, o-, m- und p-Methoxyphenyl.

Gegebenenfalls substituiertes Cycloalkyl ist vorzugsweise Cycloalkyl, das unsubstituiert oder ein- oder mehrfach, vorzugsweise bis zu dreifach durch gleiche oder verschiedene Reste aus der Gruppe Halogen, Cyano, (C₁-C₄)Alkyl, (C₁-C₄)Alkoxy, (C₁-C₄)Alkoxy-(C₁-C₄)alkoxy , (C₁-C₄)Alkoxy-(C₁-C₄)alkyl, (C₁-C₄)Halogenalkyl und (C₁-C₄)Halogenalkoxy substituiert ist, insbesondere durch einen oder zwei (C₁-C₄)Alkylreste substituiert ist,

Gegebenenfalls substituiertes Heterocyclyl ist vorzugsweise Heterocyclyl, das unsubstituiert oder ein- oder mehrfach, vorzugsweise bis zu dreifach durch gleiche oder verschiedene Reste aus der Gruppe Halogen, Cyano, (C₁-C₄)Alkyl, (C₁-C₄)Alkoxy, (C₁-C₄)Alkoxy-(C₁-C₄)alkoxy , (C₁-C₄)Alkoxy-(C₁-C₄)alkyl, (C₁-C₄)Halogenalkyl, (C₁-C₄)Halogenalkoxy, Nitro und Oxo substituiert ist, insbesondere ein- oder mehrfach durch Reste aus der Gruppe Halogen, (C₁-C₄)Alkyl, (C₁-C₄)Alkoxy, (C₁-C₄)Halogenalkyl und Oxo, ganz besonders durch einen oder zwei (C₁-C₄)Alkylreste substituiert ist.

Beispiele für Alkyl substituierte Heteroaryle sind Furylmethyl, Thienylmethyl, Pyrazolylmethyl, Imidazolylmethyl, 1,2,3- und 1,2,4-Triazolylmethyl, Isoxazolylmethyl, Thiazolylmethyl, Isothiazolylmethyl, 1,2,3-, 1,3,4-, 1,2,4- und 1,2,5-Oxadiazolylmethyl, Azepinylmethyl, Pyrrolylmethyl, Pyridylmethyl" Pyridazinylmethyl, Pyrimidinylmethyl, Pyrazinylmethyl, 1,3,5-, 1,2,4- und 1,2,3-Triazinylmethyl, 1,2,4-, 1,3,2-, 1,3,6- und 1,2,6-Oxazinylmethyl, Oxepinylmethyl, Thiepinylmethyl und 1,2,4-Diazepinylmethyl.

Erfindungsgemäß geeignete Salze der erfindungsgemäßen Verbindungen, beispielsweise Salze mit Basen oder Säureadditionssalze, sind alle üblichen nicht toxischen Salze, vorzugsweise landwirtschaftlich und/oder physiologisch annehmbare Salze. Beispielsweise Salze mit Basen oder Säureadditionssalze. Bevorzugt werden Salze mit anorganischen Basen, wie beispielsweise Alkalimetallsalze (z.B. Natrium-, Kalium- oder Cäsiumsalze), Erdalkalimetallsalze (z.B. Calzium- oder Magnesiumsalze), Ammoniumsalze oder Salze mit organischen Basen, insbesondere mit organischen Aminen, wie beispielsweise Triethylammonium-, Dicyclohexylammonium-, *N*,*N*'-Dibenzylethylen-diammonium-, Pyridinium-, Picolinium- oder Ethanolammoniumsalze, Salze mit anorganischen Säuren (z.B. Hydrochloride, Hydrobromide, Dihydrosulfate, Trihydrosulfate, oder Phosphate), Salze mit organischen Carbonsäuren oder organischen Sulfosäuren (z.b. Formiate, Acetate, Trifluoracetate, Maleate, Tartrate, Methansulfonate, Benzolsulfonate oder 4-Toluolsulfonate). Bekannterweise können t-Amine, wie beispielsweise manche der erfindungsgemäßen Verbindungen, *N*-Oxide bilden, welche ebenfalls erfindungsgemäße Salze darstellen.

Die erfindungsgemäßen Verbindungen können in Abhängigkeit von der Art der Substituenten als geometrische und/oder als optisch aktive Isomere oder entsprechende Isomerengemische in unterschiedlicher Zusammensetzung vorliegen. Diese Stereoisomere sind beispielsweise Enantiomere, Diastereomere, Atropisomere oder geometrische Isomere. Die Erfindung umfasst somit reine Stereoisomere als auch beliebige Gemische dieser Isomere.

Die erfindungsgemäßen Verbindungen können gegebenenfalls in verschiedenen polymorphen Formen oder als Mischung verschiedener polymorpher Formen vorliegen. Sowohl die reinen Polymorphe als auch die Polymorphgemische sind Gegenstand der Erfindung und können erfindungsgemäß verwendet werden.

Die Verbindungen der allgemeinen Formel (I) können mit anderen insektiziden, nematiziden akariziden oder antimikrobiellen Wirkstoffen gemischt oder gemeinsam angewendet werden. In diesen Mischungen oder gemeinsamen Anwendungen treten synergistische Wirkungen auf, d.h. die beobachtete Wirkung dieser Mischungen oder gemeinsamen Anwendungen ist höher als die Summe der Wirkungen der Einzelwirkstoffe innerhalb dieser Anwendungen. Beispiele für solche Misch- bzw. Kombinationspartner sind:
(1) Acetylcholinesterase (AChE) Inhibitoren, wie beispielsweise
   Carbamate, z.B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb; oder
   Organophosphate, z.B. Acephate, Azamethiphos, Azinphos-ethyl, Azinphos-methyl, Cadusafos, Chlorethoxyfos, Chlorfenvinphos, Chlormephos, Chlorpyrifos, Chlorpyrifos-methyl, Coumaphos, Cyanophos, Demeton-S-methyl, Diazinon, Dichlorvos/DDVP, Dicrotophos, Dimethoate, Dimethylvinphos, Disulfoton, EPN, Ethion, Ethoprophos, Famphur, Fenamiphos, Fenitrothion, Fenthion, Fosthiazate, Heptenophos, Imicyafos, Isofenphos, Isopropyl O-(methoxyaminothio-phosphoryl) salicylat, Isoxathion, Malathion, Mecarbam, Methamidophos, Methidathion, Mevinphos, Monocrotophos, Naled, Omethoate, Oxydemeton-methyl, Parathion, Parathion-methyl, Phenthoate, Phorate, Phosalone, Phosmet, Phosphamidon, Phoxim, Pirimiphos-methyl, Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Trichlorfon und Vamidothion.
(2) GABA-gesteuerte Chlorid-Kanal-Antagonisten, wie beispielsweise
   Cyclodien-organochlorine, z.B. Chlordane und Endosulfan; oder
   Phenylpyrazole (Fiprole), z.B. Ethiprole und Fipronil.
(3) Natrium-Kanal-Modulatoren / Spannungsabhängige Natrium-Kanal-Blocker, wie beispielsweise
   Pyrethroide, z.B. Acrinathrin, Allethrin, d-cis-trans Allethrin, d-trans Allethrin, Bifenthrin, Bioallethrin, Bioallethrin S-cyclopentenyl Isomer, Bioresmethrin, Cycloprothrin, Cyfluthrin, beta-Cyfluthrin, Cyhalothrin, lambda-Cyhalothrin, gamma-Cyhalothrin, Cypermethrin, alpha-Cypermethrin, beta-Cypermethrin, theta-Cypamethrin, zeta-Cypermethrin, Cyphenothrin [(1R)-trans-Isomere], Deltamethrin, Empenthrin [(EZ)-(1R)-Isomere), Esfenvalerate, Etofenprox, Fenpropathrin, Fenvalerate, Flucythrinate, Flumethrin, tau-Fluvalinate, Halfenprox, Imiprothrin, Kadethrin, Permethrin, Phenothrin [(1R)-trans-Isomer), Prallethrin, Pyrethrine (pyrethrum), Resmethrin, Silafluofen, Tefluthrin, Tetramethrin, Tetramethrin [(1R)- Isomere)], Tralomethrin und Transfluthrin; oder
   DDT; oder Methoxychlor.
(4) Nikotinerge Acetylcholin-Rezeptor (nAChR) Agonisten, wie beispielsweise
   Neonikotinoide, z.B. Acetamiprid, Clothianidin, Dinotefuran, Imidacloprid, Nitenpyram, Thiacloprid und Thiamethoxam; oder
   Nikotin; oder
   Sulfoxaflor.
(5) Nikotinerge Acetylcholin-Rezeptor (nAChR) allosterische Aktivatoren, wie beispielsweise
   Spinosine, z.B. Spinetoram und Spinosad.
(6) Chlorid-Kanal-Aktivatoren, wie beispielsweise
   Avermectine/Milbemycine, z.B. Abamectin, Emamectin-benzoat, Lepimectin und Milbemectin.
(7) Juvenilhormon-Imitatoren, wie beispielsweise
   Juvenilhormon-Analoge, z.B. Hydroprene, Kinoprene und Methoprene; oder
   Fenoxycarb; oder Pyriproxyfen.
(8) Wirkstoffe mit unbekannten oder nicht spezifischen Wirkmechanismen, wie beispielsweise Alkylhalide, z.B. Methylbromid und andere Alkylhalide; oder
   Chloropicrin; oder Sulfurylfluorid; oder Borax; oder Brechweinstein.
(9) Selektive Fraßhemmer, z.B. Pymetrozine; oder Flonicamid.
(10) Milbenwachstumsinhibitoren, z.B. Clofentezine, Hexythiazox und Diflovidazin; oder
   Etoxazole.
(11) Mikrobielle Disruptoren der Insektendarmmembran, z.B. *Bacillus thuringiensis* Subspezies *israelensis, Bacillus thuringiensis* Subspezies *aizawai, Bacillus thuringiensis* Subspezies *kurstaki, Bacillus thuringiensis* Subspezies *tenebrionis* und *B.t.* Pflanzenproteine: Cry1Ab, Cry1Ac, Cry1Fa, Cry1A.105, Cry2Ab, Vip3A, mCry3A, Cry3Ab, Cry3Bb, Cry34 Ab1/35Ab1; oder
   *Bacillus sphaericus.*
(12) Inhibitoren der oxidativen Phosphorylierung, ATP-Disruptoren, wie beispielsweise Diafenthiuron; oder
   Organozinnverbindungen, z.B. Azocyclotin, Cyhexatin und Fenbutatin-oxid; oder
   Propargite; oder Tetradifon.
(13) Entkoppler der oxidativen Phoshorylierung durch Unterbrechung des H-Protongradienten, wie beispielsweise Chlorfenapyr, DNOC und Sulfluramid.
(14) Nikotinerge Acetylcholin-Rezeptor-Antagonisten, wie beispielsweise Bensultap, Cartap-hydrochlorid, Thiocyclam und Thiosultap-Natrium.
(15) Inhibitoren der Chitinbiosynthese, Typ 0, wie beispielsweise Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.
(16) Inhibitoren der Chitinbiosynthese, Typ 1, wie beispielsweise Buprofezin.
(17) Häutungsstörende Wirkstoffe, Dipteran, wie beispielsweise Cyromazine.
(18) Ecdyson-Rezeptor Agonisten, wie beispielsweise Chromafenozide, Halofenozide, Methoxyfenozide und Tebufenozide.
(19) Oktopaminerge Agonisten, wie beispielsweise Amitraz.
(20) Komplex-III-Elektronentransportinhibitoren, wie beispielsweise Hydramethylnon; oder Acequinocyl; oder Fluacrypyrim.
(21) Komplex-I-Elektronentransportinhibitoren, beispielsweise
   METI-Akarizide, z.B. Fenazaquin, Fenpyroximate, Pyrimidifen, Pyridaben, Tebufenpyrad und Tolfenpyrad; oder
   Rotenone (Derris).
(22) Spannungsabhängige Natriumkanal-Blocker, z.B. Indoxacarb; oder Metaflumizone.
(23) Inhibitoren der Acetyl-CoA-Carboxylase, wie beispielsweise
   Tetron- und Tetramsäurederivate, z.B. Spirodiclofen, Spiromesifen und Spirotetramat.
(24) Komplex-IV-Elektronentransportinhibitoren, wie beispielsweise
   Phosphine, z.B. Aluminiumphosphid, Calciumphosphid, Phosphin und Zinkphosphid; oder
   Cyanid.
(25) Komplex-II-Elektronentransportinhibitoren, wie beispielsweise Cyenopyrafen und Cyflumetofen.
(28) Ryanodinrezeptor-Effektoren, wie beispielsweise
   Diamide, z.B. Chlorantraniliprole, Cyantraniliprole und Flubendiamide.

Weitere Wirkstoffe mit unbekanntem Wirkmechanismus, wie beispielsweise Amidoflumet, Azadirachtin, Benclothiaz, Benzoximate, Bifenazate, Bromopropylate, Chinomethionat, Cryolite, Dicofol, Diflovidazin, Fluensulfone, Flufenerim, Flufiprole, Fluopyram, Fufenozide, Imidaclothiz, Iprodione, Meperfluthrin, Pyridalyl, Pyrifluquinazon, Tetramethylfluthrin und Iodmethan; desweiteren Präparate auf Basis von Bacillus firmus (insbesondere Stamm CNCM I-1582, beispielsweise VOTiVO™, BioNem) sowie folgende bekannte wirksame Verbindungen:
3-Brom-N-{2-brom-4-chlor-6-[(1-cyclopropylethyl)carbamoyl]phenyl}-1-(3-chlorpyridin-2-yl)-1H-pyrazol-5-carboxamid (bekannt aus WO2005/077934), 4-{[(6-Brompyrid-3-yl)methyl](2-fluorethyl)amino}furan-2(5H)-on (bekannt aus WO2007/115644), 4-{[(6-Fluorpyrid-3-yl)methyl](2,2-difluorethyl)amino}furan-2(5H)-on (bekannt aus WO2007/115644), 4-{[(2-Chlor-1,3-thiazol-5-yl)methyl](2-fluorethyl)amino}furan-2(5H)-on (bekannt aus WO2007/115644), 4-{[(6-Chlorpyrid-3-yl)methyl](2-fluorethyl)amino}firan-2(5H)-on (bekannt aus WO2007/115644) Flupyradifurone, 4-{[(6-Chlor-5-fluorpyrid-3-yl)methyl](methyl)amino}furan-2(5H)-on (bekannt aus WO2007/115643), 4-{[(5,6-Dichlorpyrid-3-yl)methyl](2-fluorethyl)amino}furan-2(5H)-on (bekannt aus WO2007/115646), 4-{[(6-Chlor-5-fluorpyrid-3-yl)methyl](cyclopropyl)amino}furan-2(5H)-on (bekannt aus WO2007/115643), 4-{[(6-Chlorpyrid-3-yl)methyl](cyclopropyl)amino}furan-2(5H)-on (bekannt aus EP-A-0 539 588), 4-{[(6-Chlorpyrid-3-yl)melhyl](methyl)amino}furan-2(5H)-on (bekannt aus EP-A-0 539 588), {[1-(6-Chlorpyridin-3-yl)ethyl](methyl)oxido-λ⁴-sulfanyliden}cyanamid (bekannt aus WO2007/149134) und seine Diastereomere {[(1R)-1-(6-Chlorpyridin-3-yl)ethyl](methyl)oxido-λ⁴-sulfanyliden}cyanamid (A) und {[(1S)-1-(6-Chlorpyridin-3-yl)ethyl](methyl)oxido-λ⁴-sulfanyliden}cyanamid (B) (ebenfalls bekannt aus WO2007/149134) sowie Diastereomere [(R)-Methyl(oxido){(1R)-1-[6-(trifluormethyl)pyridin-3-yl]ethyl}-λ⁴-sulfanyliden]cyanamid (A1) und [(S)-Methyl(oxido){(1S)-1-[6-(trifluormethyl)pyridin-3-yl]ethyl}-λ⁴-sulfanyliden]cyanamid (A2), bezeichnet als Diastereomerengruppe A (bekannt aus WO 2010/074747, WO 2010/074751), [(R)-Methyl(oxido){(1S)-1-[6-(trifluormethyl)pyridin-3-yl]ethyl}-λ⁴-sulfanyliden]cyanamid (B1) und [(S)-Methyl(oxido) {(1R)-1-[6-(trifluormethyl)pyridin-3-yl]ethyl}-λ⁴-sulfanyliden]cyanamid (B2), bezeichnet als Diastereomerengruppe B (ebenfalls bekannt aus WO 2010/074747, WO 2010/074751) und 11-(4-Chlor-2,6-dimethylphenyl)-12-hydroxy-1,4-dioxa-9-azadispiro[4.2.4.2]tetradec-11-en-10-on (bekannt aus WO2006/089633), 3-(4'-Fluor-2,4-dimethylbiphenyl-3-yl)-4-hydroxy-8-oxa-1-azaspiro[4.5]dec-3-en-2-on (bekannt aus WO2008/067911), 1-{2-Fluor-4-methyl-5-[(2,2,2-trifluorethyl)sulfinyl]phenyl}-3-(trifluormethyl)-1H-1,2,4-triazol-5-amin (bekannt aus WO2006/043635), Afidopyropen (bekannt aus WO2008/066153), 2-Cyan-3-(difluormethoxy)-N,N-dimethylbenzolsulfonamid (bekannt ausWO2006/056433), 2-Cyan-3-(difluormethoxy)-N-methylbenzolsulfonamid (bekannt aus WO2006/100288), 2-Cyan-3-(difluormethoxy)-N-ethylbenzolsulfonamid (bekannt aus WO2005/035486), 4-(Difluormethoxy)-N-ethyl-N-methyl-1,2-benzothiazol-3-amin-1,1-dioxid (bekannt aus WO2007/057407), N-[1-(2,3-Dimethylphenyl)-2-(3,5-dimethylphenyl)ethyl]-4,5-dihydro-1,3-thiazol-2-amin (bekannt aus WO2008/104503), {1'-[(2E)-3-(4-Chlorphenyl)prop-2-en-1-yl]-5-fluorspiro[indol-3,4'-piperidin]-1(2H)-yl}(2-chlorpyridin-4-yl)methanon (bekannt aus WO2003/106457), 3-(2,5-Dimethylphenyl)-4-hydroxy-8-methoxy-1,8-diazaspiro[4.5]dec-3-en-2-on (bekannt aus WO2009/049851), 3-(2,5-Dimethylphenyl)-8-methoxy-2-oxo-1,8-diazaspiro[4.5]dec-3-en-4-yl-ethylcarbonat (bekannt aus WO2009/049851), 4-(But-2-in-1-yloxy)-6-(3,5-dimethylpiperidin-1-yl)-5-fluorpyrimidin (bekannt aus WO2004/099160), (2,2,3,3,4,4,5,5-Octafluorpentyl)(3,3,3-trifluorpropyl)malononitril (bekannt aus WO2005/063094), (2,2,3,3,4,4,5,5-Octafluorpentyl)(3,3,4,4,4-pentafluorbutyl)malononitril (bekannt aus WO2005/063094), 8-[2-(Cyclopropylmcthoxy)-4-(trifluormelhyl)phenoxy]-3-[6-(trifluormethyl)pyridazin-3-yl]-3-azabicyclo[3.2.1]octan (bekannt aus WO2007/040280) Flometoquin, PF1364 (CAS-Reg.Nr. 1204776-60-2) (bekannt aus JP2010/018586), 5-[5-(3,5-Dichlorphenyl)-5-(trifluormethyl)-4,5-dihydro-1,2-oxazol-3-yl]-2-(1H-1,2,4-triazol-1-yl)benzonitril (bekannt aus WO2007/075459), 5-[5-(2-Chlorpyridin-4-yl)-5-(trifluormethyl)-4,5-dihydro-1,2-oxazol-3 -yl]-2-(1H-1,2,4-triazol-1-yl)benzonitril (bekannt aus WO2007/075459), 4-[5-(3,5-Dichlorphenyl)-5-(trifluormethyl)-4,5-dihydro-1,2-oxazol-3-yl]-2-methyl-N-{2-oxo-2-[(2,2,2-trifluorethyl)amiuo]ethyl}benzamid (bekannt aus WO2005/085216), 4-{[(6-Chlorpyridin-3-yl)methyl](cyclopropyl)amino}-1,3-oxazol-2(5H)-on, 4-{[(6-Chlorpyridin-3-yl)methyl](2,2-difluorethyl)amino}-1,3-oxazol-2(5H)-on, 4-{[(6-Chlorpyridin-3-yl)methyl](ethyl)amino}-1,3-oxazol-2(5H)-on, 4-{[(6-Chlorpyridin-3-yl)methyl](methyl)amino}-1,3-oxazol-2(5H)-on (alle bekannt aus WO2010/005692), Pyflubumide (bekannt aus WO2002/096882), Methyl-2-[2-({[3-brom-1-(3-chlorpyridin-2-yl)-1H-pyrazol-5-yl] carbonyl} amino)-5-chlor-3-methylbenzoyl]-2-methylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-ethylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-methylhydrazincarboxylat (bekannt aus WO2005/085216), Methyl-2-[3,5-dibrom-2-({[3-brom-1-(3-chlorpyridin-2-yl)-1H-pyrazol-5-yl]carbonyl}amino)benzoyl]-1,2-diethylhydrazincarboxylat (bekannt aus WO2005/085216), Methyl-2-[3,5-dibrom-2-({[3-brom-1-(3-chlorpyridin-2-yl)-1H-pyrazol-5-yl]carbonyl}amino)benzoyl]-2-ethylhydrazincarboxylat (bekannt aus WO2005/085216), (5RS,7RS;5RS,7SR)-1-(6-Chlor-3-pyridylmethyl)-1,2,3,5,6,7-hexahydro-7-methyl-8-nitro-5-propoxyimidazo[1,2-a]pyridin (bekannt aus WO2007/101369), 2-{6-[2-(5-Fluorpyridin-3-yl)-1,3-thiazol-5-yl]pyridin-2-yl}pyrimidin (bekannt aus WO2010/006713), 2-{6-[2-(Pyridin-3-yl)-1,3-thiazol-5-yl]pyridin-2-yl}pyrimidin (bekannt aus WO2010/006713), 1-(3-Chlorpyridin-2-yl)-N-[4-cyan-2-methyl-6-(methylcarbamoyl)phenyl]-3-{[5-(trifluormethyl)-1H-tetrazol-1-yl]methyl}-1H-pyrazol-5-carboxamid (bekannt aus WO2010/069502), 1-(3-Chlorpyridin-2-yl)-N-[4-cyan-2-methyl-6-(methylcarbamoyl)phenyl]-3-{[5-(trifluormethyl)-2H-tetrazol-2-yl]methyl}-1H-pyrazol-5-carboxamid (bekannt aus WO2010/069502), N-[2-(tert-Butylcarbamoyl)-4-cyan-6-methylphenyl]-1-(3-chlorpyridin-2-yl)-3-{[5-(trifluormethyl)-1H-tetrazol-1-yl]methyl}-1H-pyrazol-5-carboxamid (bekannt aus WO2010/069502), N-[2-(tert-Butylcarbamoyl)-4-cyan-6-methylphenyl]-1-(3-chlorpyridin-2-yl)-3-{[5-(trifluormethyl)-2H-tetrazol-2-yl]methyl}-1H-pyrazol-5-carboxamid (bekannt aus WO2010/069502), (1E)-N-[(6-Chlorpyridin-3-yl)methyl]-N'-cyan-N-(2,2-difluorethyl)ethanimidamid (bekannt aus WO2008/009360), N-[2-(5-Amino-1,3,4-thiadiazol-2-yl)-4-chlor-6-methylphenyl]-3-brom-1-(3-chlorpyridin-2-yl)-1H-pyrazol-5-carboxamid (bekannt aus CN102057925), Methyl-2-[3,5-dibrom-2-({[3-brom-1-(3-chlorpyridin-2-yl)-1H-pyrazol-5-yl]carbonyl}amino)benzoyl]-2-ethyl-1-methylhydrazincarboxylat (bekannt aus WO2011/049233), Heptafluthrin, Pyriminostrobin, Flufenoxystrobin und 3-Chlor-N²-(2-cyanpropan-2-yl)-N¹-[4-(1,1,1,2,3,3,3-heptafluorpropan-2-yl)-2-methylphenyl]phthalamid (bekannt aus WO2012/034472).

Antimikrobiell wirkende Verbindungen:
(1) Inhibitoren der Ergosterol-Biosynthese, wie beispielsweise Aldimorph, Azaconazol, Bitertanol, Bromuconazol, Cyproconazol, Diclobutrazol, Difenoconazol, Diniconazol, Diniconazol-M, Dodemorph, Dodemorph Acetat, Epoxiconazol, Etaconazol, Fenarimol, Fenbuconazol, Fenhexamid, Fenpropidin, Fenpropimorph, Fluquinconazol, Flurprimidol, Flusilazol, Flutriafol, Furconazol, Furconazol-Cis, Hexaconazol, Imazalil, Imazalil Sulfat, Imibenconazol, Ipconazol, Metconazol, Myclobutanil, Naftifin, Nuarimol, Oxpoconazol, Paclobutrazol, Pefurazoat, Penconazol, Piperalin, Prochloraz, Propiconazol, Prothioconazol, Pyributicarb, Pyrifenox, Quinconazol, Simeconazol, Spiroxamin, Tebuconazol, Terbinafin, Tetraconazol, Triadimefon, Triadimenol, Tridemorph, Triflumizol, Triforin, Triticonazol, Uniconazol, Uniconazol-p, Viniconazol, Voriconazol, 1-(4-Chlorphenyl)-2-(1H-1,2,4-triazol-1-yl)cycloheptanol, Methyl-1-(2,2-dimethyl-2,3 -dihydro-1H-inden-1-yl)-1H-imidazol-5-carboxylat, N'- {5-(Difluormethyl)-2-methyl-4-[3-(trimethylsilyl)propoxy]phenyl}-N-ethyl-N-methylimidoformamid, N-Ethyl-N-methyl-N'-{2-methyl-5-(trifluormethyl)-4-[3-(trimethylsilyl)propoxy]phenyl}imidoformamid und O-[1-(4-Methoxyphenoxy)-3,3-dimethylbutan-2-yl]-1H-imidazol-1-carbothioat.
(2) Inhibitoren der Respiration (Atmungsketten-Inhibitoren), wie beispielsweise Bixafen, Boscalid, Carboxin, Diflumetorim, Fenfuram, Fluopyram, Flutolanil, Fluxapyroxad, Furametpyr, Furmecyclox, Isopyrazam Mischung des syn-epimeren Razemates 1RS,4SR,9RS und des anti-empimeren Razemates 1RS,4SR,9SR, Isopyrazam (anti-epimeres Razemat), Isopyrazam (anti-epimeres Enantiomer 1R,4S,9S), Isopyrazam (anti-epimeres Enantiomer 1S,4R,9R), Isopyrazam (syn-epimeres Razemat 1RS,4SR,9RS), Isopyrazam (syn-epimeres Enantiomer 1R,4S,9R), Isopyrazam (syn-epimeres Enantiomer 1S,4R,9S), Mepronil, Oxycarboxin, Penflufen, Penthiopyrad, Sedaxane, Thifluzamid, 1-Methyl-N-[2-(1,1,2,2-tetrafluorethoxy)phenyl]-3-(trifluormethyl)-1H-pyrazol-4-carboxamid, 3-(Difluormethyl)-1 -methyl-N-[2-(1,1,2,2-tetrafluorethoxy)phenyl]-1H-pyrazol-4-carboxamid, 3-(Difluormethyl)-N-[4-fluor-2-(1,1,2,3,3,3-hexafluorpropoxy)phenyl]-1-methyl-1H-pyrazol-4-carboxamid und N-[1-(2,4-Dichlorphenyl)-1-methoxypropan-2-yl]-3-(difluormethyl)-1-methyl-1H-pyrazol-4-carboxamid.
(3) Inhibitoren der Respiration (Atmungsketten-Inhibitoren) am Komplex III der Atumungskette, wie beispielsweise Ametoctradin, Amisulbrom, Azoxystrobin, Cyazofamid, Dimoxystrobin, Enestroburin, Famoxadon, Fenamidon, Fluoxastrobin, Kresoxim-Methyl, Metominostrobin, Orysastrobin, Picoxystrobin, Pyraclostrobin, Pyrametostrobin, Pyraoxystrobin, Pyribencarb, Trifloxystrobin, (2E)-2-(2-{[6-(3-Chlor-2-methylphenoxy)-5-fluorpyrimidin-4-yl]oxy}phenyl)-2-(methoxyimino)-N-methyl-ethanamid, (2E)-2-(Methoxyimino)-N-methyl-2-(2-{[({(1E)-1-[3-(trifluormethyl)phenyl]ethyliden}amino)oxy]methyl}phenyl)ethanamid, (2E)-2-(Methoxyimino)-N-methyl-2-{2-[(E)-({1-[3-(trifluormethyl)phenyl]ethoxy}imino)methyl]phenyl}ethanamid, (2E)-2-{2-[({[(1E)-1-(3-{[(E)-1-Fluor-2-phenylethenyl]oxy}phenyl)ethyliden]amino}oxy)methyl]phenyl}-2-(methoxyimino)-N-methylethan-amid, (2E)-2-{2-[({[(2E,3E)-4-(2,6-Dichlorphenyl)but-3-en-2-yliden]amino}oxy)methyl]phenyl}-2-(methoxyimino)-N-methylethanamid, 2-Chlor-N-(1,1,3-trimethyl-2,3-dihydro-1H-inden-4-yl)pyridin-3-carboxamid, 5-Methoxy-2-methyl-4-(2-{[({(1E)-1-[3-(trifluormethyl)phenyl]ethyl-iden}amino)oxy]methyl}phenyl)-2,4-dihydro-3H-1,2,4-triazol-3-on, Methyl-(2E)-2-12-[({cyclopropyl[(4-methoxyphenyl)imino]methyl}sulfanyl)methyl]phenyl}-3-methoxyprop-2-enoat, N-(3-Ethyl-3,5,5-trimethylcyclohexyl)-3-(formylamino)-2-hydroxybenzamid, 2-{2-[(2,5-Dimethylphenoxy)methyl]phenyl} -2-methoxy-N-methylacetamid und (2R)-2-{2-[(2,5-Dimethylphenoxy)methyl]phenyl}-2-methoxy-N-methylacetamid.
(4) Inhibitoren der Mitose und Zellteilung, wie beispielsweise Benomyl, Carbendazim, Chlorfenazol, Diethofencarb, Ethaboxam, Fluopicolid, Fuberidazol, Pencycuron, Thiabendazol, Thiophanat-Methyl, Thiophanat, Zoxamid, 5-Chlor-7-(4-methylpiperidin-1-yl)-6-(2,4,6-trifluorphenyl)[1,2,4]triazolo[1,5-a]pyrimidin und 3-Chlor-5-(6-chlorpyridin-3-yl)-6-methyl-4-(2,4,6-trifluorphenyl)pyridazin.
(5) Verbindungen mit Multisite-Aktivität, wie beispielsweise Bordeauxmischung, Captafol, Captan, Chlorothalonil, Kupferzubereitungen wie Kupferhydroxid, Kupfernaphthenat, Kupferoxid, Kupferoxychlorid, Kupfersulfat, Dichlofluanid, Dithianon, Dodine, Dodine freie Base, Ferbam, Fluorofolpet, Folpet, Guazatin, Guazatinacetat, Iminoctadin, Iminoctadinalbesilat, Iminoctadintriacetat, Mankupfer, Mancozeb, Maneb, Metiram, Zinkmetiram, Kupfer-Oxin, Propamidin, Propineb, Schwefel und Schwefelzubereitungen wie beispielsweise Calciumpolysulfid, Thiram, Tolylfluanid, Zineb und Ziram.
(6) Resistenzinduktoren, wie beispielsweise Acibenzolar-S-Methyl, Isotianil, Probenazol und Tiadinil.
(7) Inhibitoren der Aminosäure- und Protein-Biosynthese, wie beispielsweise Andoprim, Blasticidin-S, Cyprodinil, Kasugamycin, Kasugamycin Hydrochlorid Hydrat, Mepanipyrim und Pyrimethanil.
(8) Inhibitoren der ATP Produktion, wie beispielsweise Fentin Acetat, Fentin Chlorid, Fentin Hydroxid und Silthiofam.
(9) Inhibitoren der Zellwandsynthese, wie beispielsweise Benthiavalicarb, Dimethomorph, Flumorph, Iprovalicarb, Mandipropamid, Polyoxins, Polyoxorim, Validamycin A und Valifenalat.
(10) Inhibitoren der Lipid- und Membran-Synthese, wie beispielsweise Biphenyl, Chloroneb, Dicloran, Edifenphos, Etridiazol, Iodocarb, Iprobenfos, Isoprothiolan, Propamocarb, Propamocarb Hydrochlorid, Prothiocarb, Pyrazophos, Quintozen, Tecnazene und Tolclofos-Methyl.
(11) Inhibitoren der Melanin-Biosynthese, wie beispielsweise Carpropamid, Diclocymet, Fenoxanil, Fthalid, Pyroquilon und Tricyclazol.
(12) Inhibitoren der Nukleinsäuresynthese, wie beispielsweise Benalaxyl, Benalaxyl-M (Kiralaxyl), Bupirimat, Clozylacon, Dimethirimol, Ethirimol, Furalaxyl, Hymexazol, Metalaxyl, Metalaxyl-M (Mefenoxam), Ofurace, Oxadixyl, Oxolinsäure.
(13) Inhibitoren der Signaltransduktion, wie beispielsweise Chlozolinat, Fenpiclonil, Fludioxonil, Iprodion, Procymidon, Quinoxyfen und Vinclozolin.
(14) Entkoppler, wie beispielsweise Binapacryl, Dinocap, Ferimzon, Fluazinam und Meptyldinocap.
(15) Weitere Verbindungen, wie beispielsweise Benthiazol, Bethoxazin, Capsimycin, Carvon, Chinomethionat, Chlazafenon, Cufraneb, Cyflufenamid, Cymoxanil, Cyprosulfamide, Dazomet, Debacarb, Dichlorophen, Diclomezin, Difenzoquat, Difenzoquat Methylsulphat, Diphenylamin, Ecomat, Fenpyrazamin, Flumetover, Fluoromid, Flusulfamid, Flutianil, Fosetyl-Aluminium, Fosetyl-Calcium, Fosetyl-Natrium, Hexachlorbenzol, Irumamycin, Methasulfocarb, Methylisothiocyanat, Metrafenon, Mildiomycin, Natamycin, Nickel Dimethyldithiocarbamat, Nitrothal-Isopropyl, Octhilinone, Oxamocarb, Oxyfenthiin, Pentachlorphenol und dessen Salze, Phenothrin, Phosphorsäure und deren Salze, Propamocarb-Fosetylat, Propanosin-Natrium, Proquinazid, Pyrrolnitrin, Tebufloquin, Tecloftalam, Tolnifanid, Triazoxid, Trichlamid, Zarilamid, 1-(4-{4-[(5R)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, 1-(4-{4-[(5S)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, 1-(4-{4-[5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, 1-(4-Methoxyphenoxy)-3,3-dimethylbutan-2-yl-1H-imidazol-1-carboxylat, 2,3,5,6-Tetrachlor-4-(methylsulfonyl)pyridin, 2,3-Dibutyl-6-chlorthieno[2,3-d]pyrimidin-4(3H)-on, 2-[5-Methyl-3-(trifluormethyl)-1H-pyrazol-1-yl]-1-(4-{4-[(5R)-5-phenyl-4,5-dihydro-1,2-oxazol-3-yl]-1,3-thiazol-2-yl}piperidin-1-yl)ethanon, 2-[5-Methyl-3-(trifluormethyl)-1H-pyrazol-1-yl]-1-(4-{4-[(5S)-5-phenyl-4,5-dihydro-1,2-oxazol-3-yl]-1,3-thiazol-2-yl}piperidin-1-yl)ethanon, 2-[5-Methyl-3-(trifluormethyl)-1H-pyrazol-1-yl]-1-{4-[4-(5-phenyl-4,5-dihydro-1,2-oxazol-3-yl)-1,3-thiazol-2-yl]piperidin-1-yl}ethanon, 2-Butoxy-6-iod-3-propyl-4H-chromen-4-on, 2-Chlor-5-[2-chlor-1-(2,6-difluor-4-methoxyphenyl)-4-methyl-1-imidazol-5-yl]pyridin, 2-Phenylphenol und dessen Salze, 3,4,5-Trichlorpyridin-2,6-dicarbonitril, 3-[5-(4-Chlorphenyl)-2,3-dimethyl-1,2-oxazolidin-3-yl]pyridin, 3-Chlor-5-(4-chlorphenyl)-4-(2,6-difluorphenyl)-6-methylpyridazin, 4-(4-Chlorphenyl)-5-(2,6-difluorphenyl)-3,6-dimethylpyridazin, 5-Amino-1,3,4-thiadiazol-2-thiol, 5-Chlor-N'-phenyl-N'-(prop-2-in-1-yl)thiophen-2-sulfonohydrazid, 5-Methyl-6-octyl[1,2,4]triazolo[1,5-a]pyrimidin-7-amin, Ethyl-(2Z)-3-amino-2-cyan-3-phenylprop-2-enoat, N-(4-Chlorbenzyl)-3-[3-methoxy-4-(prop-2-in-1-yloxy)phenyl]propanamid, N-[(4-Chlorphenyl)(cyan)methyl]-3-[3-methoxy-4-(prop-2-in-1-yloxy)phenyl]propanamid, N-[(5-Brom-3-chlorpyridin-2-yl)methyl]-2,4-dichlorpyridin-3-carboxamid, N-[1-(5-Brom-3-chlorpyridin-2-yl)ethyl]-2,4-dichlorpyridin-3-carboxamid, N-[1-(5-Brom-3-chlorpyridin-2-yl)ethyl]-2-fluor-4-iodpyridin-3-carboxamid, N-{(E)-[(Cyclopropylmethoxy)imino][6-(difluormethoxy)-2,3-difluorphenyl]methyl}-2-phenylacetamid, N-{(Z)-[(Cyclopropylmethoxy)imino][6-(difluormethoxy)-2,3-difluorphenyl]methyl}-2-phenylacetamid, N-Methyl-2-(1-{[5-methyl-3-(trifluormethyl)-1H-pyrazol-1-yl]acetyl}piperidin-4-yl)-N-(1,2,3,4-tetrahydronaphthalen-1-yl)-1,3-thiazol-4-carboxamid, N-Methyl-2-(1-{[5-methyl-3-(trifluormethyl)-1H-pyrazol-1-yl]acetyl}piperidin-4-yl)-N-[(1R)-1,2,3,4-tetrahydronaphthalen-1-yl]-1,3-thiazol-4-carboxamid, N-Methyl-2-(1-{[5-methyl-3-(trifluormethyl)-1H-pyrazol-1-yl]acetyl}piperidin-4-yl)-N-[(1S)-1,2,3,4-tetrahydronaphthalen-1-yl]-1,3-thiazol-4-carboxamid, Pentyl-{6-[({[(1-methyl-1H-tetrazol-5-yl)(phenyl)methyliden]amino}oxy)methyl]pyridin-2-yl}carbamat, Phenazin-1-carbonsäure, Chinolin-8-ol und Chinolin-8-olsulfat(2:1).
(16) weitere antimikrobiell wirksame Verbindungen: (15.1) benthiazole, (15.2) bethoxazin, (15.3) capsimycin, (15.4) carvone, (15.5) chinomethionat, (15.6) pyriofenone (chlazafenone), (15.7) cufraneb, (15.8) cyflufenamid, (15.9) cymoxanil, (15.10) cyprosulfamide, (15.11) dazomet, (15.12) debacarb, (15.13) dichlorophen, (15.14) diclomezine, (15.15) difenzoquat, (15.16) difenzoquat metilsulfate, (15.17) diphenylamine, (15.18) ecomate, (15.19) fenpyrazamine, (15.20) flumetover, (15.21) fluoroimide, (15.22) flusulfamide, (15.23) flutianil, (15.24) fosetyl-aluminium, (15.25) fosetyl-calcium, (15.26) fosetyl-sodium, (15.27) hexachlorobenzene, (15.28) irumamycin, (15.29) methasulfocarb, (15.30) methyl isothiocyanate, (15.31) metrafenone, (15.32) mildiomycin, (15.33) natamycin, (15.34) nickel dimethyldithiocarbamate, (15.35) nitrothal-isopropyl, (15.37) oxamocarb, (15.38) oxyfenthiin, (15.39) pentachlorophenol and salts, (15.40) phenothrin, (15.41) phosphorous acid and its salts, (15.42) propamocarb-fosetylate, (15.43) propanosine-sodium, (15.44) pyrimorph, (15.45) (2E)-3-(4-tert-butylphenyl)-3-(2-chloropyridin-4-yl)-1-(morpholin-4-yl)prop-2-en-1-one, (15.46) (2Z)-3-(4-tert-butylphenyl)-3-(2-chloropyridin-4-yl)-1-(morpholin-4-yl)prop-2-en-1-one, (15.47) pyrrolnitrine, (15.48) tebufloquin, (15.49) tecloftalam, (15.50) tolnifanide, (15.51) triazoxide, (15.52) trichlamide, (15.53) zarilamid, (15.54) (3S,6S,7R,8R)-8-benzyl-3-[({3-[(isobutyryloxy)methoxy]-4-methoxypyridin-2-yl}carbonyl)amino]-6-methyl-4,9-dioxo-1,5-dioxonan-7-yl 2-methylpropanoate, (15.55) 1-(4-{4-[(5R)-5-(2,6-difluorophenyl)-4,5-dihydro-1,2-oxazol-3-yl]-1,3-thiazol-2-yl}piperidin-1-yl)-2-[5-methyl-3-(trifluoromethyl)-1H-pyrazol-1-yl]ethanone, (15.56) 1-(4-{4-[(5S)-5-(2,6-difluorophenyl)-4,5-dihydro-1,2-oxazol-3-yl]-1,3-thiazol-2-yl}piperidin-1-yl)-2-[5-methyl-3-(trifluoromethyl)-1H-pyrazol-1-yl]ethanone, (15.57) 1-(4-4-[5-(2,6-difluorophenyl)-4,5-dihydro-1,2-oxazol-3-yl]-1,3-thiazol-2-yl}piperidin-1-yl)-2-[5-methyl-3-(trifluoromethyl)-1H-pyrazol-1-yl]ethanone, (15.58) 1-(4-methoxyphenoxy)-3,3-dimethylbutan-2-yl 1H-imidazole-1-carboxylate, (15.59) 2,3,5,6-tetrachloro-4-(methylsulfonyl)pyridine, (15.60) 2,3-dibutyl-6-chlorothieno[2,3-d]pyrimidin-4(3H)-one, (15.61) 2,6-dimethyl-1H,5H-[1,4]dithiino[2,3-c:5,6-c']dipyrrole-1,3,5,7(2H,6H)-tetrone, (15.62) 2-[5-methyl-3-(trifluoromethyl)-1H-pyrazol-1-yl]-1-(4-{4-[(5R)-5-phenyl-4,5-dihydro-1,2-oxazol-3-yl]-1,3-thiazol-2-yl}piperidin-1-yl)ethanone, (15.63) 2-[5-methyl-3-(trifluoromethyl)-1H-pyrazol-1-yl]-1-(4-{4-[(5S)-5-phenyl-4,5-dihydro-1,2-oxazol-3-yl]-1,3-thiazol-2-yl}piperidin-1-yl)ethanone, (15.64) 2-[5-methyl-3-(trifluoromethyl)-1H-pyrazol-1-yl]-1-{4-[4-(5-phenyl-4,5-dihydro-1,2-oxazol-3-yl)-1,3-thiazol-2-yl]piperidin-1-yl}ethanone, (15.65) 2-butoxy-6-iodo-3-propyl-4H-chromen-4-one, (15.66) 2-chloro-5-[2-chloro-1-(2,6-difluoro-4-methoxyphenyl)-4-methyl-1H-imidazol-5-yl]pyridine, (15.67) 2-phenylphenol and salts, (15.68) 3-(4,4,5-trifluoro-3,3-dimethyl-3,4-dihydroisoquinolin-1-yl)quinoline, (15.69) 3,4,5-trichloropyridine-2,6-dicarbonitrile, (15.70) 3-chloro-5-(4-chlorophenyl)-4-(2,6-difluorophenyl)-6-methylpyridazine, (15.71) 4-(4-chlorophenyl)-5-(2,6-difluorophenyl)-3,6-dimethylpyridazine, (15.72) 5-amino-1,3,4-thiadiazole-2-thiol, (15.73) 5-chloro-N'-phenyl-N'-(prop-2-yn-1-yl)thiophene-2-sulfonohydrazide, (15.74) 5-fluoro-2-[(4-fluorobenzyl)oxy]pyrimidin-4-amine, (15.75) 5-fluoro-2-[(4-methylbenzyl)oxy]pyrimidin-4-amine, (15.76) 5-methyl-6-octyl[1,2,4]triazolo[1,5-a]pyrimidin-7-amine, (15.77) ethyl (2Z)-3-amino-2-cyano-3-phenylacrylate, (15.78) N'-(4-{[3-(4-chlorobenzyl)-1,2,4-thiadiazol-5-yl]oxy}-2,5-dimethylphenyl)-N-ethyl-N-methylimidoformamide, (15.79) N-(4-chlorobenzyl)-3-[3-methoxy-4-(prop-2-yn-1-yloxy)phenyl]propanamide, (15.80) N-[(4-chlorophenyl)(cyano)methyl]-3-[3-methoxy-4-(prop-2-yn-1-yloxy)phenyl]propanamide, (15.81) N-[(5-bromo-3-chloropyridin-2-yl)methyl]-2,4-dichloronicotinamide, (15.82) N-[1-(5-bromo-3-chloropyridin-2-yl)ethyl]-2,4-dichloronicotinamide, (15.83) N-[1-(5-bromo-3-chloropyridin-2-yl)ethyl]-2-fluoro-4-iodonicotinamide, (15.84) N-{(E)-[(cyclopropylmethoxy)imino][6-(difluoromethoxy)-2,3-difluorophenyl]methyl}-2-phenylacetamide, (15.85) N-{(Z)-[(cyclopropylmethoxy)imino][6-(difluoromethoxy)-2,3-difluorophenyl]methyl}-2-phenylacetamide, (15.86) N'-{4-[(3-tert-butyl-4-cyano-1,2-thiazol-5-yl)oxy]-2-chloro-5-methylphenyl}-N-ethyl-N-methylimidoformamide, (15.87) N-methyl-2-(1-{[5-methyl-3-(trifluoromethyl)-1H-pyrazol-1-yl]acetyl}piperidin-4-yl)-N-(1,2,3,4-tetrahydronaphthalen-1-yl)-1,3-thiazole-4-carboxamide, (15.88) N-methyl-2-(1-{[5-methyl-3-(trifluoromethyl)-1H-pyrazol-1-yl]acetyl}piperidin-4-yl)-N-[(1R)-1,2,3,4-tetrahydronaphthalen-1-yl]-1,3-thiazole-4-carboxamide, (15.89) N-methyl-2-(1-{[5-methyl-3-(trifluoromethyl)-1H-pyrazol-1-yl]acetyl}piperidin-4-yl)-N-[(1S)-1,2,3,4-tetrahydronaphthalen-1-yl]-1,3-thiazole-4-carboxamide, (15.90) pentyl {6-[({[(1-methyl-1H-tetrazol-5-yl)(phenyl)methylene]amino}oxy)methyl]pyridin-2-yl}carbamate, (15.91) phenazine-1-carboxylic acid, (15.92) quinolin-8-ol, (15.93) quinolin-8-ol sulfate (2:1), (15.94) tert-butyl {6-[({[(1-methyl-1H-tetrazol-5-yl)(phenyl)methylene]amino}oxy)methyl]pyridin-2-yl}carbamate, (15.95) 1-methyl-3-(trifluoromethyl)-N-[2'-(trifluoromethyl)biphenyl-2-yl]-1H-pyrazole-4-carboxamide, (15.96) N-(4'-chlorobiphenyl-2-yl)-3-(difluoromethyl)-1-methyl-1H-pyrazole-4-carboxamide, (15.97) N-(2',4'-dichlorobiphenyl-2-yl)-3-(difluoromethyl)-1-methyl-1H-pyrazole-4-carboxamide, (15.98) 3-(difluoromethyl)-1-methyl-N-[4'-(trifluoromethyl)biphenyl-2-yl]-1H-pyrazole-4-carboxamide, (15.99) N-(2',5'-difluorobiphenyl-2-yl)-1-methyl-3-(trifluoromethyl)-1H-pyrazole-4-carboxamide, (15.100) 3-(difluoromethyl)-1-methyl-N-[4'-(prop-1-yn-1-yl)biphenyl-2-yl]-1H-pyrazole-4-carboxamide, (15.101) 5-fluoro-1,3-dimethyl-N-[4'-(prop-1-yn-1-yl)biphenyl-2-yl]-1H-pyrazole-4-carboxamide, (15.102) 2-chloro-N-[4'-(prop-1-yn-1-yl)biphenyl-2-yl]nicotinamide, (15.103) 3-(difluoromethyl)-N-[4'-(3,3-dimethylbut-1-yn-1-yl)biphenyl-2-yl]-1-methyl-1H-pyrazole-4-carboxamide, (15.104) N-[4'-(3,3-dimethylbut-1-yn-1-yl)biphenyl-2-yl]-5-fluoro-1,3-dimethyl-1H-pyrazole-4-carboxamide, (15.105) 3-(difluoromethyl)-N-(4'-ethynylbiphenyl-2-yl)-1-methyl-1H-pyrazole-4-carboxamide, (15.106) N-(4'-ethynylbiphenyl-2-yl)-5-fluoro-1,3-dimethyl-1H-pyrazole-4-carboxamide, (15.107) 2-chloro-N-(4'-ethynylbiphenyl-2-yl)nicotinamide, (15.108) 2-chloro-N-[4'-(3,3-dimethylbut-1-yn-1-yl)biphenyl-2-yl]nicotinamide, (15.109) 4-(difluoromethyl)-2-methyl-N-[4'-(trifluoromethyl)biphenyl-2-yl]-1,3-thiazole-5-carboxamide, (15.110) 5-fluoro-N-[4'-(3-hydroxy-3-methylbut-1-yn-1-yl)biphenyl-2-yl]-1,3-dimethyl-1H-pyrazole-4-carboxamide, (15.111) 2-chloro-N-[4'-(3-hydroxy-3-methylbut-1-yn-1-yl)biphenyl-2-yl]nicotinamide, (15.112) 3-(difluoromethyl)-N-[4'-(3-methoxy-3-methylbut-1-yn-1-yl)biphenyl-2-yl]-1-methyl-1H-pyrazole-4-carboxamide, (15.113) 5-fluoro-N-[4'-(3-methoxy-3-methylbut-1-yn-1-yl)biphenyl-2-yl]-1,3-dimethyl-1H-pyrazole-4-carboxamide, (15.114) 2-chloro-N-[4'-(3-methoxy-3-methylbut-1-yn-1-yl)biphenyl-2-yl]nicotinamide, (15.115) (5-bromo-2-methoxy-4-methylpyridin-3-yl)(2,3,4-trimethoxy-6-methylphenyl)methanone, (15.116) N-[2-(4-{[3-(4-chlorophenyl)prop-2-yn-1-yl]oxy}-3-methoxyphenyl)ethyl]-N2-(methylsulfonyl)valinamide, (15.117) 4-oxo-4-[(2-phenylethyl)amino]butanoic acid, (15.118) but-3-yn-1-yl {6-[({[(Z)-(1-methyl-1H-tetrazol-5-yl)(phenyl)methylene]amino}oxy)methyl]pyridin-2-yl}carbamate, (15.119) 4-amino-5-fluoropyrimidin-2-ol (mesomeric form: 4-amino-5-fluoropyrimidin-2(1H)-one), (15.120) propyl 3,4,5-trihydroxybenzoate, (15.121) 1,3-dimethyl-N-(1,1,3-trimethyl-2,3-dihydro-1H-inden-4-yl)-1H-pyrazole-4-carboxamide, (15.122) 1,3-dimethyl-N-[(3R)-1,1,3-trimethyl-2,3-dihydro-1H-inden-4-yl]-1H-pyrazole-4-carboxamide, (15.123) 1,3-dimethyl-N-[(3S)-1,1,3-trimethyl-2,3-dihydro-1H-inden-4-yl]-1H-pyrazole-4-carboxamide, (15.124) [3-(4-chloro-2-fluorophenyl)-5-(2,4-difluorophenyl)-1,2-oxazol-4-yl](pyridin-3-yl)methanol, (15.125) (S)-[3-(4-chloro-2-fluorophenyl)-5-(2,4-difluorophenyl)-1,2-oxazol-4-yl](pyridin-3-yl)methanol, (15.126) (R)-[3-(4-chloro-2-fluorophenyl)-5-(2,4-difluorophenyl)-1,2-oxazol-4-yl](pyridin-3-yl)methanol, (15.127) 2-{[3-(2-chlorophenyl)-2-(2,4-difluorophenyl)oxiran-2-yl]methyl}-2,4-dihydro-3H-1,2,4-triazole-3-thione, (15.128) 1-{[3-(2-chlorophenyl)-2-(2,4-difluorophenyl)oxiran-2-yl]methyl}-1H-1,2,4-triazol-5-yl thiocyanate, (15.129) 5-(allylsulfanyl)-1-{[3-(2-chlorophenyl)-2-(2,4-difluorophenyl)oxiran-2-yl]methyl}-1H-1,2,4-triazole, (15.130) 2-[1-(2,4-dichlorophenyl)-5-hydroxy-2,6,6-trimethylheptan-4-yl]-2,4-dihydro-3H-1,2,4-triazole-3-thione, (15.131) 2-{[rel(2R,3S)-3-(2-chlorophenyl)-2-(2,4-difluorophenyl)oxiran-2-yl]methyl}-2,4-dihydro-3H-1,2,4-triazole-3-thione, (15.132) 2-{[rel(2R,3R)-3-(2-chlorophenyl)-2-(2,4-difluorophenyl)oxiran-2-yl]methyl}-2,4-dihydro-3H-1,2,4-triazole-3-thione, (15.133) 1-{[rel(2R,3S)-3-(2-chlorophenyl)-2-(2,4-difluorophenyl)oxiran-2-yl]methyl}-1H-1,2,4-triazol-5-yl thiocyanate, (15.134) 1-{[rel(2R,3R)-3-(2-chlorophenyl)-2-(2,4-difluorophenyl)oxiran-2-yl]methyl}-1H-1,2,4-triazol-5-yl thiocyanate, (15.135) 5-(allylsulfanyl)-1-{[rel(2R,3S)-3-(2-chlorophenyl)-2-(2,4-difluorophenyl)oxiran-2-yl]methyl}-1H-1,2,4-triazole, (15.136) 5-(allylsulfanyl)-1-{[rel(2R,3R)-3-(2-chlorophenyl)-2-(2,4-difluorophenyl)oxiran-2-yl]methyl}-1H-1,2,4-triazole, (15.137) 2-[(2S,4S,SS)-1-(2,4-dichlorophenyl)-5-hydroxy-2,6,6-trimethylheptan-4-yl]-2,4-dihydro-3H-1,2,4-triazole-3-thione, (15.138) 2-[(2R,4S,5S)-1-(2,4-dichlorophenyl)-5-hydroxy-2,6,6-trimethylheptan-4-yl]-2,4-dihydro-3H-1,2,4-triazole-3-thione, (15.139) 2-[(2R,4R,5R)-1-(2,4-dichlorophenyl)-5-hydroxy-2,6,6-trimethylheptan-4-yl]-2,4-dihydro-3H-1,2,4-triazole-3-thione, (15.140) 2-[(2S,4R,5R)-1-(2,4-dichlorophenyl)-5-hydroxy-2,6,6-trimethylheptan-4-yl]-2,4-dihydro-3H-1,2,4-triazole-3-thione, (15.141) 2-[(2S,4S,SR)-1-(2,4-dichlorophenyl)-5-hydroxy-2,6,6-trimethylheptan-4-yl]-2,4-dihydro-3H-1,2,4-triazole-3-thione, (15.142) 2-[(2R,4S,SR)-1-(2,4-dichlorophenyl)-5-hydroxy-2,6,6-trimethylheptan-4-yl]-2,4-dihydro-3H-1,2,4-triazole-3-thione, (15.143) 2-[(2R,4R,5S)-1-(2,4-dichlorophenyl)-5-hydroxy-2,6,6-trimethylheptan-4-yl]-2,4-dihydro-3H-1,2,4-triazole-3-thione, (15.144) 2-[(2S,4R,5S)-1-(2,4-dichlorophenyl)-5-hydroxy-2,6,6-trimethylheptan-4-yl]-2,4-dihydro-3H-1,2,4-triazole-3-thione, (15.145) 2-fluoro-6-(trifluoromethyl)-N-(1,1,3-trimethyl-2,3-dihydro-1H-inden-4-yl)benzamide, (15.146) 2-(6-benzylpyridin-2-yl)quinazoline, (15.147) 2-[6-(3-fluoro-4-methoxyphenyl)-5-methylpyridin-2-yl]quinazoline, (15.148) 3-(4,4-difluoro-3,3-dimethyl-3,4-dihydroisoquinolin-1-yl)quinoline, (15.149) Abscisic acid.

Die erfindungsgemäßen Wirkstoffe können ferner mit Mikroorganismen kombiniert werden.
Die Mikroorganismen eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Mikroorganismen gehören:
Mikroorganismen aus der Gruppe der Bakterien, z.B. Bacillus agri, Bacillus aizawai, Bacillus albolactis, Bacillus amyloliquefaciens, insbesondere der Stamm B. amyloliquefaciens IN937a, oder Stamm FZB42, Bacillus cereus, insbesondere Sporen von B. cereus CNCM I-1562, Bacillus coagulans, Bacillus endoparasiticus, Bacillus endorhythmos, Bacillus firmus, insbesondere Sporen von B. firmus CNCM I-1582, Bacillus kurstaki, Bacillus lacticola, Bacillus lactimorbus, Bacillus lactis, Bacillus laterosporus, Bacillus lentimorbus, Bacillus licheniformis, Bacillus medusa, Bacillus megaterium, Bacillus metiens, Bacillus natto, Bacillus nigrificans, Bacillus popillae, Bacillus pumilus, insbesondere der Stamm B. pumilus GB34, Bacillus siamensis, Bacillus sphaericus, Bacillus subtilis, insbesondere der Stamm B. subtilis GB03, oder der Stamm B. subtilis var. amyloliquefaciens FZB24, Bacillus thuringiensis, insbesondere B. thuringiensis var. israelensis oder B. thuringiensis ssp. aizawai Stamm ABTS-1857 oder, B. thuringiensis ssp kurstaki Stamm HD-1, B. thuringiensis var. san diego,, B. thuringiensis var. tenebrinos, Bacillus uniflagellatus, Delftia acidovorans, insbesondere Stamm RAY209, Lysobacter antibioticus, insbesondere Stamm 13-1, Metarhizium anisopliae, Pseudomonas chlororaphis, insbesondere Stamm MA342, Pseudomonas proradix, Streptomyces galbus, insbesondere Stamm K61, Streptomyces griseoviridis;
Mikroorganismen aus der Gruppe der Pilze, z.B. Ampelomyces quisqualis, insbesondere Stamm AQ10, Aureobasidium pullulans, insbesondere Blastosporen von Stamm DSM14940 oder Blastosporen von Stamm DSM14941 oder Mischungen davon, Beauveria bassiana, insbesondere Stamm ATCC74040, Beauveria brongniartii, Candida oleophila, insbesondere Stamm O, Coniothyrium minitans, insbesondere Stamm CON/M/91-8, Dilophosphora alopecuri, Gliocladium catenulatum, insbesondere Stamm J1446; Hirsutella thompsonii, Lagenidium giganteum, Lecanicillium lecanii (vormals bekannt als Verticillium lecanii), insbesondere Konidien von Stamm KV01, Metarhizium anisopliae, insbesondere Stamm F52, Metschnikovia fructicola, insbesondere Stamm NRRL Y-30752, Microsphaeropsis ochracea, Muscodor albus, insbesondere Stamm QST20799, Nomuraea rileyi, Paecilomyces lilacinus, insbesondere Sporen von Stamm P. lilacinus 251, Penicillium bilaii, insbesondere Stamm ATCC22348, Pichia anomala, insbesondere Stamm WRL-076, Pseudozyma flocculosa, insbesondere Stamm PF-A22 UL, Pytium oligandrum DV74, Trichoderma asperellum, insbesondere Stamm ICC012, Trichoderma harzianum, insbesondere T. harzianum T39, Verticillium lecanii, insbesondere die Stämme DAOM198499 und DAOM216596;
insektizide Mikroorganismen aus der Gruppe der Protozoa, z.B. Nosema locustae, Vairimorpha; insektizide Mikroorganismen aus der Gruppe der Viren, z.B. Gypsy moth (Lymantria dispar) nuclear polyhedrosis virus (NPV), Tussock moth (Lymantriidae) NPV, Heliothis NPV, Pine sawfly (Neodiprion) NPV, Codling moth (Cydia pomonella) granulosis virus (GV);
Mikroorganismen aus der Gruppe der entomopathogenen Nematoden, z.B. Steinernema scapterisci, Steinernema feltiae (Neoaplectana carpocapsae), Heterorhabditis heliothidis, Xenorhabdus luminescence.

Die hier mit ihrem "common name" genannten Wirkstoffe sind bekannt und beispielsweise im Pestizidhandbuch ("The Pesticide Manual" 14th Ed., British Crop Protection Council 2006) beschrieben oder im Internet recherchierbar (z.B. http://www.alanwood.net/pesticides).

Alle genannten Mischpartner der Klassen (1) bis (16) können, wenn sie auf Grund ihrer funktionellen Gruppen dazu imstande sind, gegebenenfalls mit geeigneten Basen oder Säuren Salze bilden.

Schließlich wurde gefunden, dass die neuen Verbindungen der Formel (I) sich bei guter Pflanzenverträglichkeit, günstiger Warmblütertoxizität und guter Umweltverträglichkeit vor allem zur Bekämpfung von tierischen Schädlingen, insbesondere von Arthropoden, Insekten, Spinnentieren, Helminthen, Nematoden und Mollusken, die in der Landwirtschaft, in den Forsten, im Vorrats- und Materialschutz sowie auf dem Hygienesektor oder im Bereich der Tiergesundheit vorkommen, eignen. Gleichfalls können die erfindungsgemäßen Verbindungen im Bereich der Tiergesundheit verwendet werden, beispielsweise zur Bekämpfung von Endo- und/oder Ectoparasiten.

Die erfindungsgemäßen Verbindungen können als Mittel zur Bekämpfung tierischer Schädlinge, vorzugsweise als Pflanzenschutzmittel eingesetzt werden. Sie sind gegen normal sensible und resistente Arten sowie gegen alle oder einzelne Entwicklungsstadien wirksam.

Die erfindungsgemäßen Verbindungen können in allgemein bekannte Formulierungen überführt werden. Solche Formulierungen enthalten im Allgemeinen von 0,01 bis 98 Gew.-% Wirkstoff, vorzugsweise von 0,5 bis 90 Gew.-%.

Die erfindungsgemäßen Verbindungen können in ihren handelsüblichen Formulierungen sowie in den aus diesen Formulierungen bereiteten Anwendungsformen in Mischung mit anderen Wirkstoffen oder Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise von 0,00001 bis 1 Gew.-% liegen.

Die Anwendung geschieht in einer den Anwendungsformen angepaßten üblichen Weise.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kultwpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Im Bereich Tiergesundheit, d.h. auf dem veterinärmedizinischen Gebiet, wirken die Wirkstoffe gemäß der vorliegenden Erfindung gegen tierische Parasiten, insbesondere Ektoparasiten oder Endoparasiten. Der Begriff Endoparasiten schließt insbesondere Helminthen wie Cestoden, Nematoden oder Trematoden, und Protozoen wie Coccidien ein. Ektoparasiten sind typischerweise und vorzugsweise Arthropoden, insbesondere Insekten wie Fliegen (stechend und leckend), parasitische Fliegenlarven, Läuse, Haarlinge, Federlinge, Flöhe und dergleichen; oder Akariden wie Zecken, zum Beispiel Schildzecken oder Lederzecken, oder Milben wie Räudemilben, Laufmilben, Federmilben und dergleichen.

Außerdem wurde gefunden, daß die erfindungsgemäßen Verbindungen eine hohe insektizide Wirkung gegen Insekten zeigen, die technische Materialien zerstören. Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam.

Unter Pflanzen werden alle Pflanzenarten, Pflanzensorten und Pflanzenpopulationen, wie erwünschte und unerwünschte Wildpflanzen oder Kulturpflanzen verstanden. Erfindungsgemäß zu behandelnde Kulturpflanzen sind Pflanzen, die natürlich vorkommen, oder solche, die durch konventionelle Züchtungs- und Optimierungsmethoden oder durch biotechnologische und gentechnologische Methoden oder durch Kombinationen der vorgenannten Methoden erhalten wurden. Der Begriff Kulturpflanze umfasst selbstverständlich auch transgene Pflanzen.

Unter Pflanzensorten versteht man Pflanzen mit neuen Eigenschaften, sogenannten Traits, die sowohl durch konventionelle Züchtung, durch Mutagenese oder durch rekombinante DNA-Techniken oder einer Kombination hieraus gezüchtet worden sind. Dies können Sorten, Rassen, Bio- und Genotypen sein.

Unter Pflanzenteilen werden alle oberirdischen und unterirdischen Teile und Organe der Pflanzen, wie Spross, Blatt, Blüte und Wurzel verstanden, insbesondere Blätter, Nadeln, Stängel, Stämme, Blüten, Fruchtkörper, Früchte, Samen, Wurzeln, Knollen und Rhizome. Der Begriff Pflanzenteilen umfasst weiterhin Erntegut sowie vegetatives und generatives Vermehrungsmaterial, wie z.B. Stecklinge, Knollen, Rhizome, Ableger und Samen bzw. Saatgut.

In einer erfindungsgemäßen Ausführungsform werden natürlich vorkommende oder durch konventionelle Züchtungs- und Optimierungsmethoden (z.B. Kreuzung oder Protoplastenfusion) erhaltene Pflanzenarten und Pflanzensorten sowie deren Pflanzenteile behandelt.

In einer weiteren erfindungsgemäßen Ausführungsform werden transgene Pflanzen, die durch gentechnologische Methoden gegebenenfalls in Kombination mit konventionellen Methoden erhalten wurden und deren Teile behandelt.

Das erfindungsgemäße Behandlungsverfahren wird vorzugsweise auf genetisch modifizierten Pflanzen oder Pflanzenteile, verwendet.

Genetisch modifizierte Pflanzen, sogenannte transgene Pflanzen, sind Pflanzen, bei denen ein heterologes Gen stabil in das Genom integriert worden ist.

Der Begriff "heterologes Gen" bedeutet im wesentlichen ein Gen, das außerhalb der Pflanze bereitgestellt oder assembliert wird und das bei Einführung in das Zellkerngenom, das Chloroplastengenom oder das Hypochondriengenom der transformierten Pflanze dadurch neue oder verbesserte agronomische oder sonstige Eigenschaften verleiht, daß es ein interessierendes Protein oder Polypeptid exprimiert oder daß es ein anderes Gen, das in der Pflanze vorliegt bzw. andere Gene, die in der Pflanze vorliegen, herunterreguliert oder abschaltet (zum Beispiel mittels Antisense-Technologie, Cosuppressionstechnologie oder RNAi-Technologie [RNA Interference]). Ein heterologes Gen, das im Genom vorliegt, wird ebenfalls als Transgen bezeichnet. Ein Transgen, das durch sein spezifisches Vorliegen im Pflanzengenom definiert ist, wird als Transformations- bzw. transgenes Event bezeichnet.

In Abhängigkeit von den Pflanzenarten oder Pflanzensorten, ihrem Standort und ihren Wachstumsbedingungen (Böden, Klima, Vegetationsperiode, Ernährung) kann die erfindungsgemäße Behandlung auch zu überadditiven ("synergistischen") Effekten führen. So sind zum Beispiel die folgenden Effekte möglich, die über die eigentlich zu erwartenden Effekte hinausgehen: verringerte Aufwandmengen und/oder erweitertes Wirkungsspektrum und/oder erhöhte Wirksamkeit der Wirkstoffe und Zusammensetzungen, die erfindungsgemäß eingesetzt werden können, besseres Pflanzenwachstum, erhöhte Toleranz gegenüber hohen oder niedrigen Temperaturen, erhöhte Toleranz gegenüber Trockenheit oder Wasser- oder Bodensalzgehalt, erhöhte Blühleistung, Ernteerleichterung, Reifebeschleunigung, höhere Erträge, größere Früchte, größere Pflanzenhöhe, intensiver grüne Farbe des Blatts, frühere Blüte, höhere Qualität und/oder höherer Nährwert der Ernteprodukte, höhere Zuckerkonzentration in den Früchten, bessere Lagerfähigkeit und/oder Verarbeitbarkeit der Ernteprodukte.

In gewissen Aufwandmengen können die erfindungsgemäßen Wirkstoffkombinationen auch eine stärkende Wirkung auf Pflanzen ausüben. Sie eignen sich daher für die Mobilisierung des pflanzlichen Abwehrsystems gegen Angriff durch unerwünschte phytopathogene Pilze und/oder Mikroorganismen und/oder Viren. Dies kann gegebenenfalls einer der Gründe für die erhöhte Wirksamkeit der erfindungsgemäßen Kombinationen sein, zum Beispiel gegen Pilze. Pflanzenstärkende (resistenzinduzierende) Substanzen sollen im vorliegenden Zusammenhang auch solche Substanzen oder Substanzkombinationen bedeuten, die fähig sind, das pflanzliche Abwehrsystem so zu stimulieren, daß die behandelten Pflanzen, wenn sie im Anschluß daran mit unerwünschten phytopathogenen Pilzen und/oder Mikroorganismen und/oder Viren inokkuliert werde, einen beträchtlichen Resistenzgrad gegen diese unerwünschten phytopathogenen Pilze und/oder Mikroorganismen und/oder Viren aufweisen. Im vorliegenden Fall versteht man unter unerwünschten phytopathogenen Pilzen und/oder Mikroorganismen und/oder Viren phytopathogene Pilze, Bakterien und Viren. Die erfindungsgemäßen Substanzen lassen sich daher zum Schutz von Pflanzen gegen Angriff durch die erwähnten Pathogene innerhalb eines gewissen Zeitraums nach der Behandlung einsetzen. Der Zeitraum, über den eine Schutzwirkung erzielt wird, erstreckt sich im allgemeinen von 1 bis 10 Tagen, vorzugsweise 1 bis 7 Tagen, nach der Behandlung der Pflanzen mit den Wirkstoffen.

Pflanzen, die weiterhin vorzugsweise erfindungsgemäß behandelt werden, sind gegen einen oder mehrere biotische Streßfaktoren resistent, d. h. diese Pflanzen weisen eine verbesserte Abwehr gegen tierische und mikrobielle Schädlinge wie Nematoden, Insekten, Milben, phytopathogene Pilze, Bakterien, Viren und/oder Viroide auf.

Neben den vorgenannten Pflanzen und Pflanzensorten, können auch solche erfindungsgemäß behandelt werden, die gegen einen oder mehrere abiotische Streßfaktoren widerstandsfähig sind.

Zu den abiotischen Streßbedingungen können zum Beispiel Dürre, Kälte- und Hitzebedingungen, osmotischer Streß, Staunässe, erhöhter Bodensalzgehalt, erhöhtes Ausgesetztsein an Mineralien, Ozonbedingungen, Starklichtbedingungen, beschränkte Verfügbarkeit von Stickstoffnährstoffen, beschränkte Verfügbarkeit von Phosphornährstoffen oder Vermeidung von Schatten zählen.

Pflanzen und Pflanzensorten, die ebenfalls erfindungsgemäß behandelt werden können, sind solche Pflanzen, die durch erhöhte Ertragseigenschaften gekennzeichnet sind. Ein erhöhter Ertrag kann bei diesen Pflanzen z. B. auf verbesserter Pflanzenphysiologie, verbessertem Pflanzenwuchs und verbesserter Pflanzenentwicklung, wie Wasserverwertungseffizienz, Wasserhalteeffizienz, verbesserter Stickstoffverwertung, erhöhter Kohlenstoffassimilation, verbesserter Photosynthese, verstärkter Keimkraft und beschleunigter Abreife beruhen. Der Ertrag kann weiterhin durch eine verbesserte Pflanzenarchitektur (unter Streß- und nicht-Streß-Bedingungen) beeinflußt werden, darunter frühe Blüte, Kontrolle der Blüte für die Produktion von Hybridsaatgut, Keimpflanzenwüchsigkeit, Pflanzengröße, Internodienzahl und -abstand, Wurzelwachstum, Samengröße, Fruchtgröße, Schotengröße, Schoten- oder Ährenzahl, Anzahl der Samen pro Schote oder Ähre, Samenmasse, verstärkte Samenfüllung, verringerter Samenausfall, verringertes Schotenplatzen sowie Standfestigkeit. Zu weiteren Ertragsmerkmalen zählen Samenzusammensetzung wie Kohlenhydratgehalt, Proteingehalt, Ölgehalt und Ölzusammensetzung, Nährwert, Verringerung der nährwidrigen Verbindungen, verbesserte Verarbeitbarkeit und verbesserte Lagerfähigkeit.

Pflanzen, die erfindungsgemäß behandelt werden können, sind Hybridpflanzen, die bereits die Eigenschaften der Heterosis bzw. des Hybrideffekts exprimieren, was im allgemeinen zu höherem Ertrag, höherer Wüchsigkeit, besserer Gesundheit und besserer Resistenz gegen biotische und abiotische Streßfaktoren führt. Solche Pflanzen werden typischerweise dadurch erzeugt, daß man eine ingezüchtete pollensterile Elternlinie (den weiblichen Kreuzungspartner) mit einer anderen ingezüchteten pollenfertilen Elternlinie (dem männlichen Kreuzungspartner) kreuzt. Das Hybridsaatgut wird typischerweise von den pollensterilen Pflanzen geerntet und an Vermehrer verkauft. Pollensterile Pflanzen können manchmal (z. B. beim Mais) durch Entfahnen (d. h. mechanischem Entfernen der männlichen Geschlechtsorgane bzw. der männlichen Blüten), produziert werden; es ist jedoch üblicher, daß die Pollensterilität auf genetischen Determinanten im Pflanzengenom beruht. In diesem Fall, insbesondere dann, wenn es sich bei dem gewünschten Produkt, da man von den Hybridpflanzen ernten will, um die Samen handelt, ist es üblicherweise günstig, sicherzustellen, daß die Pollenfertilität in Hybridpflanzen, die die für die Pollensterilität verantwortlichen genetischen Determinanten enthalten, völlig restoriert wird. Dies kann erreicht werden, indem sichergestellt wird, daß die männlichen Kreuzungspartner entsprechende Fertilitätsrestorergene besitzen, die in der Lage sind, die Pollenfertilität in Hybridpflanzen, die die genetischen Determinanten, die für die Pollensterilität verantwortlich sind, enthalten, zu restorieren. Genetische Determinanten für Pollensterilität können im Cytoplasma lokalisiert sein. Beispiele für cytoplasmatische Pollensterilität (CMS) wurden zum Beispiel für Brassica-Arten beschrieben. Genetische Determinanten für Pollensterilität können jedoch auch im Zellkerngenom lokalisiert sein. Pollensterile Pflanzen können auch mit Methoden der pflanzlichen Biotechnologie, wie Gentechnik, erhalten werden. Ein besonders günstiges Mittel zur Erzeugung von pollensterilen Pflanzen ist in WO 89/10396 beschrieben, wobei zum Beispiel eine Ribonuklease wie eine Barnase selektiv in den Tapetumzellen in den Staubblättern exprimiert wird. Die Fertilität kann dann durch Expression eines Ribonukleasehemmers wie Barstar in den Tapetumzellen restoriert werden.

Pflanzen oder Pflanzensorten (die mit Methoden der Pflanzenbiotechnologie, wie der Gentechnik, erhalten werden), die erfindungsgemäß behandelt werden können, sind herbizidtolerante Pflanzen, d. h. Pflanzen, die gegenüber einem oder mehreren vorgegebenen Herbiziden tolerant gemacht worden sind. Solche Pflanzen können entweder durch genetische Transformation oder durch Selektion von Pflanzen, die eine Mutation enthalten, die solch eine Herbizidtoleranz verleiht, erhalten werden.

Herbizidtolerante Pflanzen sind zum Beispiel glyphosatetolerante Pflanzen, d. h. Pflanzen, die gegenüber dem Herbizid Glyphosate oder dessen Salzen tolerant gemacht worden sind. So können zum Beispiel glyphosatetolerante Pflanzen durch Transformation der Pflanze mit einem Gen, das für das Enzym 5-Enolpyruvylshikimat-3-phosphatsynthase (EPSPS) kodiert, erhalten werden. Beispiele für solche EPSPS-Gene sind das AroA-Gen (Mutante CT7) des Bakterium *Salmonella typhimurium,* das CP4-Gen des Bakteriums *Agrobacterium sp.,* die Gene, die für eine EPSPS aus der Petunie, für eine EPSPS aus der Tomate oder für eine EPSPS aus Eleusine kodieren. Es kann sich auch um eine mutierte EPSPS handeln. Glyphosatetolerante Pflanzen können auch dadurch erhalten werden, daß man ein Gen exprimiert, das für ein Glyphosate-Oxidoreduktase-Enzym kodiert. Glyphosatetolerante Pflanzen können auch dadurch erhalten werden, daß man ein Gen exprimiert, das für ein Glyphosateacetyltransferase-Enzym kodiert. Glyphosatetolerante Pflanzen können auch dadurch erhalten werden, daß man Pflanzen, die natürlich vorkommende Mutationen der oben erwähnten Gene selektiert.

Sonstige herbizidresistente Pflanzen sind zum Beispiel Pflanzen, die gegenüber Herbiziden, die das Enzym Glutaminsynthase hemmen, wie Bialaphos, Phosphinotricin oder Glufosinate, tolerant gemacht worden sind. Solche Pflanzen können dadurch erhalten werden, daß man ein Enzym exprimiert, das das Herbizid oder eine Mutante des Enzyms Glutaminsynthase, das gegenüber Hemmung resistent ist, entgiftet. Solch ein wirksames entgiftendes Enzym ist zum Beispiel ein Enzym, das für ein Phosphinotricin-acetyltransferase kodiert (wie zum Beispiel das bar- oder pat-Protein aus Streptomyces-Arten). Pflanzen, die eine exogene Phosphinotricin-acetyltransferase exprimieren, sind beschrieben.

Weitere herbizidtolerante Pflanzen sind auch Pflanzen, die gegenüber den Herbiziden, die das Enzym Hydroxyphenylpyruvatdioxygenase (HPPD) hemmen, tolerant gemacht worden sind. Bei den Hydroxyphenylpyruvatdioxygenasen handelt es sich um Enzyme, die die Reaktion, in der para-Hydroxyphenylpyruvat (HPP) zu Homogentisat umgesetzt wird, katalysieren. Pflanzen, die gegenüber HPPD-Hemmern tolerant sind, können mit einem Gen, das für ein natürlich vorkommendes resistentes HPPD-Enzym kodiert, oder einem Gen, das für ein mutiertes HPPD-Enzym kodiert, transformiert werden. Eine Toleranz gegenüber HPPD-Hemmern kann auch dadurch erzielt werden, daß man Pflanzen mit Genen transformiert, die für gewisse Enzyme kodieren, die die Bildung von Homogentisat trotz Hemmung des nativen HPPD-Enzyms durch den HPPD-Hemmer ermöglichen. Die Toleranz von Pflanzen gegenüber HPPD-Hemmern kann auch dadurch verbessert werden, daß man Pflanzen zusätzlich zu einem Gen, das für ein HPPD-tolerantes Enzym kodiert, mit einem Gen transformiert, das für ein Prephenatdehydrogenase-Enzym kodiert.

Weitere herbizidresistente Pflanzen sind Pflanzen, die gegenüber Acetolactatsynthase (ALS)-Hemmern tolerant gemacht worden sind. Zu bekannten ALS-Hemmern zählen zum Beispiel Sulfonylharnstoff, Imidazolinon, Triazolopyrimidine, Pyrimidinyloxy(thio)benzoate und/oder Sulfonylaminocarbonyltriazolinon-Herbizide. Es ist bekannt, daß verschiedene Mutationen im Enzym ALS (auch als Acetohydroxysäure-Synthase, AHAS, bekannt) eine Toleranz gegenüber unterschiedlichen Herbiziden bzw. Gruppen von Herbiziden verleihen. Die Herstellung von sulfonylharnstofftoleranten Pflanzen und imidazolinontoleranten Pflanzen ist in der internationalen Veröffentlichung WO 1996/033270 beschrieben. Weitere sulfonylharnstoff-und imidazolinontolerante Pflanzen sind auch in z.B. WO 2007/024782 beschrieben.

Weitere Pflanzen, die gegenüber Imidazolinon und/oder Sulfonylharnstoff tolerant sind, können durch induzierte Mutagenese, Selektion in Zellkulturen in Gegenwart des Herbizids oder durch Mutationszüchtung erhalten werden.

Pflanzen oder Pflanzensorten (die nach Methoden der pflanzlichen Biotechnologie, wie der Gentechnik, erhalten wurden), die ebenfalls erfindungsgemäß behandelt werden können, sind insektenresistente transgene Pflanzen, d.h. Pflanzen, die gegen Befall mit gewissen Zielinsekten resistent gemacht wurden. Solche Pflanzen können durch genetische Transformation oder durch Selektion von Pflanzen, die eine Mutation enthalten, die solch eine Insektenresistenz verleiht, erhalten werden.

Der Begriff "insektenresistente transgene Pflanze" umfaßt im vorliegenden Zusammenhang jegliche Pflanze, die mindestens ein Transgen enthält, das eine Kodiersequenz umfaßt, die für folgendes kodiert:
1) ein insektizides Kristallprotein aus *Bacillus thuringiensis* oder einen insektiziden Teil davon, wie die insektiziden Kristallproteine, die online bei: http://www.lifesci.sussex.ac.uk/Home/Neil_Crickmore/Bt/ beschrieben sind, zusammengestellt wurden, oder insektizide Teile davon, z.B. Proteine der Cry-Proteinklassen Cry1Ab, Cry1Ac, Cry1F, Cry2Ab, Cry3Ae oder Cry3Bb oder insektizide Teile davon; oder
2) ein Kristallprotein aus *Bacillus thuringiensis* oder einen Teil davon, der in Gegenwart eines zweiten, anderen Kristallproteins als *Bacillus thuringiensis* oder eines Teils davon insektizid wirkt, wie das binäre Toxin, das aus den Kristallproteinen Cy34 und Cy35 besteht; oder
3) ein insektizides Hybridprotein, das Teile von zwei unterschiedlichen insektiziden Kristallproteinen aus *Bacillus thuringiensis* umfaßt, wie zum Beispiel ein Hybrid aus den Proteinen von 1) oben oder ein Hybrid aus den Proteinen von 2) oben, z. B. das Protein Cry1A.105, das von dem Mais-Event MON98034 produziert wird (WO 2007/027777); oder
4) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier-DNA während der Klonierung oder Transformation induziert wurden, wie das Protein Cry3Bb1 in Mais-Events MON863 oder MON88017 oder das Protein Cry3A im Mais-Event MIR 604;
5) ein insektizides sezerniertes Protein aus *Bacillus thuringiensis* oder *Bacillus cereus* oder einen insektiziden Teil davon, wie die vegetativ wirkenden insektentoxischen Proteine (vegetative insecticidal proteins, VIP), die unter http://www.lifesci.sussex.ac.uk/Home/Neil_Crickmore/Bt/vip.html angeführt sind, z. B. Proteine der Proteinklasse VIP3Aa; oder
6) ein sezerniertes Protein aus *Bacillus thuringiensis* oder *Bacillus cereus,* das in Gegenwart eines zweiten sezernierten Proteins aus *Bacillus thuringiensis* oder *B. cereus* insektizid wirkt, wie das binäre Toxin, das aus den Proteinen VIP1A und VIP2A besteht.
7) ein insektizides Hybridprotein, das Teile von verschiedenen sezernierten Proteinen von *Bacillus thuringiensis* oder *Bacillus cereus* umfaßt, wie ein Hybrid der Proteine von 1) oder ein Hybrid der Proteine von 2) oben; oder
8) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier-DNA während der Klonierung oder Transformation induziert wurden (wobei die Kodierung für ein insektizides Protein erhalten bleibt), wie das Protein VIP3Aa im Baumwoll-Event COT 102.

Natürlich zählt zu den insektenresistenten transgenen Pflanzen im vorliegenden Zusammenhang auch jegliche Pflanze, die eine Kombination von Genen umfaßt, die für die Proteine von einer der oben genannten Klassen 1 bis 8 kodieren. In einer Ausführungsform enthält eine insektenresistente Pflanze mehr als ein Transgen, das für ein Protein nach einer der oben genannten 1 bis 8 kodiert, um das Spektrum der entsprechenden Zielinsektenarten zu erweitern oder um die Entwicklung einer Resistenz der Insekten gegen die Pflanzen dadurch hinauszuzögern, daß man verschiedene Proteine einsetzt, die für dieselbe Zielinsektenart insektizid sind, jedoch eine unterschiedliche Wirkungsweise, wie Bindung an unterschiedliche Rezeptorbindmgsstellen im Insekt, aufweisen.

Pflanzen oder Pflanzensorten (die nach Methoden der pflanzlichen Biotechnologie, wie der Gentechnik, erhalten wurden), die ebenfalls erfindungsgemäß behandelt werden können, sind gegenüber abiotischen Streßfaktoren tolerant. Solche Pflanzen können durch genetische Transformation oder durch Selektion von Pflanzen, die eine Mutation enthalten, die solch eine Streßresistenz verleiht, erhalten werden. Zu besonders nützlichen Pflanzen mit Streßtoleranz zählen folgende:
a. Pflanzen, die ein Transgen enthalten, das die Expression und/oder Aktivität des Gens für die Poly(ADP-ribose)polymerase (PARP) in den Pflanzenzellen oder Pflanzen zu reduzieren vermag.
b. Pflanzen, die ein streßtoleranzförderndes Transgen enthalten, das die Expression und/oder Aktivität der für PARG kodierenden Gene der Pflanzen oder Pflanzenzellen zu reduzieren vermag;
c. Pflanzen, die ein streßtoleranzförderndes Transgen enthalten, das für ein in Pflanzen funktionelles Enzym des Nicotinamidadenindinukleotid-Salvage-Biosynthesewegs kodiert, darunter Nicotinamidase, Nicotinatphosphoribosyltransferase, Nicotinsäuremononukleotidadenyltransferase, Nicotinamidadenindinukleotidsynthetase oder Nicotinamidphosphoribosyltransferase.

Pflanzen oder Pflanzensorten (die nach Methoden der pflanzlichen Biotechnologie, wie der Gentechnik, erhalten wurden), die ebenfalls erfindungsgemäß behandelt werden können, weisen eine veränderte Menge, Qualität und/oder Lagerfähigkeit des Ernteprodukts und/oder veränderte Eigenschaften von bestimmten Bestandteilen des Ernteprodukts auf, wie zum Beispiel:
1) Transgene Pflanzen, die eine modifizierte Stärke synthetisieren, die bezüglich ihrer chemischphysikalischen Eigenschaften, insbesondere des Amylosegehalts oder des Amylose/Amylopektin-Verhältnisses, des Verzweigungsgrads, der durchschnittlichen Kettenlänge, der Verteilung der Seitenketten, des Viskositätsverhaltens, der Gelfestigkeit, der Stärkekorngröße und/oder Stärkekornmorphologie im Vergleich mit der synthetisierten Stärke in Wildtyppflanzenzellen oder -pflanzen verändert ist, so daß sich diese modifizierte Stärke besser für bestimmte Anwendungen eignet.
2) Transgene Pflanzen, die Nichtstärkekohlenhydratpolymere synthetisieren, oder Nichtstärkekohlenhydratpolymere, deren Eigenschaften im Vergleich zu Wildtyppflanzen ohne genetische Modifikation verändert sind. Beispiele sind Pflanzen, die Polyfructose, insbesondere des Inulin- und Levantyps, produzieren, Pflanzen, die alpha-1,4-Glucane produzieren, Pflanzen, die alpha-1,6-verzweigte alpha-1,4-Glucane produzieren und Pflanzen, die Alternan produzieren.
3) Transgene Pflanzen, die Hyaluronan produzieren.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Baumwollpflanzen mit veränderten Fasereigenschaften. Solche Pflanzen können durch genetische Transformation oder durch Selektion von Pflanzen, die eine Mutation enthalten, die solche veränderten Fasereigenschaften verleiht, erhalten werden; dazu zählen:
a) Pflanzen wie Baumwollpflanzen, die eine veränderte Form von Cellulosesynthasegenen enthalten,
b) Pflanzen wie Baumwollpflanzen, die eine veränderte Form von rsw2- oder rsw3-homologen Nukleinsäuren enthalten;
c) Pflanzen wie Baumwollpflanzen mit einer erhöhten Expression der Saccharosephosphatsynthase;
d) Pflanzen wie Baumwollpflanzen mit einer erhöhten Expression der Saccharosesynthase;
e) Pflanzen wie Baumwollpflanzen bei denen der Zeitpunkt der Durchlaßsteuerung der Plasmodesmen an der Basis der Faserzelle verändert ist, z. B. durch Herunterregulieren der faserselektiven β-1,3-Glucanase;
f) Pflanzen wie Baumwollpflanzen mit Fasern mit veränderter Reaktivität, z. B. durch Expression des N-Acetylglucosamintransferasegens, darunter auch nodC, und von Chitinsynthasegenen.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Raps oder verwandte Brassica-Pflanzen mit veränderten Eigenschaften der Ölzusammensetzung. Solche Pflanzen können durch genetische Transformation oder durch Selektion von Pflanzen, die eine Mutation enthalten, die solche veränderten Öleigenschaften verleiht, erhalten werden; dazu zählen:
a) Pflanzen wie Rapspflanzen, die Öl mit einem hohen Ölsäuregehalt produziere;
b) Pflanzen wie Rapspflanzen, die Öl mit einem niedrigen Linolensäuregehalt produzieren.
c) Pflanzen wie Rapspflanzen, die Öl mit einem niedrigen gesättigten Fettsäuregehalt produzieren.

Besonders nützliche transgene Pflanzen, die erfindungsgemäß behandelt werden können, sind Pflanzen mit einem oder mehreren Genen, die für ein oder mehrere Toxine kodieren, sind die transgenen Pflanzen, die unter den folgenden Handelsbezeichnungen angeboten werden: YIELD GARD^{®} (zum Beispiel Mais, Baumwolle, Sojabohnen), KnockOut^{®} (zum Beispiel Mais), BiteGard^{®} (zum Beispiel Mais), BT-Xtra^{®} (zum Beispiel Mais), StarLink^{®} (zum Beispiel Mais), Bollgard^{®} (Baumwolle), Nucotn^{®} (Baumwolle), Nucotn 33B^{®} (Baumwolle), NatureGard^{®} (zum Beispiel Mais), Protecta^{®} und NewLeaf^{®} (Kartoffel). Herbizidtolerante Pflanzen, die zu erwähnen sind, sind zum Beispiel Maissorten, Baumwollsorten und Sojabohnensorten, die unter den folgenden Handelsbezeichnungen angeboten werden: Roundup Ready^{®} (Glyphosatetoleranz, zum Beispiel Mais, Baumwolle, Sojabohne), Liberty Link^{®} (Phosphinotricintoleranz, zum Beispiel Raps), IMI^{®} (Imidazolinontoleranz) und SCS^{®} (Sylfonylharnstofftoleranz), zum Beispiel Mais. Zu den herbizidresistenten Pflanzen (traditionell auf Herbizidtoleranz gezüchtete Pflanzen), die zu erwähnen sind, zählen die unter der Bezeichnung Clearfield^{®} angebotenen Sorten (zum Beispiel Mais).

Besonders nützliche transgene Pflanzen, die erfindungsgemäß behandelt werden können, sind Pflanzen, die Transformations-Events, oder eine Kombination von Transformations-Events, enthalten und die zum Beispiel in den Dateien von verschiedenen nationalen oder regionalen Behörden angeführt sind (siehe zum Beispiel http://gmoinfo.jrc.it/gmp_browse.aspx und http://www.agbios.com/dbase.php).

Die erfindungsgemäße Behandlung der Pflanzen und Pflanzenteile mit den Wirkstoffkombinationen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Saatgut, weiterhin durch ein- oder mehrschichtiges Umhüllen.

Insbesondere eignen sich die erfindungsgemäßen Mischungen zur Behandlung von Saatgut. Bevorzugt sind dabei die vorstehend als bevorzugt oder besonders bevorzugt genannten erfindungsgemäßen Kombinationen zu nennen. So entsteht ein großer Teil des durch Schädlinge verursachten Schadens an Kulturpflanzen bereits durch den Befall des Saatguts während der Lagerung und nach dem Einbringen des Saatguts in den Boden sowie während und unmittelbar nach der Keimung der Pflanzen. Diese Phase ist besonders kritisch, da die Wurzeln und Sprosse der wachsenden Pflanze besonders empfindlich sind und bereits ein geringer Schaden zum Absterben der ganzen Pflanze führen kann. Es besteht daher ein insbesondere großes Interesse daran, das Saatgut und die keimende Pflanze durch den Einsatz geeigneter Mittel zu schützen.

Die Bekämpfung von Schädlingen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufriedenstellend gelöst werden können. So ist es erstrebenswert, Verfahren zum Schutz des Saatguts und der keimenden Pflanze zu entwickeln, die das zusätzliche Ausbringen von Pflanzenschutzmitteln nach der Saat oder nach dem Auflaufen der Pflanzen überflüssig machen. Es ist weiterhin erstrebenswert, die Menge des eingesetzten Wirkstoffs dahingehend zu optimieren, dass das Saatgut und die keimende Pflanze vor dem Befall durch Schädlinge bestmöglich geschützt wird, ohne jedoch die Pflanze selbst durch den eingesetzten Wirkstoff zu schädigen. Insbesondere sollten Verfahren zur Behandlung von Saatgut auch die intrinsischen insektiziden Eigenschaften transgener Pflanzen einbeziehen, um einen optimalen Schutz des Saatguts und auch der keimenden Pflanze bei einem minimalen Aufwand an Pflanzenschutzmitteln zu erreichen.

Die vorliegende Erfindung bezieht sich daher insbesondere auch auf ein Verfahren zum Schutz von Saatgut und keimenden Pflanzen vor dem Befall von Schädlingen, indem das Saatgut mit einem erfindungsgemäßen Mittel behandelt wird. Die Erfindung bezieht sich ebenfalls auf die Verwendung der erfindungsgemäßen Mittel zur Behandlung von Saatgut zum Schutz des Saatguts und der daraus entstehenden Pflanze vor Schädlingen. Weiterhin bezieht sich die Erfindung auf Saatgut, welches zum Schutz vor Schädlingen mit einem erfindungsgemäßen Mittel behandelt wurde.

Einer der Vorteile der vorliegenden Erfindung ist es, dass aufgrund der besonderen systemischen Eigenschaften der erfindungsgemäßen Mittel die Behandlung des Saatguts mit diesen Mitteln nicht nur das Saatgut selbst, sondern auch die daraus hervorgehenden Pflanzen nach dem Auflaufen vor Schädlingen schützt. Auf diese Weise kann die unmittelbare Behandlung der Kultur zum Zeitpunkt der Aussaat oder kurz danach entfallen.

Ein weiterer Vorteil besteht in der synergistischen Erhöhung der insektiziden Wirksamkeit der erfindungsgemäßen Mittel gegenüber dem insektiziden Einzelwirkstoff, die über die zu erwartende Wirksamkeit der beiden einzeln angewendeten Wirkstoffe hinausgeht. Vorteilhaft ist auch die synergistische Erhöhung der fungiziden Wirksamkeit der erfindungsgemäßen Mittel gegenüber dem fungiziden Einzelwirkstoff, die über die zu erwartende Wirksamkeit des einzeln angewendeten Wirkstoffs hinausgeht. Damit wird eine Optimierung der Menge der eingesetzten Wirkstoffe ermöglicht.

Ebenso ist es als vorteilhaft anzusehen, dass die erfindungsgemäßen Mischungen insbesondere auch bei transgenem Saatgut eingesetzt werden können, wobei die aus diesem Saatgut hervorgehenden Pflanzen zur Expression eines gegen Schädlinge gerichteten Proteins befähigt sind. Durch die Behandlung solchen Saatguts mit den erfindungsgemäßen Mitteln können bestimmte Schädlinge bereits durch die Expression des z.B. insektiziden Proteins kontrolliert werden, und zusätzlich durch die erfindungsgemäßen Mittel vor Schäden bewahrt werden.

Die erfindungsgemäßen Mittel eignen sich zum Schutz von Saatgut jeglicher Pflanzensorte wie bereits vorstehend genannt, die in der Landwirtschaft, im Gewächshaus, in Forsten oder im Gartenbau eingesetzt wird. Insbesondere handelt es sich dabei um Saatgut von Mais, Erdnuss, Canola, Raps, Mohn, Soja, Baumwolle, Rübe (z.B. Zuckerrübe und Futterrübe), Reis, Hirse, Weizen, Gerste, Hafer, Roggen, Sonnenblume, Tabak, Kartoffeln oder Gemüse (z.B. Tomaten, Kohlgewächs). Die erfindungsgemäßen Mittel eignen sich ebenfalls zur Behandlung des Saatguts von Obstpflanzen und Gemüse wie vorstehend bereits genannt. Besondere Bedeutung kommt der Behandlung des Saatguts von Mais, Soja, Baumwolle, Weizen und Canola oder Raps zu.

Wie vorstehend bereits erwähnt, kommt auch der Behandlung von transgenem Saatgut mit einem erfindungsgemäßen Mittel eine besondere Bedeutung zu. Dabei handelt es sich um das Saatgut von Pflanzen, die in der Regel zumindest ein heterologes Gen enthalten, das die Expression eines Polypeptids mit insbesondere insektiziden Eigenschaften steuert. Die heterologen Gene in transgenem Saatgut können dabei aus Mikroorganismen wie *Bacillus, Rhizobium, Pseudomonas, Serratia, Trichoderma, Clavibacter, Glomus* oder *Gliocladium* stammen. Die vorliegende Erfindung eignet sich besonders für die Behandlung von transgenem Saatgut, das zumindest ein heterologes Gen enthält, das aus *Bacillus sp.* stammt und dessen Genprodukt Wirksamkeit gegen Maiszünsler und/oder Maiswurzel-Bohrer zeigt. Besonders bevorzugt handelt es sich dabei um ein heterologes Gen, das aus *Bacillus thuringiensis* stammt.

Im Rahmen der vorliegenden Erfindung wird das erfindungsgemäßes Mittel alleine oder in einer geeigneten Formulierung auf das Saatgut aufgebracht. Vorzugsweise wird das Saatgut in einem Zustand behandelt, in dem so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde.

Im Allgemeinen muss bei der Behandlung des Saatguts darauf geachtet werden, dass die Menge des auf das Saatgut aufgebrachten erfindungsgemäßen Mittels und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Darüber hinaus können die erfindungsgemäßen Verbindungen zur Bekämpfung einer Vielzahl verschiedener Schädlinge einschließlich beispielsweise schädlicher saugender Insekten, beißender Insekten und anderen an Pflanzen parasitierenden Schädlingen, Vorratsschädlingen, Schädlingen, die industrielle Materialien zerstören und Hygieneschädlingen einschließlich Parasiten im Bereich Tiergesundheit verwendet und zu ihrer Bekämpfung wie zum Beispiel ihrer Auslöschung und Ausmerzung eingesetzt werden. Die vorliegende Erfindung schließt somit auch ein Verfahren zur Bekämpfung von an Pflanzen parasitierenden Schädlingen, Vorratsschädlingen oder Schädlingen, die industrielle Materialien zerstören, ein.

Im Bereich Tiergesundheit, d.h. auf dem veterinärmedizinischen Gebiet, wirken die Wirkstoffe gemäß der vorliegenden Erfindung gegen tierische Parasiten, insbesondere Ektoparasiten oder Endoparasiten. Der Begriff Endoparasiten schließt insbesondere Helminthen wie Cestoden, Nematoden oder Trematoden, und Protozoen wie Kozzidien ein. Ektoparasiten sind typischerweise und vorzugsweise Arthropoden, insbesondere Insekten wie Fliegen (stechend und leckend), parasitische Fliegenlarven, Läuse, Haarlinge, Federlinge, Flöhe und dergleichen; oder Akariden wie Zecken, zum Beispiel Schildzecken oder Lederzecken, oder Milben wie Räudemilben, Laufmilben, Federmilben und dergleichen.

### Zu diesen Parasiten gehören:

Aus der Ordnung der Anoplurida z.B. Haematopinus spp., Linognathus spp., Pediculus spp., Phthirus spp., Solenopotes spp.; spezielle Beispiele sind: Linognathus setosus, Linognathus vituli, Linognathus ovillus, Linognathus oviformis, Linognathus pedalis, Linognathus stenopsis, Haematopinus asini macrocephalus, Haematopinus eurysternus, Haematopinus suis, Pediculus humanus capitis, Pediculus humanus corporis, Phylloera vastatrix, Phthirus pubis, Solenopotes capillatus;
Aus der Ordnung der Mallophagida und den Unterordnungen Amblycerina und Ischnocerina z.B. Trimenopon spp., Menopon spp., Trinoton spp., Bovicola spp., Werneckiella spp., Lepikentron spp., Damalina spp., Trichodectes spp., Felicola spp.; spezielle Beispiele sind: Bovicola bovis, Bovicola ovis, Bovicola limbata, Damalina bovis, Trichodectes canis, Felicola subrostratus, Bovicola caprae, Lepikentron ovis, Werneckiella equi;
Aus der Ordnung der Diptera und den Unterordnungen Nematocerina und Brachycerina z.B. Aedes spp., Anopheles spp., Culex spp., Simulium spp., Eusimulium spp., Phlebotomus spp., Lutzomyia spp., Culicoides spp., Chrysops spp., Odagmia spp., Wilhelmia spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp., Rhinoestrus spp., Tipula spp.; spezielle Beispiele sind: Aedes aegypti, Aedes albopictus, Aedes taeniorhynchus, Anopheles gambiae, Anopheles maculipennis, Calliphora erythrocephala, Chrysozona pluvialis, Culex quinquefasciatus, Culex pipiens, Culex tarsalis, Fannia canicularis, Sarcophaga carnaria, Stomoxys calcitrans, Tipula paludosa, Lucilia cuprina, Lucilia sericata, Simulium reptans, Phlebotomus papatasi, Phlebotomus longipalpis, Odagmia ornata, Wilhelmia equina, Boophthora erythrocephala, Tabanus bromius, Tabanus spodopterus, Tabanus atratus, Tabanus sudeticus, Hybomitra ciurea, Chrysops caecutiens, Chrysops relictus, Haematopota pluvialis, Haematopota italica, Musca autumnalis, Musca domestica, Haematobia irritans irritans, Haematobia irritans exigua, Haematobia stimulans, Hydrotaea irritans, Hydrotaea albipuncta, Chrysomya chloropyga, Chrysomya bezziana, Oestrus ovis, Hypoderma bovis, Hypoderma lineatum, Przhevalskiana silenus, Dermatobia hominis, Melophagus ovinus, Lipoptena capreoli, Lipoptena cervi, Hippobosca variegata, Hippobosca equina, Gasterophilus intestinalis, Gasterophilus haemorroidalis, Gasterophilus inermis, Gasterophilus nasalis, Gasterophilus nigricomis, Gasterophilus pecorum, Braula coeca;
Aus der Ordnung der Siphonapterida z.B. Pulex spp., Ctenocephalides spp., Tunga spp., Xenopsylla spp., Ceratophyllus spp.; spezielle Beispiele sind: Ctenocephalides canis, Ctenocephalides felis, Pulex irritans, Tunga penetrans, Xenopsylla cheopis;

Aus der Ordnung der Heteropterida z.B. Cimex spp., Triatoma spp., Rhodnius spp., Panstrongylus spp.

Aus der Ordnung der Blattarida z.B. Blatta orientalis, Periplaneta americana, Blattela germanica, Supella spp. (z.B. Suppella longipalpa);
Aus der Unterklasse der Acari (Acarina) und den Ordnungen der Meta- und Mesostigmata z.B. Argas spp., Ornithodorus spp., Otobius spp., Ixodes spp., Amblyomma spp., Rhipicephalus (Boophilus) spp., Dermacentor spp., Haemophysalis spp., Hyalomma spp., Dermanyssus spp., Rhipicephalus spp. (der ursprünglichen Gattung der Mehrwirtszecken), Ornithonyssus spp., Pneumonyssus spp., Raillietia spp., Pneumonyssus spp., Sternostoma spp., Varroa spp., Acarapis spp.; spezielle Beispiele sind: Argas persicus, Argas reflexus, Ornithodorus moubata, Otobius megnini, Rhipicephalus (Boophilus) microplus, Rhipicephalus (Boophilus) decoloratus, Rhipicephalus (Boophilus) annulatus, Rhipicephalus (Boophilus) calceratus, Hyalomma anatolicum, Hyalomma aegypticum, Hyalomma marginatum, Hyalomma transiens, Rhipicephalus evertsi, Ixodes ricinus, Ixodes hexagonus, Ixodes canisuga, Ixodes pilosus, Ixodes rubicundus, Ixodes scapularis, Ixodes holocyclus, Haemaphysalis concinna, Haemaphysalis punctata, Haemaphysalis cinnabarina, Haemaphysalis otophila, Haemaphysalis leachi, Haemaphysalis longicorni, Dermacentor marginatus, Dermacentor reticulatus, Dermacentor pictus, Dermacentor albipictus, Dermacentor andersoni, Dermacentor variabilis, Hyalomma mauritanicum, Rhipicephalus sanguineus, Rhipicephalus bursa, Rhipicephalus appendiculatus, Rhipicephalus capensis, Rhipicephalus turanicus, Rhipicephalus zambeziensis, Amblyomma americanum, Amblyomma variegatum, Amblyomma maculatum, Amblyomma hebraeum, Amblyomma cajennense, Dermanyssus gallinae, Ornithonyssus bursa, Ornithonyssus sylviarum, Varroa jacobsoni;
Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp.; spezielle Beispiele sind: Cheyletiella yasguri, Cheyletiella blakei, Demodex canis, Demodex bovis, Demodex ovis, Demodex caprae, Demodex equi, Demodex caballi, Demodex suis, Neotrombicula autumnalis, Neotrombicula desaleri, Neoschöngastia xerothermobia, Trombicula akamushi, Otodectes cynotis, Notoedres cati, Sarcoptis canis, Sarcoptes bovis, Sarcoptes ovis, Sarcoptes rupicaprae (=S. caprae), Sarcoptes equi, Sarcoptes suis, Psoroptes ovis, Psoroptes cuniculi, Psoroptes equi, Chorioptes bovis, Psoergates ovis, Pneumonyssoidic mange, Pneumonyssoides caninum, Acarapis woodi.

Die erfindungsgemäßen Wirkstoffe eignen sich auch zur Bekämpfung von Arthropoden, Helminthen und Protozoen, die Tiere befallen. Zu den Tieren zählen landwirtschaftliche Nutztiere wie z.B. Rinder, Schafe, Ziegen, Pferde, Schweine, Esel, Kamele, Büffel, Kaninchen, Hühner, Puten, Enten, Gänse, Zuchtfische, Honigbienen. Zu den Tieren zählen außerdem Haustiere - die auch als Heimtiere bezeichnet werden - wie z.B. Hunde, Katzen, Stubenvögel, Aquarienfische sowie sogenannte Versuchstiere wie z.B. Hamster, Meerschweinchen, Ratten und Mäuse.

Durch die Bekämpfung dieser Arthropoden, Helminthen und/oder Protozoen sollen Todesfälle vermindert und die Leistung (bei Fleisch, Milch, Wolle, Häuten, Eiern, Honig usw.) und die Gesundheit des Wirtstieres verbessert werden, so dass durch den Einsatz der erfindungsgemäßen Wirkstoffe eine wirtschaftlichere und einfachere Tierhaltung möglich ist.

So ist es beispielsweise wünschenswert, die Aufnahme von Blut des Wirts durch die Parasiten (falls zutreffend) zu verhindern oder zu unterbrechen. Eine Bekämpfung der Parasiten kann außerdem dazu beitragen, die Übertragung infektiöser Substanzen zu verhindern.

Der Begriff "Bekämpfung", so wie er hier bezogen auf den Bereich Tiergesundheit verwendet wird, bedeutet, dass die Wirkstoffe wirken, indem sie das Vorkommen des betreffenden Parasiten in einem mit solchen Parasiten befallenen Tier auf unschädliche Niveaus reduzieren. Genauer gesagt bedeutet "Bekämpfung", wie hier verwendet, dass der Wirkstoff den betreffenden Parasiten tötet, sein Wachstum hemmt oder seine Proliferation inhibiert.

Im allgemeinen können die erfindungsgemäßen Wirkstoffe, wenn sie für die Behandlung von Tieren eingesetzt werden, direkt angewendet werden. Vorzugsweise werden sie als pharmazeutische Zusammensetzungen angewendet, die im Stand der Technik bekannte pharmazeutisch unbedenkliche Exzipienten und/oder Hilfsstoffe enthalten können.

Die Anwendung (= Verabreichung) der Wirkstoffe im Bereich Tiergesundheit und in der Tierhaltung erfolgt in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subkutan, intravenös, intraperitoneal u.a.), Implantate, durch nasale Applikation, durch dermale Applikation in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw. Die Wirkstoffe können als Shampoo oder als geeignete, in Aerosolen oder drucklosen Sprays, z.B. Pumpsprays und Zerstäubersprays, anwendbare, Formulierungen formuliert werden.

Bei der Anwendung für Nutztiere, Geflügel, Haustiere etc. kann man die erfindungsgemäßen Wirkstoffe als Formulierungen (beispielsweise Pulver, Spritzpulver [wettable powders, "WP"], Emulsionen, Emulsionskonzentrate [emulsifiable concentrates ,"EC"], fließfähige Mittel, homogene Lösungen und Suspensionskonzentrate [suspension concentrates, "SC"]), die die Wirkstoffe in einer Menge von 1 bis 80 Gew.-% enthalten, direkt oder nach Verdünnung (z.B. 100- bis 10 000facher Verdünnung) anwenden oder sie als chemisches Bad verwenden.

Beim Einsatz im Bereich Tiergesundheit können die erfindungsgemäßen Wirkstoffe in Kombination mit geeigneten Synergisten oder anderen Wirkstoffen wie beispielsweise Akariziden, Insektiziden, Anthelmintika, Mittel gegen Protozoen, verwendet werden.

Die erfindungsgemäßen Verbindungen können nach üblichen, dem Fachmann bekannten Methoden hergestellt werden.

Im Reaktionsschema 1 ist das allgemeine Darstellungsverfahren A für die erfindungsgemäßen Verbindungen (1-1) abgebildet.

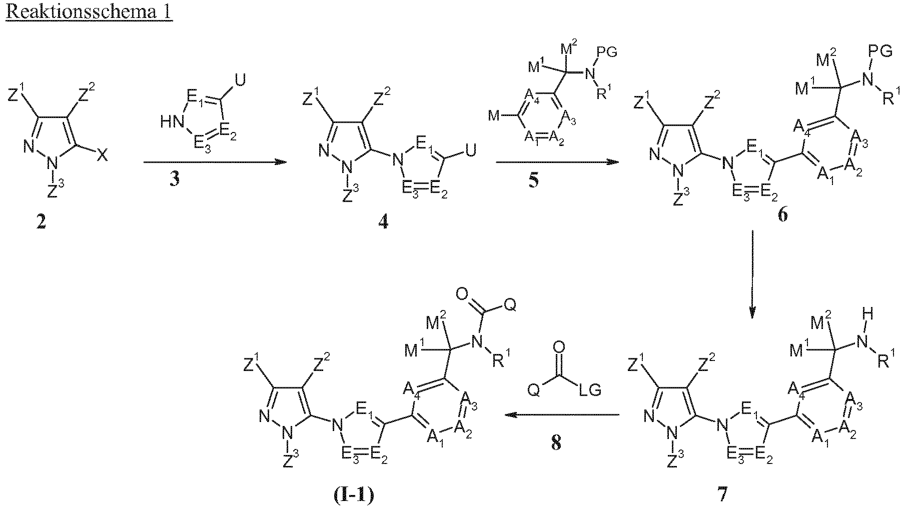

Die Reste A₁-A₄, R¹, M¹, M², Qund Z¹-Z³ haben die oben beschriebenen Bedeutungen. PG steht für eine geeignete Schutzgruppe, z.B. *t*-Butoxycarbonyl. LG steht für ein Fluchtgruppe, z.B. Chlor. Die fünfgliedrigen Zyklen aus E1-E3, Kohlenstoff und Stickstoff stehen für die unter T definierten 5 gliedrigen Heterozyklen. X steht für ein Halogen, z.B. Fluor. U steht für Brom, Iod oder Triflat, wenn M für eine Boronsäure, Boronsäureester oder Trifluorboronat steht. U steht für eine Boronsäure, Boronsäureester oder Trifluorboronat, wenn M für Brom, Iod oder Triflat steht.

Erfindungsgemäße Verbindungen der allgemeinen Struktur **(1-1)** können nach literaturbekannten Verfahren mittels Umsetzung von Intermediat **7** mit Acylierungsreagenzien der allgemeinen Struktur **8** dargestellt werden [WO2010-051926; WO2010-133312]. Intermediate der allgemeinen Struktur **7** können aus N-geschützten Derivaten der allgemeinen Struktur **6** hergestellt werden. Verbindungen der allgemeinen Struktur **6** können mittels Palladium katalysierten Reaktionen aus den Reaktionspartnern **4** und **5** hergestellt werden [WO2005-040110; WO2009-089508]. Die Verbindungen der allgemeinen Struktur **5** sind entweder kommerziell erhältlich oder können nach dem Fachmann bekannten Verfahren hergestellt werden. Die Verbindungen der allgemeinen Struktur **4** lassen sich nach literaturbekannten Verfahren entweder durch eine nucleophile Substitution am Aromaten (X = Chlor oder Fluor) [WO2007-107470; Tetrahedron Letters 2003, 44, 7629-7632] oder durch eine Übergangsmetall katalysierte Reaktion (X = Brom oder Iod) [WO2012-003405; WO2009-158371] aus den entsprechenden Ausgangsmaterialien **2** und **3** herstellen.

Alternativ können die erfindungsgemäßen Verbindungen **(1-1-1)** durch das allgemeine Darstellungsverfahren B (Reaktionsschema 2) dargestellt werden.

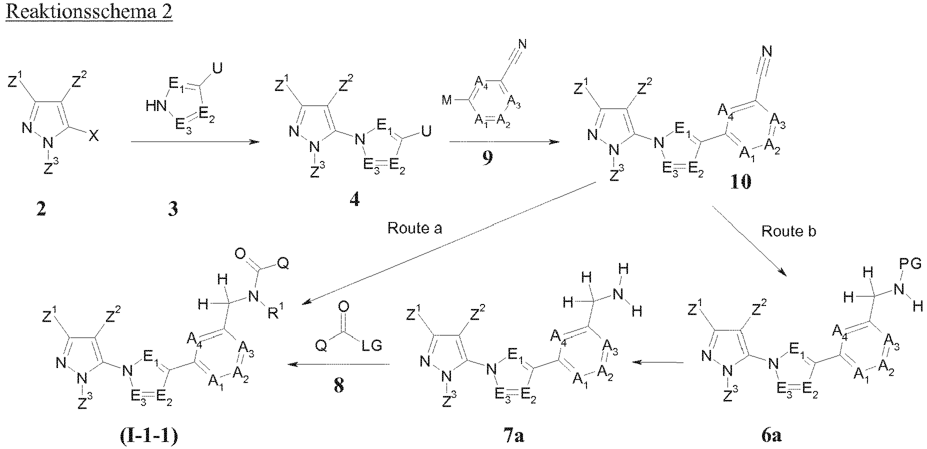

Die Reste A₁-A₄, R¹, Q und Z¹-Z³ haben die oben beschriebenen Bedeutungen. PG steht für eine geeignete Schutzgruppe, z.B. *t*-Butoxycarbonyl. LG steht für ein Fluchtgruppe, z.B. Chlor. Die fünfgliedrigen Zyklen aus E1-E3, Kohlenstoff und Stickstoff stehen für die unter T definierten 5 gliedrigen Heterozyklen. X steht für ein Halogen, z.B. Fluor. U steht für Brom, Iod oder Triflat, wenn M für eine Boronsäure, Boronsäureester oder Trifluorboronat steht. U steht für eine Boronsäure, Boronsäureester oder Trifluorboronat, wenn M für Brom, Iod oder Triflat steht.

Erfindungsgemäße Verbindungen der allgemeinen Struktur **(1-1-1)** können in Analogie zu literaturbekannten Peptidkupplungsmethoden aus den Ausgangsmaterialien **8** und **7a** hergestellt werden [WO2010-051926; WO2010-133312]. Alternativ können die erfindungsgemäßen Verbindungen der allgemeinen Struktur **(1-1-1)** auch direkt aus Verbindungen der allgemeinen Struktur **10** nach literaturbekannten Verfahren hergestellt werden [Tetrahedron Letters 2000, 41(18), 3513-3516; Journal of the American Chemical Society 1925, 47, 3051-7]. Verbindungen der allgemeinen Struktur **7a** lassen sich in Abhängigkeit der verwendeten Schutzgruppe durch geeignete Entschützung der Aminfunktion aus Verbindungen der allgemeinen Struktur **6a** herstellen [ Greene's protective groups in organic synthesis, 4. Edition, P. G. M. Wuts, T. W. Greene, John Wiley & Sons, Inc., Hoboken, New Jersey]. Verbindungen der allgemeinen Struktur **6a** können in Analgie zu literaturbekannten Verfahren aus Verbindungen der allgemeinen Struktur **10** dargestellt werden [Tetrahedron 2003, 59(29), 5417-5423; Journal of Medicinal Chemistry 2013, 56(5), 1946-1960]. Verbindungen der allgemeinen Struktur **10** können in Analogie zu der oben beschriebenen Synthese von 6 dargestellt werden. Die Darstellung der Verbindungen der allgemeinen Struktur **4** ist bereits im Darstellungsverfahren auggeführt.

Erfindungsgemäße Verbindungen der allgemeinen Struktur **(1-2)** lassen sich nach dem in Reaktionsschema 3 dargestellten Darstellungsverfahren C synthetisieren.

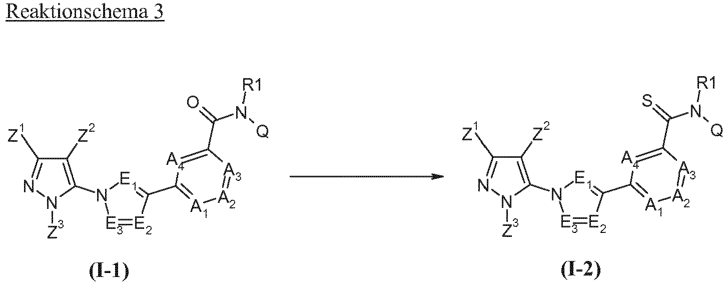

Die Reste A₁-A₄, Q, R¹ und Z¹-Z³ haben die oben beschriebenen Bedeutungen. Die fünfgliedrigen Zyklen aus E1-E3, Kohlenstoff und Stickstoff stehen für die unter T definierten 5 gliedrigen Heterozyklen.
Erfindungsgemäße Strukturen der allgemeinen Struktur **(1-2)** lassen sich in Analogie zu literaturbekannten Verfahren aus Verbindungen der allgemeinen Struktur **(1-1)** darstellen [WO2012-056372; WO2003-066050].

Erfindungsgemäße Verbindungen der allgemeinen Struktur (**II**) lassen sich nach dem in Reaktionsschema 4 dargestellten Darstellungsverfahren D synthetisieren.

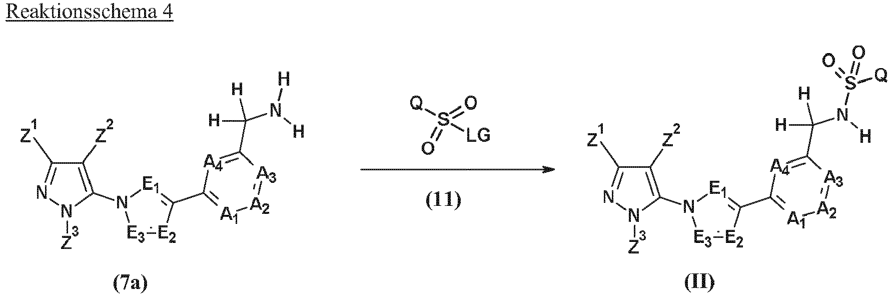

Die Reste A₁-A₄, R¹, Q und Z¹-Z³ haben die oben beschriebenen Bedeutungen. LG steht für eine Fluchtgruppe, z.B. Chlor. Die fünfgliedrigen Zyklen aus E1-E3, Kohlenstoff und Stickstoff stehen für die unter T definierten 5 gliedrigen Heterozyklen.

Erfindungsgemäße Verbindungen der allgemeinen Struktur II können in Analogie zu literaturbekannten Verfahren aus Verbindungen der allgemeinen Strutkur 7a und 11 hergestellt werden [US2007/0117858; WO2013/068462; US2009/0233878]

Die Verbindungen der allgemeinen Struktur **5** sind entweder kommerziell erhältlich oder können nach dem Fachmann bekannten Verfahren bzw. in Analogie zu diesen Verfahren hergestellt werden [WO2012004217; WO2009-130475; WO2008-107125; WO2003-099805; WO2012-0225061; WO2009-010488].

Die Verbindungen der allgemeinen Struktur **2** sind entweder kommerziell erhältlich oder können nach dem Fachmann bekannten Verfahren bzw. in Analogie zu diesen Verfahren hergestellt werden [WO2010-051926; WO2011-131615; WO2006-018725; WO2012-065932; WO2007077961; US2012-0115903; WO2010-017902; WO2010-127856; Tetrahedron Letters 2011, 44, 8451-8457]

Die Verbindungen der allgemeinen Struktur **3** sind entweder kommerziell erhältlich oder können nach dem Fachmann bekannten Verfahren bzw. in Analogie zu diesen Verfahren hergestellt werden.

Oxidationsmittel für die Oxidation alkoholischer Gruppen sind bekannt (vgl. z. B. Oxidationsreagenzien in Organic Synthesis by Oxidation with Metal Compounds, Mijs, de Jonge, Plenum Verlag, New York, 1986; Manganese Compounds as Oxidizing Agens in Organic Chemistry, Arndt, Open Court Publishing Company, La Salle, IL, 1981; The Oxidation of Organic Compounds by Permanganate Ion and Hexavalent Chromium, Lee, Open Court Publishing Company, La Salle, IL, 1980). Eine Oxidation kann beispielsweise in Gegenwart von Permanganaten (z.B. Kaliumpermanganat), Metalloxiden (z.B. Mangandioxid, Chromoxide die beispielsweise in Dipyridin-chrom(VI)-oxid als Collins Reagenz (vgl. J. C. Collins et al., Tetrahedron Lett. 30, 3363-3366, 1968) verwendet werden) durchgeführt werden. Ebenfalls in Gegenwart von Pyridiniumchlorochromat (z.B. Corey's Reagenz) (vgl. auch R. O. Hutchins et al., Tetrahedron Lett. 48, 4167-4170, 1977; D. Landini et al. Synthesis 134-136, 1979) oder Ruthenium-tetroxid (vgl. S.-I. Murahashi, N. Komiya Ruthenium-catalyzed Oxidation of Alkenes, Alcohols, Amines, Amides, β-Lactams, Phenols and Hydrocarbons, in: Modern Oxidation Methods, Baeckvall, Jan-Erling (Eds.), Wiley-VCH-Verlag Gmbh & Co. KGaA, 2004). Ebenfalls geeignet sind Ultraschall-induzierte Oxidationsreaktionen, sowie die Verwendung von Kaliumpermanganat (vgl. J. Yamawaki et al., Chem. Lett. 3, 379-380, 1983).

Zur Deblockierung/Abspaltung der Schutzgruppe SG können alle bekannten geeigneten sauren oder basischen Reaktionshilfsmittel nach den in der Literatur beschriebenen Verfahrensweises verwendet werden. Bei Verwendung von Schutzgruppen für Aminogruppen des Carbamat-Typs werden bevorzugt saure Reaktionshilfsmittel verwendet. Bei Verwendung der t-Butylcarbamat-Schutzgruppe (BOC-Gruppe) werden beispielsweise Mischungen von Mineralsäuren wie Salzsäure, Bromwasserstoffsäure, Salpetersäure, Schwefelsäure, Phosphorsäure oder organischen Säuren wie Benzoesäure, Ameisensäure, Essigsäure, Trifluoressigsäure, Methansulfonsäure, Benzolsulfonsäure oder Toluolsulfonsäure und einem geeigneten Verdünnungsmittel wie Wasser und/oder einem organischen Lösungsmittel wie Tetrahydrofuran, Dioxan, Dichlormethan, Chloroform, Essigester, Ethanol oder Methanol verwendet. Bevorzugt sind Mischungen von Salzsäure oder Essigsäure mit Wasser und/oder einem organischen Lösungsmittel wie Essigester.

Es ist bekannt, dass manche Reaktionen und Herstellungsverfahren besonders gut in Gegenwart von Verdünnungs- bzw. Lösungsmittel und basischer oder saurer Reaktionshilfsmitteln durchführbar sind. Mischungungen der Verdünnungs- bzw. Lösungsmittel sind ebenfalls einsetzbar. Die Verdünnungs- bzw. Lösungsmittel werden vorteilhafterweise in einer solchen Menge eingesetzt, dass das Reaktionsgemisch während des ganzen Verfahrens gut rührbar ist.

Als Verdünnungs- bzw. Lösungsmittel zur Durchführung der erfindungsgemäßen Verfahren kommen grundsätzlich alle unter den spezifischen Reaktionsbedingungen inerten organischen Lösungsmittel in Frage. Als Beispiele sind zu nennen: Halogenkohlenwasserstoffe (z.B. Chlorkohlenwasserstoffe, wie Tetraethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, Chlortoluol, Trichlorbenzol), Alkohole (z.B. Methanol, Ethanol, Isopropanol, Butanol), Ether (z.B. Ethylpropylether, Methyl-t-butylether, n-Butylether, Anisol, Phenetol, Cyclohexylmethylether, Dimethylether, Diethylether, Diproplether, Diisopropylether, Di-n-butylether, Diisobutylether, Diisoamylether, Ethylenglycoldimethylether, Tetrahydrofuran, Dioxan, Dichlordiethylether und Polyether des Ethylenoxids und/oder Propylenoxids), Amine (z.B. Trimethyl-, Triethyl-, Tripropyl-, Tributylamin, N-Methylmorpholin, Pyridin und Tetramethylendiamin), Nitrokohlenwasserstoffe (z.B. Nitromethan, Nitroethan, Nitropropan, Nitrobenzol, Chlornitrobenzol, o-Nitrotoluol; Nitrile wie Acetonitril, Propionitril, Butyronitril, Isobutyronitril, Benzonitril, m-Chlorbenzonitril), Tetrahydrothiophendioxid, Dimethylsulfoxid, Tetramethylensulfoxid, Dipropylsulfoxid, Benzylmethylsulfoxid, Diisobutylsulfoxid, Dibutylsulfoxid, Diisoamylsulfoxid, Sulfone (z.B. Dimethyl-, Diethyl-, Dipropyl-, Dibutyl-, Diphenyl-, Dihexyl-, Methylethyl-, Ethylpropyl-, Ethylisobutyl- und Pentamethylensulfon), aliphatische, cycloaliphatische oder aromatische Kohlenwasserstoffe (z.B. Pentan, Hexan, Heptan, Oktan, Nonan und technische Kohlenwasserstoffe), ferner sogenannte "White Spirits" mit Komponenten mit Siedepunkten im Bereich von beispielsweise 40°C bis 250°C, Cymol, Benzinfraktionen innerhalb eines Siedeinterwalles von 70°C bis 190°C, Cyclohexan, Methylcyclohexan, Petrolether, Ligroin, Octan, Benzol, Toluol, Chlorbenzol, Brombenzol, Nitrobenzol, Xylol, Ester (z.B. Methyl-, Ethyl-, Butyl-, Isobutylacetat, Dimethyl-, Dibutyl-, Ethylencarbonat); Amide (z.B. Hexamethylenphosphorsäuretriamid, Formamid, N-Methyl-formamid, *N*,*N*-Dimethyl-formamid, *N*,*N*-Dipropyl-formamid, *N*,*N*-Dibutyl-formamid, N-Methyl-pyrrolidin, N-Methyl-caprolactam, 1,3-Dimethyl-3,4,5,6-tetrahydro-2(1H)-pyrimidin, Octylpyrrolidon, Octylcaprolactam, 1,3-Dimethyl-2-imidazolindion, N-Formyl-piperidin, *N*,*N*-1,4-Diformyl-piperazin) und Ketone (z.B. Aceton, Acetophenon, Methylethylketon, Methylbutylketon).

Als basische Reaktionshilfsmittel zur Durchführung der erfindungsgemäßen Verfahren können alle geeigneten Säurebindemittel eingesetzt werden. Als Beispiele sind zu nennen: Erdalkali- oder Alkalimetallverbindungen (z.B. Hydroxide, Hydride, Oxide und Carbonate des Lithiums, Na-triums, Kaliums, Magnesiums, Calciums und Bariums), Amidinbasen oder Guanidinbasen (z.B. 7-Methyl-1,5,7-triaza-bicyclo(4.4.0)dec-5-en (MTBD); Diazabicyclo(4.3.0)nonen (DBN), Diazabicyclo(2.2.2)octan (DABCO), 1,8-Diazabicyclo(5.4.0)undecen (DBU), Cyclohexyltetrabutyl-guanidin (CyTBG), Cyclohexyltetramethylguanidin (CyTMG), *N*,*N*,*N*,*N*-Tetramethyl-1,8-naphthalindiamin, Pentamethylpiperidin) und Amine, insbesondere tertiäre Amine, (z.B. Triethylamin, Trimethylamin, Tribenzylamin, Triisopropylamin, Tributylamin, Tricyclohexylamin, Triamylamin, Trihexylamin, *N*,*N-*Dimethylanilin, *N*,*N*-Dimethyl-toluidin, *N*,*N*-Dimethyl-p-aminopyridin, N-Methyl-pyrrolidin, N-Methylpiperidin, N-Methyl-imidazol, N-Methyl-pyrazol, N-Methyl-morpholin, N-Methylhexamethylendiamin, Pyridin, 4-Pyrrolidinopyridin, 4-Dimethylamino-pyridin, Chinolin, α-Picolin, β-Picolin, Isochinolin, Pyrimidin, Acridin, *N*,*N*,N',N'-Tetramethylendiamin, *N,N*,N'*,*N'-Tetraethylendiamin, Chinoxalin, N-Propyl-diisopropylamin, N-Ethyl-diisopropylamin, *N*,*N*'-Dimethylcyclohexylamin, 2,6-Lutidin, 2,4-Lutidin oder Triethyldiamin).

Als saure Reaktionshilfsmittel zur Durchführung der erfindungsgemäßen Verfahren können alle Mineralsäuren (z.B. Halogenwasserstoff-säuren wie Fluorwasserstoffsäure, Chlorwasserstoffsäure, Bromwasserstoffsäure oder Iodwasserstoffsäure sowie Schwefelsäure, Phosphorsäure, Phosphorige Säure, Salpetersäure), Lewis Säuren (z.B. Aluminium(III)-chlorid, Bortrifluorid oder sein Etherat, Titan-(V)chlorid, Zinn(V)-chlorid, und organische Säuren (z.B. Ameisensäure, Essigsäure, Propionsäure, Malonsäure, Milchsäure, Oxalsäure, Fumarsäure, Adipinsäure, Stearinsäure, Weinsäure, Ölsäure, Methansulfonsäure, Benzoesäure, Benzolsulfonsäure oder para-Toluolsulfonsäure eingesetzt werden.

Sofern in den Reaktionsschemata Schutzgruppen vorgesehen sind, können alle allgemein bekannten Schutzgruppen verwendet werden. Insbesondere solche, die von Greene T. W., Wuts P. G. W. in Protective Groups in Organic Synthesis; John Wiley & Sons, Inc. 1999, "Protection for the hydroxyl group including 1,2- and 1,3-diols" beschrieben sind.

### Weiterhin eignen sich auch Schutzgruppen

vom Typ eines substituierten Methylethers (z.B. Methoxymethylether (MOM), Methylthiomethylether (MTM), (Phenyl-dimethylsilyl)methoxymethylether (SNOM-OR), Benzyloxymethylether (BOM-OR) para-Methoxybenzyloxymethylether (PMBM-OR), para-Nitrobenzyloxymethyl-ether, ortho-Nitrobenzyloxymethylether (NBOM-OR), (4-Methoxyphenoxy)-methylether (p-AOM-OR), Guaiacolmethylether (GUM-OR), t-Butoxymethylether, 4-Pentyloxy-methylether (POM-OR), Silyloxymethylether, 2-Methoxy-ethoxy-methylether (MEM-OR), 2,2,2-Trichlorethoxymethylether, Bis(2-chlorethoxy)-methylether, 2-(Trimethyl-silyl)ethoxymethylether (SEM-OR), Methoxymethylether (MM-OR));
vom Typ eines substituierten Ethylethers (z.B. 1-Ethoxyethylether (EE-OR), 1-(2-Chlorethoxy)ethylether (CEE-OR), 1-[2-(Trimethylsilyl)ethoxy]ethylether (SEE-OR), 1-Methyl-1-methoxyethylether (MIP-OR), 1-Methyl-1-benzyloxyethylether (MBE-OR), 1-Methyl-1-benzyloxy-2-fluor-ethylether (MIP-OR), 1-Methyl-1-phenoxyethylether, 2,2,-Trichlorethylether, 1,1-Dianisyl-2,2,2-trichlorethylether (DATE-OR), 1,1,1,3,3,3-Hexafluor-2-phenylisopropylether (HIP-OR), 2-Trimethylsilylethylether, 2-(Benzylthio)ethylether, 2-(Phenylselenyl)ethylether), eines Ethers (z.B. Tetrahydropyranylether (THP-OR), 3-Brom-tetrahydropyranylether (3-BrTHP-OR), Tetrahydrothiopyranylether, 1-Methoxy-cyclohexylether, 2- und 4-Picolylether, 3-Methyl-2-picolyl-N-oxido-ether, 2-Quinolinylmethylether (Qm-OR), 1-Pyrenylmethylether, Dipenylmethylether (DPM-OR), para, para'-Dinitrobenzhydrylether (DNB-OR), 5-Dibenzosuberylether, Triphenylmethylether (Tr-OR), alpha-Naphthyldiphenylmethylether, para-Methoxy-phenyldiphenylmethylether (MMTrOR), Di(para-methoxy-phenyl)phenylmethylether (DMTr-OR), Tri(para-methoxy-phenyl)phenylmethylether (TMTr-OR), 4-(4'-Brom-phenacyloxy) phenyldiphenylmethylether, 4,4',4"-Tris(4,5-dichlorphthalimido-phenyl)methylether (CPTr-OR), 4,4',4"-Tris(benzoyloxyphenyl)-methylether (TBTr-OR), 4,4'-Dimethoxy-3"-[N-(imidazolylmethyl)]-tritylether (IDTr-OR), 4,4'-Dimethoxy-3"-[N-(imidazolylethyl)carbamoyl]tritylether (IETr-OR), 1,1-Bis(4-methoxy-phenyl)-1'-pyrenyl-methylether (Bmpm-OR), 9-Anthrylether, 9-(9-Phenyl)xanthenylether (Pixyl-OR), 9-(9-Phenyl-10-oxo)anthryl (Tritylon-Ether), 4-Methoxy-tetrahydropyranylether (MTHP-OR), 4-Methoxy-tetrahydrothiopyranylether, 4-Methoxy-tetrahydrothiopyranyl-S,S-dioxid, 1-[(2-Chlor-4-methyl)phenyl]-4-methoxypiperidin-4-yl-ether (CTMP-OR), 1-(2-Fluorphenyl)-4-methoxy-piperidin-4-yl-ether (Fpmp-OR), 1,4-Dioxan-2-yl-ether, Tetrahydrofuranylether, Tetrahydrothiofuranylether, 2,3,3a,4,5,6,7,7a-Octahydro-7,8,8-trimethyl-4,7-methanbenzofuran-2-yl-ether (MBF-OR), t-Butylether, Allylether, Propargylether, para-Chlorphenylether, para-Methoxy-phenylether, para-Nitro-phenylether, para-2,4-Dinitro-phenylether (DNP-OR), 2,3,5,6-Tetrafluor-4-(trifluormethyl)phenylether, Benzylether (Bn-OR));
vom Typ eines sustituierten Benzylethers (z.B. para-Methoxy-benzylether (MPM-OR), 3,4-Dimethoxybenzylether (DMPM-OR), ortho-Nitro-benzylether, para-Nitro-benzylether, para-Halo-benzylether, 2,6-Dichlor-benzylether, para-Aminoacyl-benzylether (PAB-OR), para-Azido-benzylether (Azb-OR), 4-Azido-3-chlor-benzylether, 2-Trifluormethyl-benzylether, para-(Methylsulfinyl)benzylether (Msib-OR));
vom Typ eines Silylethers (z.B. Trimethylsilylether (TMS-OR), Triethylsilylether (TES-OR), Triisopropylsilylether (TIPS-OR), Dimethylisopropylsilylether (IPDMS-OR), Diethylisopropylsilylether (DEIPS-OR), Dimethylhexylsilylether (TDS-OR), t-Butyldimethylsilylether (TBDMS-OR), t-Butyldiphenylsilylether(TBDPS-OR), Tribenzylsilylether, Tri-para-xylylsilylether, Triphenylsilylether (TPS-OR), Diphenylmethylsilylether (DPMS-OR), Di-t-butylmethylsilylether (DTBMS-OR), Tris(trimethylsilyl)silylether (Sisylether), Di-t-butylmethylsilylether (DTBMS-OR), Tris(trimethylsilyl)silylether (Sisylether), (2-Hydroxystyryl)-dimethylsilylether (HSDMS-OR), (2-Hydroxystyryl)diisopropylsilylether (HSDIS-OR), t-Butylmethoxyphenyl-silylether (TBMPS-OR), t-Butoxydiphenylsilylether (DPTBOS-OR));
vom Typ eines Esters (z.B. Formiatester, Benzoylformiatester, Acetatester (Ac-OR), Chloracetatester, Dichloracetatester, Trichloracetatester, Trifluoracetatester, (TFA-OR), Methoxyacetatester, Triphenylmethoxyacetatester, Phenoxyacetatester, para-Chlor- phenoxyacetatester, Phenylacetatester, Diphenylacetatester (DPA-OR), Nicotinatester, 3-Phenyl-propionatester, 4-Pentoatester, 4-Oxopentoatester (Levulinate) (Lev-OR) 4,4-(Ethylendithio)-pentanoatester (LevS-OR), 5-[3-Bis(4-methoxyphenyl)hydroxy-methoxyphenoxy]-levulinatester, Pivaloatester (Pv-OR), 1-Adamantanoatester, Crotonatester, 4-Methoxy-crotonatester, Benzoatester (Bz-OR), para-Phenyl-benzoatester, 2,4,6-Trimethyl-benzoatester (Mesitoate), 4-(Methylthiomethoxy)-butyratester (MTMB-OR), 2-(Methylthiomethoxymethyl)-benzoatester (MTMT-OR),
vom Typ eines Esters (z.B. Methylcarbonat, Methoxymethylcarbonat, 9-Fluorenylmethylcarbonat (Fmoc-OR), Ethylcarbonat, 2,2,2-Trichlorethylcarbonat (Troc-OR), 1,1-Dimethyl-2,2,2-trichlorethylcarbonat (TCBOC-OR), 2-(Trimethylsilyl)ethylcarbonat (TMS-OR), 2-(Phenylsulfonyl)-ethylcarbonat (Ps-OR), 2-(Triphenylphosphonio)-ethylcarbonat (Peoc-OR), t-Butylcarbonat (Boc-OR), Isobutylcarbonat, Vinylcarbonat, Allylcarbonat (Alloc-OR), para-Nitro-phenylcarbonat, Benzylcarbonat (Z-OR), para-Methoxy-benzylcarbonat, 3,4-Dimethoxy-benzylcarbonat, ortho-Nitro-benzylcarbonat, para-Nitro-benzylcarbonat, 2-Dansylethylcarbonat (Dnseoc-OR), 2-(4-Nitrophenyl)ethylcarbonat (Npeoc-OR), 2-(2,4-Dinitrophenyl)ethylcarbonat (Dnpeoc)), und
vom Typ eines Sulfats (z.B. Allylsulfonat (Als-OR), Methansulfonat (Ms-OR), Benzylsulfonat, Tosylat (Ts-OR), 2-[(4-Nitrophenyl)ethyl]sulfonat (Npes-OR)).

Als Katalysatoren zur Durchführung einer katalytischen Hydrierung im erfindungsgemäßen Verfahren sind alle üblichen Hydrierkatalysatoren, wie beispielsweise Platin-Katalysatoren (z.B. Platin-Platte, Platin-Schwamm, Platin-Schwarz, kolloidales Platin, Platinoxid, Platindraht), Palladium-Katalysatoren (z.B. Palladium-Schwamm, Palladium-Schwarz, Palladiumoxid, Palladium-Kohle, kolloidales Palladium, Palladium-Bariumsulfat, Palladium-Bariumcarbonat, Palladium-Hydroxid , NickelKatalysatoren (z.B. reduziertes Nickel, Nickeloxid, Raney-Nickel), Ruthenium-Katalysatoren, Cobalt-Katalysatoren (z.B. reduziertes Cobalt, Raney-Cobalt), Kupfer-Katalysatoren (z.B. reduziertes Kupfer, Raney-Kupfer, Ullmann-Kupfer) geeignet. Bevorzugt werden Edelmetalllkatalysatoren (z.B. Platin- und Palladium- oder Ruthenium-Katalysatoren) verwendet, die gegebenenfalls auf einem geeigneten Träger (z.B. Kohlenstoff oder Silizium) aufgebraucht sind, Rhodium-Katalysatoren (z.B. Tris(triphenylphosphin)rhodium(I)-chlorid in Gegenwart von Triphenylphosphin). Ferner können "chiralen Hydrierkatalysatoren" (z. B. solche die chirale Diphosphinliganden enhalten wie (2S,3S)-(-)-2,3-Bis(diphenylphosphino)-butan [(S,S)-Chiraphos] oder (R)-(+)-2,2'- bzw. (S)-(-)-2,2'-Bis(diphenylphosphino)-1,1'-binaphthalin [R(+)-BINAP bzw. S(-)-BINAP] ) verwendet werden, wodurch der Anteil eines Isomers im Isomerengemisch erhöht wird bzw. das Entstehen eines anderen Isomers nahezu vollständig unterdrückt wird.

Die Herstellung von Salzen der erfindungsgemäßen Verbindungen erfolgt nach Standardverfahren. Repräsentative Säureadditionssalze sind beispielsweise solche die durch Reaktion mit anorganischen Säuren, wie beispielsweise Schwefelsäure, Salzsäure, Bromwasserstoffsäure, Phosphorsäure oder organischen Carbonsäuren wie Essigsäure, Trifluoressigsäure, Zitronensäure, Bernsteinsäure, Buttersäure, Milchsäure, Ameisensäure, Fumarsäure, Maleinsäure, Malonsäure, Camphersäure, Oxalsäure, Phthalsäure, Propionsäure, Glycolsäure, Glutarsäure, Stearinsäure, Salicylsäure, Sorbinsäure, Weinsäure, Zimtsäure, Valeriansäure, Pikrinsäure, Benzoesäure oder organischen Sulfonsäuren wie Methansulfonsäure und 4-Toluolsulfonsäure gebildet werden.

Repräsentativ sind auch Salze von erfindungsgemäßen Verbindungen, die aus organischen Basen, wie beispielsweise Pyridin oder Triethylamine gebildet werden oder solche, die aus anorganischen Basen, wie beispielsweise Hydride, Hydroxide oder Karbonate des Natriums, Lithiums, Calciums, Magnesiums oder Bariums, gebildet werden, wenn die Verbindungen der allgemeinen Formel (I) ein zu dieser Salzbildung geeignetes Strukturelement aufweist.

Synthesemethoden zur Darstellung heterocyclischer N-Oxide und t-Aminen sind bekannt. Sie können mit Peroxysäuren (z.B. Peressigsäure und meta-Chlor-perbenzoesäure (MCPBA), Wasserstoffperoxid), Alkylhydroperoxide (z.B. t-Butylhydroperoxid), Natriumperborat und Dioxiranen (z.B. Dimethyldioxiran) erhalten werden. Diese Methoden sind beispielsweise von T. L. Gilchrist, in Comprehensive Organic Synthesis, Vol. 7, S. 748-750, 1992, S. V. Ley, (Ed.), Pergamon Press; M. Tisler, B. Stanovnik, in Comprehensive Heterocyclic Chemistry, Vol. 3, S. 18-20, 1984, A. J. Boulton, A. McKillop, (Eds.), Pergamon Press; M. R. Grimmett, B. R. T. Keene in Advances in Heterocyclic Chemistry, Vol. 43, S. 149-163, 1988, A. R. Katritzky, (Ed.), Academic Press; M. Tisler, B. Stanovnik, in Advances in Heterocyclic Chemistry, Vol. 9, S. 285-291, 1968, A. R. Katritzky, A. J. Boulton (Eds.), Academic Press; G. W. H. Cheeseman, E. S. G. Werstiuk in Advances in Heterocyclic Chemistry, Vol. 22, S. 390-392, 1978, A. R. Katritzky, A. J. Boulton, (Eds.), Academic Press beschrieben.

### Experimenteller Teil

### Darstellungsverfahren A

### Herstellung von N-{2-Fluor-5-[5'-(pentafluorethyl)-4'-(trifluormethyl)-2'H-1,3'-bipyrazol-4-yl]benzyl}propanamid (Beispiel Ic-1) und tert-Butyl-{3-[2'-methyl-5'-(pentafluorethyl)-4'-(trifluormethyl)-2'H-1,3'-bipyrazol-4-yl]benzyl}carbamat (Beispiel Ic-18)

Das Reaktionsschema 5 zeigt die Synthese der erfindungsgemäßen Verbindungen (**Ic-18**) und (**Ic-1**).

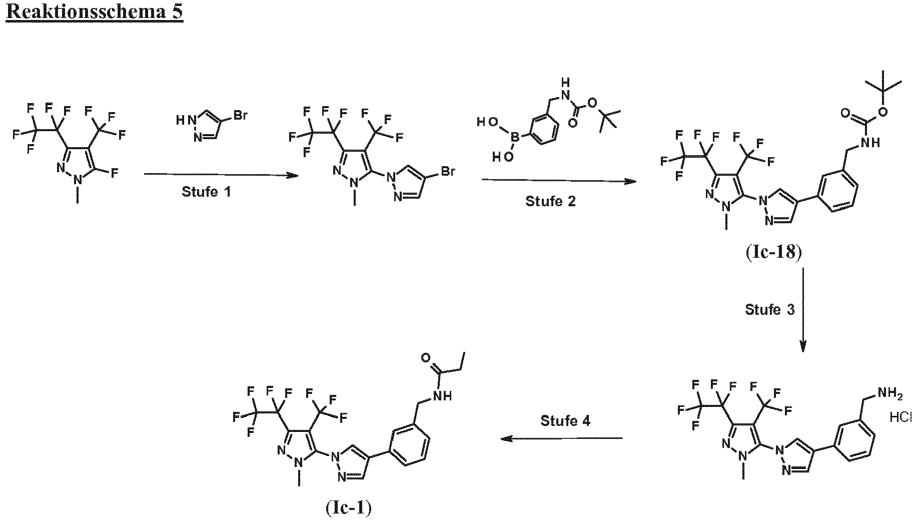

### .Stufe 1: Synthese von 4-Brom-2'-methyl-5'-(pentafluorethyl)-4'-(trifluormethyl)-2'H-1,3'-bipyrazol

16,0 g (55,9 mmol) 5-Fluor-1-methyl-3-(pentafluorethyl)-4-(trifluormethyl)-1H-pyrazol, 8,22 g (55,9 mmol) 4-Brom-1H-pyrazol und 15,5 g (112 mmol) Kaliumcarbonat werden in 250 mL Tetrahydrofuran suspendiert. Die Rekationsmischung wird 16h unter Rückfluß erhitzt..Die festen Reaktionsbestandteile werden abfiltriert und mit Tetrahydrofuran nachgewaschen. Die vereinigten organischen Phasen werden am Rotationsverdampfer unter vermindertem Druck eingeengt.
Man erhält 23,0 g **4-Brom-2'-methyl-5'-(pentafluorethyl)-4'-(trifluormethyl)-2'H-1,3'-bipyrazol** ¹H-NMR (400 MHz, d₆-DMSO): δ = 8,58 (s, 1H); 8,16 (s, 1H); 3,32 (s, 3H);
HPLC-MS: logP^{a)} = 4,17

### Stufe 2: Synthese von tert-Butyl-{3-[2'-methyl-5'-(pentafluorethyl)-4'-(trifluormethyl)-2'H-1,3'-bipyrazol-4-yl]benzyl}carbamat (Ic-18)

500 mg (1,21 mmol) 4-Brom-2'-methyl-5'-(pentafluorethyl)-4'-(trifluormethyl)-2'H-1,3'-bipyrazol, 304 mg (1,21 mmol) (3-{[(tert-Butoxycarbonyl)amino]methyl}phenyl)boronsäure und 69,9 mg (0,06 mmol) Tetrakis(triphenylphosphin)palladium(0) werden in einer Mischung aus 10 mL 2-Propanol und 3,69 mL 1N Natriumhydrogencarbonatlösung gelöst. Die Lösungen wurden vorher gründlich entgast. Die Reaktionsmischung wird unter Schutzgasatmosphäre 16h auf 90°C erhitzt. Das Reaktionsgemisch wird mit Wasser verdünnt und mehrfach mit Chloroform extrahiert. Die vereinigten organischen Phasen werden über Natriumsulfat getrocknet, filtriert un dam Rotationsverdampfer unter vermindertem Druck eingeengt Das Rohprodukt wird an der RP Phase gereinigt.
Man erhält 410 mg **tert-Butyl-{3-[2'-methyl-5'-(pentafluorethyl)-4'-(trifluormethyl)-2'H-1,3'-bipyrazol-4-yl]benzyl}carbamat (Ic-18)**
¹H-NMR (400 MHz, d₆-DMSO): δ = 8,68 (s, 1H); 8,44 (s, 1H); 7,64-7,19 (5H, m); 4,19 (m, 2H); 3,82 (s, 3H); 1,40 (s, 9H)
HPLC-MS: logP^{a)} = 4,74; Masse (m/z) = 540,1 [M+H]⁺.

### Stufe 3: Synthese von 1-{3-[2'-Methyl-5'-(pentafluorethyl)-4'-(trifluormethyl)-2'H-1,3'-bipyrazol-4-yl]phenyl}methanaminhydrochlorid(1:1)

400 mg (0,74 mmol) tert-Butyl-{3-[2'-methyl-5'-(pentafluorethyl)-4'-(trifluormethyl)-2'H-1,3'-bipyrazol-4-yl]benzyl}carbamat werden in 5 mL 4N Chlorwasserstoff in Dioxan gelöst und 1h gerührt. Das Lösungsmittel wird Rotationsverdampfer unter vermindertem Druck entfernt.

Man erhält 410 mg 1-{3-[2'-Methyl-5'-(pentafluorethyl)-4'-(trifluormethyl)-2'H-1,3'-bipyrazol-4-yl]phenyl}methanaminhydrochlorid(1:1)
¹H-NMR (400 MHz, d₆-DMSO): δ = 8,71 (s, 1H); 8,48 (s, 1H); 7,87-7,42 (m, 4H)
HPLC-MS: logP^{a)} = 1,93 , Masse (m/z) = 440,1 [M+H]⁺.

### Stufe 4: Synthese von N-{3-[2'-Methyl-5'-(pentafluorethyl)-4'-(trifluormethyl)-2'H-1,3'-bipyrazol-4-yl]benzyl}propanamid (Ic-1)

133 mg (0,32 mmol) 1-{3-[2'-Methyl-5'-(pentafluorethyl)-4'-(trifluormethyl)-2'H-1,3'-bipyrazol-4-yl]phenyl}methanamin und 0,13 mL N,N-Diethyl-N-isopropylamin werden in 3 mL Chloroform gelöst und dann mit 29 µL (0,35 mmol) Propionylchlorid versetzt. Nach einer Stunde wird die Reaktionsmischung mit 5%-iger Natriumhydrogenphosphatlösung versetzt. Die wässrige Phase wird mehrfach mit Chloroform extrahiert. Die vereinigten organischen Phasen werden über Natriumsulfat getrocknet, filtriert und unter vermindertem Druck am Rotationsverdampfer eingeengt. Das Rohprodukt wird an Kieselgel säulenchromatographisch gereinigt.
Man erhält 40 mg von N-{3-[2'-Methyl-5'-(pentafluorethyl)-4'-(trifluormethyl)-2'H-1,3'-bipyrazol-4-yl]benzyl}propanamid (Ic-1)
¹H-NMR siehe NMR Daten in Peakliste
HPLC-MS: logP^{a)} = 3,59, Masse (m/z) = 496,1 [M+H]⁺.

### Herstellung von N-(3-{1-[1-Methyl-3-(pentafluorethyl)-4-(trifluormethyl)-1H-pyrazol-5-yl]-1H-imidazol-4-yl}benzyl)propanamid (Ib-5) und tert-Butyl-(3-{1-[1-methyl-3-(pentafluorethyl)-4-(trifluormethyl)-1H-pyrazol-5-yl]-1H-imidazol-4-yl}benzyl)carbamat (Ib-1)

Das Reaktionsschema 6 zeigt die Synthese der erfindungsgemäßen Verbindungen (**Ib-5**) und (**Ib-1**)

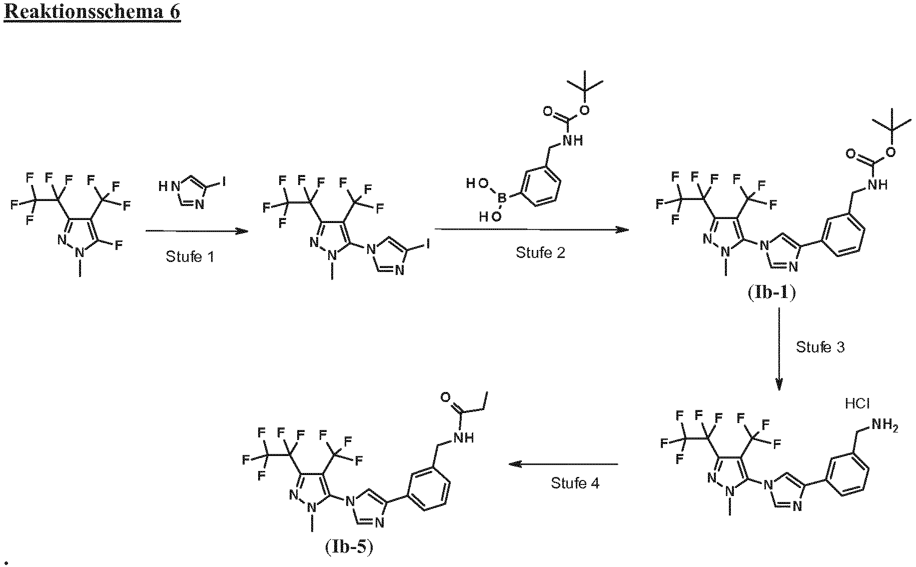

### Stufe 1: Synthese von 5-(4-Iod-1H-imidazol-1-yl)-1-methyl-3-(pentafluorethyl)-4-(trifluormethyl)-1H-pyrazol

7,0 g (24,5 mmol) 5-Fluor-1-methyl-3-(pentafluorethyl)-4-(trifluormethyl)-1H-pyrazol, 4,75 g (24,5 mmol) 4-Iod-1H-imidazol und 6,76 g (48,9 mmol) Kaliumcarbonat werden in 100 mL Tetrahydrofuran suspendiert. Die Reaktionsmischung wurde 16h unter Rückfluß erhitzt. Die festen Bestandteile werden abfiltriert und mit Tetrahydrofuran gewaschen. Das Lösungsmittel wird unter vermindertem Druck am Rotationsverdampfer eingeengt. Das Rohprodukt wird an Kieselgel säulenchromatographisch gereinigt. Man erhält 8,73 g 5-(4-Iod-1H-imidazol-1-yl)-1-methyl-3-(pentafluorethyl)-4-(trifluormethyl)-1H-pyrazol.
¹H-NMR (400 MHz, d₆-DMSO): δ = 8,073 (s, 1H); 7,86 (s, 1H); 3,73 (s, 3H);
HPLC-MS: logP^{a)} = 3,47 Masse (m/z) = 460,8; 461,8 [M+H]⁺.

### Stufe 2: Synthese von tert-Butyl-(3-{1-[1-methyl-3-(pentafluorethyl)-4-(trifluormethyl)-1H-pyrazol-5-yl]-1H-imidazol-4-yl}benzyl)carbamat (Ib-1)

2,00 g (4,35 mmol) 5-(4-Iod-1H-imidazol-1-yl)-1-methyl-3-(pentafluorethyl)-4-(trifluormethyl)-1H-pyrazol, 1,09 g (4,35 mmol) (3-{[(tert-Butoxycarbonyl)amino]methyl}phenyl)boronsäure und 251 mg (0,22 mmol) Tetrakis(triphenylphosphin)palladium(0) werden in einer Mischung aus 40 mL 2-Propanol und 13 mL 1N wässriger Natriumhydrogencarbonatlösung gelöst. Die Lösungsmittel wurden vorher gründlich entgast. Die Reaktionslösung wird 16h unter Schutzgasatmosphäre auf 90°C erhitzt. Nach dem Ende der Reaktion wird das Reaktionsgemisch mit Wasser und Chloroform versetzt. Die organische Phase wird mit Chlorofonn extrahiert. Die vereinigten organischen Phasen werden dann am Rotationsverdampfer unter vermindertem Druck eingeengt. Das Rohprodukt wird dann an der RP Phase gereinigt.
Man erhält 1,43 g tert-Butyl-(3-{1-[1-methyl-3-(pentafluorethyl)-4-(trifluormethyl)-1H-pyrazol-5-yl]-1H-imidazol-4-yl}benzyl)carbamat (Ib-1)
¹H-NMR (400 MHz, d₆-DMSO): δ = 8,17 (s, 1H); 8,06 (s, 1H); 7,72 (s, 1H); 7,65 (d, 1H); 7,55 (t, 1H); 7,45 (t, 1H); 7,25 (d, 1H); 4,17 (d, 2H); 3,78 (s, 3H); 1,40 (s, 9H)
HPLC-MS: logP^{a)} = 4,21; Masse (m/z) = 540,1 [M+H]⁺.

### Stufe 3: Synthese von 1-(3-{1-[1-Methyl-3-(pentafluorethyl)-4-(trifluormethyl)-1H-pyrazol-5-yl]-1H-imidazol-4-yl}phenyl)methanaminhydrochlorid

600 mg (1,11 mmol) tert-Butyl-(3-{1-[1-methyl-3-(pentafluorethyl)-4-(trifluormethyl)-1H-pyrazol-5-yl]-1H-imidazol-4-yl}benzyl)carbamat werden in 8,34 mL 4N Chlorwasserstoff in Dioxan gelöst und 1h gerührt. Das Lösungsmittel wird Rotationsverdampfer unter vermindertem Druck entfernt.

Man erhält 650 mg Rohprodukt, welche ohne weitere Aufreinigung in den weiteren Reaktionen eingesetzt wird.
HPLC-MS: logP^{a)} = 1,83 , Masse (m/z) = 440,1 [M+H-HCl]⁺.

### Stufe 4: Synthese von N-(3-{1-[1-Methyl-3-(pentafluorethyl)-4-(trifluormethyl)-1H-pyrazol-5-yl]-1H-imidazol-4-yl}benzyl)propanamid (Ib-5)

216 mg (0,45 mmol) 1-(3-{1-[1-Methyl-3-(pentafluorethyl)-4-(trifluormethyl)-1H-pyrazol-5-yl]-1H-imidazol-4-yl}phenyl)methanaminhydrochlorid und 190 µL (1,1 mmol) N,N-Diethyl-N-isopropylamin werden in Chloroform gelöst und dann mit 46 mg (0,5 mmol) Propionylchlorid versetzt. Es wird eine katalytische Menge 4-(Dimethylamino)-pyridin hinzugefügt. Nach einer Stunde wird die Reaktionsmischung mit 5%-iger Natriumhydrogenphosphatlösung versetzt. Die wässrige Phase wird mehrfach mit Chloroform extrahiert. Die vereinigten organischen Phasen werden über Natriumsulfat getrocknet, filtriert und unter vermindertem Druck am Rotationsverdampfer eingeengt. Das Rohprodukt wird an Kieselgel säulenchromatographisch gereinigt.
Man erhält 70 mg N-(3-{1-[1-Methyl-3-(pentafluorethyl)-4-(trifluormethyl)-1H-pyrazol-5-yl]-1H-imidazol-4-yl}benzyl)propanamid (Ib-5)
¹H-NMR siehe NMR Daten in Peakliste
HPLC-MS: logP^{a)} = 3,14, Masse (m/z) = 496,1 [M+H]⁺.

### Darstellungsverfahren B

### Herstellung von N-{2-Fluor-5-[2'-methyl-5'-(pentafluorethyl)-4'-(trifluormethyl)-2'H-1,3'-bipyrazol-4-yl]benzyl}propanamid (Ib-8)

Das Reaktionsschema 7 zeigt die Synthese der erfindungsgemaäßen Verbindung (**Ib-8**)

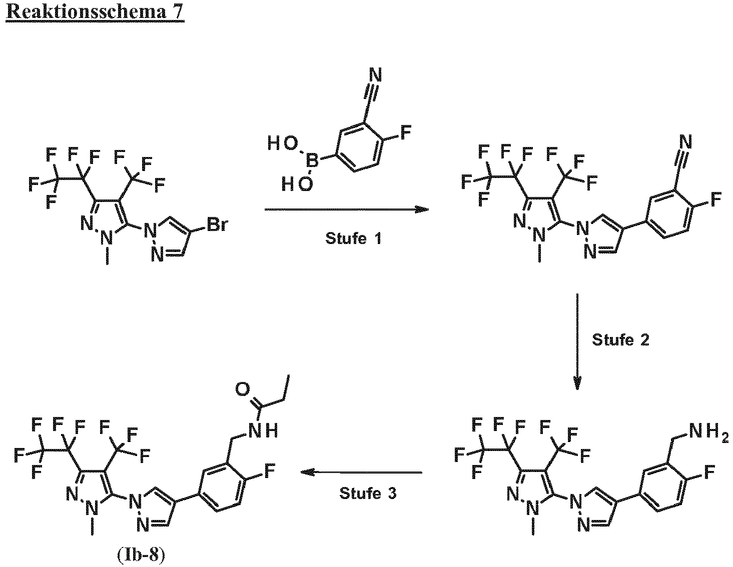

### Stufe 1: Synthese von 2-Fluor-5-[2'-methyl-5'-(pentafluorethyl)-4'-(trifluormethyl)-2'H-1,3'-bipyrazol-4-yl]benzonitril

4,00 g (10 mmol) 4-Brom-2'-methyl-5'-(pentafluorethyl)-4'-(trifluormethyl)-2'H-1,3'-bipyrazol, 1,60 g (10 mmol) 3-Cyan-4-fluorphenyl)boronsäure und 0,56 g (0,2 mmol) Tetrakis(triphenylphosphin)palladium(0) werden in einer Mischung aus 80 mL 2-Propanol und 30 mL 1N wässriger Natriumhydrogencarbonatlösung gelöst. Die Lösungsmittel wurden vorher gründlich entgast. Die Reaktionslösung wird 5h unter Schutzgasatmosphäre auf 85°C erhitzt. Nach dem Ende der Reaktion wird das Reaktionsgemisch mit Wasser und Chloroform versetzt. Die organische Phase wird mit Chloroform extrahiert. Die vereinigten organischen Phasen werden dann am Rotationsverdampfer unter vermindertem Druck eingeengt. Das Rohprodukt wird dann an der RP Phase gereinigt.
Man erhält 3,31 g 2-Fluor-5-[2'-methyl-5'-(pentafluorethyl)-4'-(trifluormethyl)-2'H-1,3'-bipyrazol-4-yl]benzonitril.
¹H-NMR (400 MHz, d₆-DMSO): δ = 8,83 (s, 1H); 8,60 (s, 1H); 8,37 (dd, 1H); 8,14-8,12 (m, 1H); 7,67-7,61 (m, 1H); 3,83 (s, 3H)
HPLC-MS: logP^{a)} = 4,37 , Masse (m/z) = 454,0 [M+H]⁺.

### Stufe 2: Synthese von 1-{2-Fluor-5-[5'-(pentafluorethyl)-4'-(trifluormethyl)-2'H-1,3'-bipyrazol-4-yl]phenyl}methanamin

4,60 g (10 mmol) 2-Fluor-5-[2'-methyl-5'-(pentafluorethyl)-4'-(trifluormethyl)-2'H-1,3'-bipyrazol-4-yl]benzonitril werden in 120 mL Methanol gelöst. Die Lösung wird auf 0°C gekühlt und dann mit 13 mg (0,1 mmol) Nickel(II)chlorid x 6H₂O versetzt. 768 mg (20 mmol) Natriumborhydrid werden in kleinen Portionen zu der Mischung hinzugegeben. Nach der letzten Zugabe wird das Reaktionsgemisch langsam auf Raumtemperatur erwärmt.. Nach 1h bei Raumtemperatur werden nochmal 4 mg (0.03 mmol) Nickel(II)chlorid x 6H₂O und 220 mg (5,7 mmol) Natriumborhydrid zu der Reaktionsmischung hinzugefügt. Die Reaktionsmischung wird eine weitere Stunde bei Raumtemperatur gerührt. Dann wird das Reakionsgemisch mit Wasser verdünnt. Das Reaktionsgemisch wird dreimal mit Chloroform extrahiert. Die vereinigten organischen Phasen werden über Natriumsulfat getrocknet, filtriert und dann am Rotatiosnverdampfer unter vermindertem Druck eingeengt. Der Rückstand wird an der RP Phase aufgereinigt.

Man erhält 1,25 g 1-{2-Fluor-5-[5'-(pentafluorethyl)-4'-(trifluormethyl)-2'H-1,3'-bipyrazol-4-yl]phenyl}methanamin.
¹H-NMR (400 MHz, d₆-DMSO): δ = 8,68 (s, 1H); 8,45 (s, 1H); 7,83 (dd, 1H); 7,60-7,56 (m, 1H); 7,20 (t, 1H); 3,82 (s, 3H); 3,78 (s, 2H); 1,83 (s, 2H)
HPLC-MS: logP^{a)} = 1,90 , Masse (m/z) = 458.0 [M+H-HCl]⁺.

### Stufe 3: Synthese von N-{2-Fluor-5-[5'-(pentafluorethyl)-4'-(trifluormethyl)-2'H-1,3'-bipyrazol-4-yl]benzyl}propanamid (Ib-8)

250 mg (0,55 mmol) 1-{2-Fluor-5-[5'-(pentafluorethyl)-4'-(trifluormethyl)-2'H-1,3'-bipyrazol-4-yl]phenyl}methanamin und 0,23 mL (1,31 mmol) N,N-Diethyl-N-isopropylamin werden in '3 mL Chloroform gelöst und mit 52 µL (0,61 mmol) Propionylchlorid versetzt. Nach einer Stunde wird die Reaktionsmischung mit 5%-iger Natriumhydrogenphosphatlösung versetzt. Die wässrige Phase wird mehrfach mit Chloroform extrahiert. Die vereinigten organischen Phasen werden über Natriumsulfat getrocknet, filtriert und unter vermindertem Druck am Rotationsverdampfer eingeengt. Das Rohprodukt wird an Kieselgel säulenchromatographisch gereinigt. After extraction and evaporation, the residue obtained was purified by flash chromatography on silica gel with cyclohexane and ethylacetate as eluents. This afforded 100 mg (32%) of the title compound.

Man erhält 100 mg N-{2-Fluor-5-[5'-(pentafluorethyl)-4'-(trifluormethyl)-2'H-1,3'-bipyrazol-4-yl]benzyl}propanamid (Ib-8).
¹H-NMR siehe NMR Daten in Peakliste
HPLC-MS: logP^{a)} = 3,27, Masse (m/z) = 514.0 [M+H]⁺.

^{a)} Anmerkung zur Bestimmung der logP-Werte und Massendetektion: Die Bestimmung der angegebenen logP-Werte erfolgte gemäß EEC-Direktive 79/831 Annex V.A8 durch HPLC (High Performance Liquid Chromatography) an einer Phasenumkehrsäule (C18).Agilent 1100 LC-System; 50*4,6 Zorbax Eclipse Plus C18 1,8 microm; Eluent A: Acetonitril (0,1 % Ameisensäure); Eluent B: Wasser (0,09 % Ameisensäure); linearer Gradient von 10 % Acetonitril bis 95 % Acetonitril in 4,25 min, dann 95% Acetonitril für weitere 1,25 min; Ofentemperatur 55 °C; Fluß:2,0 mL/min. Die Massendetektion erfolgt über ein Agilend MSD-System.

Bei der angegebenen Masse handelt es sich um den Peak des Isotopenmusters des [M+H]⁺ Ions mit der höchsten Intensität; falls das [M-H]⁻ Ion detektiert wurde, ist die Massenangabe mit² gekennzeichnet. ² Bei der angegebenen Masse handelt es sich um den Peak des Isotopenmusters des [M-H]⁻ Ions mit der höchsten Intensität.

In Analogie zu den oben beschriebenen Darstellungsverfahren A, B & D wurden die in der Tabelle 1 aufgeführten Verbindungen dargestellt.

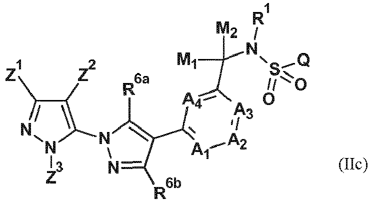

**Tabelle 1**

| **Beisp.-Nr.** | **Z¹** | **Z²** | **Z³** | **R¹** | **R^{6a}** | **R^{6b}** | **A₄** | **A₃** | **A₂** | **A₁** | **M₁** | **M₂** | **Q** | **logP^{a)}** | **Masse^{a)}** |
|---|---|---|---|---|---|---|---|---|---|---|---|---|---|---|---|
| IIc-1 | C₂F₅ | CF₃ | CH₃ | H | H | H | C-H | C-F | C-H | C-H | H | H | cyclopentyl | 4,37 | 590,1 |
| IIc-2 | C₂F₅ | CF₃ | CH₃ | H | H | H | C-H | C-F | C-H | C-H | H | H | propan-2-yl | 4,06 | 564,0 |
| IIc-3 | C₂F₅ | CF₃ | CH₃ | H | H | H | C-H | C-F | C-H | C-H | H | H | ethyl | 3,85 | 550,1 |
| IIc-4 | C₂F₅ | CF₃ | CH₃ | H | H | H | C-H | C-F | C-H | C-H | H | H | CH₃ | 3,58 | 536,100 |

| | | | | | | | | | | | | | | | |
|---|---|---|---|---|---|---|---|---|---|---|---|---|---|---|---|
| ^{a)} Wenn nicht anders gekennzeichntet wurde folgende Methode zur Bestimmung der logP-Werte und Massen verwendet: Die Bestimmung der angegebenen logP-Werte erfolgte gemäß EEC-Direktive 79/831 Annex V.A8 durch HPLC (High Performance Liquid Chromatography) an einer Phasenumkehrsäule (C18).Agilent 1100 LC-System; 50*4,6 Zorbax Eclipse Plus C18 1,8 microm; Eluent A: Acetonitril (0,1 % Ameisensäure); Eluent B: Wasser (0,09 % Ameisensäure); linearer Gradient von 10 % Acetonitril bis 95 % Acetonitril in 4,25 min, dann 95% Acetonitril für weitere 1,25 min; Ofentemperatur 55 °C; Fluß:2,0 mL/min. Die Massendetektion erfolgt über ein Agilend MSD-System. ^{b)} Anmerkung zur alternativen Methode zur Bestimmung der logP-Werte und Massen: Die Bestimmung der angegebenen logP-Werte erfolgte gemäß EEC-Direktive 79/831Annex V.A8 durch HPLC (High Performance Liquid Chromatography) an einer Phasenumkehrsäule (C18). Waters ACQUITY UPLC-MS System; 2,1*50mm Zorbax Eclipse Plus C18 1,8µm 600bar; Injektions Voumen: 0,5ul ca. 1000ppm; Eluent A: Acetonitril (0,1% Ameisensäure); Eluent B: Wasser (0,085% Ameisensäure); linearer Gradient von 10% Acetonitril bis 95% Acetonitril in 1,5min, dann 95% Acetonitril für weitere 0,5min; Ofentemperatur 60°C; Fluß: 1,0mL/min. Die Massendetektion erfolgt über ein Waters SQD System. | | | | | | | | | | | | | | | |

Bei der angegebenen Masse handelt es sich um den Peak des Isotopenmusters des [M+H]⁺ Ions mit der höchsten Intensität; falls das [M-H]⁻ Ion detektiert wurde, ist die Massenangabe mit² gekennzeichnet. ² Bei der angegebenen Masse handelt es sich um den Peak des Isotopenmusters des [M-H]⁻ Ions mit der höchsten Intensität.

### NMR-Daten ausgewählter Beispiele

Die ¹H-NMR-Daten ausgewählter Beispiele sind in Form von ¹H-NMR-Peaklisten notiert. Zu jedem Signalpeak sind der δ-Wert in ppm und die Signalintensität in Klammern aufgeführt.

| |
|---|
| Beispiel IIc-1: ¹H-NMR(400.0 MHz, DMSO): |
| δ= 10.267(0.6); 8.853(0.4); 8.678(5.4); 8.584(0.5); 8.424(5.7); 8.415(0.6); 8.316(0.4); 7.774(1.4); 7.768(1.5); 7.756(1.4); 7.750(1.3); 7.690(0.9); 7.684(0.9); 7.678(1.0); 7.670(1.2); 7.663(1.0); 7.657(1.0); 7.651(0.8); 7.620(0.9); 7.605(2.0); 7.590(1.0); 7.303(1.5); 7.279(1.9); 7.257(1.4); 5.756(0.5); 4.260(3.4); 4.245(3.3); 3.829(1.6); 3.813(16.0); 3.537(1.1); 3.518(1.7); 3.498(1.2); 3.479(0.3); 3.325(114.5); 2.675(0.6); 2.671(0.9); 2.666(0.7); 2.541(0.5); 2.511(51.6); 2.506(101.6); 2.502(132.7); 2.497(96.4); 2.493(46.9); 2.333(0.7); 2.329(0.9); 2.324(0.7); 1.887(1.5); 1.871(6.3); 1.853(4.1); 1.836(1.8); 1.664(0.9); 1.657(1.3); 1.646(1.7); 1.628(1.3); 1.614(0.4); 1.603(0.5); 1.576(0.6); 1.570(0.5); 1.564(0.5); 1.551(1.5); 1.544(0.9); 1.533(1.6); 1.520(1.2); 1.513(0.7); 1.504(0.5); 1.235(1.5); 1.218(1.3); 0.146(0.7); 0.008(6.7); 0.000(165.4); -0.009(6.3); -0.150(0.8) |
| Beispiel IIc-2: ¹H-NMR(400.0 MHz, DMSO): |
| δ=8.687(0.4); 8.676(4.6); 8.430(0.5); 8.419(5.0); 8.315(0.5); 7.777(1.1); 7.772(1.2); 7.760(1.1); 7.754(1.2); 7.689(0.7); 7.683(0.7); 7.677(0.8); 7.668(0.9); 7.662(0.8); 7.656(0.8); 7.650(0.7); 7.607(0.9); 7.592(1.7); 7.577(0.8); 7.302(1.3); 7.281(1.5); 7.278(1.6); 7.256(1.2); 4.256(2.9); 4.241(3.0); 3.813(12.7); 3.325(147.5); 3.294(0.3); 3.201(0.4); 3.184(1.1); 3.167(1.6); 3.150(1.2); 3.133(0.4); 2.676(0.5); 2.671(0.7); 2.666(0.5); 2.524(2.0); 2.511(43.1); 2.507(86.0); 2.502(112.8); 2.497(81.9); 2.493(39.9); 2.333(0.6); 2.329(0.8); 2.324(0.6); 1.236(16.0); 1.219(15.7); 0.915(0.6); 0.146(0.6); 0.008(5.9); 0.000(145.5); -0.009(5.8); -0.150(0.6) |
| Beispiel IIc-3: ¹H-NMR(400.0 MHz, DMSO): |
| δ= 8.690(5.9); 8.677(0.6); 8.433(6.8); 7.775(1.4); 7.770(1.6); 7.757(1.4); 7.752(1.5); 7.695(0.9); 7.689(0.8); 7.683(1.0); 7.674(1.2); 7.668(1.0); 7.661(1.0); 7.656(0.9); 7.637(1.1); 7.621(2.2); 7.606(1.1); 7.308(1.7); 7.286(1.9); 7.284(2.0); 7.262(1.5); 5.757(2.1); 4.235(3.9): |
| 4.220(3.9); 3.814(16.0); 3.328(77.6); 3.042(1.5); 3.024(4.8); 3.006(4.9); 2.987(1.6); 2.671(0.4); 2.667(0.3); 2.524(1.3); 2.511(27.4); 2.507(53.8); 2.502(69.7); 2.498(50.0); 2.333(0.3); 2.329(0.4); 1.235(0.4); 1.204(5.2); 1.185(11.0); 1.167(4.9); 0.146(0.4); 0.008(3.2); 0.000(81.8); -0.009(3.3); -0.150(0.4) |
| Beispiel IIc-4: ¹H-NMR(400.0 MHz, DMSO): |
| δ= 8.701(3.0); 8.442(3.3); 7.765(0.7); 7.760(0.8); 7.748(0.7); 7.743(0.8); 7.696(0.4); 7.690(0.4); 7.684(0.5); 7.676(0.6); 7.669(0.5); 7.663(0.5); 7.657(0.4); 7.596(0.5); 7.580(1.1); 7.566(0.5); 7.312(0.8); 7.290(1.0); 7.288(1.0); 7.266(0.8); 4.254(2.0); 4.238(2.0); 3.813(7.9); 3.400(0.4); 3.328(164.6); 2.936(11.0); 2.675(0.5); 2.671(0.6); 2.667(0.5); 2.506(79.9); 2.502(103.3); 2.497(75.0); 2.333(0.5); 2.329(0.7); 2.324(0.5); 2.086(1.8); 1.398(16.0); 1.141(0.6); 1.125(0.4); 0.000(0.4) |

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Die Listen der ¹H-NMR-Peaks sind ähnlich den klassischen ¹H-NMR-Ausdrucken und enthalten somit gewöhnlich alle Peaks, die bei einer klassischen NMR-Interpretation aufgeführt werden.

Darüber hinaus können sie wie klassische ¹H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im delta-Bereich von Lösungsmitteln und/oder Wasser sind in unseren Listen von ¹H-NMR-Peaks die gewöhnlichen Lösungsmittelpeaks, zum Beispiel Peaks von DMSO in DMSO-d₆ und der Peak von Wasser, gezeigt, die gewöhnlich im Durchschnitt eine hohe Intensität aufweisen.

Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%).

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen.

Ein Fachmann, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen ¹H-NMR-Interpretation.

### Biologische Ausführungsbeispiele für Verwendungen im Bereich Tiergesundheit und Pflanzenschutz

### Boophilus microplus -Injektionstest (BOOPMI Inj)

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Lösungsmittel und verdünnt das Konzentrat mit Lösungsmittel auf die gewünschte Konzentration.

1µl der Wirkstofflösung wird in das Abdomen von 5 vollgesogenen, adulten, weiblichen Rinderzecken *(Boophilus microplus)* injiziert. Die Tiere werden in Schalen überführt und in einem klimatisierten Raum aufbewahrt.

Die Wirkungskontrolle erfolgt nach 7 Tagen auf Ablage fertiler Eier. Eier, deren Fertilität nicht äußerlich sichtbar ist, werden bis zum Larvenschlupf nach etwa 42 Tagen im Klimaschrank aufbewahrt. Eine Wirkung von 100 % bedeutet, dass keine der Zecken fertile Eier gelegt hat, 0% bedeutet, dass alle Eier fertil sind.

Bei diesem Test zeigen z.B. die folgenden Verbindungen der Herstellungsbeispiele eine Wirkung von 100% bei einer Aufwandmenge von 20µg/Tier: IIc-3, IIc-4

### Ctenocephalides felis - Oraltest (CTECFE)

| | |
|---|---|
| Lösungsmittel: | Dimethylsulfoxid |

Zwecks Herstellung einer zweckmäßigen Wirkstoffzubereitung vermischt man 10 mg Wirkstoff mit 0,5 ml Dimethylsulfoxid. Durch Verdünnen mit citriertem Rinderblut erhält man die gewünschte Konzentration.

Ca. 20 nüchterne adulte Katzenflöhe (*Ctenocephalides felis*) werden in eine Kammer eingesetzt, die oben und unten mit Gaze verschlossen ist. Auf die Kammer wird ein Metallzylinder gestellt, dessen Unterseite mit Parafilm verschlossen ist. Der Zylinder enthält die Blut-Wirkstoffzubereitung, die von den Flöhen durch die Parafilmmembran aufgenommen werden kann.

Nach 2 Tagen wird die Abtötung in % bestimmt. Dabei bedeutet 100 %, dass alle Flöhe abgetötet wurden; 0 % bedeutet, dass keiner der Flöhe abgetötet wurde.

Bei diesem Test zeigen z.B. die folgenden Verbindungen der Herstellungsbeispiele eine Wirkung von 100% bei einer Aufwandmenge von 100ppm: IIc-4

Bei diesem Test zeigen z.B. die folgenden Verbindungen der Herstellungsbeispiele eine Wirkung von 95% bei einer Aufwandmenge von 100ppm: IIc-3

### Lucilia cuprina - Test (LUCICU)

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.

Ca. 20 L1-Larven der Australischen Schafgoldfliege (*Lucilia cuprina*) werden in ein Testgefäß überführt, welches gehacktes Pferdefleisch und die Wirkstoffzubereitung der gewünschten Konzentration enthält.

Nach 2 Tagen wird die Abtötung in % bestimmt. Dabei bedeutet 100 %, dass alle Larven abgetötet wurden; 0 % bedeutet, dass keine Larven abgetötet wurden.

Bei diesem Test zeigen z.B. die folgenden Verbindungen der Herstellungsbeispiele eine Wirkung von 100% bei einer Aufwandmenge von 100ppm: IIc-3, IIc-4

### Musca domestica-Test (MUSCDO)

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.

Gefäße, die einen Schwamm enthalten, der mit Zuckerlösung und der Wirkstoffzubereitung der gewünschten Konzentration behandelt wurde, werden mit 10 adulten Stubenfliegen (*Musca domestica*) besetzt.

Nach 2 Tagen wird die Abtötung in % bestimmt. Dabei bedeutet 100 %, dass alle Fliegen abgetötet wurden; 0 % bedeutet, dass keine der Fliegen abgetötet wurde.

Bei diesem Test zeigen z.B. die folgenden Verbindungen der Herstellungsbeispiele eine Wirkung von 100% bei einer Aufwandmenge von 100ppm: IIc-4

### Meloidogyne incognita- Test (MELGIN)

| | |
|---|---|
| Lösungsmittel: | 125,0 Gewichtsteile Aceton |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.

Gefäße werden mit Sand, Wirkstofflösung, einer Ei-Larven-Suspension des südlichen Wurzelgallenälchens (*Meloidogyne incognita*) und Salatsamen gefüllt. Die Salatsamen keimen und die Pflänzchen entwickeln sich. An den Wurzeln entwickeln sich die Gallen.

Nach 14 Tagen wird die nematizide Wirkung anhand der Gallenbildung in % bestimmt. Dabei bedeutet 100 %, dass keine Gallen gefunden wurden; 0 % bedeutet, dass die Zahl der Gallen an den behandelten Pflanzen der unbehandelten Kontrolle entspricht.

Bei diesem Test zeigen z. B. die folgenden Verbindungen der Herstellungsbeispiele Wirkung von 90% bei einer Aufwandmenge von 20ppm: IIc-3, IIc-4

### Myzus persicae - Sprühtest (MYZJPE)

| | |
|---|---|
| Lösungsmittel: | 78 Gewichtsteile Aceton |
| | 1,5 Gewichtsteile Dimethylformamid |
| | |
| Emulgator: | Alkylarylpolyglykolether |

Zur Herstellung einer zweckmäßigen Wirkstoffzubereitung löst man 1 Gewichtsteil Wirkstoff mit den angegebenen Gewichststeilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.

Nach 6 Tagen wind die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele Wirkung von 90% bei einer Aufwandmenge von 100g/ha: IIc-3

### Phaedon cochleariae - Sprühtest (PHAECO)

| | |
|---|---|
| Lösungsmittel: | 78,0 Gewichtsteile Aceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben *(Brassica pekinensis*) werden mit einer Wirkstoffzubereitung der gewünschten Konzentration gespritzt und nach dem Abtrocknen mit Larven des Meerrettichblattkäfers *(Phaedon cochleariae)* besetzt.

Nach 7 Tagen wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele Wirkung von 100% bei einer Aufwandmenge von 100g/ha: IIc-1, IIc-2, IIc-3, IIc-4

### Tetranychus urticae - Sprühtest, OP-resistent (TETRUR)

| | |
|---|---|
| Lösungsmittel: | 78,0 GewichtsteileAceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Bohnenblattscheiben *(Phaseolus vulgaris),* die von allen Stadien der Gemeinen Spinnmilbe *(Tetranychus urticae*) befallen sind, werden mit einer Wirkstoffzubereitung der gewünschten Konzentration gespritzt.

Nach 6 Tagen wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele eine Wirkung von 100% bei einer Aufwandmenge von 100g/ha: IIc-2, IIc-3, IIc-4

Bei diesem Test zeigen z. B. die folgenden Verbindungen der Herstellungsbeispiele eine Wirkung von 90% bei einer Aufwandmenge von 100g/ha: IIc-1

## Patentansprüche

1. Verbindungen der allgemeinen Formel (II), 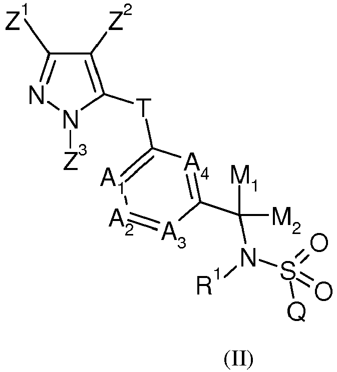 in denen
R¹ für Wasserstoff, gegebenenfalls substituiertes C₁-C₆-Alkyl, C₃-C₆-Alkenyl, C₃-C₆-Alkinyl, C₃-C₇-Cycloalkyl, C₁-C₆-Alkylcarbonyl, C₁-C₆-Alkoxycarbonyl, Aryl(C₁-C₃)-alkyl, Heteroaryl(C₁-C₃)-alkyl,
M¹ und M² jeweils unabhängig voneinander für Wasserstoff, Cyano oder für ein gegebenenfalls ein oder mehrfach substituiertes C₁-C₆-Alkyl, C₂-C₆-Alkenyl, C₂-C₆-Alkinyl, C₃-C₆-Cycloalkyl, C₁-C₆-Alkoxycarbonyl stehen, oder
M¹ und M² mit dem Kohlenstoffatom, an das sie gebunden sind, einen gegebenenfalls substituierten 3-, 4-, 5- oder 6-gliedrigen Ring bilden, der gegebenenfalls 0, 1 oder 2 Stickstoffatome und/oder 0, 1 oder 2 Sauerstoffatome und/oder 0, 1 oder 2 Schwefelatome enthält,
die chemischen Gruppierungen
A₁ für CR² oder Stickstoff,
A₂ für CR³ oder Stickstoff,
A₃ für CR⁴ oder Stickstoff, und
A₄ für CR⁵ oder Stickstoff stehen,
wobei aber nicht mehr als drei der chemischen Gruppierungen A₁ bis A₄ gleichzeitig für Stickstoff stehen;
R², R³, R⁴ und R⁵ unabhängig voneinander für Wasserstoff, Halogen, Cyano, Nitro, gegebenenfalls substituiertes C₁-C₆-Alkyl, C₃-C₆-Cycloalkyl, C₁-C₆-Alkoxy, *N*-C₁-C₆-Alkoxy-imino-C₁-C₃-alkyl, C₁-C₆-Alkylsulfanyl, C₁-C₆-Alkylsulfinyl, C₁-C₆-Alkylsulfonyl, *N*-C₁-C₆-Alkylamino oder *N*,*N*-Di-C₁-C₆-alkylamino, stehen;
wenn keine der Gruppierungen A₂ und A₃ für Stickstoff steht, können R³ und R⁴ gemeinsam mit dem Kohlenstoffatom, an das sie gebunden sind, einen 5- oder 6-gliedrigen Ring bilden, der 0, 1 oder 2 Stickstoffatome und/oder 0 oder 1 Sauerstoffatom und/oder 0 oder 1 Schwefelatom enthält, oder
wenn keine der Gruppierungen A₁ und A₂ für Stickstoff steht, können R² und R³ gemeinsam mit dem Kohlenstoffatom, an das sie gebunden sind, einen 6-gliedrigen Ring bilden, der 0, 1 oder 2 Stickstoffatome enthält;
W für Sauerstoff oder Schwefel steht;
Q für Wasserstoff, Hydroxy, Amino oder eine der gegebenenfalls substituierten Gruppierungen Alkyl, Alkoxy, Alkenyl, Alkinyl, Cycloalkyl, Heterocycloalkyl, Cycloalkylalkyl, Arylalkyl, Heteroarylalkyl oder für eine Gruppierung *N*-Alkylamino, *N*-Alkylcarbonylamino, *N*,*N*-Dialkylamino steht; oder
Q für einen gegebenenfalls einfach bis mehrfach mit V substituierten, ungesättigten 6-gliedrigen Carbozyklus steht, oder für einen gegebenenfalls einfach bis mehrfach mit V substituierten, ungesättigten 5- bzw. 6-gliedrigen, heterozyklischen Ring steht, wobei
V unabhängig voneinander für Halogen, Cyano, Nitro, gegebenenfalls substituiertes Alkyl, Alkenyl, Alkinyl, Cycloalkyl, Alkoxy, *N*-Alkoxyiminoalkyl, Alkylsulfanyl, Alkylsulfinyl, Alkylsulfonyl, *N*,*N*-Dialkylamino steht
T für einen der nachfolgend aufgeführten 5 gliedrigen Heteroaromaten T1-T8 steht, wobei die Bindung zur Pyrazolkopfgruppe mit einem Sternchen gekennzeichnet ist, 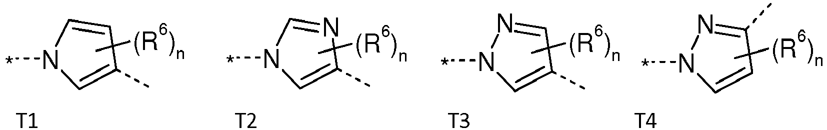 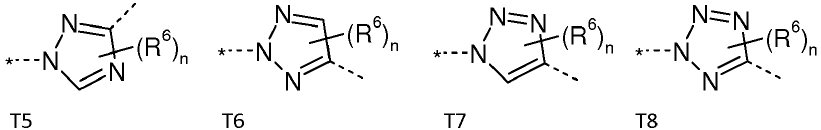
wobei
R⁶ unabhängig voneinander für Halogen, Cyano, Nitro, Amino oder ein gegebenenfalls substituiertes C₁-C₆-Alkyl, C₁-C₆-Alkoxy, C₁-C₆-Alkylcarbonyl, C₁-C₆-Alkylsulfanyl, C₁-C₆-Alkylsulfinyl, C₁-C₆-Alkylsulfonyl, und
n für die Werte 0-2 stehen;
Z¹ für ein gegebenenfalls substituiertes Alkyl und Cycloalkyl, und
Z² für Wasserstoff, Halogen, Cyano, Nitro, Amino oder ein gegebenenfalls substituiertes Alkyl, Alkylcarbonyl, Alkylsulfanyl, Alkylsulfinyl, Alkylsulfonyl, und
Z³ für Wasserstoff oder ein gegebenenfalls substituiertes Alkyl, Cycloalkyl, Alkenyl, Alkinyl, Aryl oder Hetaryl stehen.

2. Verbindungen gemäß Anspruch 1, in denen
R¹ für Wasserstoff, gegebenenfalls substituiertes C₁-C₆-Alkyl, C₃-C₆-Alkenyl, C₃-C₆-Alkinyl, C₃-C₇-Cycloalkyl, C₁-C₆-Alkylcarbonyl, C₁-C₆-Alkoxycarbonyl, Aryl(C₁-C₃)-alkyl, Heteroaryl(C₁-C₃)-alkyl steht;
M¹ und M² jeweils unabhängig voneinander für Wasserstoff, Cyano oder für ein gegebenenfalls ein oder mehrfach substituiertes C₁-C₆-Alkyl, C₂-C₆-Alkenyl, C₂-C₆-Alkinyl, C₃-C₆-Cycloalkyl, C₁-C₆-Alkoxycarbonyl stehen, oder
M¹ und M² mit dem Kohlenstoffatom, an das sie gebunden sind, einen gegebenenfalls substituierten 3-, 4-, 5- oder 6-gliedrigen Ring bilden, der gegebenenfalls 0, 1 oder 2 Stickstoffatome und/oder 0, 1 oder 2 Sauerstoffatome und/oder 0, 1 oder 2 Schwefelatome enthält,
die chemischen Gruppierungen
A₁ für CR² oder Stickstoff,
A₂ für CR³ oder Stickstoff,
A₃ für CR⁴ oder Stickstoff, und
A₄ für CR⁵ oder Stickstoff stehen,
wobei aber nicht mehr als drei der chemischen Gruppierungen A₁ bis A₄ gleichzeitig für Stickstoff stehen;
R², R³, R⁴ und R⁵ unabhängig voneinander für Wasserstoff, Halogen, Cyano, Nitro, gegebenenfalls substituiertes C₁-C₆-Alkyl, C₃-C₆-Cycloalkyl, C₁-C₆-Alkoxy, *N*- C₁-C₆-Alkoxyimino- C₁-C₃-alkyl, C₁-C₆-Alkylsulfanyl, C₁-C₆-Alkylsulfinyl, C₁-C₆-Alkylsulfonyl, *N*-C₁-C₆-Alkylamino, *N,N*-Di-C₁-C₆-alkylamino, stehen;
wenn keine der Gruppierungen A₂ und A₃ für Stickstoff steht, können R³ und R⁴ gemeinsam mit dem Kohlenstoffatom, an das sie gebunden sind, einen 5- oder 6-gliedrigen Ring bilden, der 0, 1 oder 2 Stickstoffatome und/oder 0 oder 1 Sauerstoffatom und/oder 0 oder 1 Schwefelatom enthält, oder
wenn keine der Gruppierungen A₁ und A₂ für Stickstoff steht, können R² und R³ gemeinsam mit dem Kohlenstoffatom, an das sie gebunden sind, einen 6-gliedrigen Ring bilden, der 0, 1 oder 2 Stickstoffatome enthält;
W für Sauerstoff oder Schwefel steht;
Q für Wasserstoff, Formyl, Hydroxy, Amino oder eine der gegebenenfalls substituierten Gruppierungen C₁-C₆-Alkyl, C₂-C₆-Alkenyl, C₂-C₆-Alkinyl, C₃-C₆-Cycloalkyl, C₂-C₇-Heterocycloalkyl, C₁-C₄-Alkoxy, C₃-C₆-Cycloalkyl-C₁-C₆-alkyl, Aryl-(C₁-C₃)-alkyl, Heteroaryl-(C₁-C₃)-alkyl oder für eine Gruppierung *N*-C₁-C₄-Alkylamino, *N-*C₁-C₄-Alkylcarbonylamino, *N,N*-Di-C₁-C₄-alkylamino steht; oder
Q für einen gegebenenfalls einfach bis mehrfach mit V substituierten, ungesättigten 6-gliedrigen Carbozyklus steht, oder für einen gegebenenfalls einfach bis mehrfach mit V substituierten, ungesättigten 5- bzw. 6-gliedrigen, heterozyklischen Ring steht, wobei
V unabhängig voneinander für Halogen, Cyano, Nitro, gegebenenfalls substituiertes C₁-C₆-Alkyl, C₂-C₄-Alkenyl, C₂-C₄-Alkinyl, C₃-C₆-Cycloalkyl, C₁-C₆-Alkoxy, *N*-C₁-C₆-Alkoxy-imino-C₁-C₃-alkyl, C₁-C₆-Alkylsulfanyl, C₁-C₆-Alkylsulfinyl, C₁-C₆-Alkylsulfonyl, *N,N*-Di-(C₁-C₆-alkyl)amino steht;
T für einen der nachfolgend aufgeführten 5 gliedrigen Heteroaromaten T1-T8 steht, wobei die Bindung zur Pyrazolkopfgruppe mit einem Sternchen gekennzeichnet ist, 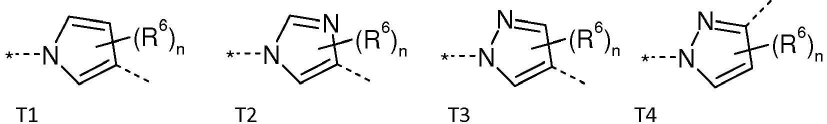 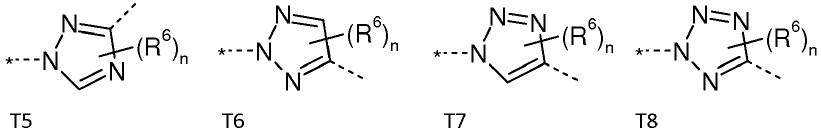
wobei
R⁶ unabhängig voneinander für Halogen, Cyano, Nitro, Amino oder ein gegebenenfalls mit Halogen substituiertes C₁-C₆-Alkyl, C₁-C₆-Alkoxy, C₁-C₆-Alkylcarbonyl, C₁-C₆-Alkylsulfanyl, C₁-C₆-Alkylsulfinyl, C₁-C₆-Alkylsulfonyl, und
n für die Werte 0-1 stehen;
Z¹ für ein gegebenenfalls substituiertes C₁-C₆-Halogenalkyl, C₃-C₆-Halogencycloalkyl,
und
Z² für Wasserstoff Halogen, Cyano, Nitro, Amino oder ein gegebenenfalls substituiertes C₁-C₆-Alkyl, C₁-C₆-Alkylcarbonyl, C₁-C₆-Alkylsulfanyl, C₁-C₆-Alkylsulfinyl, C₁-C₆-Alkylsulfonyl, und
Z³ für Wasserstoff oder ein gegebenenfalls substituiertes C₁-C₆-Alkyl, C₃-C₆-Cycloalkyl, C₃-C₆-Alkenyl, C₃-C₆-Alkinyl, Aryl oder Hetaryl stehen.

3. Verbindungen gemäß Anspruch 1, in denen
R¹ für Wasserstoff, gegebenenfalls ein- bis siebenfach unabhängig voneinander mit Fluor, Chlor, Cyano, C₁-C₆-Alkoxy und C₁-C₆-Alkoxycarbonyl substituiertes C₁-C₆-Alkyl, C₃-C₆-Alkenyl, C₃-C₆-Alkinyl, C₃-C₇-Cycloalkyl, ,C₁-C₆-Alkylcarbonyl, C₁-C₆-Alkoxycarbonyl, Aryl(C₁-C₃)-alkyl, Heteroaryl(C₁-C₃)-alkyl,
M¹ für Wasserstoff steht,
M² für Wasserstoff, Cyano, gegebenenfalls ein- bis fünffach unabhängig voneinander durch Fluor, Chlor, Cyano oder C₁-C₃-Alkoxy substituiertes C₁-C₆-Alkyl, C₂-C₆-Alkenyl, C₃-C₆-Cycloalkyl, C₁-C₃-Alkoxycarbonyl steht,
M¹ und M² mit dem Kohlenstoffatom, an das sie gebunden sind, einen gegebenenfalls substituierten 3-gliedrigen Ring bilden,
die chemischen Gruppierungen
A₁ für CR² oder Stickstoff,
A₂ für CR³ oder Stickstoff,
A₃ für CR⁴ oder Stickstoff, und
A₄ für CR⁵ oder Stickstoff stehen,
wobei aber nicht mehr als drei der chemischen Gruppierungen A₁ bis A₄ gleichzeitig für Stickstoff stehen;
R², R³, R⁴ und R⁵ unabhängig voneinander für Wasserstoff, Fluor, Chlor, Brom, Iod, Cyano, Nitro, gegebenenfalls einfach bis fünffach unabhängig voneinander durch Fluor, Chlor, Cyano, Hydroxycarbonyl, C₁-C₆-Alkoxycarbonyl, C₁-C₆-Alkylcarbonyl oder Phenyl substituiertes C₁-C₆-Alkyl, C₃-C₆-Cycloalkyl, C₁-C₆-Alkoxy, *N*-C₁-C₆-Alkoxy-imino-C₁-C₃-alkyl, C₁-C₆-Alkylsulfanyl, C₁-C₆-Alkylsulfinyl, C₁-C₆-Alkylsulfonyl, *N-*C₁-C₆-Alkylamino oder *N,N*-Di-C₁-C₆-alkylamino stehen;
W für Sauerstoff oder Schwefel steht;
Q für Wasserstoff, Amino oder eine der gegebenenfalls unabhängig von einander einbis fünffach mit Hydroxy, Nitro, Amino, Fluor, Chlor, C₁-C₆-Alkyl, C₁-C₆-Alkoxy, Cyano, Hydroxycarbonyl, C₁-C₄-Alkoxycarbonyl, C₁-C₄-Alkylcarbamoyl, C₃-C₇-Cycloalkylcarbamoyl, Phenyl substituierten Gruppierungen C₁-C₆-Alkyl, C₂-C₆-Alkenyl, C₂-C₆-Alkinyl, C₃-C₆-Cycloalkyl, C₂-C₅-Heterocycloalkyl, C₁-C₄-Alkoxy, C₃-C₆-Cycloalkyl-C₁-C₆-alkyl, Aryl(C₁-C₃)-alkyl, Heteroaryl(C₁-C₃)-alkyl, *N-*C₁-C₄-Alkylamino, *N*-C₁-C₄-Alkylcarbonylamino oder *N,N*-Di-C₁-C₄-alkylamino steht; oder
Q für ein mit 0 - 4 Substituenten V substituiertes Aryl oder für ein mit 0 - 4 Substituenten V substituierten 5 bzw. 6 gliedrigen Heteroaromaten steht, wobei
V unabhängig voneinander für Halogen, Cyano, Nitro, gegebenenfalls substituiertes C₁-C₆-Alkyl, C₂-C₄-Alkenyl, C₂-C₄-Alkinyl, C₃-C₆-Cycloalkyl, C₁-C₆-Alkoxy, *N*-C₁-C₆-Alkoxy-imino-C₁-C₃-alkyl, C₁-C₆-Alkylsulfanyl, C₁-C₆-Alkylsulfinyl, C₁-C₆-Alkylsulfonyl, *N,N*-Di-(C₁-C₆-alkyl)amino steht;
T für einen der nachfolgend aufgeführten 5 gliedrigen Heteroaromaten T1-T8 steht, wobei die Bindung zur Pyrazolkopfgruppe mit einem Sternchen gekennzeichnet ist, 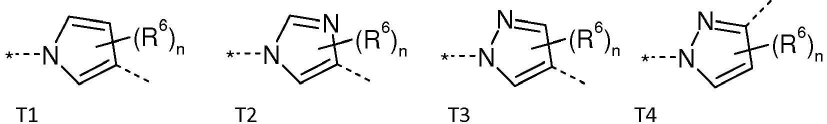 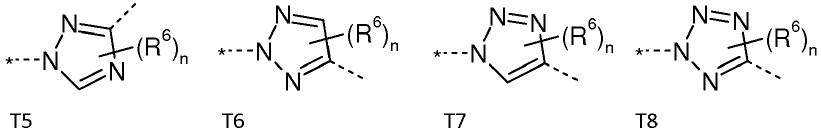
wobei
R⁶ unabhängig voneinander für Fluor, Chlor, Brom, Iod, Cyano, Nitro, Amino oder ein gegebenenfalls unabhängig voneinander ein- bis fünffach mit Fluor und/oder Chlor substituiertes C₁-C₆-Alkyl, C₁-C₆-Alkox, C₁-C₆-Alkylcarbonyl, C₁-C₆-Alkylsulfanyl, C₁-C₆-Alkylsulfinyl, C₁-C₆-Alkylsulfonyl, und
n für die Werte 0-1 stehen;
Z¹ für ein gegebenenfalls substituiertes C₁-C₆-Halogenalkyl, C₃-C₆-Halogencycloalkyl,
und
Z² für Wasserstoff, Fluor, Chlor, Brom, Iod, Cyano, Nitro, Amino oder ein gegebenenfalls unabhängig voneinander ein- bis fünffach mit Fluor und/oder Chlor substituiertes C₁-C₆-Alkyl, C₁-C₆-Alkylcarbonyl, C₁-C₆-Alkylsulfanyl, C₁-C₆-Alkylsulfinyl, C₁-C₆-Alkylsulfonyl, und
Z³ für Wasserstoff oder ein gegebenenfalls substituiertes C₁-C₆-Alkyl, C₃-C₆-Cycloalkyl C₃-C₄-Alkenyl, C₃-C₄-Alkinyl, Aryl und Hetaryl stehen.

4. Verbindungen gemäß Anspruch 1, in denen
R¹ für Wasserstoff, Methyl, Ethyl, n-Propyl, Isopropyl, n-Butyl, Isobutyl, s-Butyl, t-Butyl, Methoxymethyl, Ethoxymethyl, Propoxymethyl, Methylcarbonyl, Ethylcarbonyl, n-Propylcarbonyl, Isopropylcarbonyl, n-Butylcarbonyl, i-Butylcarbonyl, s-Butylcarbonyl, t-Butylcarbonyl, Methoxycarbonyl, Ethoxycarbonyl, n-Propoxycarbonyl, Isopropoxycarbonyl, n-Butoxycarbonyl, i-Butoxycarbonyl, s-Butoxycarbonyl, t-Butoxycarbonyl, Cyanomethyl, 2-Cyanoethyl, Benzyl, 4-Methoxybenzyl, Pyrid-2-yl-methyl, Pyrid-3-yl-methyl, Pyrid-4-yl-methyl, 6-Chlor-pyrid-3-yl-methyl steht;
M¹ für Wasserstoff steht,
M² Wasserstoff, Methyl, Ethyl, Difluormethyl, Trichlormethyl, Dichlorfluormethyl, Trifluormethyl, Cyclopropyl, Cyclobutyl, Methoxycarbonyl, Ethoxycarbonyl,
M¹ und M² mit dem Kohlenstoffatom, an das sie gebunden sind, einen 3-gliedrigen Carbozyklus bilden,
die chemischen Gruppierungen
A₁ für CR² oder Stickstoff,
A₂ für CR³ oder Stickstoff,
A₃ für CR⁴ oder Stickstoff, und
A₄ für CR⁵ oder Stickstoff stehen,
wobei aber nicht mehr als drei der chemischen Gruppierungen A₁ bis A₄ gleichzeitig für Stickstoff stehen;
R² und R⁵ unabhängig voneinander für Wasserstoff, Methyl, Fluor und Chlor stehen und
R³ und R⁴ unabhängig voneinander für Wasserstoff, Fluor, Chlor, Brom, Iod, Cyano, Nitro, Methyl, Ethyl, Fluormethyl, Difluormethyl, Chlordifluormethyl, Trifluormethyl, 2,2,2-Trifluorethyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N-*Methoxyiminomethyl, 1-(*N*-Methoxyimino)-ethyl, Methylsulfanyl, Trifluormethylsulfanyl, Methylsulfonyl, Methylsulfinyl, Trifluormethylsulfonyl, Trifluormethylsulfinyl stehen
W für Sauerstoff oder Schwefel steht;
Q für für Wasserstoff, Methyl, Ethyl, n-Propyl, Isopropyl, t-Butyl, 1-Methylpropyl, n-Butyl, 2-Methylpropyl, 2-Methylbutyl, Hydroxymethyl, 2-Hydroxyethyl, 2-Hydroxypropyl, Cyanomethyl, 2-Cyanoethyl, Trifluormethyl, 1-Fluorethyl, 2-Fluorethyl, 2,2-Difluorethyl, 2,2,2-Trifluorethyl, 1-Trifluormethylethyl, 2,2-Difluorpropyl, 3,3,3-Trifluorpropyl, 2,2-Dimethyl-3-fluorpropyl, Cyclopropyl, 1-Cyano-cyclopropyl, Cyclopropyl-methyl, Cyclobutyl, Cyclopentyl, Cyclohexyl, 1-Cyclopropylethyl, Bis(cyclopropyl)methyl, 2,2-Dimethylcyclopropyl-methyl, 2-Phenylcyclopropyl, 2,2-Dichlorcyclopropyl, trans-2-Chlorcyclopropyl, cis-2-Chlorcyclopropyl, 2,2-Difluorcyclopropyl, trans-2-Fluorcyclopropyl, cis-2-Fluorcyclopropyl, trans-4-Hydroxycyclohexyl, 4-Trifluormethylcyclohexyl, Ethenyl, 1-Methylethenyl, Prop-1-enyl, 2-Methylprop-1-enyl, 3-Methylbut-1-enyl, 3,3,3-Trifluorprop-1-enyl, 1-Ethyl-ethenyl, 1-Methylprop-1-enyl, Prop-2-inyl, 3-Fluor-prop-2-enyl" Oxetan-3-yl, Thietan-3-yl, 1-Oxido-thietan-3-yl, 1,1-Dioxido-thietan-3-yl, Tetrahydrofuran-3-yl, 1,1-Dioxido-tetrahydrothiophen-3 -yl, Isoxazol-3-ylmethyl, 1,2,4-Triazol-3-ylmethyl, 3-Methyloxetan-3-ylmethyl, Benzyl, 2,6-Difluorphenylmethyl, 3-Fluorphenylmethyl, 2-Fluorphenylmethyl, 2,5-Difluorphenylmethyl, 1-Phenylethyl, 4-Chlorphenylethyl, 2-Trifluormethylphenylethyl, 1-Pyridin-2-ylethyl, Pyridin-2-ylmethyl, 5-Fluorpyridin-2-ylmethyl, (6-Chlor-pyridin-3-yl)methyl, Pyrimidin-2-ylmethyl, Thiophen-2-yl-methyl. 2-Ethoxyethyl, 2-Methoxyethyl, 1-(Methylsulfanyl)ethyl, 2-(Methylsulfanyl)ethyl, 1-Methyl-2-(ethylsulfanyl)ethyl, 2-Methyl-1-(methylsulfanyl)propan-2-yl, Methoxycarbonyl, Ethoxycarbonyl, Methoxycarbonylmethyl, NH₂, *N*-Ethylamino, *N-*Allylamino, *N,N*-Dimethylamino, *N,N*-Diethylamino, Methoxy, Ethoxy, Propoxy, iso-Propoxy, tert-Butoxy steht; oder
Q für ein mit 0,1,2 oder 3Substituenten V substituiertes Phenyl, Naphthyl, Pyridazin, Pyrazin, Pyrimidin, Triazin, Pyridin, Pyrazol, Thiazol, Isothiazol, Oxazol, Isoxazol, Triazol, Imidazol, Furan, Thiophen, Pyrrol, Oxadiazol, Thiadiazol steht, wobei
V unabhängig voneinander für Fluor, Chlor, Brom, Iod, Cyano, Nitro, Methyl, Ethyl, Difluormethyl, Trichlormethyl, Chlordifluormethyl, Dichlorfluormethyl, Trifluormethyl, 1-Fluorethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Dilfluorethyl, Pentafluorethyl Heptafluor-n-propyl, Heptafluor-isopropyl, Nonafluor-n-butyl, Cyclopropyl, Cyclobutyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N*-Methoxyiminomethyl, 1-(*N*-Methoxyimino)-ethyl, Methylsulfanyl, Methylsulfonyl, Methylsulfinyl, Trifluormethylsulfonyl, Trifluormethylsulfinyl, Trifluormethylsulfanyl, *N,N*-Dimethylamino steht;
T für einen der nachfolgend aufgeführten 5 gliedrigen Heteroaromaten T1-T8 steht, wobei die Bindung zur Pyrazolkopfgruppe mit einem Sternchen gekennzeichnet ist, 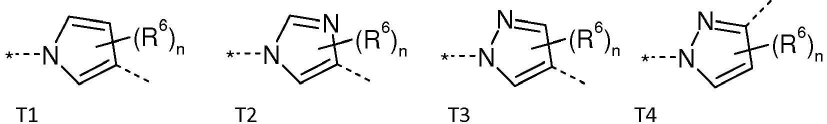 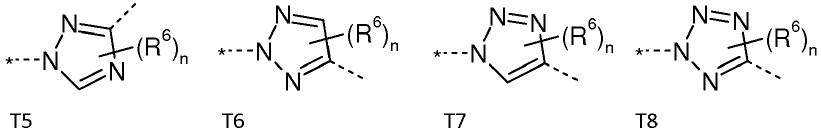
wobei
R⁶ unabhängig voneinander für Fluor, Chlor, Cyano, Nitro, Amino, Methyl, Ethyl, Propyl, 1-Methylethyl, tert-Butyl, Trifluormethyl, Difluormethyl, Methoxy, Ethoxy, Trifluormethoxy, 2,2-Difluorethoxy, 2,2,2-Trifluorethoxy, Methylcarbonyl, Ethylcarbonyl, Trifluormethylcarbonyl, Methylsulfanyl, Methylsulfinyl, Methylsulfonyl, Trifluormethylsulfonyl, Trifluormethylsulfanyl, Trilfuormethylsulfinyl, und
n für die Werte 0-1 stehen;
Z¹ für Difluormethyl, Trichlormethyl, Chlordifluormethyl, Dichlorfluormethyl, Trifluormethyl, Bromdichlormethyl, 1-Fluorethyl, 1-Fluor-1-methylethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Difluorethyl, Pentafluorethyl Heptafluor-n-propyl, Heptafluor-isopropyl, Nonafluor-n-butyl, Cyclopropyl, 1-Chlorcyclopropyl, 1-Fluorcyclopropyl, 1-Bromcyclopropyl, 1-Cyan-cyclopropyl, 1-Trifluormehtyl-cyclopropyl, Cyclobutyl und 2,2-Difluor-1-methyl-cyclopropyl, und
Z² für Wasserstoff, Fluor, Chlor, Brom, Iod, Cyano, Nitro,, Amino, Methyl, Ethyl, 1,1-t-Butyl, Difluormethyl, Trichlormethyl, Chlordifluormethyl, Dichlorfluormethyl, Trifluormethyl, Bromdichlormethyl, 1-Fluorethyl, 1-Fluor-1-methylethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Difluorethyl, Pentafluorethyl Heptafluor-n-propyl, Heptafluor-isopropyl, Nonafluor-n-butyl, Methylsulfanyl, Methylsulfinyl, Methylsulfonyl, Ethylthio, Ethylsulfinyl, Ethylsulfonyl, Trifluormethylsulfanyl, Trifluormethylsulfinyl, Trifluormethylsulfonyl, Chlor-difluormethylsulfanyl, Chlor-difluormethylsulfinyl, Chlor-difluormethylsulfonyl, Dichlor-fluormethylsulfanyl, Dichlor-fluormethylsulfinyl, Dichlor-fluormethylsulfonyl und
Z³ für Wasserstoff, Methyl, Ethyl, n-Propyl, Isopropyl, n-Butyl, Isobutyl, s-Butyl, t-Butyl, 1-Propenyl, 1-Propinyl, 1-Butinyl, Difluormethyl, Trichlormethyl, Chlordifluormethyl, Dichlorfluormethyl, Trifluormethyl, 1-Fluorethyl, 1-Fluor-1-methylethyl, 2-Fluorethyl, 2,2-Difluorethyl, 2,2,2-Trifluorethyl, Phenyl, 2-Chlorphenyl, 3-Chlorphenyl. 4-Chlorphenyl, 2,4-Dichlorpenyl, 2,5-Dichlorphenyl, 3,4-Dichlorphenyl, 2,6-Dichlorphenyl 2,6-Dichlor-4-trifluormehtylphenyl, 3-Chlor-5-trifluormethylpyridin-2-yl stehen.

5. Verbindungen gemäß Anspruch 1, in denen
Z¹ für Trifluormethyl, 1-Chlor-cyclopropyl, 1-Fluor-cyclopropyl oder Pentafluorethyl steht,
Z² für Trifluormethyl, Nitro, Methylsulfanyl, Methylsulfinyl, Methylsulfonyl, Fluor, Chlor, Brom, Cyano oder Iod steht,
Z³ für Methyl, Ethyl, n-Propyl oder Wasserstoff steht,
R¹ für Wasserstoff, Methyl, Ethyl, n-Propyl, Isopropyl, n-Butyl, i-Butyl, s-Butyl, t-Butyl, Methoxymethyl, Ethoxymethyl, Propoxymethyl, Methylcarbonyl, Ethylcarbonyl, n-Propylcarbonyl, Isopropylcarbonyl, n-Butylcarbonyl, i-Butylcarbonyl, s-Butylcarbonyl, t-Butylcarbonyl, Methoxycarbonyl, Ethoxycarbonyl, n-Propoxycarbonyl, Isopropoxycarbonyl, n-Butoxycarbonyl, i-Butoxycarbonyl, s-Butoxycarbonyl, t-Butoxycarbonyl, Cyanomethyl, 2-Cyanoethyl, Benzyl, 4-Methoxybenzyl, Pyrid-2-yl-methyl, Pyrid-3-yl-methyl, Pyrid-4-yl-methyl, 6-Chlor-pyrid-3-yl-methyl steht,
M¹ für Wasserstoff steht;
M² für Wasserstoff oder Methyl steht;
M¹ und M² mit dem Kohlenstoffatom, an das sie gebunden sind, einen 3-gliedrigen Carbozyklus bilden,
A¹, und A⁴ für CH steht
A2 für CH oder N steht,
A₃ für CR⁴ und
R⁴ für Methyl, Ethyl, Fluor, Chlor, Brom oder Iod steht
T für einen der nachfolgend aufgeführten 5 gliedrigen Heteroaromaten T1-T8 steht, wobei die Bindung zur Pyrazolkopfgruppe mit einem Sternchen gekennzeichnet ist, 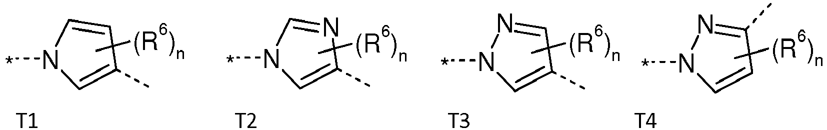 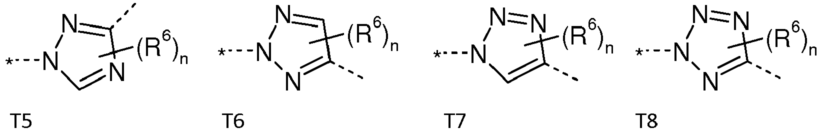
wobei
R⁶ für Wasserstoff, Methyl, Ethyl, 2-Methylethyl, 2,2-Dimethylethyl, Fluor, Chlor, Brom, Iod, Nitro, Trifluormethyl, Amino steht,
W für Sauerstoff steht und
Q für für Wasserstoff, Methyl, Ethyl, n-Propyl, 1-Methylethyl, 1,1-Dimethylethyl, n-Butyl, 1-Methylpropyl, 2-Methylpropyl, 2-Methylbutyl, Hydroxymethyl2-Hydroxypropyl, Cyanomethyl, 2-Cyanoethyl, 2-Fluorethyl, 2,2-Difluorethyl, 2,2,2-Trifluorethyl, 1-Trifluormethylethyl, 2,2-Difluorpropyl, 3,3,3-Triflurpropyl, 2,2-Dimethyl-3-fluorpropyl, Cyclopropyl, Cyclopropyl-methyl, Cyclobutyl, Cyclopentyl, Cyclohexyl, 1-Cyclopropylethyl, Bis(cyclopropyl)methyl, 2,2-Dimethylcyclopropyl-methyl, 2-Phenylcyclopropyl, 2,2-Dichlorcyclopropyl, trans-2-Chlorcyclopropyl, cis-2-Chlorcyclopropyl, 2,2-Difluorcyclopropyl, trans-2-Fluorcyclopropyl, cis-2-Fluorcyclopropyl, trans-4-Hydroxycyclohexyl, 4-Trifluormethylcyclohexyl, Ethenyl, 1-Methylethenyl, Prop-1-enyl, 2-Methylprop-1-enyl, 3-Methylbut-1-enyl, 3,3,3-Trifluorprop-1-enyl, 1-Ethyl-ethenyl, 1-Methylprop-1-enyl, Prop-2-inyl, 3-Fluor-prop-2-enyl, Oxetan-3-yl, Thietan-3-yl, 1-Oxido-thietan-3-yl, 1,1-Dioxido-thietan-3-yl, Isoxazol-3-ylmethyl, 1,2,4-Triazol-3-ylmethyl, 3-Methyloxetan-3-ylmethyl, Benzyl, 2,6-Difluorphenylmethyl, 3-Fluorphenylmethyl, 2-Fluorphenylmethyl, 2,5-Difluorphenylmethyl, 1-Phenylethyl, 4-Chlorphenylethyl, 2-Trifluormethylphenylethyl, 1-Pyridin-2-ylethyl, Pyridin-2-ylmethyl, (6-Chlor-pyridin-3-yl)methyl, 5-Fluorpyridin-2-ylmethyl, Pyrimidin-2-ylmethyl, Methoxy, 2-Ethoxyethyl, 2-(Methylsulfanyl)ethyl, 1-Methyl-2-(ethylsulfanyl)ethyl, 2-Methyl-1-(methylsulfanyl)propan-2-yl, Methoxycarbonyl, Methoxycarbonylmethyl, NH₂, *N-*Ethylamino, *N*-Allylamino, *N,N*-Dimethylamino, *N,N*-Diethylamino, Methoxy, Ethoxy, Propoxy, iso-Propoxy, tert-Butoxy steht; oder
Q für ein mit 0, 1, 2 oer 3 Substituenten V substituiertes Phenyl, Naphthyl, Pyridazin,
Pyrazin, Pyrimidin, Triazin, Pyridin, Pyrazol, Thiazol, Isothiazol, Oxazol, Isoxazol, Triazol, Imidazol, Furan, Thiophen, Pyrrol, Oxadiazol, Thiadiazol steht, wobei
V unabhängig voneinander für Fluor, Chlor, Brom, Iod, Cyano, Nitro, Methyl, Ethyl, Difluormethyl, Trichlormethyl, Chlordifluormethyl, Dichlorfluormethyl, Trifluormethyl, 1-Fluorethyl, 2-Fluorethyl, 2,2-Difluorethyl, 2,2,2-Trifluorethyl, 1,2,2,2-Tetrafluorethyl, 1-Chlor-1,2,2,2-tetrafluorethyl, 2,2,2-Trichlorethyl, 2-Chlor-2,2-difluorethyl, 1,1-Dilfluorethyl, Pentafluorethyl, Heptafluor-n-propyl, Heptafluor-isopropyl, Nonafluor-n-butyl, Cyclopropyl, Cyclobutyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, *N*-Methoxyiminomethyl, 1-(*N*-Methoxyimino)-ethyl, Methylsulfanyl, Methylsulfonyl, Methylsulfinyl, Trifluormethylsulfonyl, Trifluormethylsulfinyl, Trifluormethylsulfanyl, *N,N*-Dimethylamino steht.

6. Verbindungen der allgeneinen Formeln (IIa), (IIb), (IIc), (IId), (IIe), (IIf), (IIg), (IIh), in denen die Reste A₁-A₄, n, W, Q, R¹, R⁶, M¹, M² und Z¹-Z³ die Bedeutungen gemäß einem der Ansprüche 1 bis 5annehmen können.

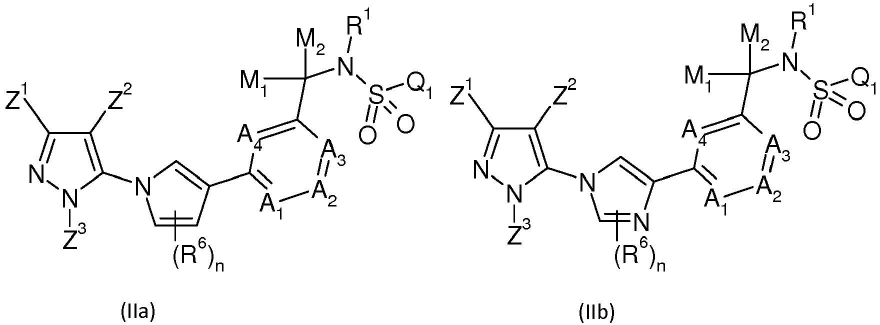

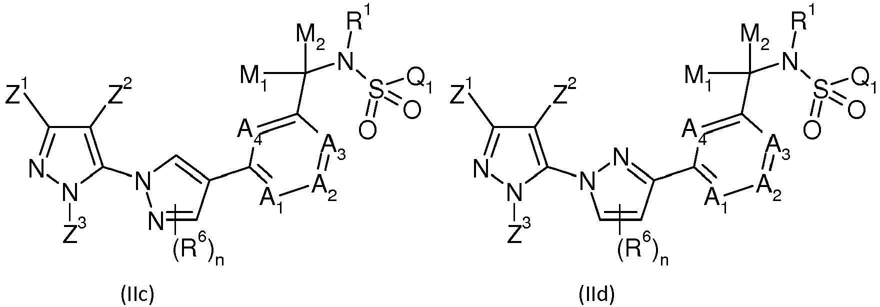

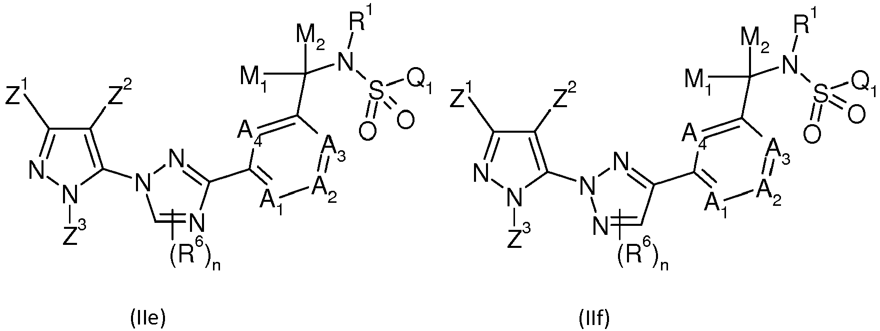

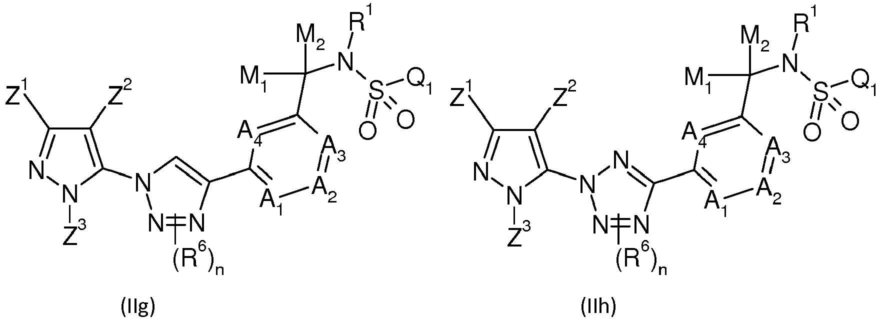

7. Verwendung von Verbindungen der allgemeinen Formel (II) oder der allgemeinen (IIa) bis (IIh) gemäß einem der Ansprüche 1 bis 6 zur Bekämpfung von an Pflanzen parasitierenden Schädlingen, Vorratsschädlingen oder Schädlingen, die industrielle Materialien zerstören.

8. Pharmazeutische Zusammensetzungen, enthaltend wenigstens eine Verbindung gemäß einem der Ansprüche 1 bis 6.

9. Verbindungen gemäß einem der Ansprüche 1 bis 6 zur Verwendung als Arzneimittel.

10. Verwendung von Verbindungen gemäß einem der Ansprüche 1 bis 6 zur Herstellung pharmazeutischer Zusammensetzungen zur Bekämpfung von Parasiten auf Tieren.

11. Verfahren zur Herstellung von Pflanzenschutzmitteln enthaltend Verbindungen gemäß einem der Ansprüche 1 bis 6, sowie übliche Streckmittel und/oder oberflächenaktive Substanzen.

12. Verfahren zum Bekämpfen von an Pflanzen parasitierenden Schädlingen, Vorratsschädlingen oder Schädlingen, die industrielle Materialien zerstören, **dadurch gekennzeichnet, dass** man eine Verbindung gemäß einem der Ansprüche 1 bis 6 auf die Schädlinge und/oder ihren Lebensraum einwirken lässt.

13. Verwendung von Verbindungen gemäß einem der Ansprüche 1 bis 6 zum Schutz des Vermehrungsmaterials von Pflanzen.

## Claims

1. Compounds of the general formula (II) 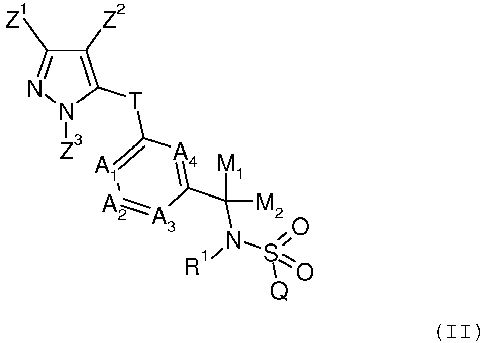 in which
R¹ represents hydrogen, optionally substituted C₁-C₆-alkyl, C₃-C₆-alkenyl, C₃-C₆-alkynyl, C₃-C₇-cycloalkyl, C₁-C₆-alkylcarbonyl, C₁-C₆-alkoxycarbonyl, aryl-(C₁-C₃)-alkyl, heteroaryl-(C₁-C₃)-alkyl,
M¹ and M² each independently of one another represent hydrogen, cyano or represent optionally mono- or polysubstituted C₁-C₆-alkyl, C₂-C₆-alkenyl, C₂-C₆-alkynyl, C₃-C₆-cycloalkyl, C₁-C₆-alkoxycarbonyl, or
M¹ and M² with the carbon atom to which they are attached form an optionally substituted 3-, 4-, 5- or 6-membered ring which optionally contains 0, 1 or 2 nitrogen atoms and/or 0, 1 or 2 oxygen atoms and/or 0, 1 or 2 sulphur atoms,
the chemical groupings
A₁ represents CR² or nitrogen,
A₂ represents CR³ or nitrogen,
A₃ represents CR⁴ or nitrogen and
A₄ represents CR⁵ or nitrogen,
but where not more than three of the chemical groupings A₁ to A₄ simultaneously represent nitrogen;
R², R³, R⁴ and R⁵ independently of one another represent hydrogen, halogen, cyano, nitro, optionally substituted C₁-C₆-alkyl, C₃-C₆-cycloalkyl, C₁-C₆-alkoxy, *N*-C₁-C₆-alkoxy-imino-C₁-C₃-alkyl, C₁-C₆-alkylsulphanyl, C₁-C₆-alkylsulphinyl, C₁-C₆-alkylsulphonyl, *N*-C₁-C₆-alkylamino or *N, N*-di-C₁-C₆-alkylamino;
if none of the groupings A₂ and A₃ represents nitrogen, R³ and R⁴ together with the carbon atom to which they are attached may form a 5- or 6-membered ring which contains 0, 1 or 2 nitrogen atoms and/or 0 or 1 oxygen atom and/or 0 or 1 sulphur atom, or
if none of the groupings A₁ and A₂ represents nitrogen, R² and R³ together with the carbon atom to which they are attached may form a 6-membered ring which contains 0, 1 or 2 nitrogen atoms;
W represents oxygen or sulphur;
Q represents hydrogen, hydroxy, amino or one of the optionally substituted groupings alkyl, alkoxy, alkenyl, alkynyl, cycloalkyl, heterocycloalkyl, cycloalkylalkyl, arylalkyl, heteroarylalkyl or represents a grouping *N-*alkylamino, *N*-alkylcarbonylamino, *N,N-*dialkylamino; or
Q represents an unsaturated 6-membered carbocycle which is optionally mono- or polysubstituted by V or an unsaturated 5- or 6-membered heterocyclic ring which is optionally mono- or polysubstituted by V, where
V independently of one another represent halogen, cyano, nitro, optionally substituted alkyl, alkenyl, alkynyl, cycloalkyl, alkoxy, *N*-alkoxyiminoalkyl, alkylsulphanyl, alkylsulphinyl, alkylsulphonyl, *N,N-*dialkylamino,
T represents one of the 5-membered heteroaromatics T1-T8 listed below, where the bond to the pyrazole head group is marked with an asterisk, 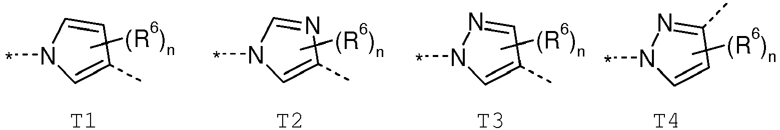 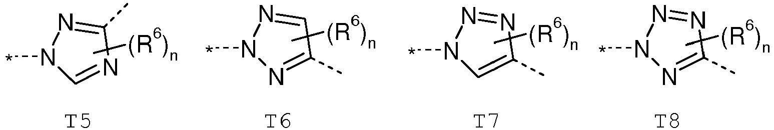
where
R⁶ independently of one another represent halogen, cyano, nitro, amino or optionally substituted C₁-C₆-alkyl, C₁-C₆-alkoxy, C₁-C₆-alkylcarbonyl, C₁-C₆-alkylsulphanyl, C₁-C₆-alkylsulphinyl, C₁-C₆-alkylsulphonyl, and
n represents the values 0-2;
Z¹ represents optionally substituted alkyl and cycloalkyl, and
Z² represents hydrogen, halogen, cyano, nitro, amino or optionally substituted alkyl, alkylcarbonyl, alkylsulphanyl, alkylsulphinyl, alkylsulphonyl, and
Z³ represents hydrogen or optionally substituted alkyl, cycloalkyl, alkenyl, alkynyl, aryl or hetaryl.

2. Compounds according to Claim 1 in which
R¹ represents hydrogen, optionally substituted C₁-C₆-alkyl, C₃-C₆-alkenyl, C₃-C₆-alkynyl, C₃-C₇-cycloalkyl, C₁-C₆-alkylcarbonyl, C₁-C₆-alkoxycarbonyl, aryl-(C₁-C₃)-alkyl, heteroaryl-(C₁-C3)-alkyl,
M¹ and M² each independently of one another represent hydrogen, cyano or represent optionally mono- or polysubstituted C₁-C₆-alkyl, C₂-C₆-alkenyl, C₂-C₆-alkynyl, C₃-C₆-cycloalkyl, C₁-C₆-alkoxycarbonyl, or
M¹ and M² with the carbon atom to which they are attached form an optionally substituted 3-, 4-, 5- or 6-membered ring which optionally contains 0, 1 or 2 nitrogen atoms and/or 0, 1 or 2 oxygen atoms and/or 0, 1 or 2 sulphur atoms,
the chemical groupings
A₁ represents CR² or nitrogen,
A₂ represents CR³ or nitrogen,
A₃ represents CR⁴ or nitrogen and
A₄ represents CR⁵ or nitrogen,
but where not more than three of the chemical groupings A₁ to A₄ simultaneously represent nitrogen;
R², R³, R⁴ and R⁵ independently of one another represent hydrogen, halogen, cyano, nitro, optionally substituted C₁-C₆-alkyl, C₃-C₆-cycloalkyl, C₁-C₆-alkoxy, N-C₁-C₆-alkoxyimino-C₁-C₃-alkyl, C₁-C₆-alkylsulphanyl, C₁-C₆-alkylsulphinyl, C₁-C₆-alkylsulphonyl, N-C₁-C₆-alkylamino or *N,N*-di-C₁-C₆-alkylamino;
if none of the groupings A₂ and A₃ represents nitrogen, R³ and R⁴ together with the carbon atom to which they are attached may form a 5- or 6-membered ring which contains 0, 1 or 2 nitrogen atoms and/or 0 or 1 oxygen atom and/or 0 or 1 sulphur atom, or
if none of the groupings A₁ and A₂ represents nitrogen, R² and R³ together with the carbon atom to which they are attached may form a 6-membered ring which contains 0, 1 or 2 nitrogen atoms;
W represents oxygen or sulphur;
Q represents hydrogen, formyl, hydroxy, amino or one of the optionally substituted groupings C₁-C₆-alkyl, C₂-C₆-alkenyl, C₂-C₆-alkynyl, C₃-C₆-cycloalkyl, C₂-C₇-heterocycloalkyl, C₁-C₄-alkoxy, C₃-C₆-Cycloalkyl-C₁-C₆-alkyl, aryl-(C₁-C₃)-alkyl, heteroaryl-(C₁-C₃)-alkyl or represents a grouping N-C₁-C₄-alkylamino, N-C₁-C₄-alkylcarbonylamino, N,N-di-C₁-C₄-alkylamino; or
Q represents an unsaturated 6-membered carbocycle which is optionally mono- or polysubstituted by V or an unsaturated 5- or 6-membered heterocyclic ring which is optionally mono- or polysubstituted by V, where
V independently of one another represent halogen, cyano, nitro, optionally substituted C₁-C₆-alkyl, C₂-C₄-alkenyl, C₂-C₄-alkynyl, C₃-C₆-cycloalkyl, C₁-C₆-alkoxy, *N*-C₁-C₆-alkoxy-imino-C₁-C₃-alkyl, C₁-C₆-alkylsulphanyl, C₁-C₆-alkylsulphinyl, C₁-C₆-alkylsulphonyl, *N,N*-di-(C₁-C₆-alkyl)amino;
T represents one of the 5-membered heteroaromatics T1-T8 listed below, where the bond to the pyrazole head group is marked with an asterisk, 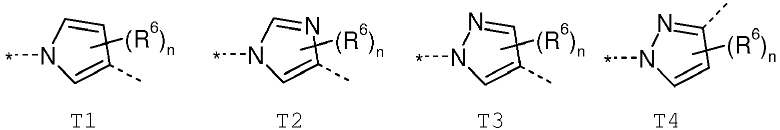 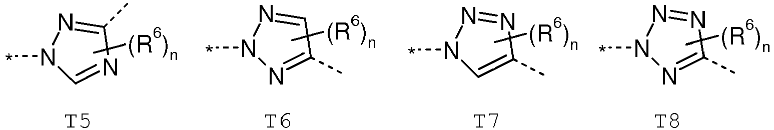
where
R⁶ independently of one another represent halogen, cyano, nitro, amino or optionally halogen-substituted C₁-C₆-alkyl, C₁-C₆-alkoxy, C₁-C₆-alkylcarbonyl, C₁-C₆-alkylsulphanyl, C₁-C₆-alkylsulphinyl, C₁-C₆-alkylsulphonyl, and
n represents the values 0-1;
Z¹ represents optionally substituted C₁-C₆-haloalkyl, C₃-C₆-halocycloalkyl, and
Z² represents hydrogen, halogen, cyano, nitro, amino or optionally substituted C₁-C₆-alkyl, C₁-C₆-alkylcarbonyl, C₁-C₆-alkylsulphanyl, C₁-C₆-alkylsulphinyl, C₁-C₆-alkylsulphonyl, and
Z³ represents hydrogen or optionally substituted C₁-C₆-alkyl, C₃-C₆-cycloalkyl, C₃-C₆-alkenyl, C₃-C₆-alkynyl, aryl or hetaryl.

3. Compounds according to Claim 1 in which
R¹ represents hydrogen or represents C₁-C₆-alkyl, C₃-C₆-alkenyl, C₃-C₆-alkynyl, C₃-C₇-cycloalkyl, C₁-C₆-alkylcarbonyl, C₁-C₆-alkoxycarbonyl, aryl- (C₁-C₃) -alkyl, heteroaryl- (C₁-C₃) -alkyl which are optionally mono- to heptasubstituted independently of one another by fluorine, chlorine, cyano, C₁-C₆-alkoxy and C₁-C₆-alkoxycarbonyl,
M¹ represents hydrogen,
M² represents hydrogen, cyano or represents C₁-C₆-alkyl, C₂-C₆-alkenyl, C₃-C₆-cycloalkyl, C₁-C₃-alkoxycarbonyl which are optionally mono-to pentasubstituted independently of one another by fluorine, chlorine, cyano or C₁-C₃-alkoxy,
M¹ and M² with the carbon atom to which they are attached form an optionally substituted 3-membered ring,
the chemical groupings
A₁ represents CR² or nitrogen,
A₂ represents CR³ or nitrogen,
A₃ represents CR⁴ or nitrogen and
A₄ represents CR⁵ or nitrogen,
but where not more than three of the chemical groupings A₁ to A₄ simultaneously represent nitrogen;
R², R³, R⁴ and R⁵ independently of one another represent hydrogen, fluorine, chlorine, bromine, iodine, cyano, nitro or represent C₁-C₆-alkyl, C₃-C₆-cycloalkyl, C₁-C₆-alkoxy, *N*-C₁-C₆-alkoxyimino-C₁-C₃-alkyl, C₁-C₆-alkylsulphanyl, C₁-C₆-alkylsulphinyl, C₁-C₆-alkylsulphonyl, *N*-C₁-C₆-alkylamino or *N,N*-di-C₁-C₆-alkylamino which are optionally mono- to pentasubstituted independently of one another by fluorine, chlorine, cyano, hydroxycarbonyl, C₁-C₆-alkoxycarbonyl, C₁-C₆-alkylcarbonyl or phenyl;
W represents oxygen or sulphur;
Q represents hydrogen, amino or one of the groupings C₁-C₆-alkyl, C₂-C₆-alkenyl, C₂-C₆-alkynyl, C₃-C₆-cycloalkyl, C₂-C₅-heterocycloalkyl, C₁-C₄-alkoxy, C₃-C₆-cycloalkyl-C₁-C₆-alkyl, aryl-(C₁-C₃)-alkyl, heteroaryl-(C₁-C₃)-alkyl, *N*-C₁-C₄-alkylamino, *N*-C₁-C₄-alkylcarbonylamino or *N,N*-di-C₁-C₄-alkylamino which are optionally independently of one another mono- to pentasubstituted by hydroxy, nitro, amino, fluorine, chlorine, C₁-C₆-alkyl, C₁-C₆-alkoxy, cyano, hydroxycarbonyl, C₁-C₄-alkoxycarbonyl, C₁-C₄-alkylcarbamoyl, C₃-C₇-cycloalkylcarbamoyl, phenyl; or
Q represents aryl substituted by 0 - 4 substituents V or a 5- or 6-membered heteroaromatic substituted by 0 - 4 substituents V, where
V independently of one another represent halogen, cyano, nitro, optionally substituted C₁-C₆-alkyl, C₂-C₄-alkenyl, C₂-C₄-alkynyl, C₃-C₆-cycloalkyl, C₁-C₆-alkoxy, *N*-C₁-C₆-alkoxyimino-C₁-C₃-alkyl, C₁-C₆-alkylsulphanyl, C₁-C₆-alkylsulphinyl, C₁-C₆-alkylsulphonyl, *N, N*-di-(C₁-C₆-alkyl) amino;
T represents one of the 5-membered heteroaromatics T1-T8 listed below, where the bond to the pyrazole head group is marked with an asterisk, 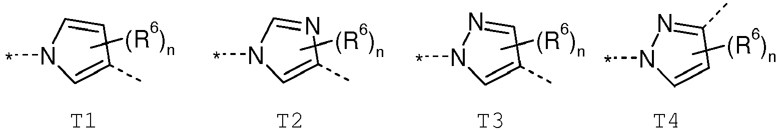 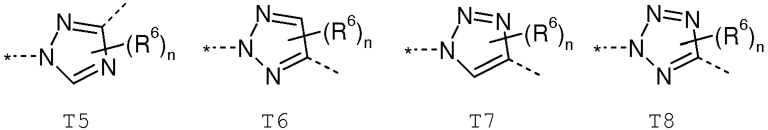
where
R⁶ independently of one another represent fluorine, chlorine, bromine, iodine, cyano, nitro, amino or represents C₁-C₆-alkyl, C₁-C₆-alkoxy, C₁-C₆-alkylcarbonyl, C₁-C₆-alkylsulphanyl, C₁-C₆-alkylsulphinyl, C₁-C₆-alkylsulphonyl which are optionally independently of one another mono- to pentasubstituted by fluorine and/or chlorine, and
n represents the values 0-1;
Z¹ represents optionally substituted C₁-C₆-haloalkyl, C₃-C₆-halocycloalkyl, and
Z² represents hydrogen, fluorine, chlorine, bromine, iodine, cyano, nitro, amino or represents C₁-C₆-alkyl, C₁-C₆-alkylcarbonyl, C₁-C₆-alkylsulphanyl, C₁-C₆-alkylsulphinyl, C₁-C₆-alkylsulphonyl which are optionally independently of one another mono- to pentasubstituted by fluorine and/or chlorine, and
Z³ represents hydrogen or optionally substituted C₁-C₆-alkyl, C₃-C₆-cycloalkyl, C₃-C₄-alkenyl, C₃-C₄-alkynyl, aryl or hetaryl.

4. Compounds according to Claim 1 in which
R¹ represents hydrogen, methyl, ethyl, n-propyl, isopropyl, n-butyl, isobutyl, s-butyl, t-butyl, methoxymethyl, ethoxymethyl, propoxymethyl, methylcarbonyl, ethylcarbonyl, n-propylcarbonyl, isopropylcarbonyl, n-butylcarbonyl, isobutylcarbonyl, s-butylcarbonyl, t-butylcarbonyl, methoxycarbonyl, ethoxycarbonyl, n-propoxycarbonyl, isopropoxycarbonyl, n-butoxycarbonyl, isobutoxycarbonyl, s-butoxycarbonyl, t-butoxycarbonyl, cyanomethyl, 2-cyanoethyl, benzyl, 4-methoxybenzyl, pyrid-2-ylmethyl, pyrid-3-ylmethyl, pyrid-4-ylmethyl, 6-chloropyrid-3-ylmethyl;
M¹ represents hydrogen,
M² represents hydrogen, methyl, ethyl, difluoromethyl, trichloromethyl, dichlorofluoromethyl, trifluoromethyl, cyclopropyl, cyclobutyl, methoxycarbonyl, ethoxycarbonyl,
M¹ and M² with the carbon atom to which they are attached form a 3-membered carbocycle,
the chemical groupings
A₁ represents CR² or nitrogen,
A₂ represents CR³ or nitrogen,
A₃ represents CR⁴ or nitrogen and
A₄ represents CR⁵ or nitrogen,
but where not more than three of the chemical groupings A₁ to A₄ simultaneously represent nitrogen;
R² and R⁵ independently of one another represent hydrogen, methyl, fluorine or chlorine and
R³ and R⁴ independently of one another represent hydrogen, fluorine, chlorine, bromine, iodine, cyano, nitro, methyl, ethyl, fluoromethyl, difluoromethyl, chlorodifluoromethyl, trifluoromethyl, 2,2,2-trifluoroethyl, methoxy, ethoxy, n-propoxy, 1-methylethoxy, fluoromethoxy, difluoromethoxy, chlorodifluoromethoxy, dichlorofluoromethoxy, trifluoromethoxy, 2,2,2-trifluoroethoxy, 2-chloro-2,2-difluoroethoxy, pentafluoroethoxy, *N-*methoxyiminomethyl, 1-(*N*-methoxyimino)ethyl, methylsulfanyl, trifluoromethylsulphanyl, methylsulphonyl, methylsulphinyl, trifluoromethylsulphonyl, trifluoromethylsulphinyl;
W represents oxygen or sulphur;
Q represents hydrogen, methyl, ethyl, n-propyl, isopropyl, t-butyl, 1-methylpropyl, n-butyl, 2-methylpropyl, 2-methylbutyl, hydroxymethyl, 2-hydroxyethyl, 2-hydroxypropyl, cyanomethyl, 2-cyanoethyl, trifluoromethyl, 1-fluoroethyl, 2-fluoroethyl, 2,2-difluoroethyl, 2,2,2-trifluoroethyl, 1-trifluoromethylethyl, 2,2-difluoropropyl, 3,3,3-trifluoropropyl, 2,2-dimethyl-3-fluoropropyl, cyclopropyl, 1-cyanocyclopropyl, cyclopropylmethyl, cyclobutyl, cyclopentyl, cyclohexyl, 1-cyclopropylethyl, bis(cyclopropyl)methyl, 2,2-dimethylcyclopropylmethyl, 2-phenylcyclopropyl, 2,2-dichlorocyclopropyl, trans-2-chlorocyclopropyl, cis-2-chlorocyclopropyl, 2,2-difluorocyclopropyl, trans-2-fluorocyclopropyl, cis-2-fluorocyclopropyl, trans-4-hydroxycyclohexyl, 4-trifluoromethylcyclohexyl, ethenyl, 1-methylethenyl, prop-1-enyl, 2-methylprop-1-enyl, 3-methylbut-1-enyl, 3,3,3-trifluoroprop-1-enyl, 1-ethylethenyl, 1-methylprop-1-enyl, prop-2-ynyl, 3-fluoroprop-2-enyl, oxetan-3-yl, thietan-3-yl, 1-oxidothietan-3-yl, 1,1-dioxidothietan-3-yl, tetrahydrofuran-3-yl, 1,1-dioxidotetrahydrothiophen-3-yl, isoxazol-3-ylmethyl, 1,2,4-triazol-3-ylmethyl, 3-methyloxetan-3-ylmethyl, benzyl, 2,6-difluorophenylmethyl, 3-fluorophenylmethyl, 2-fluorophenylmethyl, 2,5-difluorophenylmethyl, 1-phenylethyl, 4-chlorophenylethyl, 2-trifluoromethylphenylethyl, 1-pyridin-2-ylethyl, pyridin-2-ylmethyl, 5-fluoropyridin-2-ylmethyl, (6-chloropyridin-3-yl)methyl, pyrimidin-2-ylmethyl, thiophen-2-yl-methyl, 2-ethoxyethyl, 2-methoxyethyl, 1-(methylsulphanyl)ethyl, 2-(methylsulphanyl)ethyl, 1-methyl-2-(ethylsulphanyl)ethyl, 2-methyl-1-(methylsulphanyl)propan-2-yl, methoxycarbonyl, ethoxycarbonyl, methoxycarbonylmethyl, NH₂, *N*-ethylamino, *N-*allylamino, *N,N*-dimethylamino, *N,N-*diethylamino, methoxy, ethoxy, propoxy, isopropoxy, tert-butoxy; or
Q represents phenyl, naphthyl, pyridazine, pyrazine, pyrimidine, triazine, pyridine, pyrazole, thiazole, isothiazole, oxazole, isoxazole, triazole, imidazole, furan, thiophene, pyrrole, oxadiazole, thiadiazole substituted by 0, 1, 2 or 3 substituents V, where
V independently of one another represent fluorine, chlorine, bromine, iodine, cyano, nitro, methyl, ethyl, difluoromethyl, trichloromethyl, chlorodifluoromethyl, dichlorofluoromethyl, trifluoromethyl, 1-fluoroethyl, 2-fluoroethyl, 2,2-difluoroethyl, 2,2,2-trifluoroethyl, 1,2,2,2-tetrafluoroethyl, 1-chloro-1,2,2,2-tetrafluoroethyl, 2,2,2-trichloroethyl, 2-chloro-2,2-difluoroethyl, 1,1-difluoroethyl, pentafluoroethyl, heptafluoro-n-propyl, heptafluoroisopropyl, nonafluoro-n-butyl, cyclopropyl, cyclobutyl, methoxy, ethoxy, n-propoxy, 1-methylethoxy, fluoromethoxy, difluoromethoxy, chlorodifluoromethoxy, dichlorofluoromethoxy, trifluoromethoxy, 2,2,2-trifluoroethoxy, 2-chloro-2,2-difluoroethoxy, pentafluoroethoxy, *N-*methoxyiminomethyl, 1-(*N*-methoxyimino)ethyl, methylsulphanyl, methylsulphonyl, methylsulphinyl, trifluoromethylsulphonyl, trifluoromethylsulphinyl, trifluoromethylsulphanyl, *N,N*-dimethylamino;
T represents one of the 5-membered heteroaromatics T1-T8 listed below, where the bond to the pyrazole head group is marked with an asterisk, 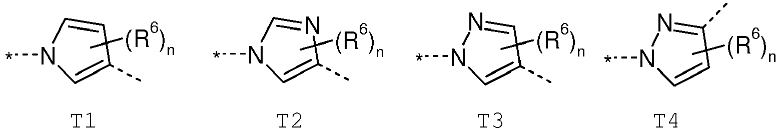 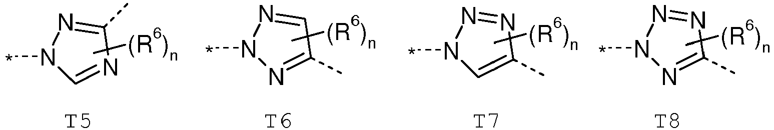
where
R⁶ independently of one another represent fluorine, chlorine, cyano, nitro, amino, methyl, ethyl, propyl, 1-methylethyl, tert-butyl, trifluoromethyl, difluoromethyl, methoxy, ethoxy, trifluoromethoxy, 2,2-difluoroethoxy, 2,2,2-trifluoroethoxy, methylcarbonyl, ethylcarbonyl, trifluoromethylcarbonyl, methylsulphanyl, methylsulphinyl, methylsulphonyl, trifluoromethylsulphonyl, trifluoromethylsulphanyl, trifluoromethylsulphinyl, and
n represents the values 0-1;
Z¹ represents difluoromethyl, trichloromethyl, chlorodifluoromethyl, dichlorofluoromethyl, trifluoromethyl, bromodichloromethyl, 1-fluoroethyl, 1-fluoro-1-methylethyl, 2-fluoroethyl, 2,2-difluoroethyl, 2,2,2-trifluoroethyl, 1,2,2,2-tetrafluoroethyl, 1-chloro-1,2,2,2-tetrafluoroethyl, 2,2,2-trichloroethyl, 2-chloro-2,2-difluoroethyl, 1,1-difluoroethyl, pentafluoroethyl, heptafluoro-n-propyl, heptafluoroisopropyl, nonafluoro-n-butyl, cyclopropyl, 1-chlorocyclopropyl, 1-fluorocyclopropyl, 1-bromocyclopropyl, 1-cyanocyclopropyl, 1-trifluoromethylcyclopropyl, cyclobutyl or 2,2-difluoro-1-methylcyclopropyl, and
Z² represents hydrogen, fluorine, chlorine, bromine, iodine, cyano, nitro, amino, methyl, ethyl, 1,1-t-butyl, difluoromethyl, trichloromethyl, chlorodifluoromethyl, dichlorofluoromethyl, trifluoromethyl, bromodichloromethyl, 1-fluoroethyl, 1-fluoro-1-methylethyl, 2-fluoroethyl, 2,2-difluoroethyl, 2,2,2-trifluoroethyl, 1,2,2,2-tetrafluoroethyl, 1-chloro-1,2,2,2-tetrafluoroethyl, 2,2,2-trichloroethyl, 2-chloro-2,2-difluoroethyl, 1,1-difluoroethyl, pentafluoroethyl, heptafluoro-n-propyl, heptafluoroisopropyl, nonafluoro-n-butyl, methylsulphanyl, methylsulphinyl, methylsulphonyl, ethylthio, ethylsulphinyl, ethylsulphonyl, trifluoromethylsulphanyl, trifluoromethylsulphinyl, trifluoromethylsulphonyl, chlorodifluoromethylsulphanyl, chlorodifluoromethylsulphinyl, chlorodifluoromethylsulphonyl, dichlorofluoromethylsulphanyl, dichlorofluoromethylsulphinyl, dichlorofluoromethylsulphonyl and
Z³ represents hydrogen, methyl, ethyl, n-propyl, isopropyl, n-butyl, isobutyl, s-butyl, t-butyl, 1-propenyl, 1-propynyl, 1-butynyl, difluoromethyl, trichloromethyl, chlorodifluoromethyl, dichlorofluoromethyl, trifluoromethyl, 1-fluoroethyl, 1-fluoro-1-methylethyl, 2-fluoroethyl, 2,2-difluoroethyl, 2,2,2-trifluoroethyl, phenyl, 2-chlorophenyl, 3-chlorophenyl, 4-chlorophenyl, 2,4-dichlorophenyl, 2,5-dichlorophenyl, 3,4-dichlorophenyl, 2,6-dichlorophenyl, 2,6-dichloro-4-trifluoromethylphenyl, 3-chloro-5-trifluoromethylpyridin-2-yl.

5. Compounds according to Claim 1 in which
Z¹ represents trifluoromethyl, 1-chlorocyclopropyl, 1-fluorocyclopropyl or pentafluoroethyl,
Z² represents trifluoromethyl, nitro, methylsulphanyl, methylsulphinyl, methylsulphonyl, fluorine, chlorine, bromine, cyano or iodine,
Z³ represents methyl, ethyl, n-propyl or hydrogen,
R¹ represents hydrogen, methyl, ethyl, n-propyl, isopropyl, n-butyl, isobutyl, s-butyl, t-butyl, methoxymethyl, ethoxymethyl, propoxymethyl, methylcarbonyl, ethylcarbonyl, n-propylcarbonyl, isopropylcarbonyl, n-butylcarbonyl, isobutylcarbonyl, s-butylcarbonyl, t-butylcarbonyl, methoxycarbonyl, ethoxycarbonyl, n-propoxycarbonyl, isopropoxycarbonyl, n-butoxycarbonyl, isobutoxycarbonyl, s-butoxycarbonyl, t-butoxycarbonyl, cyanomethyl, 2-cyanoethyl, benzyl, 4-methoxybenzyl, pyrid-2-ylmethyl, pyrid-3-ylmethyl, pyrid-4-ylmethyl, 6-chloropyrid-3-ylmethyl;
M¹ represents hydrogen;
M² represents hydrogen or methyl;
M¹ and M² with the carbon atom to which they are attached form a 3-membered carbocycle,
A¹ and A⁴ represent CH,
A² represents CH or N,
A₃ represents CR⁴ and
R⁴ represents methyl, ethyl, fluorine, chlorine, bromine or iodine,
T represents one of the 5-membered heteroaromatics T1-T8 listed below, where the bond to the pyrazole head group is marked with an asterisk, 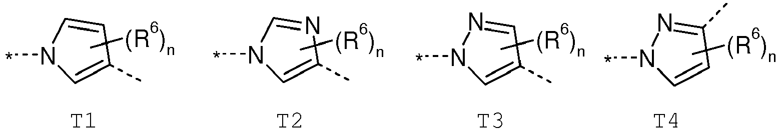 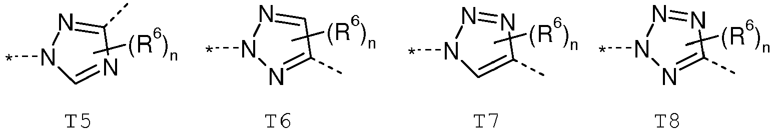 where
R⁶ represents hydrogen, methyl, ethyl, 2-methylethyl, 2,2-dimethylethyl, fluorine, chlorine, bromine, iodine, nitro, trifluoromethyl, amino,
W represents oxygen and
Q represents hydrogen, methyl, ethyl, n-propyl, 1-methylethyl, 1,1-dimethylethyl, n-butyl, 1-methylpropyl, 2-methylpropyl, 2-methylbutyl, hydroxymethyl, 2-hydroxypropyl, cyanomethyl, 2-cyanoethyl, 2-fluoroethyl, 2,2-difluoroethyl, 2,2,2-trifluoroethyl, 1-trifluoromethylethyl, 2,2-difluoropropyl, 3,3,3-trifluoropropyl, 2,2-dimethyl-3-fluoropropyl, cyclopropyl, cyclopropylmethyl, cyclobutyl, cyclopentyl, cyclohexyl, 1-cyclopropylethyl, bis(cyclopropyl)methyl, 2,2-dimethylcyclopropylmethyl, 2-phenylcyclopropyl, 2,2-dichlorocyclopropyl, trans-2-chlorocyclopropyl, cis-2-chlorocyclopropyl, 2,2-difluorocyclopropyl, trans-2-fluorocyclopropyl, cis-2-fluorocyclopropyl, trans-4-hydroxycyclohexyl, 4-trifluoromethylcyclohexyl, ethenyl, 1-methylethenyl, prop-1-enyl, 2-methylprop-1-enyl, 3-methylbut-1-enyl, 3,3,3-trifluoroprop-1-enyl, 1-ethylethenyl, 1-methylprop-1-enyl, prop-2-ynyl, 3-fluoroprop-2-enyl, oxetan-3-yl, thietan-3-yl, 1-oxidothietan-3-yl, 1,1-dioxidothietan-3-yl, isoxazol-3-ylmethyl, 1,2,4-triazol-3-ylmethyl, 3-methyloxetan-3-ylmethyl, benzyl, 2,6-difluorophenylmethyl, 3-fluorophenylmethyl, 2-fluorophenylmethyl, 2,5-difluorophenylmethyl, 1-phenylethyl, 4-chlorophenylethyl, 2-trifluormethylphenylethyl, 1-pyridin-2-ylethyl, pyridin-2-ylmethyl, (6-chloropyridin-3-yl)methyl, 5-fluoropyridin-2-ylmethyl, pyrimidin-2-ylmethyl, methoxy, 2-ethoxyethyl, 2-(methylsulphanyl)ethyl, 1-methyl-2-(ethylsulphanyl)ethyl, 2-methyl-1-(methylsulphanyl)propan-2-yl, methoxycarbonyl, methoxycarbonylmethyl, NH₂, *N*-ethylamino, *N*-allylamino, *N, N-*dimethylamino, *N, N*-diethylamino, methoxy, ethoxy, propoxy, isopropoxy, tert-butoxy; or
Q represents phenyl, naphthyl, pyridazine, pyrazine, pyrimidine, triazine, pyridine, pyrazole, thiazole, isothiazole, oxazole, isoxazole, triazole, imidazole, furan, thiophene, pyrrole, oxadiazole, thiadiazole substituted by 0, 1, 2 or 3 substituents V, where V independently of one another represent fluorine, chlorine, bromine, iodine, cyano, nitro, methyl, ethyl, difluoromethyl, trichloromethyl, chlorodifluoromethyl, dichlorofluoromethyl, trifluoromethyl, 1-fluoroethyl, 2-fluoroethyl, 2,2-difluoroethyl, 2,2,2-trifluoroethyl, 1,2,2,2-tetrafluoroethyl, 1-chloro-1,2,2,2-tetrafluoroethyl, 2,2,2-trichloroethyl, 2-chloro-2,2-difluoroethyl, 1,1-difluoroethyl, pentafluoroethyl, heptafluoro-n-propyl, heptafluoroisopropyl, nonafluoro-n-butyl, cyclopropyl, cyclobutyl, methoxy, ethoxy, n-propoxy, 1-methylethoxy, fluoromethoxy, difluoromethoxy, chlorodifluoromethoxy, dichlorofluoromethoxy, trifluoromethoxy, 2,2,2-trifluoroethoxy, 2-chloro-2,2-difluoroethoxy, pentafluoroethoxy, *N-*methoxyiminomethyl, 1-(*N*-methoxyimino)ethyl, methylsulphanyl, methylsulphonyl, methylsulphinyl, trifluoromethylsulphonyl, trifluoromethylsulphinyl, trifluoromethylsulphanyl, *N,N*-dimethylamino.

6. Compounds of the general formulae (IIa), (IIb), (IIc), (IId), (IIe), (IIf), (IIg), (IIh) in which the radicals A₁-A₄, n, W, Q, R¹, R⁶, M¹, M² and Z¹-Z³ may assume the meanings according to any of Claims 1 to 5.

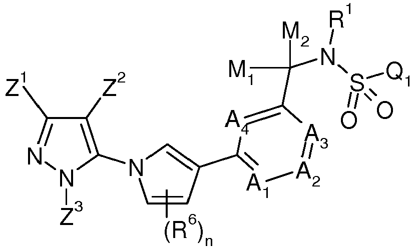

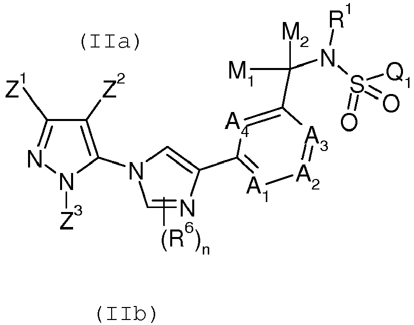

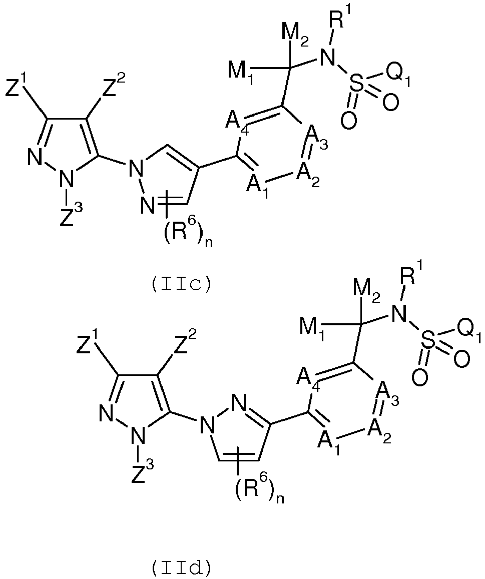

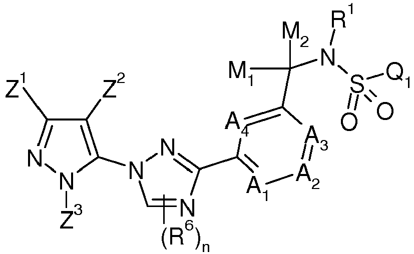

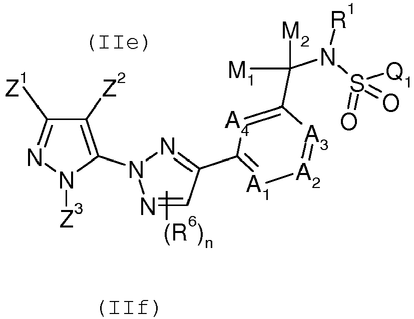

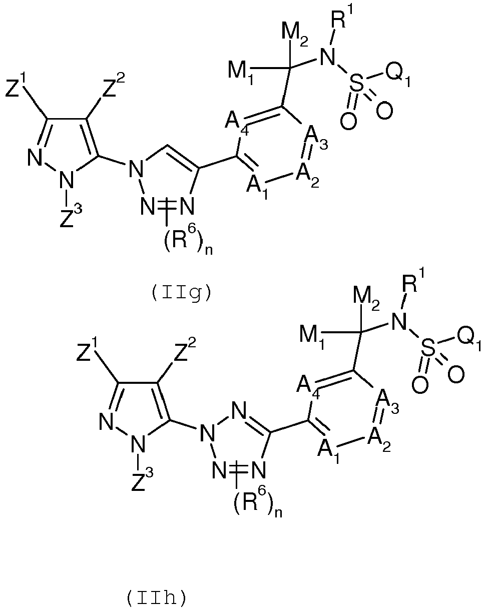

7. Use of compounds of the general formula (II) or the general formulae (IIa) to (IIh) according to any of Claims 1 to 6 for controlling pests which parasitize on plants, storage pests or pests which destroy industrial materials.

8. Pharmaceutical compositions comprising at least one compound according to any of Claims 1 to 6.

9. Compounds according to any of Claims 1 to 6 for use as medicaments.

10. Use of compounds according to any of Claims 1 to 6 for preparing pharmaceutical compositions for controlling parasites on animals.

11. Process for preparing crop protection compositions comprising compounds according to any of Claims 1 to 6 and customary extenders and/or surfactants.

12. Method for controlling pests which parasitize on plants, storage pests or pests which destroy industrial materials, **characterized in that** a compound according to any of Claims 1 to 6 is allowed to act on the pests and/or their habitat.

13. Use of compounds according to any of Claims 1 to 6 for protecting the propagation material of plants.

## Revendications

1. Composés de formule générale (II), 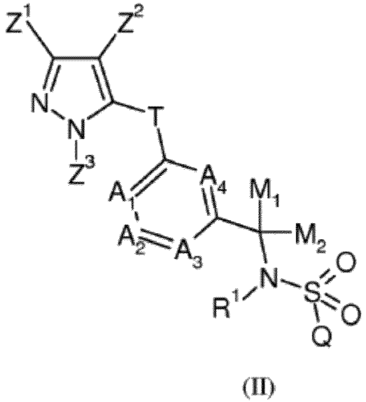 dans laquelle
R¹ représente un atome d'hydrogène, un groupe alkyle en C₁-C₆, alcényle en C₃-C₆, alcynyle en C₃-C₆, cycloalkyle en C₃-C₇, alkyl(C₁-C₆)carbonyle, alcoxy(C₁-C₆)carbonyle, aryl-alkyle(C₁-C₃₎, hétéroaryl-alkyle(C₁-C₃), éventuellement substitué,
M¹ et M² représentent chacun, indépendamment l'un de l'autre, un atome d'hydrogène, un groupe cyano ou un groupe alkyle en C₁-C₆, alcényle en C₂-C₆, alcynyle en C₂-C₆, cycloalkyle en C₃-C₆, alcoxy(C₁-C₆)carbonyle, éventuellement une ou plusieurs fois substitué, ou
M¹ et M² forment avec l'atome de carbone, auquel ils sont liés, un cycle à 3, 4, 5 ou 6 chaînons, éventuellement substitué, qui contient éventuellement 0, 1 ou 2 atomes d'azote et/ou 0, 1 ou 2 atomes d'oxygène et/ou 0, 1 ou 2 atomes de soufre,
les groupements chimiques ci-dessous ont les significations suivantes :
A₁ représente CR² ou un atome d'azote
A₂ représente CR³ ou un atome d'azote,
A₃ représente CR⁴ ou un atome d'azote,
A₄ représente CR⁵ ou un atome d'azote,
mais au maximum trois des groupements chimiques A₁ à A₄ représentent simultanément un atome d'azote ;
R², R³, R⁴ et R⁵ représentent indépendamment les uns des autres un atome d'hydrogène ou d'halogène, un groupe cyano, nitro, un groupe alkyle en C₁-C₆, cycloalkyle en C₃-C₆, alcoxy en C₁-C₆, N-alcoxy (C₁-C₆)-imino-alkyle (C₁-C₃), alkyl (C₁-C₆)-sulfanyle, alkyl(C₁-C₆) sulfinyle, alkyl(C₁-C₆)-sulfonyle, *N*-alkyl(C₁-C₆) amino ou *N,N-*di-alkyl(C₁-C₆)amino, éventuellement substitué ;
si aucun des groupements A₂ et A₃ ne représente un atome d'azote, R³ et R⁴ peuvent former ensemble avec l'atome de carbone, auquel ils sont liés, un cycle à 5 ou 6 chaînons, qui contient 0, 1 ou 2 atomes d'azote et/ou 0 ou 1 atome d'oxygène et/ou 0 ou 1 atome de soufre, ou
si aucun des groupements A₁ et A₂ ne représente un atome d'azote, R² et R³ peuvent former ensemble avec l'atome de carbone, auquel ils sont liés, un cycle à 6 chaînons, qui contient 0, 1 ou 2 atomes d'azote ;
W représente un atome d'oxygène ou de soufre ;
Q représente un atome d'hydrogène, un groupe hydroxy, amino ou un des groupements alkyle, alcoxy, alcényle, alcynyle, cycloalkyle, hétérocycloalkyle, cycloalkylalkyle, arylalkyle, hétéroarylalkyle éventuellement substitués ou un groupement *N*-alkylamino, N-alkylcarbonylamino, N,N-dialkylamino ; ou
Q représente un carbocycle insaturé à 6 chaînons, éventuellement une à plusieurs fois substitué par V, ou un cycle hétérocyclique insaturé à 5 ou 6 chaînons, éventuellement une à plusieurs fois substitué par V,
V représentant chaque fois indépendamment halogéno, cyano, nitro, un groupe alkyle, alcényle, alcynyle, cycloalkyle, alcoxy, *N*-alcoxyimino-alkyle, alkylsulfanyle, alkylsulfinyle, alkylsulfonyle, *N,N*-dialkylamino, éventuellement substitué ;
T représente un des groupes hétéroaromatiques à 5 chaînons T1-T8 indiqués ci-après, la liaison au groupe de tête pyrazole étant indiquée par un astérisque, 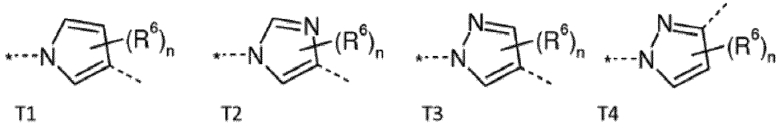 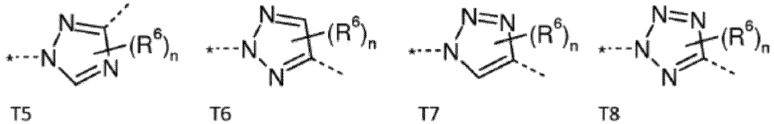
où
R⁶ représente chaque fois indépendamment un atome d'halogène, un groupe cyano, nitro, amino ou un groupe alkyle en C₁-C₆, alcoxy en C₁-C₆, alkyl(C₁-C₆) carbonyle, alkyl (C₁-C₆) sulfanyle alkyl (C₁-C₆) sulfinyle, alkyl (C₁-C₆) sulfonyle, éventuellement substitué, et
n représente les valeurs 0 à 2 ;
Z¹ représente un groupe alkyle ou cycloalkyle éventuellement substitué, et
Z² représente un atome d'hydrogène ou d'halogène, un groupe cyano, nitro, amino ou un groupe alkyle, alkylcarbonyle, alkylsulfanyle, alkylsulfinyle, alkylsulfonyle, éventuellement substitué, et
Z³ représente un atome d'hydrogène ou un groupe alkyle, cycloalkyle, alcényle, alcynyle, aryle ou hétéroaryle, éventuellement substitué.

2. Composés selon la revendication 1, dans lesquels
R¹ représente un atome d'hydrogène, un groupe alkyle en C₁-C₆, alcényle en C₃-C₆, alcynyle en C₃-C₆, cycloalkyle en C₃-C₇, alkyl(C₁-C₆) carbonyle, alcoxy(C₁-C₆)carbonyle, aryl-alkyle(C₁-C₃), hétéroaryl-alkyle(C₁-C₃), éventuellement substitué,
M¹ et M² représentent chacun, indépendamment l'un de l'autre, un atome d'hydrogène, un groupe cyano ou un groupe alkyle en C₁-C₆, alcényle en C₂-C₆, alcynyle en C₂-C₆, cycloalkyle en C₃-C₆, alcoxy(C₁-C₆) carbonyle, éventuellement une ou plusieurs fois substitué, ou
M¹ et M² forment avec l'atome de carbone, auquel ils sont liés, un cycle à 3, 4, 5 ou 6 chaînons, éventuellement substitué, qui contient éventuellement 0, 1 ou 2 atomes d'azote et/ou 0, 1 ou 2 atomes d'oxygène et/ou 0, 1 ou 2 atomes de soufre,
les groupements chimiques ci-dessous ont les significations suivantes :
A₁ représente CR² ou un atome d'azote
A₂ représente CR³ ou un atome d'azote,
A₃ représente CR⁴ ou un atome d'azote, et
A₄ représente CR⁵ ou un atome d'azote,
mais au maximum trois des groupements chimiques A₁ à A₄ représentant simultanément un atome d'azote ;
R², R³, R⁴ et R⁵ représentent indépendamment les uns des autres un atome d'hydrogène ou d'halogène, un groupe cyano, nitro, un groupe alkyle en C₁-C₆, cycloalkyle en C₃-C₆, alcoxy en C₁-C₆, *N*-alcoxy(C₁-C₆)-imino-alkyle(C₁-C₃), alkyl(C₁-C₆)-sulfanyle, alkyl(C₁-C₆) sulfinyle, alkyl(C₁-C₆)-sulfonyle, *N*-alkyl(C₁-C₆) amino ou *N,N-*di-alkyl(C₁-C₆)amino, éventuellement substitué ;
si aucun des groupements A₂ et A₃ ne représente un atome d'azote, R³ et R⁴ peuvent former ensemble avec l'atome de carbone, auquel ils sont liés, un cycle à 5 ou 6 chaînons, qui contient 0, 1 ou 2 atomes d'azote et/ou 0 ou 1 atome d'oxygène et/ou 0 ou 1 atome de soufre, ou
si aucun des groupements A₁ et A₂ ne représente un atome d'azote, R² et R³ peuvent former ensemble avec l'atome de carbone, auquel ils sont liés, un cycle à 6 chaînons, qui contient 0, 1 ou 2 atomes d'azote ;
W représente un atome d'oxygène ou de soufre ;
Q représente un atome d'hydrogène, un groupe formyle, hydroxy, amino ou un des groupements alkyle en C₁-C₆, alcényle en C₂-C₆, alcynyle en C₂-C₆, cycloalkyle en C₃-C₆, hétérocycloalkyle en C₂-C₇, alcoxy en C₁-C₄, cycloalkyl (C₃-C₆)-alkyle(C₁-C₆), aryl-alkyle(C₁-C₃), hétéroaryl-alkyle(C₁-C₃) éventuellement substitués ou un groupement *N*-alkyl(C₁-C₄)amino, *N*-alkyl(C₁-C₄)-carbonylamino, *N*,*N*-dialkyl(C₁-C₄)amino ; ou
Q représente un carbocycle insaturé à 6 chaînons, éventuellement une à plusieurs fois substitué par V, ou un cycle hétérocyclique insaturé à 5 ou 6 chaînons, éventuellement une à plusieurs fois substitué par V,
V représentant chaque fois indépendamment halogéno, cyano, nitro, un groupe alkyle en C₁-C₆, alcényle en C₂-C₄, alcynyle en C₂-C₄, cycloalkyle en C₃-C₆, alcoxy en C₁-C₆, *N*-alcoxy(C₁-C₆)-iminoalkyle(C₁-C₃), alkyl(C₁-C₆) sulfanyle, alkyl (C₁-C₆) sulfinyle, alkyl(C₁-C₆) sulfonyle, *N*,*N*-dialkyl(C₁-C₆)amino, éventuellement substitué ;
T représente un des groupes hétéroaromatiques à 5 chaînons T1-T8 indiqués ci-après, la liaison au groupe de tête pyrazole étant indiquée par un astérisque, 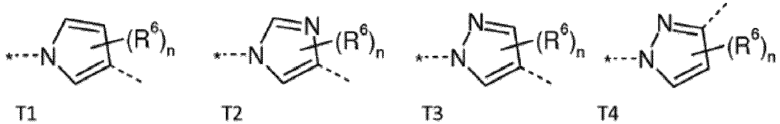 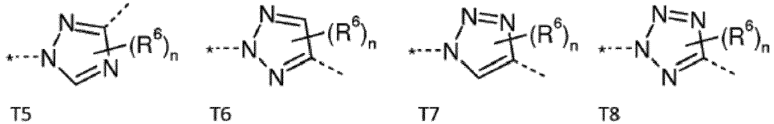
où
R⁶ représente chaque fois indépendamment un atome d'halogène, un groupe cyano, nitro, amino ou un groupe alkyle en C₁-C₆, alcoxy en C₁-C₆, alkyl(C₁-C₆)carbonyle, alkyl(C₁-C₆)sulfanyle alkyl(C₁-C₆)sulfinyle, alkyl(C₁-C₆)sulfonyle, éventuellement substitué, et
n représente les valeurs 0-1 ;
Z¹ représente un groupe halogénoalkyle(C₁-C₆) ou halogénocycloalkyle(C₃-C₆) éventuellement substitué, et
Z² représente un atome d'hydrogène ou d'halogène, un groupe cyano, nitro, amino ou un groupe alkyle en C₁-C₆, alkyl(C₁-C₆)carbonyle, alkyl(C₁-C₆)sulfanyle, alkyl(C₁-C₆)sulfinyle, alkyl(C₁-C₆)sulfonyle, éventuellement substitué, et
Z³ représente un atome d'hydrogène ou un groupe alkyle en C₁-C₆, cycloalkyle en C₃-C₆, alcényle en C₃-C₆, alcynyle en C₃-C₆, aryle ou hétéroaryle, éventuellement substitué.

3. Composés selon la revendication 1, dans lesquels
R¹ représente un atome d'hydrogène, un groupe alkyle en C₁-C₆, alcényle en C₃-C₆, alcynyle en C₃-C₆, cycloalkyle en C₃-C₇, alkyl(C₁-C₆)carbonyle, alcoxy(C₁-C₆)carbonyle, aryl-alkyle(C₁-C₃), hétéroaryl-alkyle(C₁-C₃), éventuellement une à sept fois substitué, chaque fois indépendamment, par fluoro, chloro, cyano, alcoxy en C₁-C₆ et alcoxy(C₁-C₆)carbonyle,
M¹ représente un atome d'hydrogène,
M² représente un atome d'hydrogène, un groupe cyano, un groupe alkyle en C₁-C₆, alcényle en C₂-C₆, cycloalkyle en C₃-C₆, alcoxy(C₁-C₃)carbonyle, éventuellement une à cinq fois substitué, chaque fois indépendamment, par fluoro, chloro, cyano ou alcoxy en C₁-C₃,
M¹ et M² forment avec l'atome de carbone, auquel ils sont liés, un cycle à 3 chaînons, éventuellement substitué,
les groupements chimiques ci-dessous ont les significations suivantes :
A₁ représente CR² ou un atome d'azote
A₂ représente CR³ ou un atome d'azote,
A₃ représente CR⁴ ou un atome d'azote, et
A₄ représente CR⁵ ou un atome d'azote,
mais au maximum trois des groupements chimiques A₁ à A₄ représentant simultanément un atome d'azote ;
R², R³, R⁴ et R⁵ représentent indépendamment les uns des autres un atome d'hydrogène, de fluor, de chlore, de brome, d'iode, un groupe cyano, nitro, un groupe alkyle en C₁-C₆, cycloalkyle en C₃-C₆, alcoxy en C₁-C₆, *N*-alcoxy(C₁-C₆)-imino-alkyle(C₁-C₃), alkyl(C₁-C₆)-sulfanyle, alkyl(C₁-C₆)-sulfinyle, alkyl(C₁-C₆)-sulfonyle, *N*-alkyl(C₁-C₆)-amino ou *N*,*N*-dialkyl(C₁-C₆)amino, éventuellement une à cinq fois substitué, chaque fois indépendamment, par fluoro, chloro, cyano, hydroxycarbonyle, alcoxy(C₁-C₆)carbonyle, alkyl(C₁-C₆)carbonyle ou phényle ;
W représente un atome d'oxygène ou de soufre ;
Q représente un atome d'hydrogène, un groupe amino ou un des groupements alkyle en C₁-C₆, alcényle en C₂-C₆, alcynyle en C₂-C₆, cycloalkyle en C₃-C₆, hétérocycloalkyle en C₂-C₅, alcoxy en C₁-C₄, cycloalkyl(C₃-C₆)-alkyle(C₁-C₆), aryl-alkyle(C₁-C₃), hétéroaryl-alkyle(C₁-C₃), *N*-alkyl(C₁-C₄)amino, *N*-alkyl(C₁-C₄)carbonylamino ou *N*,*N*-dialkyl(C₁-C₄)-amino, éventuellement une à cinq fois substitués, indépendamment les uns des autres, par hydroxy, nitro, amino, fluoro, chloro, alkyle en C₁-C₆, alcoxy en C₁-C₆, cyano, hydroxycarbonyle, alcoxy(C₁-C₄)carbonyle, alkyl(C₁-C₄)carbamoyle, cycloalkyl(C₃-C₇)carbamoyle, phényle ; ou
Q représente un groupe aryle substitué par 0-4 substituants V ou un cycle hétéroaromatique à 5 ou 6 chaînons, substitué par 0-4 substituants V,
V représentant chacun indépendamment halogéno, cyano, nitro, un groupe alkyle en C₁-C₆, alcényle en C₂-C₄, alcynyle en C₂-C₄, cycloalkyle en C₃-C₆, alcoxy en C₁-C₆, *N*-alcoxy(C₁-C₆)-iminoalkyle(C₁-C₃), alkyl(C₁-C₆) sulfanyle, alkyl(C₁-C₆) sulfinyle, alkyl (C₁-C₆) sulfonyle, *N*,*N*-dialkyl(C₁-C₆)amino, éventuellement substitué ;
T représente un des groupes hétéroaromatiques à 5 chaînons T1-T8 indiqués ci-après, la liaison au groupe de tête pyrazole étant indiquée par un astérisque, 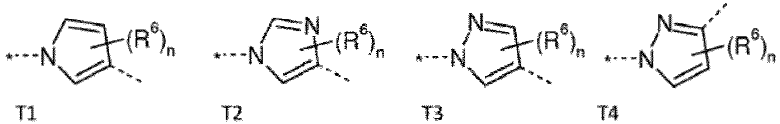 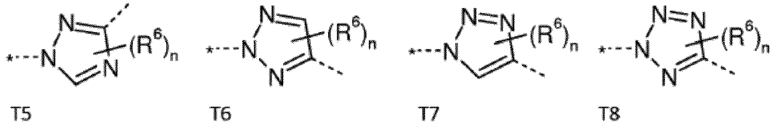
où
R⁶ représente chaque fois indépendamment un atome de fluor, de chlore, de brome, d'iode, un groupe cyano, nitro, amino ou un groupe alkyle en C₁-C₆, alcoxy en C₁-C₆, alkyl(C₁-C₆)carbonyle, alkyl(C₁-C₆)sulfanyle alkyl(C₁-C₆)sulfinyle, alkyl(C₁-C₆)sulfonyle, éventuellement une à cinq fois substitué, chaque fois indépendamment, par fluoro et/ou chloro, et
n représente les valeurs 0-1 ;
Z¹ représente un groupe halogénoalkyle(C₁-C₆) ou halogénocycloalkyle(C₃-C₆) éventuellement substitué, et
Z² représente un atome d'hydrogène, de fluor, de chlore, de brome, d'iode, un groupe cyano, nitro, amino ou un groupe alkyle en C₁-C₆, alkyl(C₁-C₆)carbonyle, alkyl(C₁-C₆)sulfanyle, alkyl(C₁-C₆) sulfinyle, alkyl(C₁-C₄)sulfonyle, éventuellement une à cinq fois substitué, chaque fois indépendamment, par fluoro et/ou chloro, et
Z³ représente un atome d'hydrogène ou un groupe alkyle en C₁-C₆, cycloalkyle en C₃-C₆, alcényle en C₃-C₄, alcynyle en C₃-C₄, aryle ou hétéroaryle, éventuellement substitué.

4. Composés selon la revendication 1, dans lesquels
R¹ représente un atome d'hydrogène, un groupe méthyle, éthyle, n-propyle, isopropyle, n-butyle, isobutyle, sec-butyle, tert-butyle, méthoxyméthyle, éthoxyméthyle, propoxyméthyle, méthylcarbonyle, éthylcarbonyle, n-propylcarbonyle, isopropylcarbonyle, n-butylcarbonyle, isobutylcarbonyle, sec-butylcarbonyle, tert-butylcarbonyle, méthoxycarbonyle, éthoxycarbonyle, n-propoxycarbonyle, isopropoxycarbonyle, n-butoxycarbonyle, isobutoxycarbonyle, sec-butoxycarbonyle, tert-butoxycarbonyle, cyanométhyle, 2-cyanoéthyle, benzyle, 4-méthoxybenzyle, pyrid-2-yl-méthyle, pyrid-3-yl-méthyle, pyrid-4-yl-méthyle, 6-chloropyrid-3-yl-méthyle,
M¹ représente un atome d'hydrogène,
M² représente un atome d'hydrogène, un groupe méthyle, éthyle, difluorométhyle, trichlorométhyle, dichlorofluorométhyle, trifluorométhyle, cyclopropyle, cyclobutyle, méthoxycarbonyle, éthoxycarbonyle,
M¹ et M² forment avec l'atome de carbone, auquel ils sont liés, un carbocycle à 3 chaînons,
les groupements chimiques ci-dessous ont les significations suivantes :
A₁ représente CR² ou un atome d'azote
A₂ représente CR³ ou un atome d'azote,
A₃ représente CR⁴ ou un atome d'azote, et
A₄ représente CR⁵ ou un atome d'azote,
mais au maximum trois des groupements chimiques A₁ à A₄ représentant simultanément un atome d'azote ;
R² et R⁵ représentent indépendamment l'un de l'autre un atome d'hydrogène, un groupe méthyle, un atome de fluor ou de chlore et
R³ et R⁴ représentent indépendamment l'un de l'autre un atome d'hydrogène, de fluor, de chlore, de brome, d'iode, un groupe cyano, nitro, un groupe méthyle, éthyle, fluorométhyle, difluorométhyle, chlorodifluorométhyle, trifluorométhyle, 2,2,2-trifluoroéthyle, méthoxy, éthoxy, n-propoxy, 1-méthyléthoxy, fluorométhoxy, difluorométhoxy, chlorodifluorométhoxy, dichlorofluorométhoxy, trifluorométhoxy, 2,2,2-trifluoroéthoxy, 2-chloro-2,2-difluoroéthoxy, pentafluoroéthoxy, *N*-méthoxyiminométhyle, 1-(*N*-méthoxyimino)-éthyle, méthylsulfanyle, trifluorométhylsulfanyle, méthylsulfonyle, méthylsulfinyle, trifluorométhylsulfonyle, trifluorométhylsulfinyle,
W représente un atome d'oxygène ou de soufre ;
Q représente un atome d'hydrogène, un groupe méthyle, éthyle, n-propyle, isopropyle, tert-butyle, 1-méthylpropyle, n-butyle, 2-méthylpropyle, 2-méthylbutyle, hydroxyméthyle, 2-hydroxyéthyle, 2-hydroxypropyle, cyanométhyle, 2-cyanoéthyle, trifluorométhyle, 1-fluoroéthyle, 2-fluoroéthyle, 2,2-difluoroéthyle, 2,2,2-trifluoroéthyle, 1-trifluorométhyléthyle, 2,2-difluoropropyle, 3,3,3-trifluoropropyle, 2,2-diméthyl-3-fluoropropyle, cyclopropyle, 1-cyano-cyclopropyle, cyclopropyl-méthyle, cyclobutyle, cyclopentyle, cyclohexyle, 1-cyclopropyléthyle, bis(cyclopropyl)méthyle, 2,2-diméthylcyclopropylméthyle, 2-phénylcyclopropyle, 2,2-dichlorocyclopropyle, trans-2-chlorocyclopropyle, cis-2-chlorocyclopropyle, 2,2-difluorocyclopropyle, trans-2-fluorocyclopropyle, cis-2-fluorocyclopropyle, trans-4-hydroxycyclohexyle, 4-trifluorométhylcyclohexyle, éthényle, 1-méthyléthényle, prop-1-ényle, 2-méthylprop-1-ényle, 3-méthylbut-1-ényle, 3,3,3-trifluoroprop-1-ényle, 1-éthyl-éthényle, 1-méthylprop-1-ényle, prop-2-ynyle, 3-fluoro-prop-2-ényle, oxétan-3-yle, thiétan-3-yle, 1-oxydo-thiétan-3-yle, 1,1-dioxydo-thiétan-3-yle, tétrahydrofuran-3-yle, 1,1-dioxydo-tétrahydrothiophén-3-yle, isoxazol-3-ylméthyle, 1,2,4-triazol-3-ylméthyle, 3-méthyloxétan-3-yl-méthyle, benzyle, 2,6-difluorohénylméthyle, 3-fluorophénylméthyle, 2-fluorophénylméthyle, 2,5-difluorophénylméthyle, 1-phényléthyle, 4-chlorophényléthyle, 2-trifluorométhylphényléthyle, 1-pyridin-2-yléthyle, pyridin-2-ylméthyle, 5-fluoropyridin-2-ylméthyle, (6-chloro-pyridin-3-yl)méthyle, pyrimidin-2-ylméthyle, thiophén-2-yl-méthyle, 2-éthoxyéthyle, 2-méthoxyéthyle, 1-(méthylsulfanyl)éthyle, 2-(méthylsulfanyl)-éthyle, 1-méthyl-2-(éthylsulfanyl)éthyle, 2-méthyl-1-(méthylsulfanyl)propan-2-yle, méthoxycarbonyle, éthoxycarbonyle, méthoxycarbonylméthyle, NH₂, *N*-éthylamino, *N*-allylamino, *N,N-*diméthylamino, *N,N*-diéthylamino, méthoxy, éthoxy, propoxy, isopropoxy, tert-butoxy ; ou
Q représente un groupe phényle, naphtyle, pyridazine, pyrazine, pyrimidine, triazine, pyridine, pyrazole, thiazole, isothiazole, oxazole, isoxazole, triazole, imidazole, furane, thiophène, pyrrole, oxadiazole, thiadiazole, substitué par 0, 1, 2 ou 3 substituants V,
V représentant chaque fois indépendamment fluoro, chloro, bromo, iodo, cyano, nitro, méthyle, éthyle, difluorométhyle, trichlorométhyle, chlorodifluorométhyle, dichlorofluorométhyle, trifluorométhyle, 1-fluoroéthyle, 2-fluoroéthyle, 2,2-difluoroéthyle, 2,2,2-trifluoroéthyle, 1,2,2,2-tétrafluoroéthyle, 1-chloro-1,2,2,2-tétrafluoroéthyle, 2,2,2-trichloréthyle, 2-chloro-2,2-difluoroéthyle, 1,1-difluoroéthyle, pentafluoroéthyle, heptafluoro-n-propyle, heptafluoroisopropyle, nonafluoro-n-butyle, cyclopropyle, cyclobutyle, méthoxy, éthoxy, n-propoxy, 1-méthyléthoxy, fluorométhoxy, difluorométhoxy, chlorodifluorométhoxy, dichlorofluorométhoxy, trifluorométhoxy, 2,2,2-trifluoroéthoxy, 2-chloro-2,2-difluoroéthoxy, pentafluoroéthoxy, n-méthoxyiminométhyle, 1-(*N*-méthoxyimino)-éthyle, méthylsulfanyle, méthylsulfonyle, méthylsulfinyle, trifluorométhylsulfonyle, trifluorométhylsulfinyle, trifluorométhylsulfanyle, *N,N*-diméthyl-amino ;
T représente un des groupes hétéroaromatiques à 5 chaînons T1-T8 indiqués ci-après, la liaison au groupe de tête pyrazole étant indiquée par un astérisque, 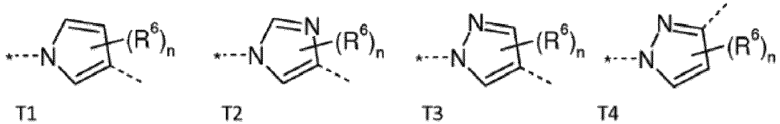 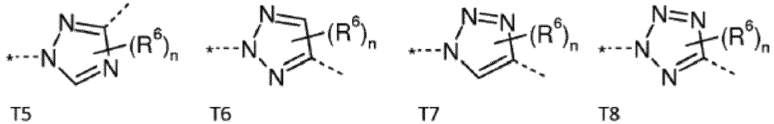
où
R⁶ représente chaque fois indépendamment un atome de fluor, de chlore, un groupe cyano, nitro, amino, méthyle, éthyle, propyle, 1-méthyléthyle, tert-butyle, trifluorométhyle, difluorométhyle, méthoxy, éthoxy, trifluorométhoxy, 2,2-difluoroéthoxy, 2,2,2-trifluoroéthoxy, méthylcarbonyle, éthylcarbonyle, trifluorométhylcarbonyle, méthylsulfanyle, méthylsulfinyle, méthylsulfonyle, trifluorométhylsulfonyle, trifluorométhylsulfanyle, trifluorométhylsulfinyle, et
n représente les valeurs 0-1 ;
Z¹ représente un groupe difluorométhyle, trichlorométhyle, chlorodifluorométhyle, dichlorofluorométhyle, trifluorométhyle, bromodichlorométhyle, 1-fluoroéthyle, 1-fluoro-1-méthyléthyle, 2-fluoroéthyle, 2,2-difluoroéthyle, 2,2,2-trifluoroéthyle, 1,2,2,2-tétrafluoroéthyle, 1-chloro-1,2,2,2-tétrafluoroéthyle, 2,2,2-trichloroéthyle, 2-chloro-2,2-difluoroéthyle, 1,1-difluoroéthyle, pentafluoroéthyle, heptafluoro-n-propyle, heptafluoro-isopropyle, nonafluoro-n-butyle, cyclopropyle, 1-chlorocyclopropyle, 1-fluorocyclopropyle, 1-bromocyclopropyle, 1-cyanocyclopropyle, 1-trifluorométhyl-cyclopropyle, cyclobutyle ou 2,2-difluoro-1-méthylcyclopropyle, et
Z² représente un atome d'hydrogène, de fluor, de chlore, de brome, d'iode, un groupe cyano, nitro, amino, méthyle, éthyle, 1,1-tert-butyle, difluorométhyle, trichlorométhyle, chlorodifluorométhyle, dichlorofluorométhyle, trifluorométhyle, bromodichlorométhyle, 1-fluoroéthyle, 1-fluoro-1-méthyléthyle, 2-fluoroéthyle, 2,2-difluoroéthyle, 2,2,2-trifluoroéthyle, 1,2,2,2-tétrafluoroéthyle, 1-chloro-1,2,2,2-tétrafluoroéthyle, 2,2,2-trichloréthyle, 2-chloro-2,2-difluoroéthyle, 1,1-difluoroéthyle, pentafluoroéthyle, heptafluoro-n-propyle, heptafluoro-isopropyle, nonafluoro-n-butyle, méthylsulfanyle, méthylsulfinyle, méthylsulfonyle, éthylthio, éthylsulfinyle, éthylsulfonyle, trifluorométhylsulfanyle, trifluorométhylsulfinyle, trifluorométhylsulfonyle, chloro-difluorométhylsulfanyle, chlorodifluorométhylsulfinyle, chlorodifluorométhylsulfonyle, dichlorofluorométhylsulfanyle, dichlorofluorométhylsulfinyle, dichlorofluorométhylsulfonyle et
Z³ représente un atome d'hydrogène ou un groupe méthyle, éthyle, n-propyle, isopropyle, n-butyle, isobutyle, sec-butyle, tert-butyle, 1-propényle, 1-propynyle, 1-butynyle, difluorométhyle, trichlorométhyle, chlorodifluorométhyle, dichlorofluorométhyle, trifluorométhyle, 1-fluoroéthyle, 1-fluoro-1-méthyléthyle, 2-fluoroéthyle, 2,2-difluoroéthyle, 2,2,2-trifluoroéthyle, phényle, 2-chlorophényle, 3-chlorophényle. 4-chlorophényle, 2,4-dichlorophényle, 2,5-dichlorophényle, 3,4-dichlorophényle, 2,6-dichlorophényle, 2,6-dichloro-4-trifluorométhylphényle, 3-chloro-5-trifluorométhylpyridin-2-yle.

5. Composés selon la revendication 1, dans lesquels
Z¹ représente un groupe trifluorométhyle, 1-chlorocyclopropyle, 1-fluoro-cyclopropyl ou pentafluoroéthyle,
Z² représente un groupe trifluorométhyle, nitro, méthylsulfanyle, méthylsulfinyle, méthylsulfonyle, un atome de fluor, de chlore, de brome, cyano ou un atome d'iode,
Z³ représente un groupe méthyle, éthyle, n-propyle ou un atome d'hydrogène,
R¹ représente un atome d'hydrogène, un groupe méthyle, éthyle, n-propyle, isopropyle, n-butyle, isobutyle, sec-butyle, tert-butyle, méthoxyméthyle, éthoxyméthyle, propoxyméthyle, méthylcarbonyle, éthylcarbonyle, n-propylcarbonyle, isopropylcarbonyle, n-butylcarbonyle, isobutylcarbonyle, sec-butylcarbonyle, tert-butylcarbonyle, méthoxycarbonyle, éthoxycarbonyle, n-propoxycarbonyle, isopropoxycarbonyle, n-butoxycarbonyle, isobutoxycarbonyle, sec-butoxycarbonyle, tert-butoxycarbonyle, cyanométhyle, 2-cyanoéthyle, benzyle, 4-méthoxybenzyle, pyrid-2-yl-méthyle, pyrid-3-yl-méthyle, pyrid-4-yl-méthyle, 6-chloropyrid-3-yl-méthyle,
M¹ représente un atome d'hydrogène,
M² représente un atome d'hydrogène ou un groupe méthyle,
M¹ et M² forment avec l'atome de carbone, auquel ils sont liés, un carbocycle à 3 chaînons,
A₁ et A₄ représentent CH,
A₂ représente CH ou N,
A₃ représente CR⁴ et
R⁴ représente un groupe méthyle, éthyle, un atome de fluor, de chlore, de brome ou d'iode,
T représente un des groupes hétéroaromatiques à 5 chaînons T1-T8 indiqués ci-après, la liaison au groupe de tête pyrazole étant indiquée par un astérisque, 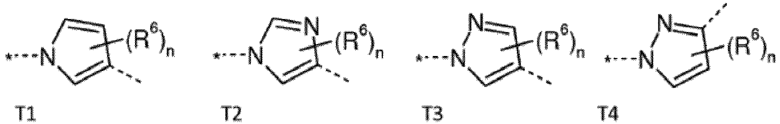 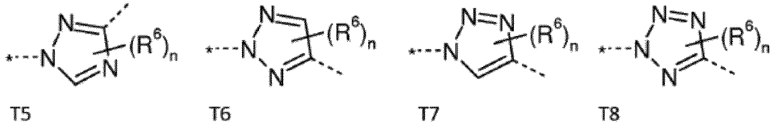
où
R⁶ représente un atome d'hydrogène, un groupe méthyle, éthyle, 2-méthyléthyle, 2,2-diméthyléthyle, un atome de fluor, de chlore, de brome, d'iode, un groupe nitro, trifluorométhyle, amino,
W représente un atome d'oxygène et
Q représente un atome d'hydrogène, un groupe méthyle, éthyle, n-propyle, 1-méthyléthyle, 1,1-diméthyléthyle, n-butyle, 1-méthylpropyle, 2-méthylpropyle, 2-méthylbutyle, hydroxyméthyle, 2-hydroxypropyle, cyanométhyle, 2-cyanoéthyle, 2-fluoroéthyle, 2,2-difluoroéthyle, 2,2,2-trifluoroéthyle, 1-trifluorométhyléthyle, 2,2-difluoropropyle, 3,3,3-trifluoropropyle, 2,2-diméthyl-3-fluoropropyle, cyclopropyle, cyclopropyl-méthyle, cyclobutyle, cyclopentyle, cyclohexyle, 1-cyclopropyléthyle, bis(cyclopropyl)méthyle, 2,2-diméthylcyclopropylméthyle, 2-phénylcyclopropyle, 2,2-dichlorocyclopropyle, trans-2-chlorocyclopropyle, cis-2-chlorocyclopropyle, 2,2-difluorocyclopropyle, trans-2-fluorocyclopropyle, cis-2-fluorocyclopropyle, trans-4-hydroxycyclohexyle, 4-trifluorométhylcyclohexyle, éthényle, 1-méthyléthényle, prop-1-ényle, 2-méthylprop-1-ényle, 3-méthylbut-1-ényle, 3,3,3-trifluoroprop-1-ényle, 1-éthyl-éthényle, 1-méthylprop-1-ényle, prop-2-ynyle, 3-fluoro-prop-2-ényle, oxétan-3-yle, thiétan-3-yle, 1-oxydo-thiétan-3-yle, 1,1-dioxydothiétan-3-yle, isoxazol-3-ylméthyle, 1,2,4-triazol-3-ylméthyle, 3-méthyloxétan-3-ylméthyle, benzyle, 2,6-difluorophénylméthyle, 3-fluorophénylméthyle, 2-fluorophénylméthyle, 2,5-difluorophénylméthyle, 1-phényléthyle, 4-chlorophényléthyle, 2-trifluorométhylphényléthyle, 1-pyridin-2-yléthyle, pyridin-2-ylméthyle, (6-chloro-pyridin-3-yl)méthyle, 5-fluoropyridin-2-ylméthyle, pyrimidin-2-ylméthyle, méthoxy, 2-éthoxyéthyle, 2-(méthylsulfanyl)éthyle, 1-méthyl-2-(éthylsulfanyl)éthyle, 2-méthyl-1-(méthylsulfanyl)propan-2-yle, méthoxycarbonyle, méthoxycarbonylméthyle, NH₂, *N*-éthylamino, *N*-allylamino, *N*,*N*-diméthylamino, *N*,*N*-diéthylamino, méthoxy, éthoxy, propoxy, isopropoxy, tert-butoxy ; ou
Q représente un groupe phényle, naphtyle, pyridazine, pyrazine, pyrimidine, triazine, pyridine, pyrazole, thiazole, isothiazole, oxazole, isoxazole, triazole, imidazole, furane, thiophène, pyrrole, oxadiazole, thiadiazole, substitué par 0, 1, 2 ou 3 substituants V,
V représentant chaque fois indépendamment fluoro, chloro, bromo, iodo, cyano, nitro, méthyle, éthyle, difluorométhyle, trichlorométhyle, chlorodifluorométhyle, dichlorofluorométhyle, trifluorométhyle, 1-fluoroéthyle, 2-fluoroéthyle, 2,2-difluoroéthyle, 2,2,2-trifluoroéthyle, 1,2,2,2-tétrafluoroéthyle, 1-chloro-1,2,2,2-tétrafluoroéthyle, 2,2,2-trichloroéthyle, 2-chloro-2,2-difluoroéthyle, 1,1-difluoroéthyle, pentafluoroéthyle heptafluoro-n-propyle, heptafluoro-isopropyle, nonafluoro-n-butyle, cyclopropyle, cyclobutyle, méthoxy, éthoxy, n-propoxy, 1-méthyléthoxy, fluorométhoxy, difluorométhoxy, chlorodifluorométhoxy, dichlorofluorométhoxy, trifluorométhoxy, 2,2,2-trifluoroéthoxy, 2-chloro-2,2-difluoroéthoxy, pentafluoroéthoxy, N-méthoxyiminométhyle, 1-(*N*-méthoxyimino)-éthyle, méthylsulfanyle, méthylsulfonyle, méthylsulfinyle, trifluorométhylsulfonyle, trifluorométhylsulfinyle, trifluorométhylsulfanyle, *N*,*N*-diméthylamino.

6. Composés de formules générales (IIa), (IIb), (IIc), (IId), (IIe), (IIf), (IIg), (IIh), dans lesquelles les radicaux A₁-A₄, n, W, Q, R¹, R⁶, M¹, M² et Z¹-Z³ peuvent avoir les significations selon l'une quelconque des revendications 1 à 5.

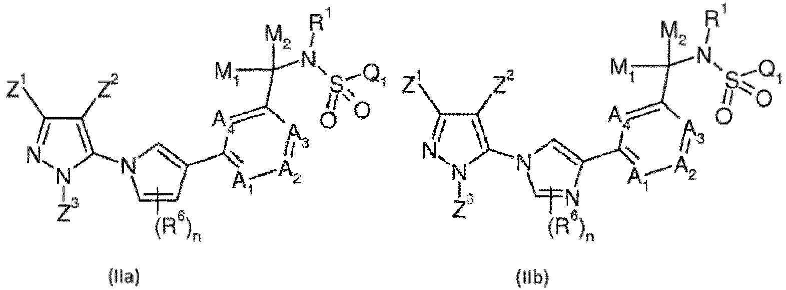

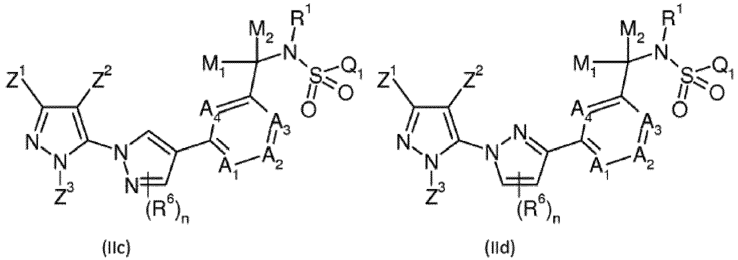

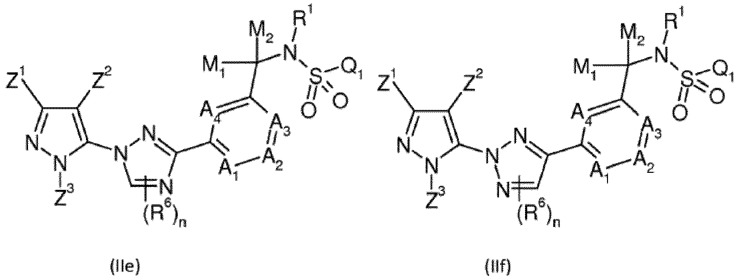

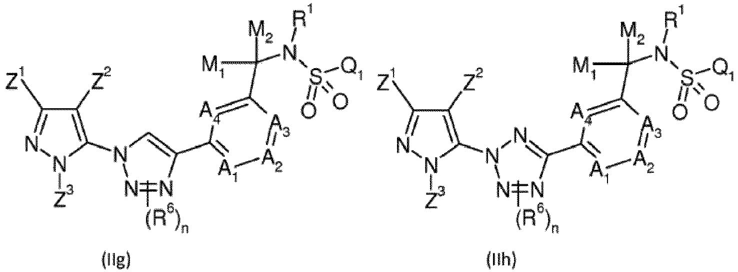

7. Utilisation des composés de formule générale (II) ou des formules générales (IIa) à (IIh) selon l'une quelconque des revendications 1 à 6, pour la lutte contre des ravageurs parasitant les plantes, des ravageurs des stocks ou des ravageurs qui détruisent des matériaux industriels.

8. Compositions pharmaceutiques, contenant au moins un composé selon l'une quelconque des revendications 1 à 6.

9. Composés selon l'une quelconque des revendications 1 à 6, pour utilisation en tant que médicament.

10. Utilisation des composés selon l'une quelconque des revendications 1 à 6, pour la fabrication de compositions pharmaceutiques destinées à la lutte contre des parasites d'animaux.

11. Procédé pour la fabrication de produits phytosanitaires contenant des composés selon l'une quelconque des revendications 1 à 6, ainsi que des excipients usuels et/ou des substances tensioactives usuelles.

12. Procédé pour la lutte contre des ravageurs parasitant les plantes, des ravageurs de stocks ou des ravageurs qui détruisent des matériaux industriels, **caractérisé en ce qu'**on fait agir sur les ravageurs et/ou leur habitat un composé selon l'une quelconque des revendications 1 à 6.

13. Utilisation des composés selon l'une quelconque des revendications 1 à 6, pour la protection du matériel de multiplication de plantes.
